# EUROPEAN PATENT SPECIFICATION

(11) **EP 0 938 497 B1**
(45) Date of publication and mention of the grant of the patent: **28.02.2007**
(21) Application number: 97948165.2
(22) Date of filing: 05.11.1997
(51) Int. Cl.: C07K 14/16, A61K 39/21, C07K 16/10

(54) **CONSTRAINED HELICAL PEPTIDES AND METHODS OF MAKING SAME**
GESPANNTE, HELIXFORMENDE PEPTIDE UND VERFAHREN UM SIE HERZUSTELLEN
PEPTIDE A STRUCTURE HELICOIDALE CONTRAINTE ET LEURS PROCEDES DE FABRICATION

(30) Priority: 06.11.1996 US 743698; 16.06.1997 US 876698
(43) Date of publication of application: 01.09.1999
(73) Proprietor: GENENTECH, INC., South San Francisco, CA 94080-4990 (US)
(72) Inventor: BRAISTED, Andrew, San Francisco, CA 94127 (US); JUDICE, J., Kevin, Montara, CA 94037 (US); MCDOWELL, Robert, S., San Francisco, CA 94114 (US); PHELAN, J., Christopher, San Francisco, CA 94117 (US); STAROVASNIK, Melissa, A., San Francisco CA 94116 (US); WELLS, James, A., Burlingame, CA 94080 (US)
(74) Representative: Kremer, Simon Mark
(86) International application number: PCT/US1997/020069
(87) International publication number: WO 1998/020036

(56) References cited:
- WO-A-91/15512
- WO-A-92/09625
- WO-A-95/34312
- J.C. PHELAN ET AL.: "A General Method for Constraining Short Peptides to an alpha-Helical Conformation" JOURNAL OF THE AMERICAN CHEMICAL SOCIETY, vol. 119, no. 3, 22 January 1997, DC US, pages 455-460, XP002059088
- M.K. LAWLESS ET AL.: "HIV-1 Membrane Fusion Mechanism: Structural Studies of the Interactions between Biologically-Active Peptides from gp41" BIOCHEMISTRY, vol. 35, no. 42, 22 October 1996, EASTON, PA US, pages 13697-13708, XP002059093
- X. ZHANG ET AL.: "Synthetic CD4 exocyclics inhibit binding of human immunodeficiency virus type 1 envelope to CD4 and virus repication in T lymphocytes" NATURE BIOTECHNOLOGY., vol. 15, no. 2, February 1997, UBLISHING US, pages 150-154, XP002059089

## Description

The invention relates to the conformational constraint of peptides. In particular, the invention relates to constraining peptides to an α-helical conformation. This invention also relates to the rational design and preparation of HIV vaccines based on HIV gp41 polypeptide sequences. This invention further relates to improved methods for HIV infection diagnosis and immunogens which induce antibodies useful in the diagnostic methods.

### BACKGROUND OF THE INVENTION

A variety of methods for stabilizing α-helical peptides have been described previously. Addition of trifluoroethanolor hexafluoroisopropanolhas frequently been used to stabilize α-helices in aqueous solution. Dimerization of α-helices at hydrophobic interfaces has also provided exogenous stabilization. Short α-helical peptides have been stabilized by incorporating groups at the termini to stabilize the intrinsic helix dipole. Naturally occurring capping motifs as well as organic templates have been used to stabilize α-helices by end-nucleation. Several non-covalent side chain constraints have been investigated for α-helix stabilization, including hydrophobic interactions, salt bridges, and metal ion chelation by both natural and unnatural amino acids.

Finally, α-helices have been stabilized by covalent side chain tethers. Chorev *et al.,* Biochemistry, 30: 5968-5974(1991), Osapay *et al.,* J. Am. Chem. Soc., 112: 6046-6051(1990), Osapay *et al.,* J. Am. Chem. Soc., 114: 6966-6973 (1990), Bracken *et al.,* J. Am. Chem. Soc., 116: 6431-6432 (1994), and Houston *et al.,* J. Peptide Science, 1: 274-282 (1995) described the stabilization of α-helices by side chain to side chain lactamization. Ravi *et al.,* J. Am. Chem. Soc., 105: 105-109 (1983) and Jackson *et al.,* J. Am. Chem. Soc., 113: 9391-9392 (1991) described the constraint of peptides by disulfide bonds between residues. The naturally occurring peptide apamin has been used as a scaffold for the presentation of α-helical peptide sequences constrained in helical conformation by disulfide bonds to scaffold cysteine residues.

Acquired immunodeficiency syndrome (AIDS) is caused by a retrovirus identified as the human immunodeficiency virus (HIV). There have been intense efforts to develop a vaccine that induces a protective immune response based on induction of antibodies or cellular responses. Recent efforts have used subunit vaccines where an HIV protein, rather than attenuated or killed virus, is used as the immunogen in the vaccine for safety reasons. Subunit vaccines generally include gp120, the portion of the HIV envelope protein which is on the surface of the virus.

The HIV envelope protein has been extensively described, and the amino acid and nucleic acid sequences encoding HIV envelope from a number of HIV strains are known (Myers, G. et al., 1992. Human Retroviruses and AIDS. A compilation and analysis of nucleic acid and amino acid sequences. Los Alamos National Laboratory, Los Alamos, New Mexico). The HIV envelope protein is a glycoprotein of about 160 kd (gp160) which is anchored in the membrane bilayer at its carboxyl terminal region. The N-terminal segment. gp 120, protrudes into the aqueous environment surrounding the virion and the C-terminal segment, gp41, spans the membrane. Via a host-cell mediated process, gp 160 is cleaved to form gp120 and the integral membrane protein gp41. As there is no covalent attachment between gp120 and gp41, free gp120 is sometimes released from the surface of virions and infected cells.

gp 120 has been the object of intensive investigation as a vaccine candidate for subunit vaccines, as the viral protein which is most likely to be accessible to immune attack. At present, clinical trials using gp120 MN strain are underway.

However, effective vaccines based on gp 120 or another HIV protein for protection against additional strains of HIV are still being sought to prevent the spread of this disease.

### SUMMARY OF THE INVENTION

The invention provides a method for constructing a constrained helical peptide comprising the steps of: (1) synthesizing a peptide, wherein the peptide comprises a sequence of eight amino acid residues, wherein the sequence of eight amino acid residues has a first terminal residue and a second terminal residue, wherein the first terminal residue and the second terminal residue flank an internal sequence of six amino acid residues, and wherein the first terminal residue has a side chain containing an amide bond-forming substituent and the second terminal residue has a side chain containing an amide bond-forming substituent; (2) providing a difunction al linker having a first functional group capable of forming an amide linkage with the side chain amide bond-fortningsubstituentof the first terminal residue and having a second functional group capable of forming an amide linkage with the side chain amide bond-forming substituent of the second terminal residue; and (3) cyclizing the peptide by reacting the side chain amide bond-forming substituent of the first terminal residue with the first functional group of the difunctional linker to form an amide linkage and reacting the side chain amide bond-foriningsubstituentof the second terminal residue with the second functional group of the difunctional linker to form an amide linkage, yielding a constrained helical peptide.

The invention also provides a method for constructing a constrained helical peptide comprising the steps of: (1) synthesizing a peptide, wherein the peptide comprises a sequence of eight amino acid residues, wherein the sequence of eight amino acid residues has a first terminal residue and a second terminal residue, wherein the first terminal residue and the second terminal residue flank an internal sequence of six amino acid residues, wherein the first terminal residue has a side chain containing an amide bond-forming substituent and the second terminal residue has a side chain containing an amide bond-forming substituent, and wherein the side chain amide bond-forming substituent of the first terminal residue is protected with a first protecting group and the side chain amide bond-forming substituent of the second terminal residue is protected with a second protecting group such that the first protecting group and the second protecting group are differentially removable; (2) removing the first protecting group such that the side chain amide bond-forming substituent of the first terminal residue is deprotected and the side chain amide bond-forming substituent of the second terminal residue is not deprotected; (3) providing a difunctional linker having a first functional group capable of forming an amide linkage with the side chain amide bond-forming substituent of the first terminal residue and having a second functional group capable of forming an amide linkage with the side chain amide bond-forming substituent of the second terminal residue; (4) reacting the peptide with the difunctional linker to form an amide linkage between the first functional group of the difunctional linker and the side chain amide bond-forming substituentof the first terminal residue; (5) removing the second protecting group to deprotectthe side chain amide bond-forming substituent of the second terminal residue; and (6) cyclizing the peptide by intramolecularlyreactingthe side chain amide bond-forming substituent of the second terminal residue with the second functional group of the difunctional linker to form an amide linkage and yield a constrained helical peptide.

The invention further provides a method for constructing a constrained helical peptide, comprising the steps of: (a) synthesizing a peptide, wherein the peptide comprises a sequence of eight amino acid residues, wherein the sequence of eight amino acid residues has a first terminal residue and a second terminal residue, wherein the first terminal residue and the second terminal residue flank an internal sequence of six amino acid residues, wherein the first terminal residue has a side chain containing an amide bond-forming substituent and the second terminal residue has side chain containing an amide bond-forming substituent, wherein the first terminal residue is coupled to a difunctional linker having a first functional group and a second functional group, wherein the first functional group is in an amide linkage with the side chain amide bond-forming substituent of the first terminal residue, and wherein the second functional group of the difunctional linker is capable of forming an amide linkage with the side chain amide bond-forming substituent of the second terminal residue; and (b) cyclizing the peptide by intramolecularlyreacting the side chain amide bond-forming substituent of the second terminal residue with the second functional group of the difunctional linker to form an amide linkage and yield a constrained helical peptide.

The invention additionally provides a method for constructing a constrained helical peptide comprising the steps of: (1) synthesizing a peptide, wherein the peptide comprises a sequence of eight amino acid residues, and wherein the sequence of eight amino acid residues has a first terminal residue and a second terminal residue, wherein the first terminal residue and the second terminal residue are independently selected from Asp and Glu; (2) providing a diamine linker having a first amino group capable of forming an amide linkage with the carboxy side chain of the first terminal residue and a second amino group capable of forming an amide linkage with the carboxy side chain of the second terminal residue; and (3) cyclizing the peptide by reacting the first amino group of the diamine linker with the carboxy side chain of the first terminal residue to form an amide linkage and reacting the second amino group of the diamine linker with the carboxy side chain of the second terminal residue to form an amide linkage, yielding a constrained helical peptide.

The invention also encompasses a method for constructing a constrained helical peptide comprising the steps of: (1) synthesizing a peptide, wherein the peptide comprises a sequence of eight amino acid residues, wherein the sequence of eight amino acid residues has a first terminal residue and a second terminal residue, wherein the first terminal residue and the second terminal residue flank an internal sequence of six amino acid residues, wherein the first terminal residue and the second terminal residue are independently selected from Asp and Glu, and wherein the carboxy side chain of the first terminal residue is protected with a first protecting group and the carboxy side chain of the second terminal residue is protected with a second protecting group such that the first protecting group and the second protecting group are differentially removable; (2) removing the first protecting group such that the carboxy side chain of the first terminal residue is deprotected and the carboxy side chain of the second terminal residue is not deprotected; (3) reacting the peptide with a diamine linker having a first amino group and a second amino group to form an amide linkage between the deprotected carboxy side chain of the first terminal residue and the first amino group of the diamine linker; (4) removing the second protecting group to deprotect the carboxy side chain of the second terminal residue; and (5) cyclizing the peptide by intramolecularlyreacting the deprotectedcarboxy side chain of the second terminal residue with the second amino group of the diamine linker to form an amide linkage and yield a constrained helical peptide.

The invention further encompasses a method for constructing a constrained helical peptide comprising the steps of: (I) synthesizing peptide, wherein the peptide comprises a sequence of eight amino acid residues, wherein the sequence of eight amino acid residues has a first terminal residue and a second terminal residue, wherein the first terminal residue and the second terminal residue flank an internal sequence of six amino acid residues, wherein the first terminal residue and the second terminal residue are independently selected from Asp and Glu, and wherein the carboxy side chain of the first terminal residue is coupled to a diamine linker having a first amino group and a second amino group, such that the carboxy side chain of the first terminal residue is in an amide linkage with the first amino group of the diamine linker; and (2) cyclizing the peptide by intramolecularlyreacting the carboxy side chain of the second terminal residue with the second amino group of the diamine linker to form an amide linkage and yield a constrained helical peptide.

The invention also encompasses a compound selected from the group consisting of:
the compound represented by Formula (1): 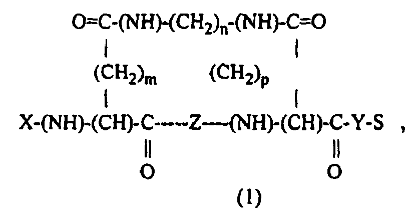
wherein S is absent or is a macromolecule, X is hydrogen or is any amino acid or amino acid sequence, Y is absent, or is hydroxyl if S is absent, or is any amino acid or amino acid sequence, Z is any amino acid sequence consisting of six amino acids; m and p are independently selected from the integers 0 to 6 inclusive, provided that m+p is less than or equal to 6, and n is any integer in the range defined by (7-(m+p)) to (9-(m+p)) inclusive, provided that n is greater than 1;
the compound represented by Formula (6): 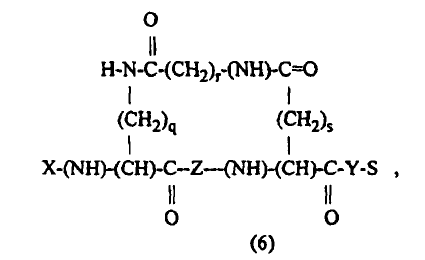
wherein S is absent or is a macromolecule, X is hydrogen or is any amino acid or amino acid sequence, Y is absent, or is hydroxyl if S is absent, or is any amino acid or amino acid sequence, Z is any amino acid sequence consisting of six amino acids, q is selected from the integers 1 to 7 inclusive, s is selected from the integers 0 to 6 inclusive, provided that q+s is less than or equal to 7, and r is any integer in the range defined by (7-(q+s)) to (9-(q+s)) inclusive, provided that r is greater than 0;
the compound represented by Formula (11): 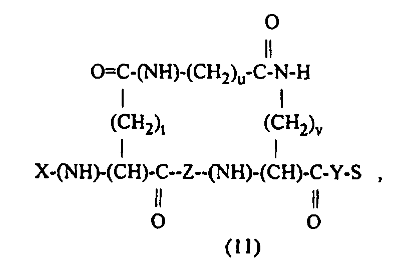
wherein S is absent or is a macromolecule, X is hydrogen or is any amino acid or amino acid sequence, Y is absent, or is hydroxyl if S is absent, or is any amino acid or amino acid sequence, Z is any amino acid sequence consisting of six amino acids; t is selected from the integers 0 to 6 inclusive, and v is selected from the integers 1 to 7 inclusive, provided that t+v is less than or equal to 7; and u is any integer in the range defined by (7-(t+v)) to (9-(t+v)) inclusive, provided that u is greater than 0; and
the compound represented by Formula (16): 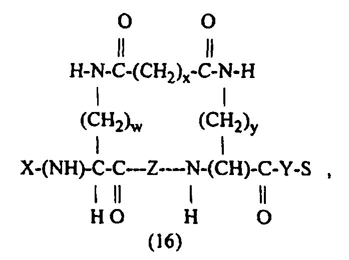
wherein S is absent or is a macromolecule, X is hydrogen or is any amino acid or amino acid sequence, Y is absent, or is hydroxyl if S is absent, or is any amino acid or amino acid sequence, Z is any amino acid sequence consisting of six amino acids; w and y are independently selected from the integers 1 to 7 inclusive, provided that w+y is less than or equal to 8, and x is any integer in the range defined by (7-(w+y)) to (9-(w+y)) inclusive, provided that x is greater than or equal to 0.

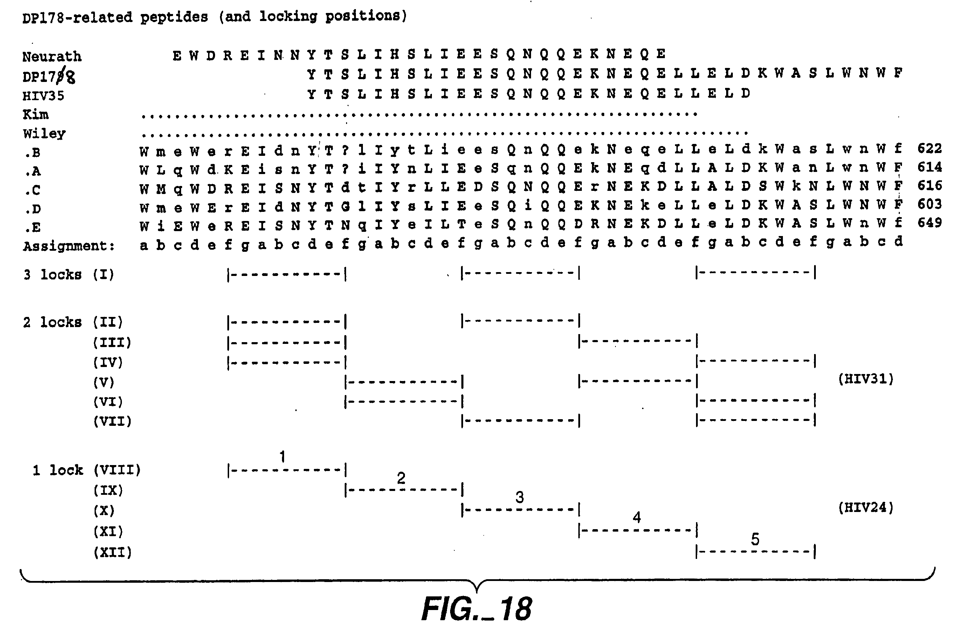

In a preferred embodiment is provided a compound containing a constrained helical peptide that in turn contains a peptide of a sequence of eight amino acid residues, in which the sequence of eight amino acid residues has a first terminal residue and a second terminal residue that flank an internal sequence of six amino acids and that have a side chain that are linked to each other forming a locking moiety to form a constrained helical peptide. The internal sequence of six amino acids has the form **gabcde, defgab,** or **cdefga** and is selected from the group of sequences consisting of a sequence of six contiguous amino acids in HIV-1LAI strain gp41 amino acid sequence 633 to 678, in its homolog sequence from another HIV strain, in a consensus sequence of its homolog sequences from any one HIV clade, or an amino acid substituted variant thereof, in which amino acid 633 or its corresponding amino acid in the homolog, consensus or variant sequence is assigned position a of a repeating **abcdefg** assignment for the 633-678 sequence (as shown in Figure 18). In these compounds the locking moiety or tether is between adjacent **f** positions when the internal sequence is of the form **gabcde**, adjacent c positions when the internal sequence is of the form **defgab**, or adjacent b positions when the internal sequence is of the form **cdefga**. Most preferably the lock is between adjacent **f** positions. Figure 18 provides the alignment of the repeating abcdefg assignment to the amino acids in the 633-678 region. In a preferred embodiment the internal sequence of six amino acids has the form **gabcde**. The compounds preferably have HIV anti-fusogenic or anti-infection activity.

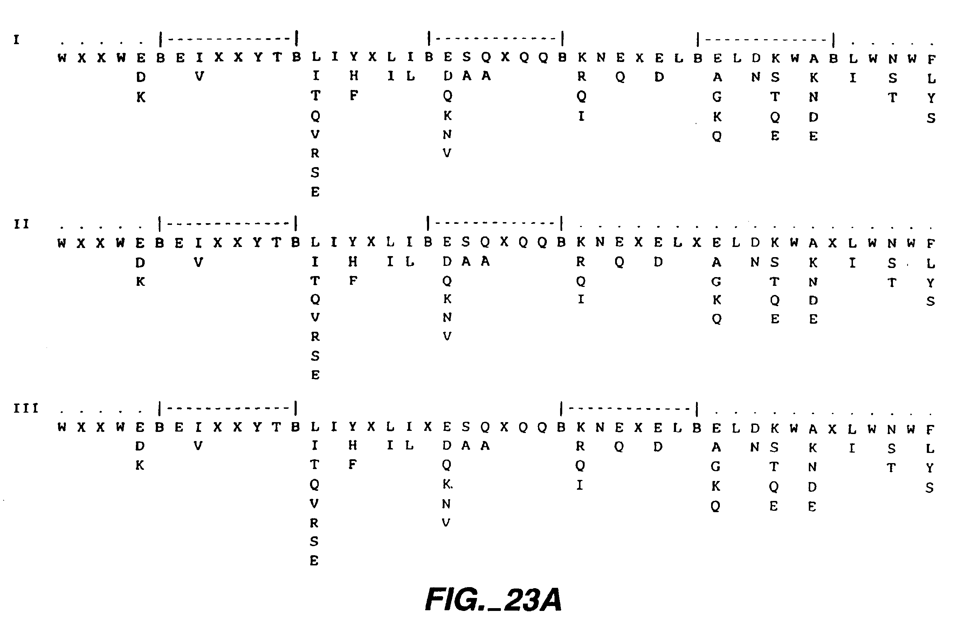

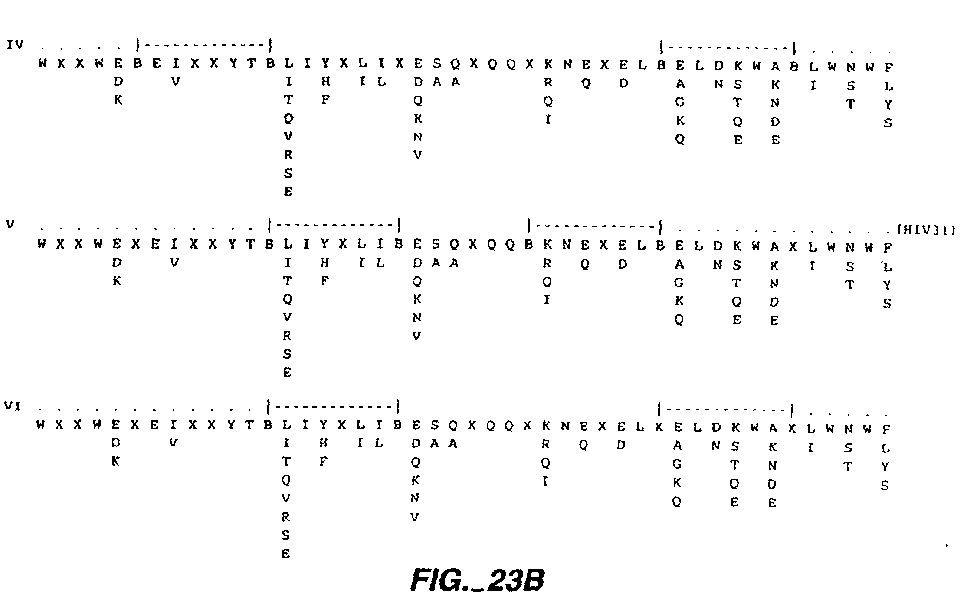

Preferred compounds are those selected from the group consisting constrained helical peptides of each possible sequence having any one or any combination of amino acid substitutions indicated in the constrained helical peptide series I to XII as shown in Figures 23A and 23B in combination with any one or any combination of amino acid truncations indicated in the constrained helical peptide series I to XII as shown in Figures 23A and 23B. Peptides HIV24 and HIV31 are particularly preferred compounds of this type.

In another embodiment the compounds of the invention are used as haptens, preferably attached to carriers, for use as an immunogen to raise antibodies that have a diagnostic use or as a vaccine for prophylactic or therapeutic treatment of patients at risk for or infected with HIV. Examples of such prophylactic use of the peptides may include, but are not limited to, prevention of virus transmission from mother to infant and other settings where the likelihood of HIV transmission exists, such as, for example, accidents in health care settings wherein workers are exposed to HIV-containing blood products. The constrained peptides of the invention can serve the role of a prophylactic vaccine, wherein the host raises antibodies against the peptides of the invention, which then serve to neutralize HIV viruses by, for example, inhibiting further HIV infection.

### BRIEF DESCRIPTION OF THE DRAWINGS

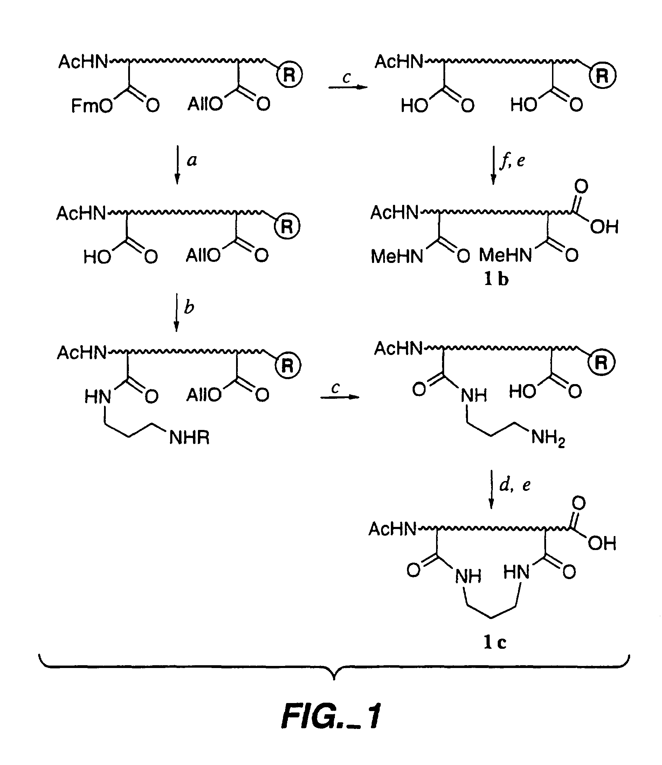

Fig. 1 is a diagram depicting the synthesis of peptide 1b and 1c. Reagent *α* represents 20% piperidine/DMA; reagent *b* represents H₂NCH₂CH ₂CH₂NHR (R=H or BOC), BOP, DIPEA, CH₂Cl₂ ; reagent *c* represents Pd(PPh3)4, 20% piperidine/DMA, (R=BOC) TFA/CH₂Cl₂/anisole/1,2-ethanedithiol 45:45:5:5 v/v; reagent *d* represents BOP, DIPEA, CH₂Cl₂; reagent e represents HF/anisole/EtSMe20:2:1 v/v, 0 °C; and reagent *f* represents CH₃NH₂, BOP, CH₂Cl₂.

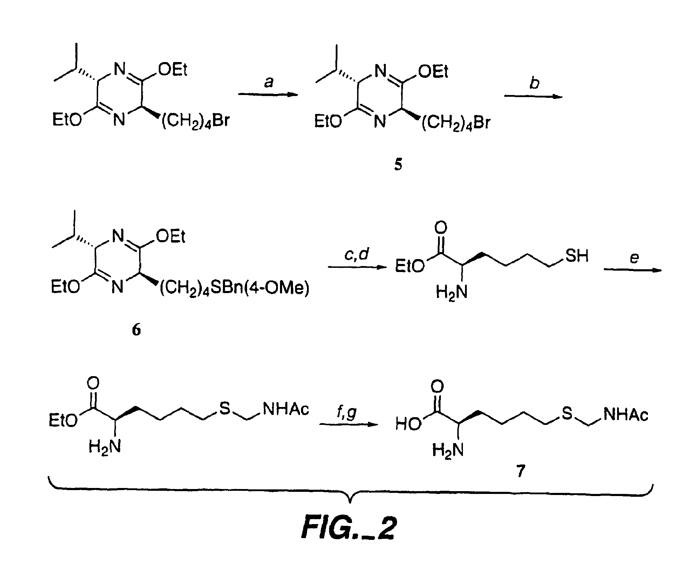

Fig. 2 is a diagram depicting the synthesis of N-Fmoc-S-Acm-D-thiolysine (compound 7). Reagent α represents *ⁿ*BuLi, THF, -78 °C; Br(CH₂)₄Br; reagent *b* represents 4-MeOBnSH, KO*^{t}*Bu, THF; reagent *c* represents 0.25 M HCl, THF/H₂O; reagent *d* represents Hg(OAc)₂, TFA; H₂S; reagent *e* represents acetamidomethanol, TFA; reagent *f* represents LiOH, THF/H₂O; and reagent g representsFmoc-OSu, dioxane, NaHCO₃.

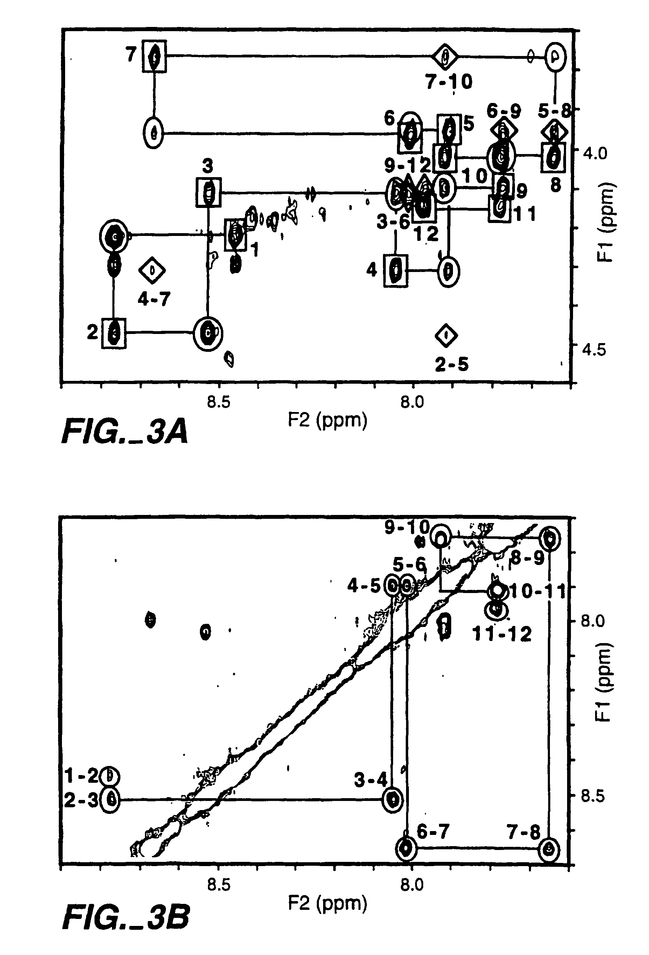

Figs. 3a and 3b are graphs depicting the H^{N}-H^{α} and H^{N}-H^{N} sections, respectively, of the ROESY spectrum of peptide **1c**. The spectrum was collected at 280 K, pH 5.0, 500 MHZ and a peptide concentration of 1.5 mM with a 4.5 kHz spin-lock mixing pulse of 200 ms duration. Lines connect the ROEs by which sequential assignments were made. Rectangular, oval and diamond shaped boxes denote intra residue, sequential and (*I, I+3*) correlations, respectively.

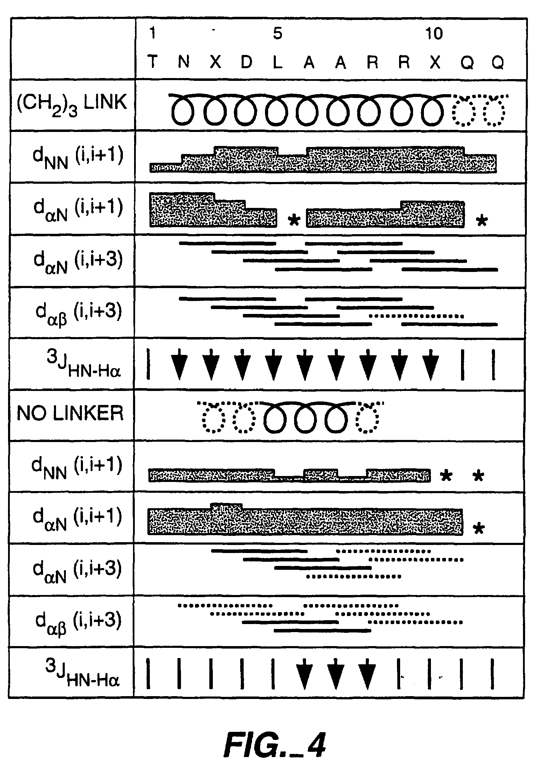

Fig. 4 is a graph depicting ROE and ³J_{HN-Hα} data for peptides **1c** and **1b**. For the d_{NN} and d_{αN} rows, observation of the sequential ROE is indicated by a bar connecting two residues, the thickness of the bar indicating the relative intensity of the ROE. The downward pointing arrows indicate ³J_{HN-Hα} less than 6.0 Hz. Observed medium range ROEs (H^{α}-H^{N} *I, 1+3* and H^{α}-H^{β} *I, 1+3*) are indicated by the lines in the lower part of the figure; dotted lines and stars indicate ROEs that could not be unambiguously observed because of chemical shift degeneracy. The coil motif above the primary sequence indicates the region deduced to have helical structure from the NMR data; the dashed coil indicates sections of peptide where only some of the NMR data indicate helical character.

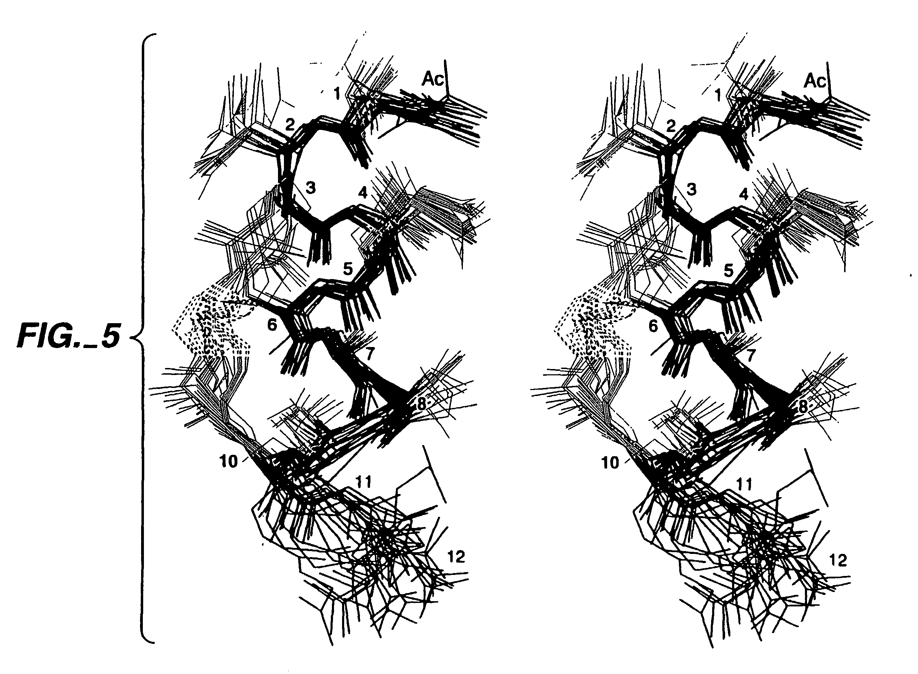

Fig. 5 is a molecular model depicting an ensemble of 20 rMD structures calculated using NMR data for peptide **1c.** The structures were overlayed using the N, Cα and C atoms of residue Thr1 to Gln10. Backbone and side-chain heavy atoms are connected by solid and dotted lines, respectively. The side-chains of Arg8 and Arg9 are truncated at C^{γ}, and all side-chain atoms of Gln11 and Gln12 are omitted for clarity.

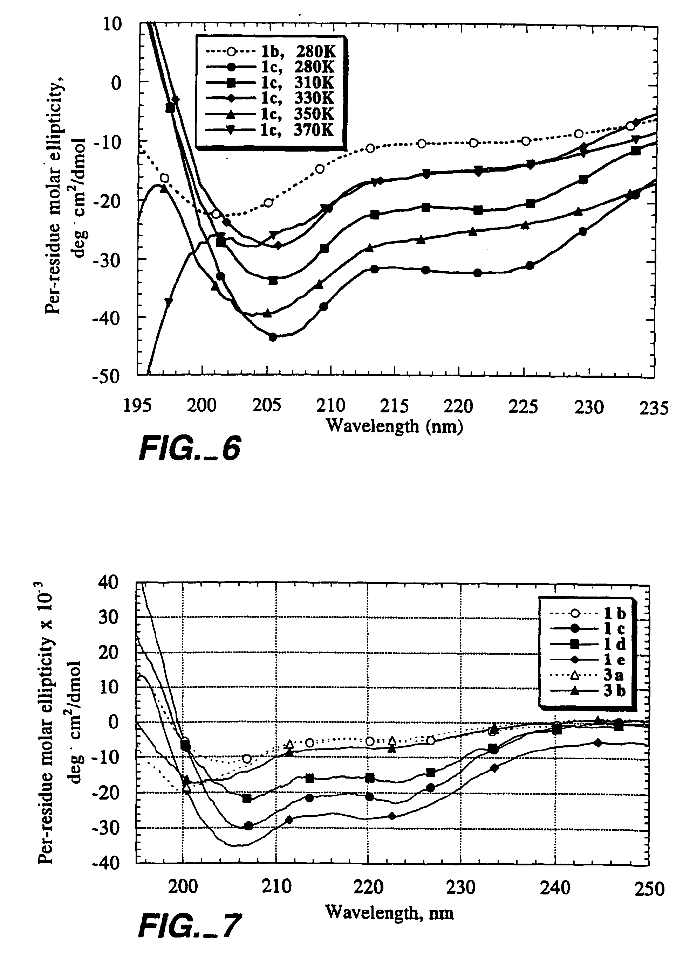

Fig. 6 is a graph depicting the CD spectra of peptide **1c** at 280, 310, 330, 350, and 370 K.
Fig. 7 is a graph depicting the CD spectra of peptides **1** and **3** (Apamin-based sequences) at 280 K.

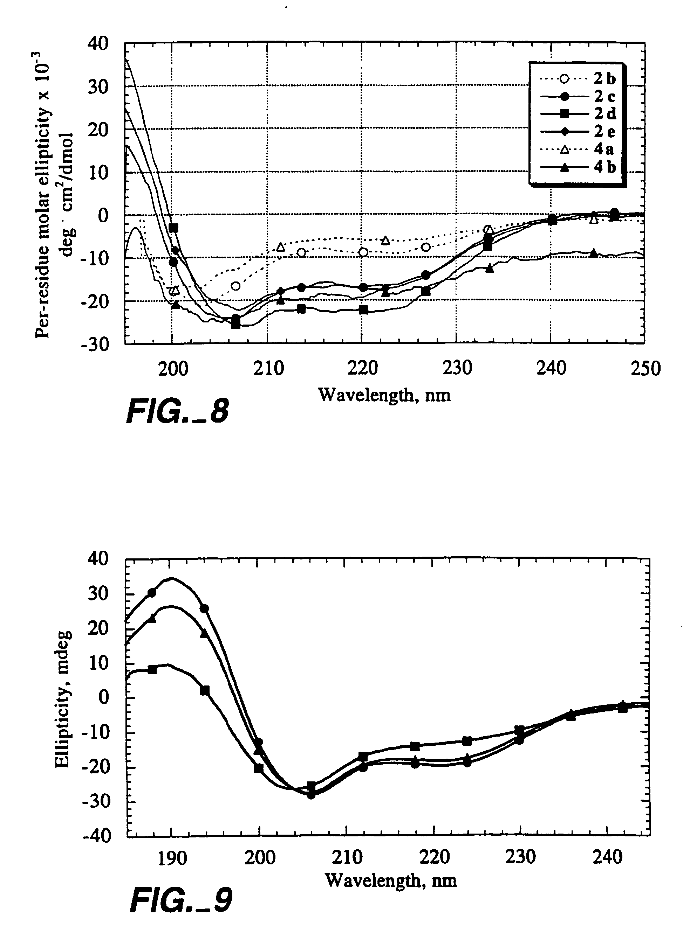

Fig. 8 is a graph depicting the CD spectra of peptides **2** and 4 (C-peptide-based sequences) at 280 K.
Fig. 9 is a graph depicting the thermal denaturation profile of peptide 1c as determined by CD spectra obtained before, during and after heating for 1 day at 87 °C. Circles indicate the initial spectrum obtained from a sample before heating; squares indicate the spectrum obtained from a sample at 87°C during incubation; triangles indicate the spectrum obtained from a sample after recooling to 7 °C at 0.2 °C/min.

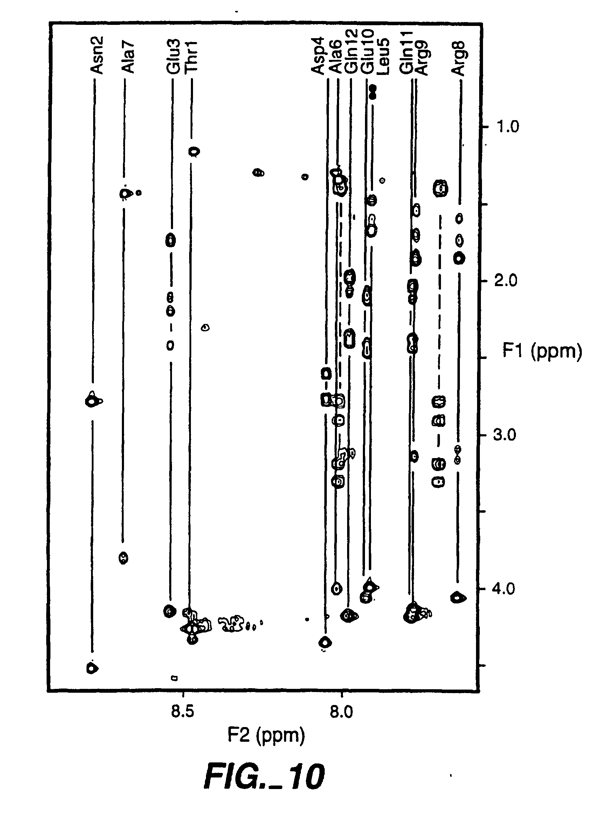

Fig. 10 is a graph depicting a section of the TOCSY spectrum of peptide 1c. The data were collected at 280 K, pH 5.0, 500 MHZ and a peptide concentration of 1.5 mM with a mixing time of 90 ms. The solid lines connect cross-peaks between backbone amide and side chain protons; assignments are indicated at the top of each line. Dashed lines connect cross-peaks between the side chain amide protons of Gln3 and Gln10 and the methylene linker resonances.

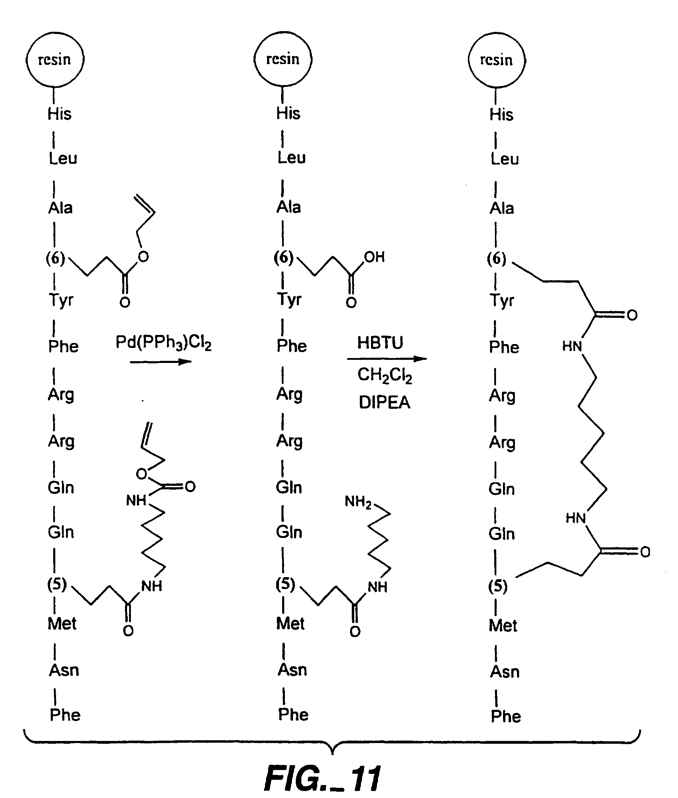

Fig. 11 is a diagram depicting the synthesis of a locked helix species of the peptide Asn-Met-Glu-Gln-Gln-Arg-Arg-Phe-Tyr-Glu-Ala-Leu-His where the carboxy side chains of the Glu residues are covalently linked with a 1,5-pentanediamine linker.

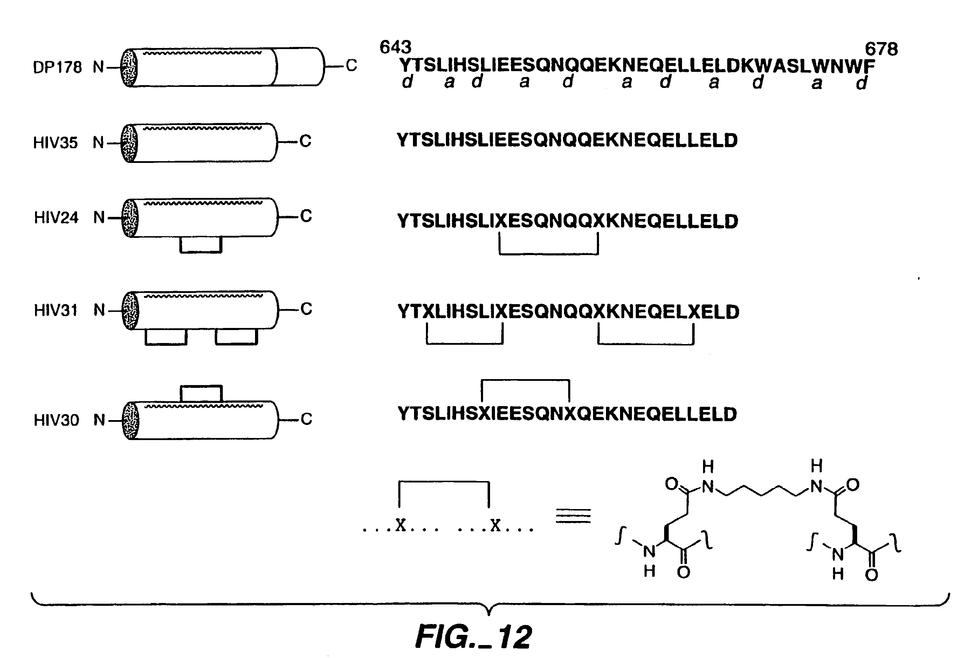

Figure 12 depicts sequences and schematic representationsof the locked-helix peptide embodiments of the invention. The cylinders represent α-helices, with the stippled faces corresponding to the 4, 3 hydrophobic repeat. Covalent restraints linking sidechains at *I* and *I* + 7 are represented as dark lines.

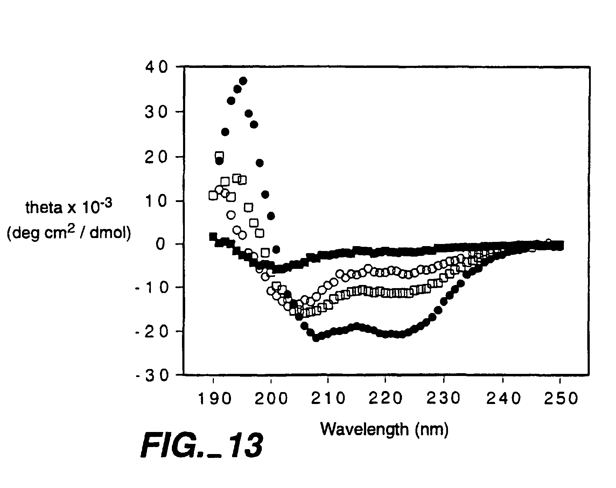

Figure 13 is a circular dichroism spectra of peptides HIV24 (open squares), HIV30 (open circles), HIV31 (closed circles),and HIV35 (closed squares). Spectra were acquired at 7° C in 10 mM Tris-HCI, pH 7.5(21).

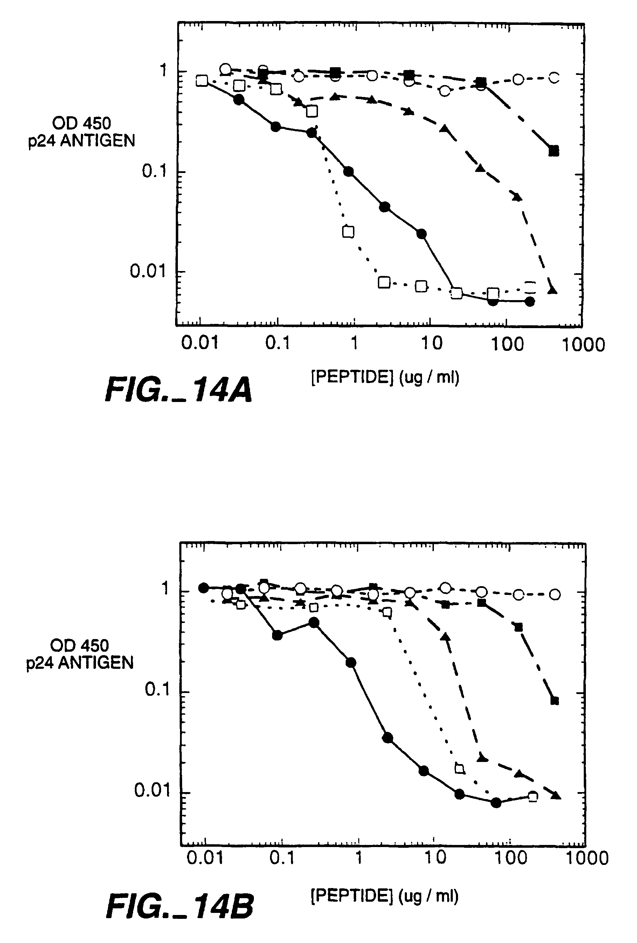

Figures 14A and 14B are graphs depicting the effect of inhibitory peptides in primary infectivity assays using PBMCs with virus JRCSF, an NSI strain (Figure 14A), and BZ167, an SI strain (Figure 14B) (22). HIV24 (closed triangles); HIV30 (open circles); HIV31 (closed circles); HIV35 (closed squares); DP178, (open squares).

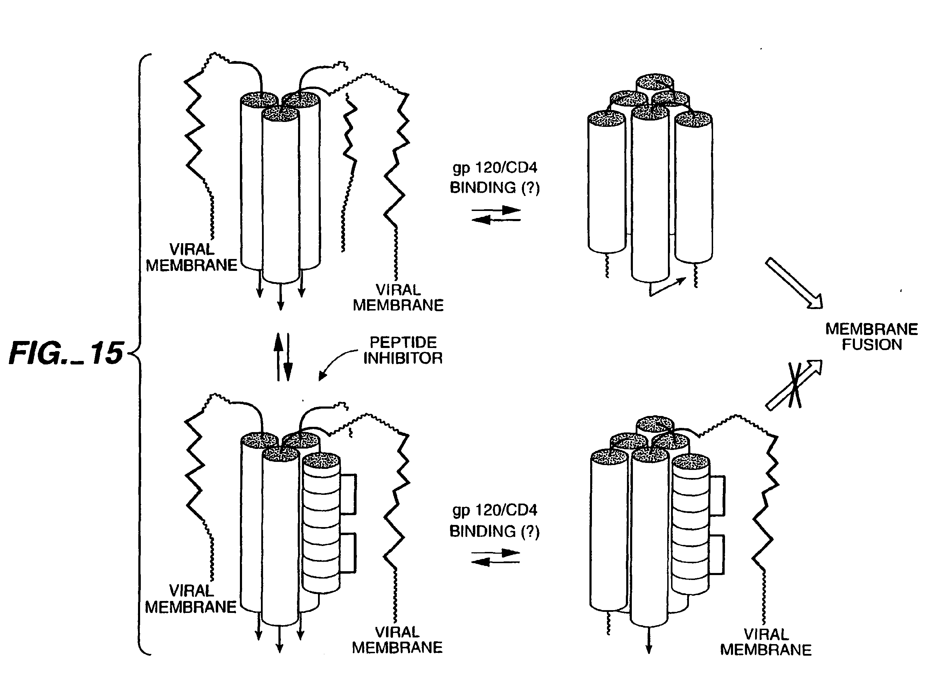

Figure 15 is a schematic of a proposed mechanism for assembly of the fusogenic state of gp41 (top) and inhibition by constrained peptides (bottom).

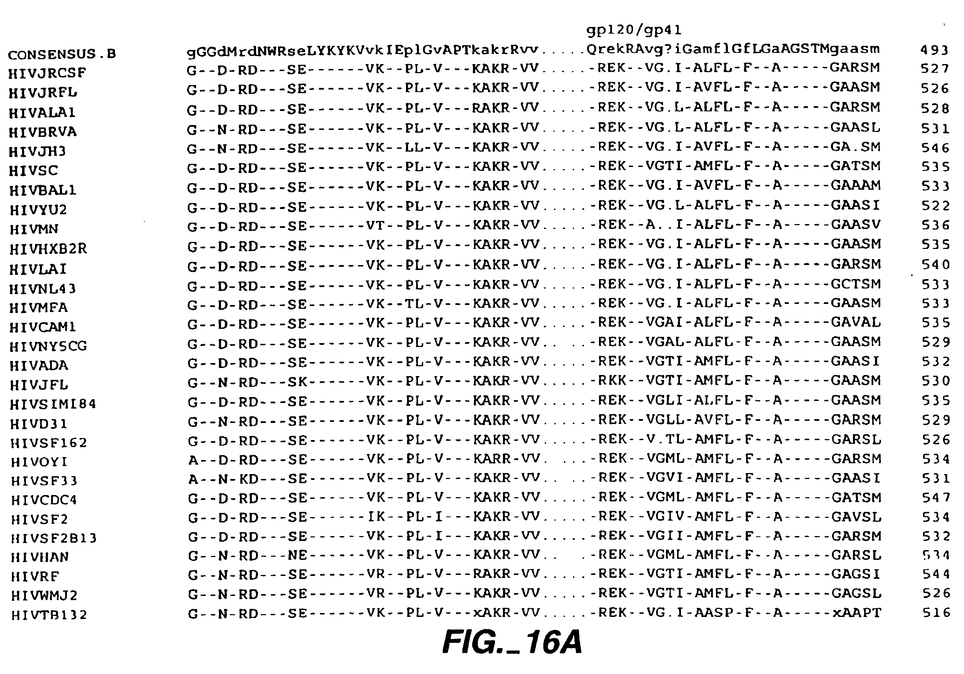

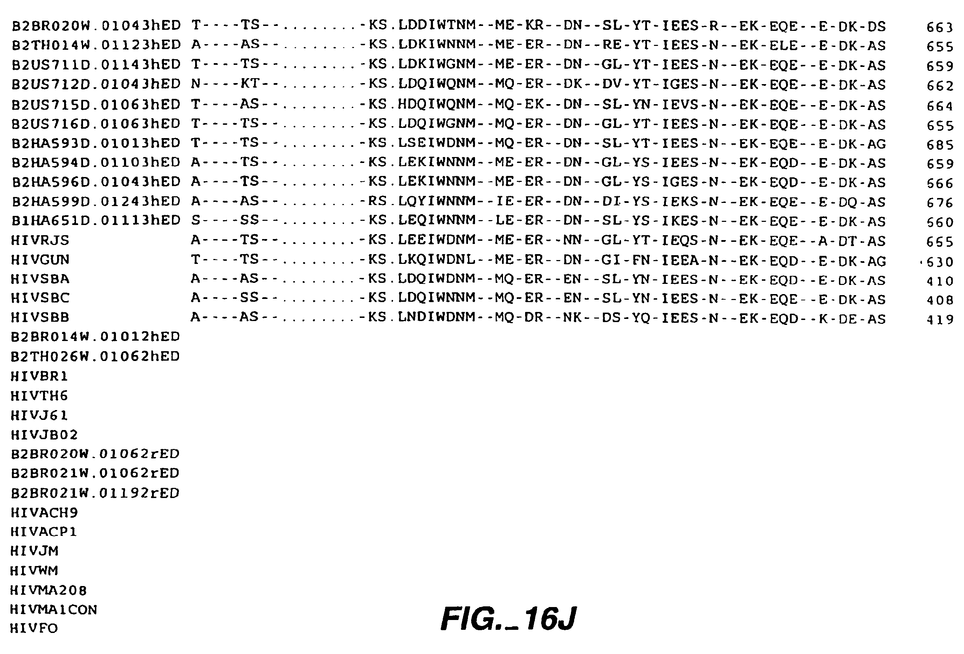

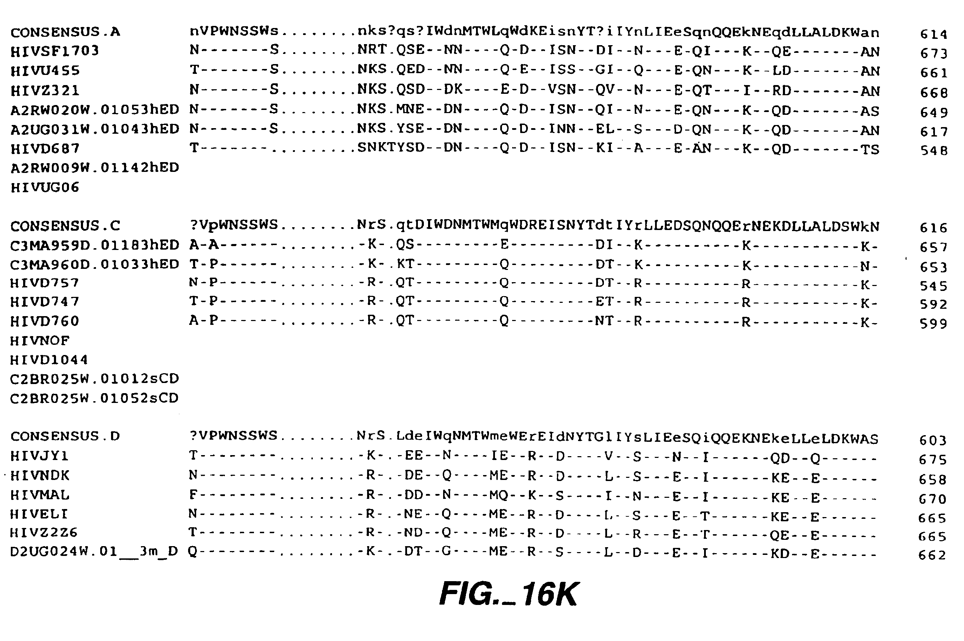

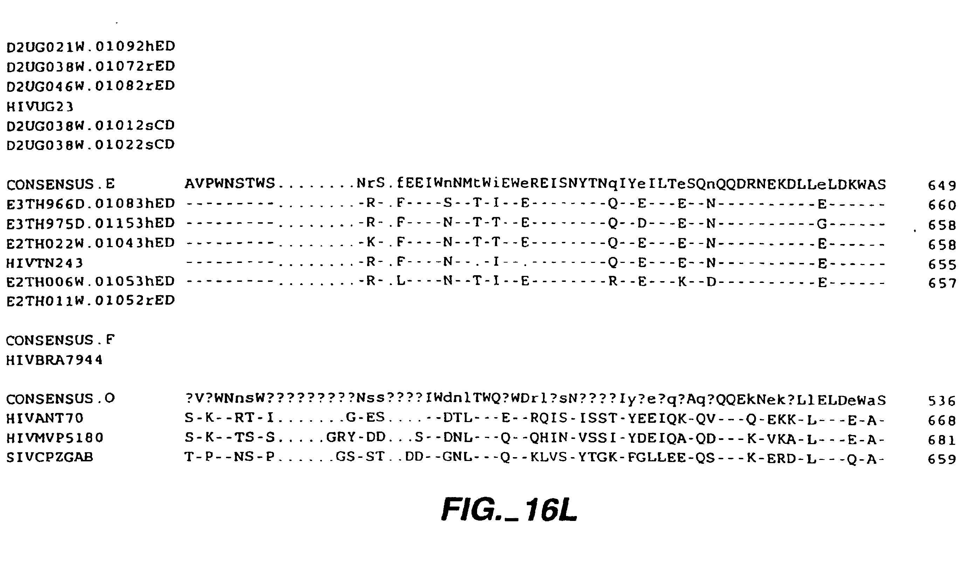

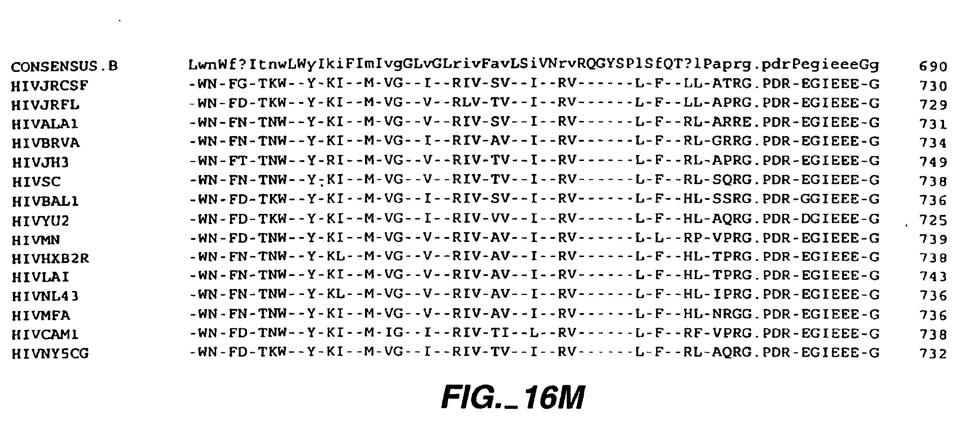

Figures 16A to 16M present amino acid sequences of gp41 from known HIV virus strains and their consensus sequences based on statistical amino acid frequency. Amino acids are represented by the standard single letter code. The strains within each HIV clade are presented. A "-" in a sequence represents the amino corresponding position in viral sequences within that clade. An upper case amino acid in a consensus sequence indicates that only that amino acid is found at that corresponding position in viral sequences within that clade. Strain designations with no sequence information indicate that the complete gp41 sequence has not been determined.

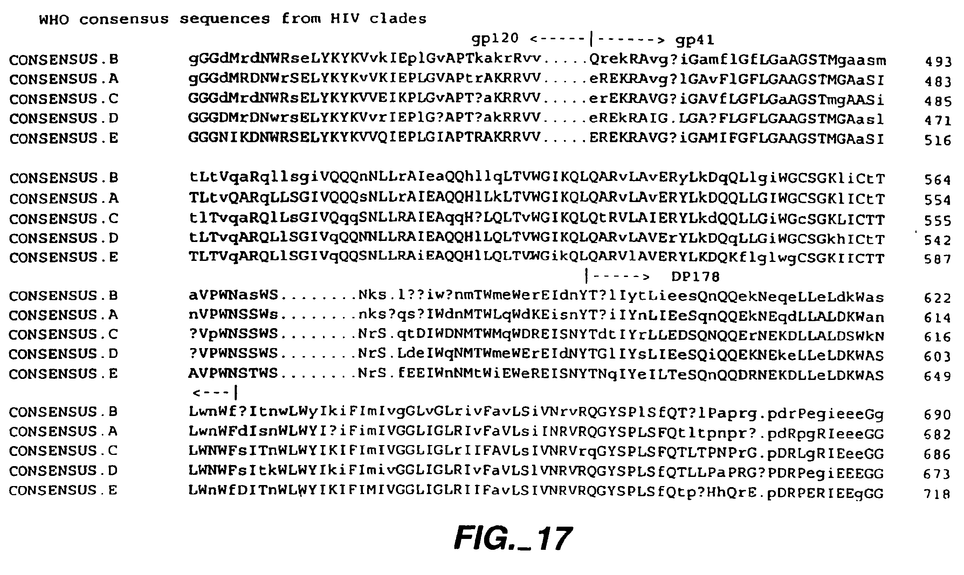

Figure 17 is a summary of consensus sequences from known strains. The peptide sequence of DP178 is delineated. The nomenclature is the same as in Figures 16A to 16G.
Figure 18 is a schematic presenting an alignment of sequences from clades A, B, C, D, and E consensus sequences, peptides DP178, HIV35 and the Neurath peptide, in which the repeating heptad abcdefg assignment as taught herein is provided, and positions of some constraining locks are indicated. For example, amino acids in the sequence ESQNQQ of DP178 are assigned positions **g, a, b, c, d,** and **e**, respectively, and thus has the form **gabcde**, for purposes o the present invention. This sequence is the internal sequence of six amino acids present in peptide HIV24, which is a single-lock form of the HIV35 sequence. Locations of internal sequences of the invention are those found between locking residues, whose positions are indicated by the "l" symbols and each of which, in this example, correspond to assigned position **f**. Positions for placing either one, two or three locks in the representative presented sequences are shown. The figure delineates five **gabcde** form helical sections suitable for locking when locks occur at adjacent **f** positions. Also shown are locations of **gabcde** form helical sections when one, two or three *i* to *i*+7 locks are present in a 633-678 sequence or variant thereof.. The two-lock variants are labeled (II), (III), HIV31, (VI) and (VII), and the one-lock variants (VIII), (IX), HIV24, (XI) and (XII). Three-lock variant is labeled (I).

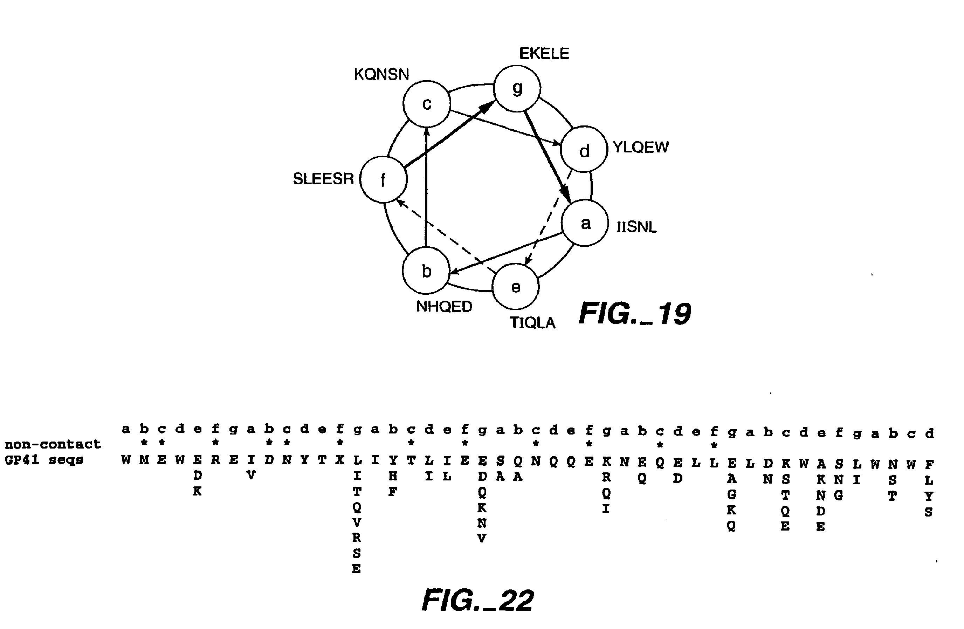

Figure 19 is a helical wheel representation of the representative gp41 fusion peptide sequence from the HIV-1 LAI strain, showing the **"abcdefg"** heptad reading frame and the heptad repeat pattern as assigned herein (see Figure 18) for the purposes of the present invention.

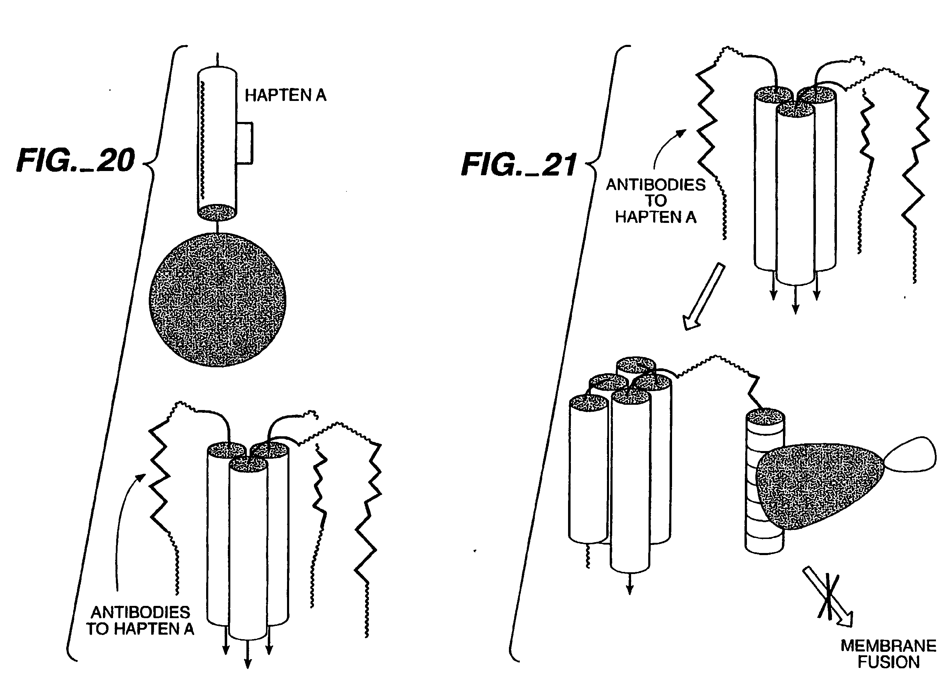

Figure 20 is a schematic depicting the use of the compounds of the invention as haptens for immunization and shows the gp41 core trimer, its DP178 binding groove and the 633-678 region that binds this grove. Hapten a presents the 4,3 repeat surface from HIV 24 in a constrained helix. This face would presumably never be exposed to the immune system since it likely does not form until exposed to the trimer "groove" binding site. An antibody raised to it, however, would essentially be an anti-idiotypic antibody to the trimer grooves. When the resting state gp41 is exposed to this antibody it would induce the C-terminus of gp41 to form a helix in a non-productive state, *i.e.* bound to the antibody, thus sequestering the protein off the fusion pathway. Antibodies to Hapten a are expected to bind to the indicated region, inducing this region to form a helix in a non-productive orientation, thus preventing final assembly of the fusogenic helical bundle.
Figure 21 is a schematic depicting a proposed mechanism for antibody intervention in HIV viral infectivity.
Figure 22 is presents a consensus sequence of the HIV gp41 sequences from Figure 17 with all allowed amino acid substitutions in each position listed. For example, at the fifth amino acid position (starting from the N-terminal amino acid (left end)), the amino acids E (glutamic acid), D (aspartic acid) and K (lysine) are allowed without disrupting H-bonding, thus without disrupting helicity or significantly interfering with the peptide's interaction with the core coiled-coil trimer of gp41. "X" indicates positions that can be substituted with any non-helix breaking amino acid. The repeating heptad abcdefg assignment for each amino acid position in the 633 to 678 sequence, for purposes of the present invention, is shown. The "*" indicate b, c, and f positions that, when not used for locking the helix, can be replaced with a non helix-breaking amino acid without significantly disturbing H-bonding, helicity and trimer groove binding.

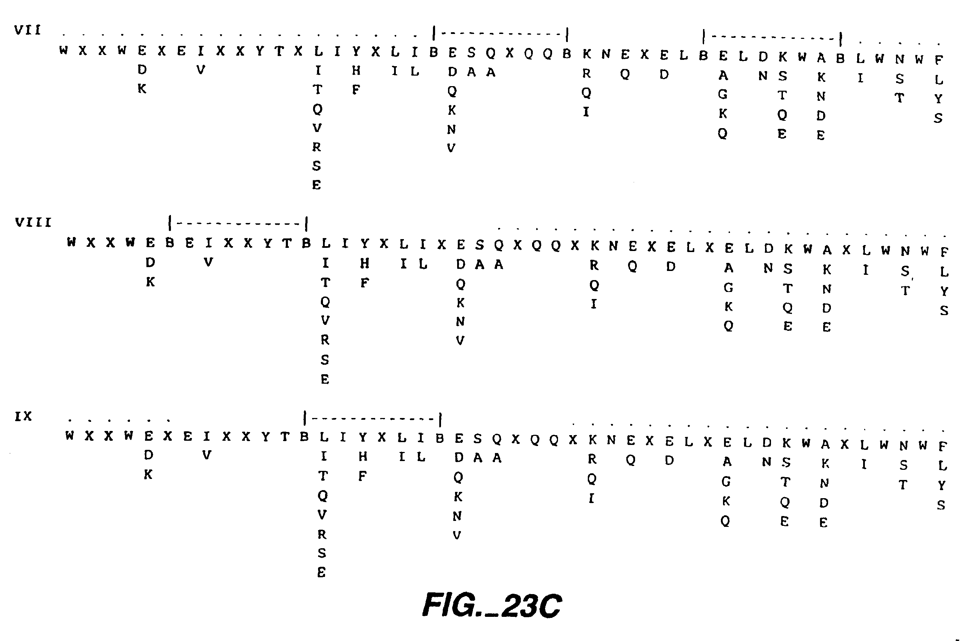

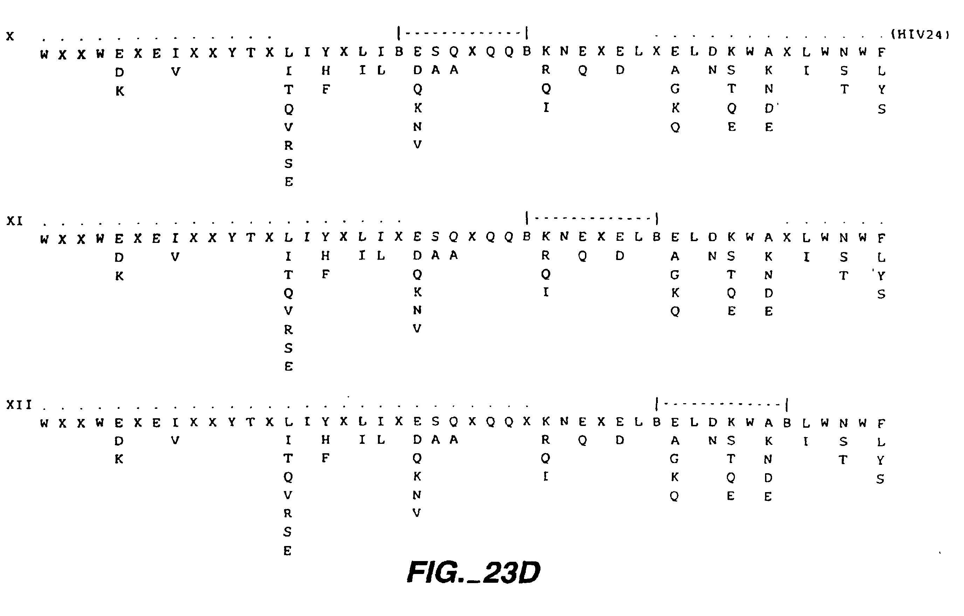

Figure 23 presents a shorthand notation of specific peptides in peptide series I through XII (as in Figure 18), indicating locking positions, amino acid substitution variant peptides, and truncation variant peptides of each. The "X" indicates a position that can be substituted with any non helix-breaking amino acid,

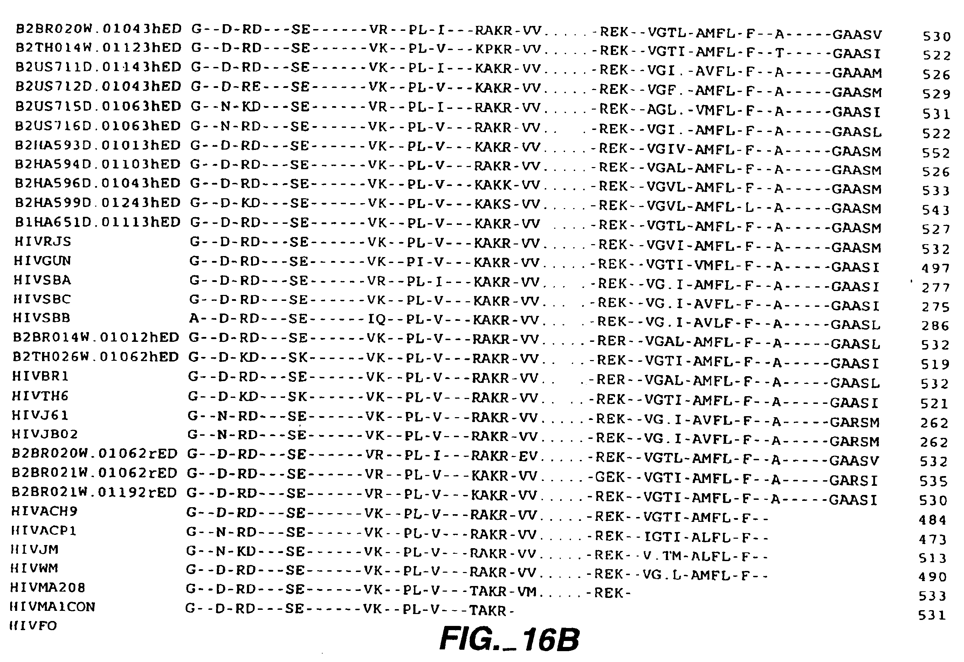

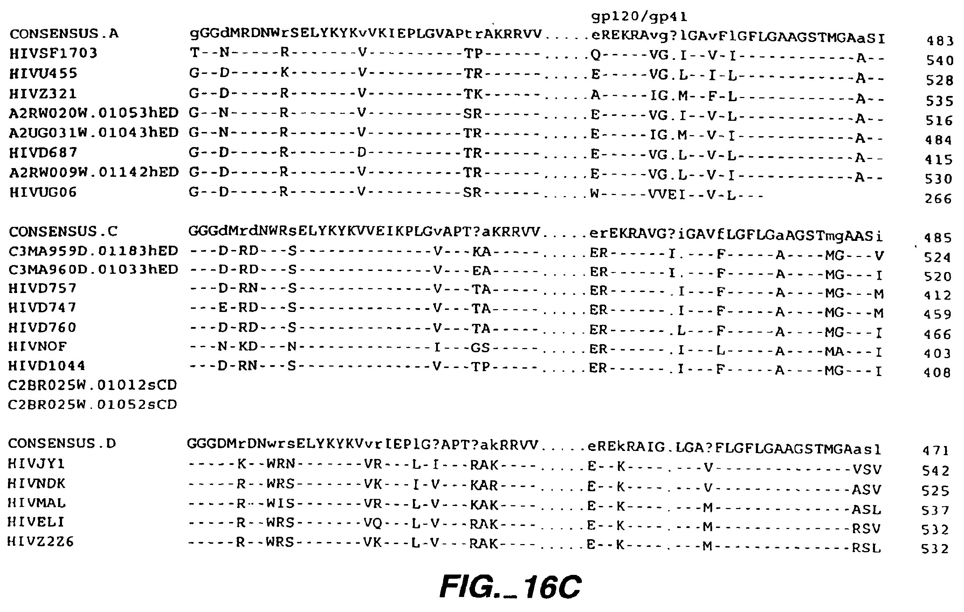

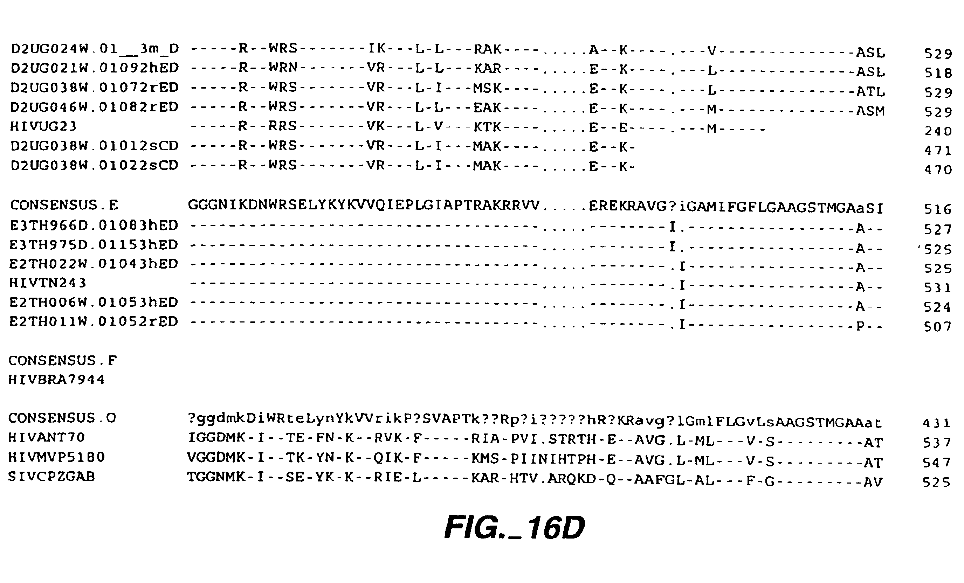

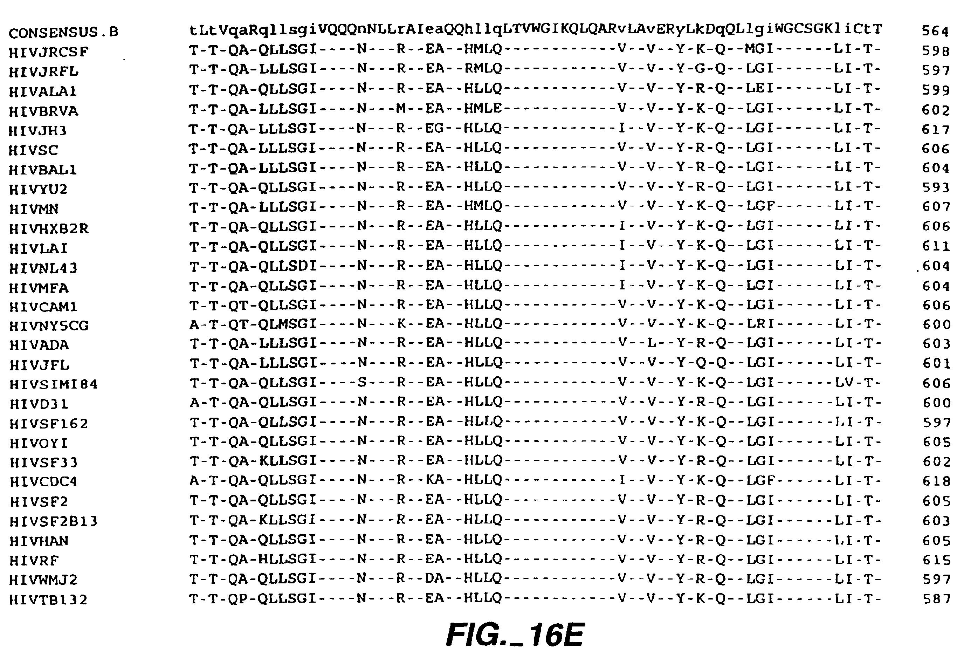

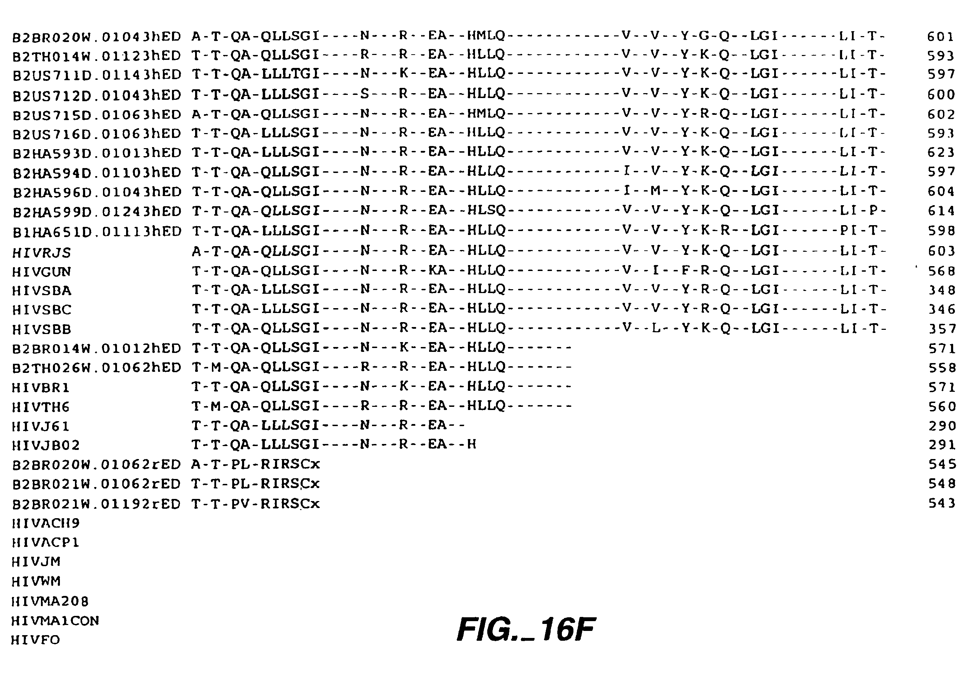

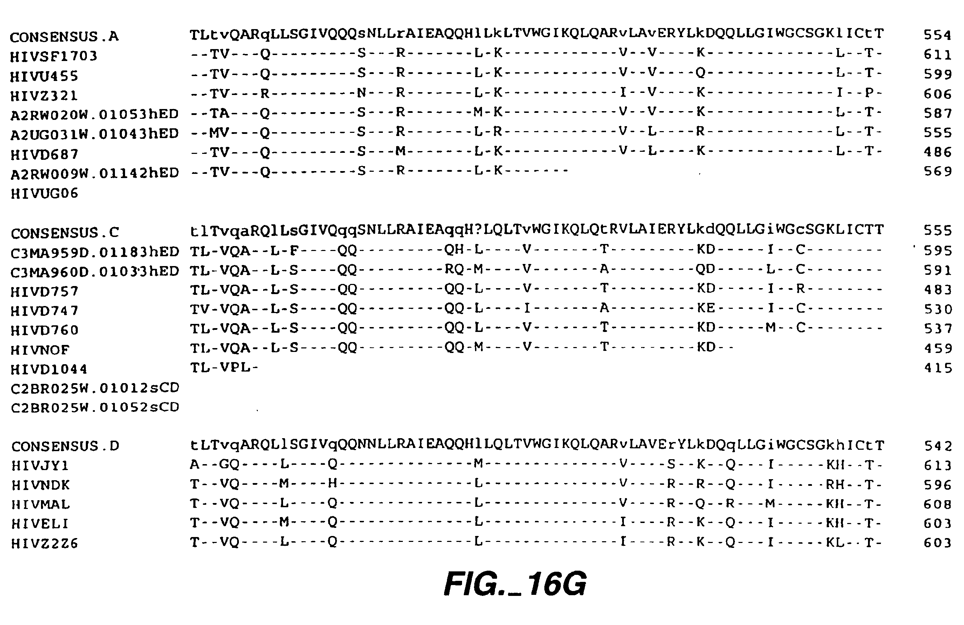

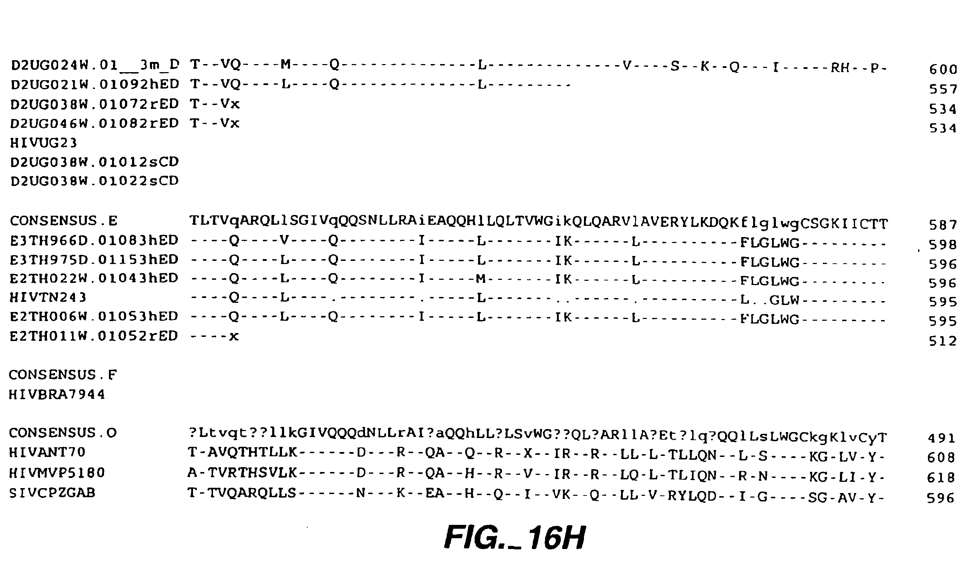

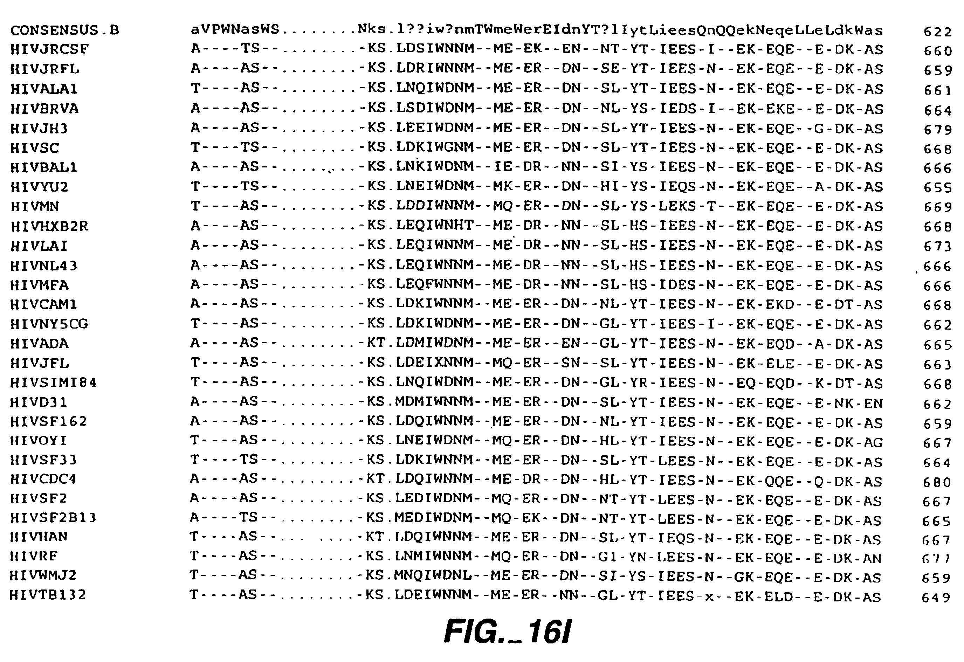

Figure 23 presents a shorthand notation of specific peptides in peptide series I through XII (as in Figure 18), indicating locking positions, amino acid substitution variant peptides, and truncation variant peptides of each. The "X" indicates a position that can be substituted with any non helix-breakingamino acid, but preferably with an amino acid present in that position from any one of the known HIV sequences shown in Figure 16. "B" indicates a position used for the bridging (or tethering or locking) residues. Preferred **f** positions are presented for locking; however in less preferred embodiments the **c** and some **b** positions can be used for locking. As in Figure 18, locations of internal sequences relevant to the invention are those found between locking residues whose positions are indicated by the "|" symbols and correspond to assigned position **f**, in this example. Positions for placing either one, two or three locks in the representative presented sequences are shown. The figure delineates five **gabcde** form helical sections suitable for locking when locks occur at adjacent **f** positions. The "." indicates positions that can be optionally absent from the final constrained helical peptide compound without substantially effecting the helical properties and groove binding properties of the final constrained helical peptide. For example, a peptide based on peptide I, having the three locks placed as indicated, can optionally lack any one or all of the five N-terminal amino acids WXXWE, which are marked by a ".". Further, another series of truncated variants is indicated in the figure-C-terminal truncated variants--sincethe five C-terminal residues (LWNWF) are marked with a "." can be absent. When the lock is placed more centrally in the 633-678 sequence, as shown in peptide series II, peptides in this series can lack additional amino acids at the C-terminal end as indicated by the "." marked positions.

### DETAILED DESCRIPTION OF THE PREFERRED EMBODIMENTS

### A. DEFINITIONS

Amino acids and amino acid residues described herein may be referred to according to the accepted one or three letter code provided in the table below. Unless otherwise specified, these amino acids or residues are of the naturally occurring L stereoisomer form.

| Common Name | One-Letter Symbol | Three-Letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |

In general, unless otherwise specified, the abbreviations used for the designation of amino acids and the protective groups used therefor are based on the recommendations of the IUPAC-IUB Commission of Biochemical Nomenclature (Biochemistry, 11: 1726-1732 (1972)).

As used herein, the term -(CH₂)ₙ- is used to denote a straight chain alkyl substituent of n carbons in length, wherein -(CH₂)₀- is defined as a chemical bond. i.e. indicating that no alkyl substituent is present, - (CH₂)₁- is defined as a methyl substituent, -(CH₂)₂- is defined as an ethyl substituent, etc.

As used herein, the term "C₁-C₆alkyl means a saturated aliphatic hydrocarbon substituent having the number of carbon atoms specified. C₁-C₆alkyl encompasses cyclic and straight chain hydrocarbons, unbranched and branched hydrocarbons, substituted and unsubstituted hydrocarbons, and primary, secondary and tertiary hydrocarbon substituents. Representative examples of these alkyl substituents include methyl, fluorenylmethyl, ethyl, n-propyl, isopropyl, n-butyl, isobutyl, sec-butyl, tert-butyl, n-pentyl, 2-methylbutyl, 2,2-dimethylpropyl,n-hexyl,2-methylpentyl,2,2-dimethylbutyl, cyclohexyl, and the like. The terms "lower alkyl", "simple alkyl" and "C₁-C₆alkyl" are synonymous and used interchangeably.

As used herein, the terms "peptide", "polypeptide", and "protein" are used synonymously and refer to any proteinaceous compound comprising an amino acid sequence of two or more amino acid residues.

As used herein, an "amide bond-forming substituentcontained in an amino acid side chain", a "side chain amide bond-formingsubsituent", and their grammatical variants, are defined to include (1) any carboxy substituent contained in the side chain ("R" group) of an amino acid wherein the carboxy substituent is capable of forming an amide linkage with an amino group contained in another molecule, i.e. the carboxy substitutent reacts with an amino group contained in another molecule to form an amide linkage; and (2) any amino substituent contained in the side chain ("R" group) of an amino acid wherein the amino substituent is capable of forming an amide linkage with a carboxy group contained in another molecule, i.e. the amino substitutent reacts with a carboxy group contained in another molecule to form an amide linkage.

As used herein, "differentially removable" protecting or protective groups are defined as any pair of protective groups capable of protecting a first amide bond-forming substituent and a second amide bond-forming substituent, wherein it is possible to deprotect the first amide bond-forming substituent protected with one member of the pair under conditions which do not deprotect the second amide bond-forming substituent protected with the other member of the pair. Differentially removable protecting groups are also referred to herein as "orthogonal" protecting groups, and the differentially removable protection conferred by such protective groups is referred to herein as "orthogonal" protection.

The term "epitope" as used herein, designates the structural component of a molecule that is responsible for specific interactions with correspondingantibody (immunoglobulin) molecules elicited by the same or related antigen. More generally, the term refers to a peptide having the same or similar immunoreactive properties, such as specific antibody binding affinity, as the antigenic protein or peptide used to generate the antibody. Therefore, an epitope that is formed by a specific peptide sequence generally refers to any peptide which is reactive with antibodies directed against the specific sequence.

The term "antigen" as used herein, means a molecule which is used to induce production of antibodies. The term is alternatively used to denote a molecule which is reactive with a specific antibody.

The term "immunogen" as used herein, describes an entity that induces antibody production in a host animal. In some instances the antigen and the immunogen are the same entity, while in other instances the two entities are different.

The term "subunit vaccine" is used herein, as in the art, to refer to a viral vaccine that does not contain virus, but rather contains one or more viral proteins or fragments of viral proteins. As used herein, the term "multivalent",means that the vaccine contains a constrained helical peptide or peptides having a gp41-based sequence from at least two HIV isolates having different amino acid sequences.

The term "breakthrough isolate" or "breakthroughvirus" is used herein, as in the art, to refer to a virus isolated from a vaccinee.

### B. GENERAL METHODS

In general, the invention provides a method for removing elements of α-helical secondary structure from the context of a protein without losing the well defined structure found within the protein's α-helix. In one aspect, the method is useful for artificially reconstructing and characterizing the binding determinants that exist within an α-helical binding domain of a protein of interest. The design of molecules which are capable of binding competitively at a protein interface requires the ability to mimic the higher level structure of the natural ligand. If the ligand's structure at the site of protein interface can be mimicked with a short peptide, then the peptide can be used to determine whether it is feasible to design small molecules that competitively bind at the protein interface. A short peptide's ability to compete with the natural ligand for binding at the protein interface would indicate that the ligand's structure at the contact point with the protein interface is such that the short peptide could be used as a model for designing small molecules that compete with the natural ligand for binding at the protein interface.

In another aspect, the methods of the invention are used to stabilize the conformational structure of a protein or peptide. The present methods can be employed to lock in place one (or more) α-helical determinant(s) of interest in a protein or peptide such that the protein (or peptide) retains an α-helical conformation in environments or conditions that would destabilize or deteriorate the α-helical secondary structure of an unconstrained protein or peptide species.

The methods of the invention are also useful for the replication of protein function without an intact protein or intact functional domain. For example, the replication of a protein's binding activity by a constrained helical peptide of the invention would allow the use of affinity purification procedures for the protein's ligand without requiring a supply of intact protein or large fragments thereof. Thus, a constrained helical peptide possessing a particular protein's binding activity could overcome supply or cost problems preventing the use of the protein in affinity purification. In yet another example, a constrained helical peptide possessing the conformational structure at the site of interest in a particular protein could be used to isolate a conformational epitope from the rest of the protein and raise antibodies against the single epitope of interest without interference from the other antigenic sites existing in the intact protein.

Particularly preferred are the use of the compounds of the invention having constrained helical peptides having internal amino acid sequences from the HIV isolate LAI gp41 amino acid sequence 633-678 and homologs thereof, for use as haptens, vaccines, and in diagnostics.

In another aspect, the methods and peptides of the invention can be used to create combinatorial constrained helical peptide libraries that are useful in chemical selection systems.

### I. Locked Helix Peptides and Uses Therefor

The invention provides locked helix peptides of formula (I): 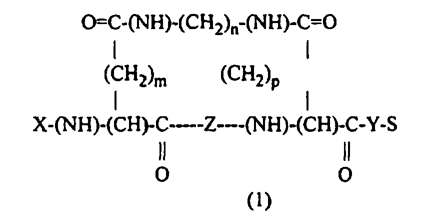
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; m and p are independently selected from the integers 0 to 6 inclusive, provided that m+p is less than or equal to 6; and n is any integer in the range defined by (7-(m+p)) to (9-(m+p)) inclusive, provided that n is greater than 1.

In another embodiment, the invention provides locked helix peptides of formula (2): 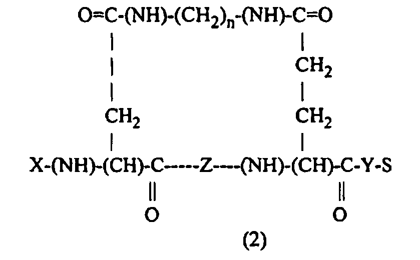
wherein S is absent or is a macromolecule; X is hydrogen or is any amino acid or amino acid sequence: Y is absent, or is hydroxyl if S is absent, or is any amino acid or amino acid sequence;
Z is any amino acid sequence consisting of six amino acids; and n is any integer from 4 to 6 inclusive.

In yet another embodiment, the invention provides locked helix peptides of formula (3): 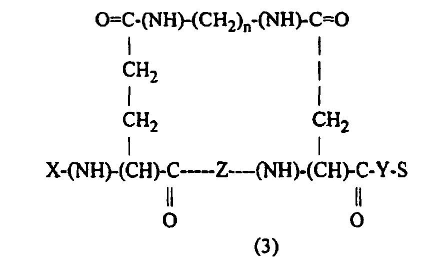
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 4 to 6 inclusive.

In still another embodiment, the invention provides locked helix peptides of formula (4): 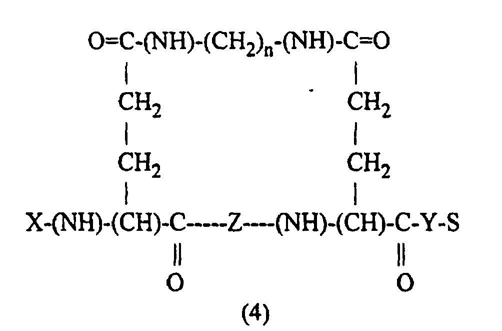
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 3 to 5 inclusive.

In still another embodiment, the invention provides locked helix peptides of formula (5): 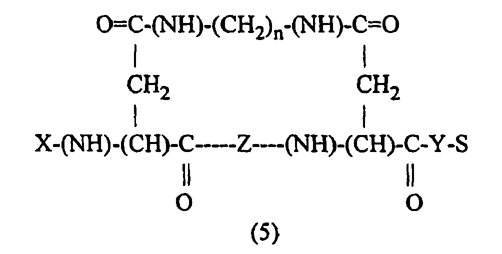
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 5 to 7 inclusive.

In still another embodiment, the invention provides locked helix peptides of formula (6): 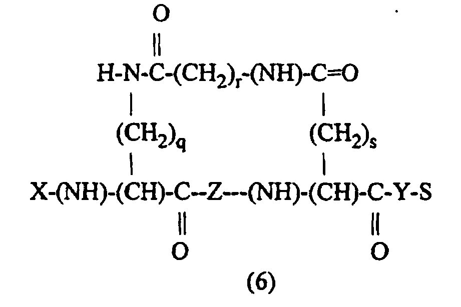
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; q is selected from the integers 1 to 7 inclusive, and s is selected from the integers 0 to 6 inclusive, provided that q+s is less than or equal to 7; and r is any integer in the range defined by (7-(q+s)) to (9-(q+s)) inclusive, provided that r is greater than 0.

In still another embodiment, the invention provides locked helix peptides of formula (7): 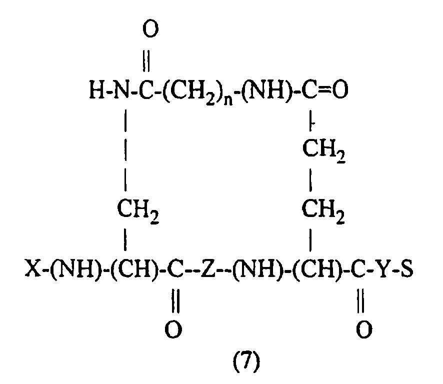
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 4 to 6 inclusive.

In still another embodiment, the invention provides locked helix peptides of formula (8): 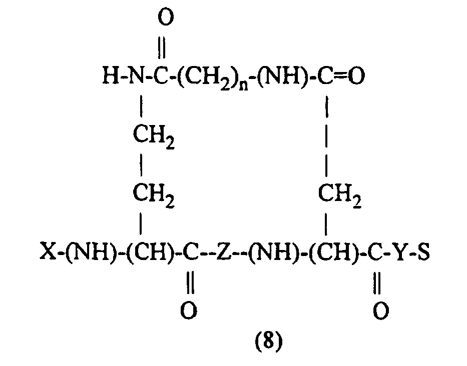
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 4 to 6 inclusive.

In still another embodiment, the invention provides locked helix peptides of formula (9): 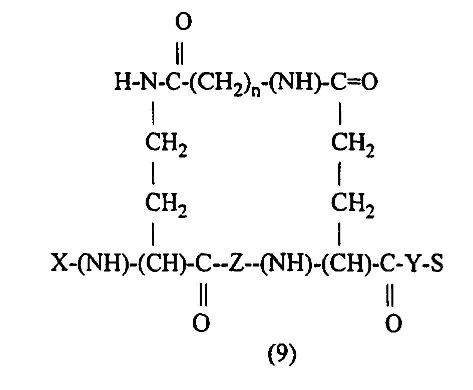
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 3 to 5 inclusive.

In still another embodiment, the invention provides locked helix peptides of formula (10): 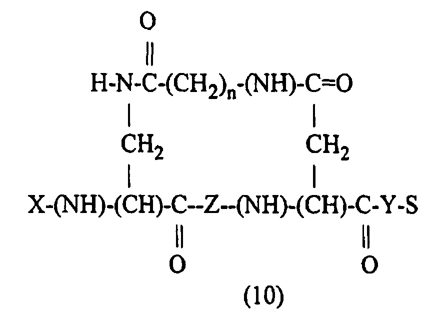
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 5 to 7 inclusive.

In still another embodiment, the invention provides locked helix peptides of formula (11): 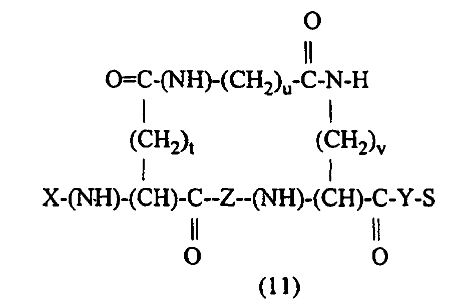
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; t is selected from the integers 0 to 6 inclusive, and
v is selected from the integers 1 to 7 inclusive, provided that t+v is less than or equal to 7; and u is any integer in the range defined by (7-(t+v)) to (9-(t+v)) inclusive, provided that u is greater than zero.

In still another embodiment, the invention provides locked helix peptides of formula (12): 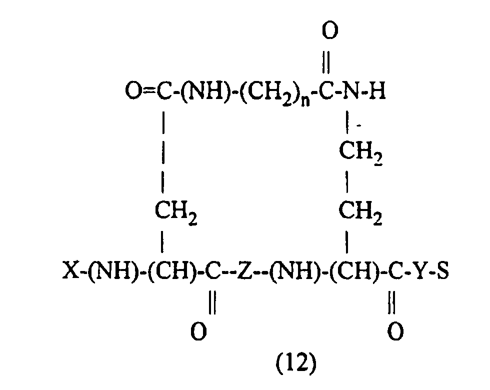
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 4 to 6 inclusive.

In still another embodiment, the invention provides locked helix peptides of formula (13): 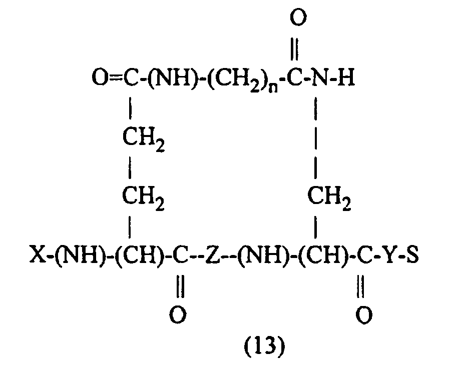
wherein S is absent or is a macromolecule, X is hydrogen or is any amino acid or amino acid sequence; Y is absent, or is hydroxyl if S is absent, or is any amino acid or amino acid sequence;
Z is any amino acid sequence consisting of six amino acids; and n is any integer from 4 to 6 inclusive.

In still another embodiment, the invention provides locked helix peptides of formula (14): 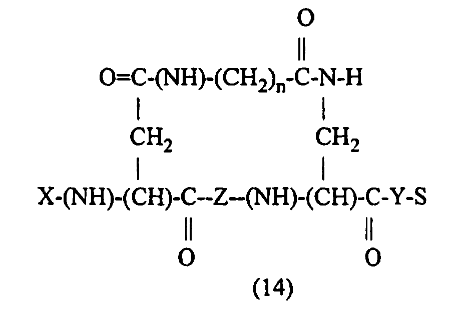
wherein S is absent or is a macromolecule; X is hydrogen or is any amino acid or amino acid sequence; Y is absent, or is hydroxyl if S is absent, or is any amino acid or amino acid sequence:
Z is any amino acid sequence consisting of six amino acids; and n is any integer from 5 to 7 inclusive.

In still another embodiment, the invention provides locked helix peptides of formula (15): 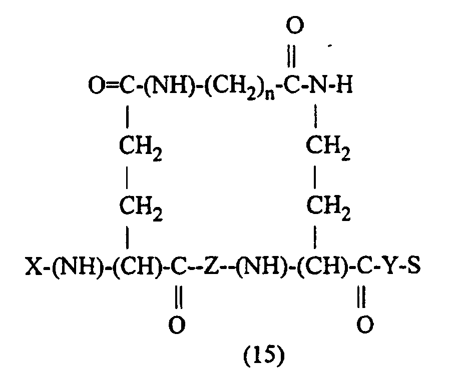
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 3 to 5 inclusive.

In still another embodiment, the invention provides locked helix peptides of formula (16): 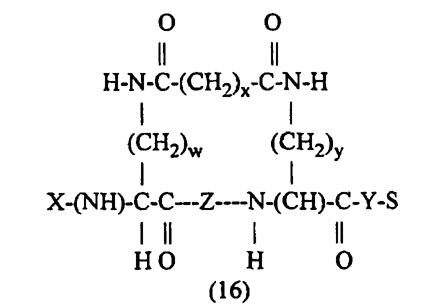
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; w and y are independently selected from the integers 1 to 7 inclusive, provided that w+y is less than or equal to 8; and x is any integer in the range defined by (7-(w+y)) to (9-(w+y)) inclusive, provided that x is greater than or equal to 0.

In still another embodiment, the invention provides locked helix peptides of formula (17): 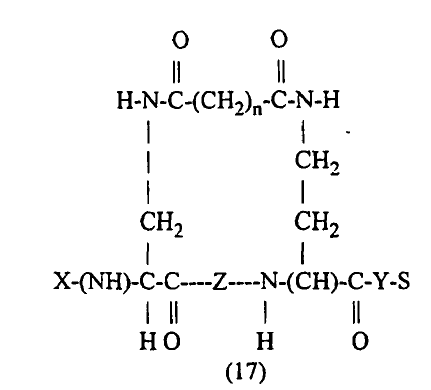
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 4 to 6 inclusive.

In still another embodiment, the invention provides locked helix peptides of formula (18): 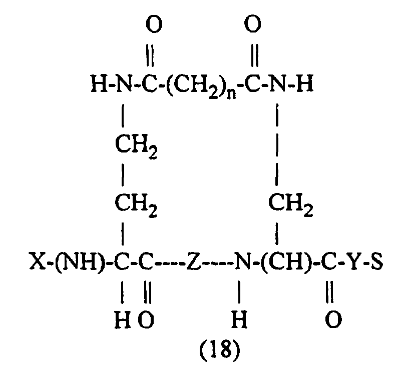
wherein S is absent or is a macromolecule; X is hydrogen or is any amino acid or amino acid sequence; Y is absent, or is hydroxyl if S is absent, or is any amino acid or amino acid sequence:
Z is any amino acid sequence consisting of six amino acids; and n is any integer from 4 to 6 inclusive.

In still another embodiment, the invention provides locked helix peptides of formula (19): 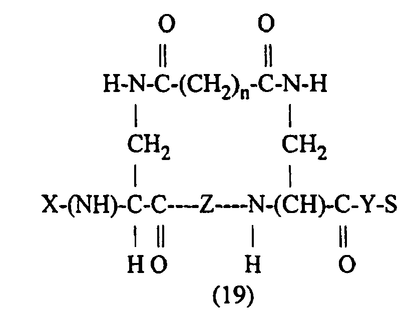
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 5 to 7 inclusive.

In still another embodiment, the invention provides locked helix peptides of formula (20): 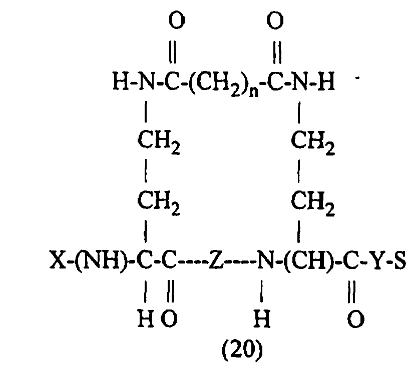
wherein S is absent or is a macromolecule; X is hydrogen or is any amino acid or amino acid sequence; Y is absent, or is hydroxyl if S is absent, or is any amino acid or amino acid sequence;
Z is any amino acid sequence consisting of six amino acids; and n is any integer from 3 to 5 inclusive.

In a further embodiment, the invention provides locked helix peptides of formula (1), formula (2), formula (3), formula (4), formula (5), formula (6), formula (7), formula (8), formula (9), formula (10), formula (11), formula (12), formula (13), formula (14), formula (15), formula (16), formula(17), formula (18), formula (19) and formula (20) wherein X, Y, and Z collectively contain up to or about 35 amino acids (i.e. locked helix peptides of formulas (1), (2), (3), (4), (5), (6), (7), (8), (9), (10), (11), (12), (13), (14), (15), (16), (17), (18), (19) and (20) each of which contains a total of no more than or about 35 amino acid residues).

Also provided herein are locked helix peptides of formula (1), formula (2), formula (3), formula (4), formula (5), formula (6), formula (7), formula (8), formula (9), formula (10), formula (11), formula (12), formula (13), formula (14), formula (15), formula (16), formula (17), formula (18), formula (19) and formula (20) wherein X and/or Y contain(s) up to or about 30 amino acid residues.

Further provided herein are locked helix peptides of formula (1), formula (2), formula (3), formula (4), formula(5), formula(6), formula(7), formula(8), formula (9), formula (10), formula (11), formula (12), formula(13), formula(14), formula(15), formula(16), formula (17), formula(18), formula (19) and formula (20) wherein X and/or Y contain(s) up to or about 25 amino acid residues.

Additionally provided herein are locked helix peptides of formula (1), formula (2), formula (3), formula (4), formula (5), formula (6), formula (7), formula (8), formula (9), formula (10), formula (11), formula (12), formula (13), formula (14), formula (15), formula (16), formula (17), formula (18), formula (19) and formula (20) wherein X and/or Y contain(s) up to or about 20 amino acid residues.

Also encompassed herein are locked helix peptides of formula (1), formula (2), formula (3), formula (4), formula (5), formula(6), formula (7), formula(8), formula (9), formula (10), formula (11), formula (12), formula (13), formula (14), formula (15), formula (16), formula (17), formula (18), formula (19) and formula (20) wherein X and/or Y contain(s) up to or about 15 amino acid residues.

Further encompassed herein are locked helix peptides of formula (1), formula (2), formula (3), formula (4), formula (5), formula (6), formula (7), formula (8), formula (9), formula (10), formula (11), formula (12), formula(13), formula (14), formula (15), formula (16), formula (17), formula (18), formula (19) and formula (20) wherein X and/or Y contain(s) up to or about 10 amino acid residues.

Additionally encompassed herein are locked helix peptides of formula (1), formula (2), formula (3), formula (4), formula (5), formula (6), formula (7), formula (8), formula (9), formula (10), formula (11), formula (12), formula (13), formula (14), formula (15), formula (16), formula (17), formula (18), formula (19) and formula (20) wherein X and/or Y contain(s) up to or about 5 amino acid residues.

Also within the scope of the invention are locked helix peptides of formula (1), formula (2), formula (3), formula (4), formula (5), formula (6), formula (7), formula (8), formula (9), formula (10), formula (11), formula (12), formula (13), formula (14), formula (15), formula (16), formula (17), formula (19), formula (19) and formula (20) wherein X and/or Y contain(s) up to or about 3 amino acid residues.

The invention also provides locked helix peptides of formula (1a): 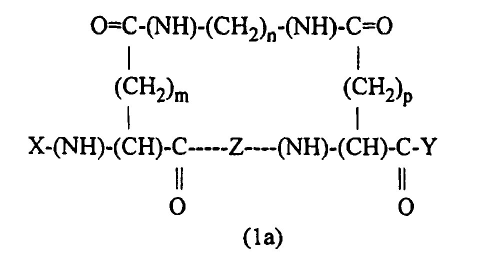
wherein X is hydrogen or is any amino acid or amino acid sequence; Y is hydroxyl or is any amino acid or amino acid sequence; Z is any amino acid sequence consistingof six amino acids; m and p are independently selected from the integers 0 to 6 inclusive, provided that m+p is less than or equal to 6; and n is any integer in the range defined by (7-(m+p)) to (9-(m+p)) inclusive, provided that n is greater than 1.

In another embodiment, the invention provides locked helix peptides of formula (2a): 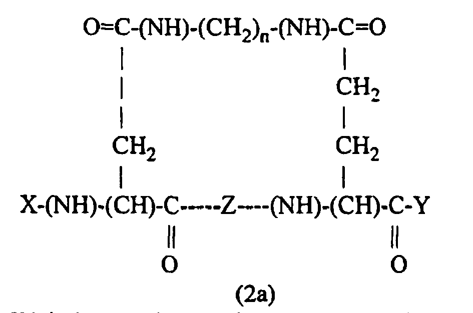
wherein X is hydrogen or is any amino acid or amino acid sequence; Y is hydroxyl or is any amino acid or amino acid sequence; Z is any amino acid sequence consisting of six amino acids; and n is any integer from 4 to 6 inclusive.

In yet another embodiment, the invention provides locked helix peptides of formula (3a): 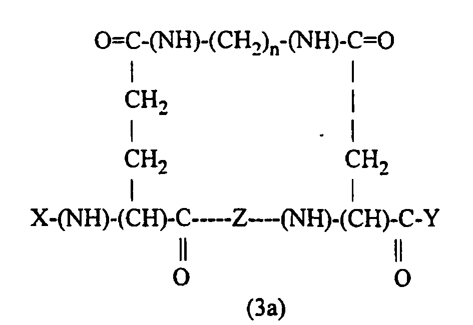
wherein X is hydrogen or is any amino acid or amino acid sequence; Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (4a): 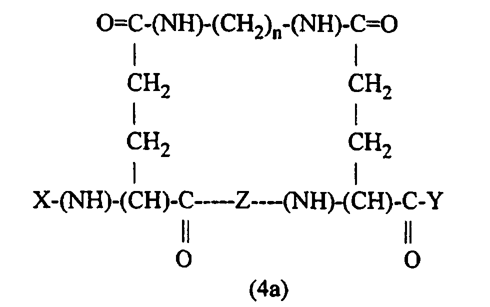
wherein X is hydrogen or is any amino acid or amino acid sequence; Y is hydroxyl or is any amino acid or amino acid sequence; Z is any amino acid sequence consisting of six amino acids; and n is any integer from 3 to 5 inclusive.

In still another embodiment, the invention provides locked helix peptides of formula (5a): 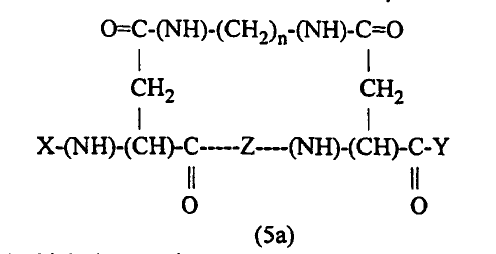
wherein X is hydrogen or is any amino acid or amino acid sequence; Y is hydroxyl or is any amino acid or amino acid sequence; Z is any amino acid sequence consisting of six amino acids; and n is any integer from 5 to 7 inclusive.

In still another embodiment, the invention provides locked helix peptides of formula (6a): 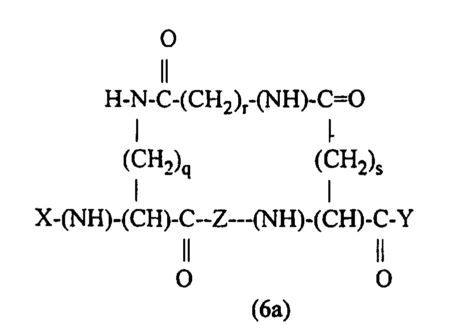
wherein X is hydrogen or is any amino acid or amino acid sequence; Y is hydroxyl or is any amino acid or amino acid sequence; Z is any amino acid sequence consisting of six amino acids; q is selected from the integers 1 to 7 inclusive, and s is selected from the integers 0 to 6 inclusive, provided that q+s is less than or equal to 7; and r is any integer in the range defined by (7-(q+s)) to (9-(q+s)) inclusive, provided that r is greater than 0.

In still another embodiment, the invention provides locked helix peptides of formula (7a): 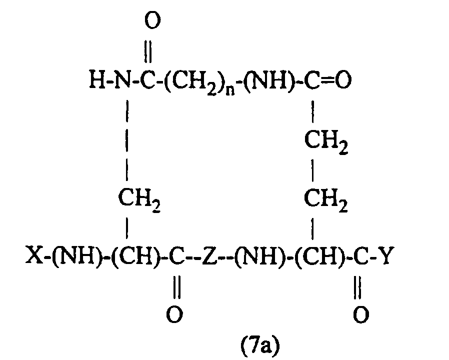
wherein X is hydrogen or is any amino acid or amino acid sequence; Y is hydroxyl or is any amino acid or amino acid sequence; Z is any amino acid sequence consisting of six amino acids: and n is any integer from 4 to 6 inclusive.

In still another embodiment, the invention provides locked helix peptides of formula (8a): 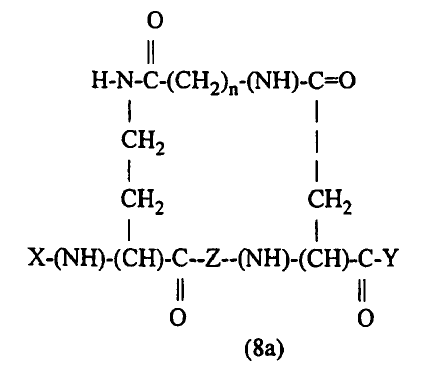
wherein X is hydrogen or is any amino acid or amino acid sequence; Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (9a): 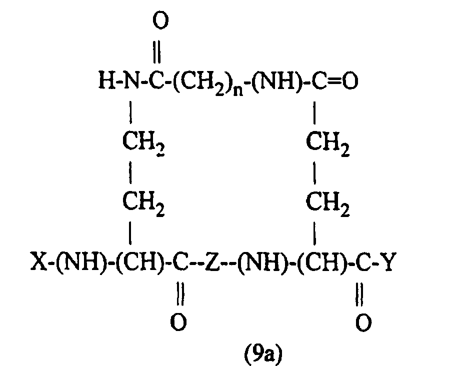
wherein X is hydrogen or is any amino acid or amino acid sequence; Y is hydroxyl or is any amino acid or amino acid sequence; Z is any amino acid sequence consisting of six amino acids; and n is any integer from 3 to 5 inclusive.

In still another embodiment, the invention provides locked helix peptides of formula (10a): 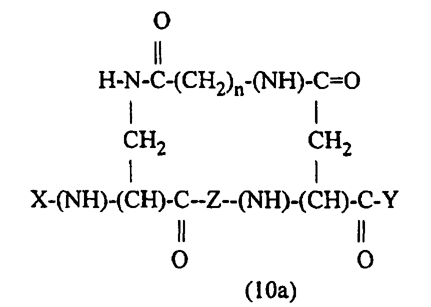
wherein X is hydrogen or is any amino acid or amino acid sequence; Y is hydroxyl or is any amino acid or amino acid sequence; Z is any amino acid sequence consisting of six amino acids; and n is any integer from 5 to 7 inclusive.

In still another embodiment, the invention provides locked helix peptides of formula (11a): 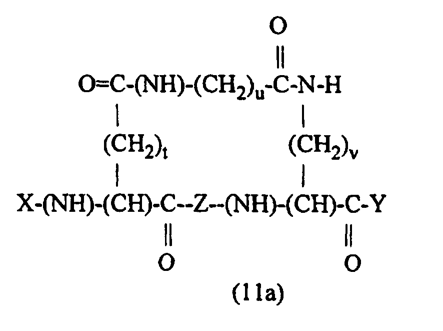
wherein X is hydrogen or is any amino acid or amino acid sequence; Y is hydroxyl or is any amino acid or amino acid sequence; Z is any amino acid sequence consisting of six amino acids; t is selected from the integers 0 to 6 inclusive, and v is selected from the integers 1 to 7 inclusive, provided that t+v is less than or equal to 7; and u is any integer in the range defined by (7-(t+v)) to (9-(t+v)) inclusive, provided that u is greater than 0.

In still another embodiment, the invention provides locked helix peptides of formula (12a): 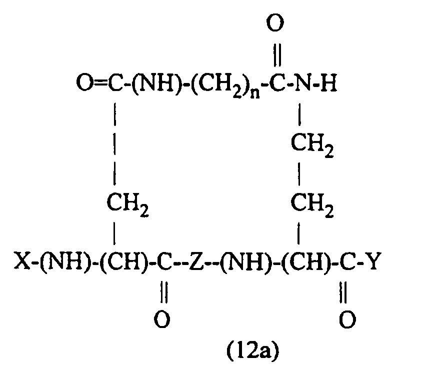
wherein X is hydrogen or is any amino acid or amino acid sequence; Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (13a): 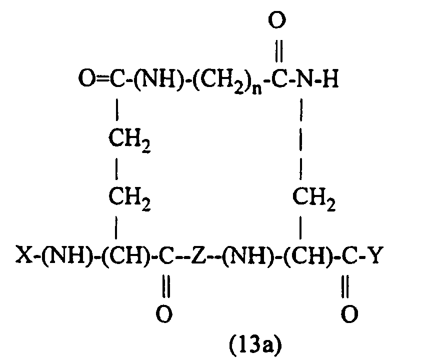
wherein X is hydrogen or is any amino acid or amino acid sequence; Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (14a): 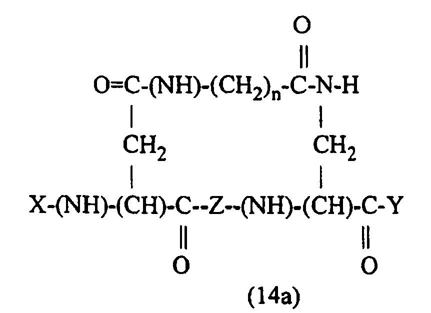
wherein X is hydrogen or is any amino acid or amino acid sequence; Y is hydroxyl or is any amino acid or amino acid sequence; Z is any amino acid sequence consisting of six amino acids; and n is any integer from 5 to 7 inclusive.

In still another embodiment, the invention provides locked helix peptides of formula (15a): 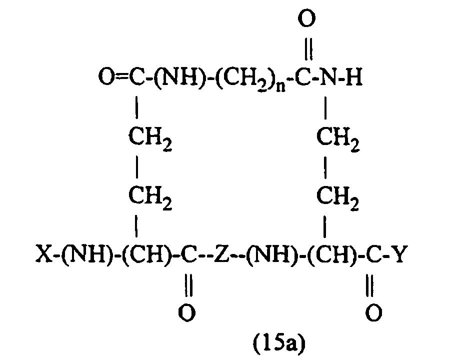
wherein X is hydrogen or is any amino acid or amino acid sequence: Y is hydroxyl or is any amino acid or amino acid sequence; Z is any amino acid sequence consisting of six amino acids; and n is any integer from 3 to 5 inclusive.

In still another embodiment, the invention provides locked helix peptides of formula (16a): 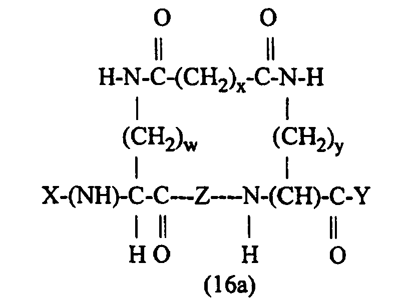
wherein X is hydrogen or is any amino acid or amino acid sequence; Y is hydroxyl or is any amino acid or amino acid sequence; Z is any amino acid sequence consisting of six amino acids; w and y are independently selected from the integers 1 to 7 inclusive, provided that w+y is less than or equal to 8; and x is any integer in the range defined by (7-(w+y)) to (9-(w+y)) inclusive, provided that x is greater than or equal to 0.

In still another embodiment, the invention provides locked helix peptides of formula (17a): 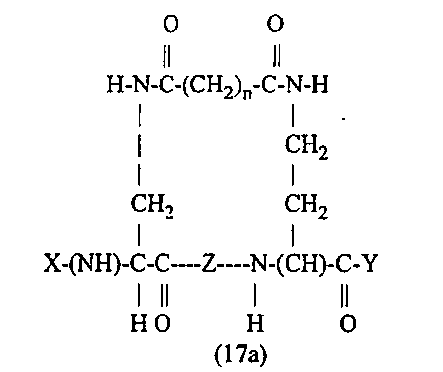
wherein X is hydrogen or is any amino acid or amino acid sequence; Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (18a): 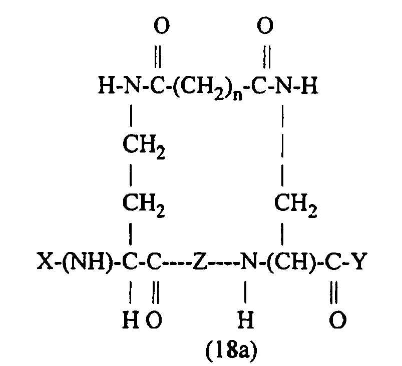
wherein X is hydrogen or is any amino acid or amino acid sequence; Y is hydroxyl or is any amino acid or amino acid sequence: Z is any amino acid sequence consisting of six amino acids; and n is any integer from 4 to 6 inclusive.

In still another embodiment, the invention provides locked helix peptides of formula (19a): 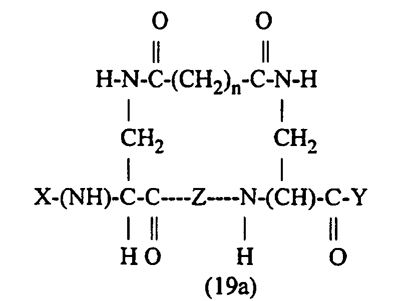
wherein X is hydrogen or is any amino acid or amino acid sequence; Y is hydroxyl or is any amino acid or amino acid sequence; Z is any amino acid sequence consisting of six amino acids; and n is any integer from 5 to 7 inclusive.

In still another embodiment, the invention provides locked helix peptides of formula (20a): 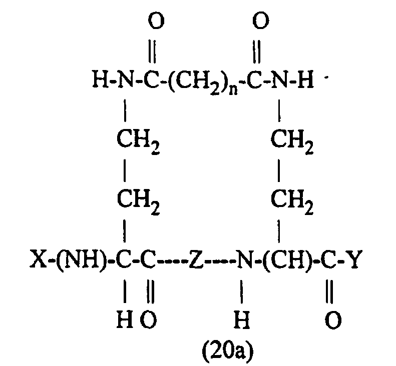
wherein X is hydrogen or is any amino acid or amino acid sequence; Y is hydroxyl or is any amino acid or amino acid sequence; Z is any amino acid sequence consisting of six amino acids; and n is any integer from 3 to 5 inclusive.

In a further embodiment, the invention provides locked helix peptides of formula (1a), formula (2a), formula (3a), formula (4a), formula (5a), formula (6a), formula (7a), formula (8a), formula (9a), formula (10a), formula (11a), formula (12a), formula (13a), formula (14a), formula (15a), formula (16a), formula (17a), formula (18a), formula (19a) and formula (20a) wherein X, Y, and Z collectively contain up to or about 12 amino acids (i.e. locked helix peptides of formulas (1a), (2a), (3a), (4a), (5a), (6a), (7a), (8a), (9a), (10a), (11a), (12a), (13a), (14a), (15a), (16a), (17a), (18a), (19a) and (20a) each of which contains a total of no more than about 12 amino acid residues).

Also provided herein are locked helix peptides of formula (1a), formula (2a), formula (3a), formula (4a), formula (5a), formula (6a), formula (7a), formula (8a), formula (9a), formula (10a), formula (11a), formula (12a), formula (13a), formula (14a), formula (15a), formula (16a), formula (17a), formula (18a), formula (19a) and formula (20a) wherein X and/or Y contain(s) up to or about 30 amino acid residues.

Further provided herein are lockedhelix peptidesof formula (1a), formula (2a), formula (3a), formula (4a), formula (5a), formula (6a), formula (7a), formula (8a), formula (9a), formula (10a), formula (11a), formula (12a), formula (13a), formula (14a), formula (15a), formula (16a), formula (17a), formula (18a), formula (19a) and formula (20a) wherein X and/or Y contain(s) up to or about 25 amino acid residues.

Additionallyprovided herein are locked helix peptides of formula (1a), formula (2a), formula (3a), formula (4a), formula (5a), formula (6a), formula (7a), formula (8a), formula (9a), formula (10a), formula (11a), formula (12a), formula (13a), formula (14a), formula (15a), formula (16a), formula (17a), formula (18a), formula (19a) and formula (20a) wherein X and/or Y contain(s) up to or about 20 amino acid residues.

Also encompassed herein are locked helix peptides of formula (1a), formula (2a), formula (3a), formula (4a), formula (5a), formula (6a), formula (7a), formula (8a), formula (9a), formula (10a), formula (11a), formula (12a), formula (13a), formula (14a), formula (15a), formula (16a), formula (17a), formula (18a), formula (19a) and formula (20a) wherein X and/or Y contain(s) up to or about 15 amino acid residues.

Further encompassed herein are locked helix peptides of formula (1a), formula (2a), formula (3a), formula (4a), formula (5a), formula (6a), formula (7a), formula (8a), formula (9a), formula (10a), formula (11a), formula (12a), formula (13a), formula (14a), formula (15a), formula (16a), formula (17a), formula (18a), formula (19a) and formula (20a) wherein X and/or Y contain(s) up to or about 10 amino acid residues.

Additionally encompassed herein are locked helix peptides of formula (1a), formula (2a), formula (3a), formula (4a), formula (5a), formula (6a), formula (7a), formula (8a), formula (9a), formula (10a), formula (11a), formula (12a), formula (13a), formula (14a), formula (15a), formula (16a), formula (17a), formula (18a), formula (19a) and formula (20a) wherein X and/or Y contain(s) up to or about 5 amino acid residues.

Also within the scope of the invention are locked helix peptides of formula (1a), formula (2a), formula (3a), formula (4a), formula (5a), formula (6a), formula (7a), formula (8a), formula (9a), formula (10a), formula (11a), formula (12a), formula (13a), formula (14a), formula (15a), formula (16a), formula (17a), formula (18a), formula (19a) and formula (20a) wherein X and/or Y contain(s) up to or about 3 amino acid residues.

The invention also provides locked helix peptides of formula (1b): 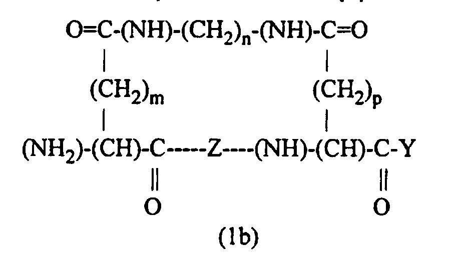
wherein Y is hydroxyl or is any amino acid or amino acid sequence; Z is any amino acid sequence consisting of six amino acids; m and p are independently selected from the integers 0 to 6 inclusive, provided that m+p is less than or equal to 6; and n is any integer in the range defined by (7-(m+p)) to (9-(m+p)) inclusive, provided that n is greater than 1.

In another embodiment, the invention provides locked helix peptides of formula (2b): 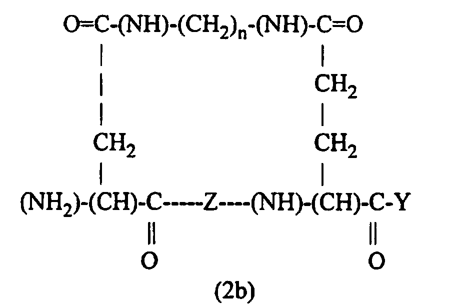
wherein Y is hydroxyl or is any amino acid or amino acid sequence; Z is any amino acid sequence consisting of six amino acids; and n is any integer from 4 to 6 inclusive.

In yet another embodiment, the invention provides locked helix peptides of formula (3b): 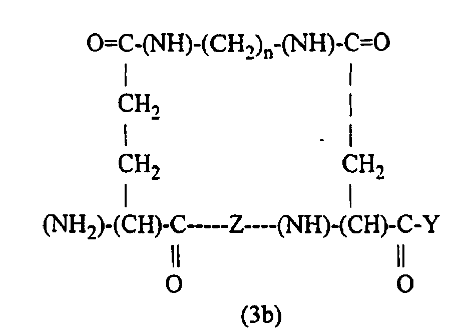
wherein Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (4b): 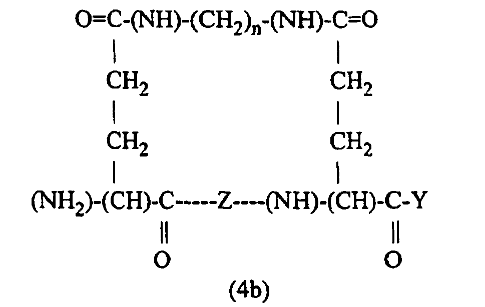
wherein Y is hydroxyl or is any amino acid or amino acid sequence; Z is any amino acid sequence consisting of six amino acids; and n is any integer from 3 to 5 inclusive.

In still another embodiment, the invention provides locked helix peptides of formula (5b): 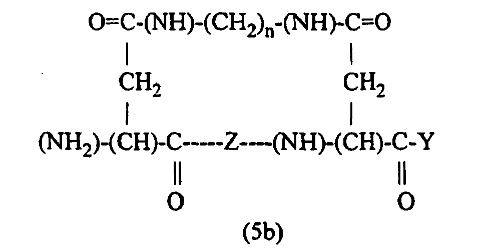
wherein Y is hydroxyl or is any amino acid or amino acid sequence; Z is any amino acid sequence consisting of six amino acids; and n is any integer from 5 to 7 inclusive.

In still another embodiment, the invention provides locked helix peptides of formula (6b): 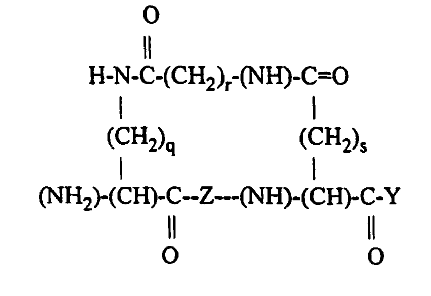 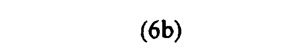
wherein Y is hydroxyl or is any amino acid or amino acid sequence; Z is any amino acid sequence consisting of six amino acids; q is selected from the integers 1 to 7 inclusive, and s is selected from the integers 0 to 6 inclusive, provided that q+s is less than or equal to 7; and r is any integer in the range defined by (7-(q+s)) to (9-(q+s)) inclusive, provided that r is greater than 0.

In still another embodiment, the invention provides locked helix peptides of formula (7b): 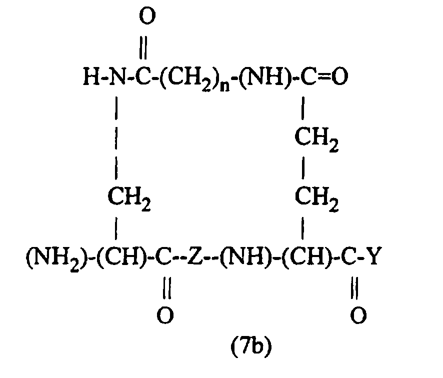
wherein Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (8b): 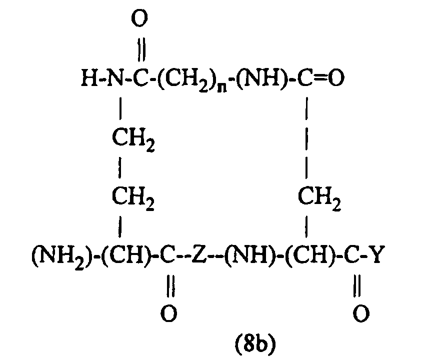
wherein Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (9b): 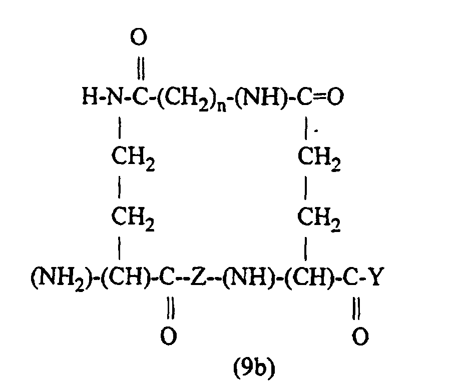
wherein Y is hydroxyl or is any amino acid or amino acid sequence; Z is any amino acid sequence consisting of six amino acids; and n is any integer from 3 to 5 inclusive.

In still another embodiment, the invention provides locked helix peptides of formula (10b): 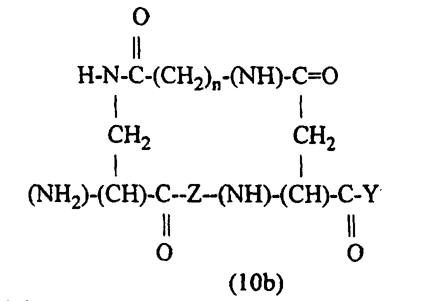
wherein Y is hydroxyl or is any amino acid or amino acid sequence; Z is any amino acid sequence consisting of six amino acids; and n is any integer from 5 to 7 inclusive.

In still another embodiment, the invention provides locked helix peptides of formula (11b): 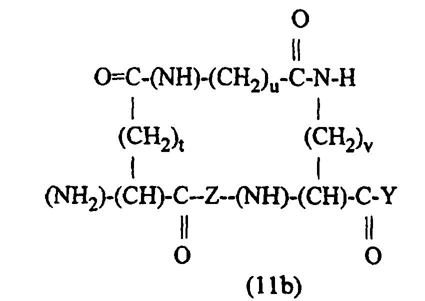
wherein Y is hydroxyl or is any amino acid or amino acid sequence; Z is any amino acid sequence consisting of six amino acids; t is selected from the integers 0 to 6 inclusive, and v is selected from the integers 1 to 7 inclusive, provided that t+v is less than or equal to 7; and u is any integer in the range defined by (7-(t+v)) to (9-(t+v)) inclusive, provided that u is greater than 0.

In still another embodiment, the invention provides locked helix peptides of formula (12b): 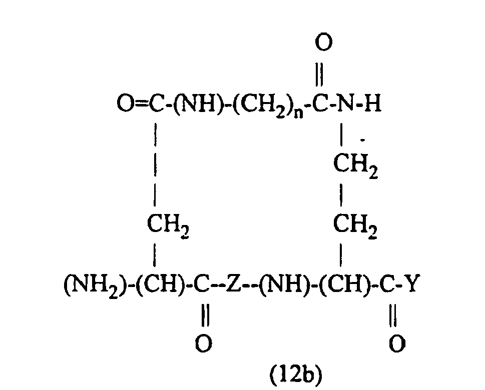
wherein Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (13b): 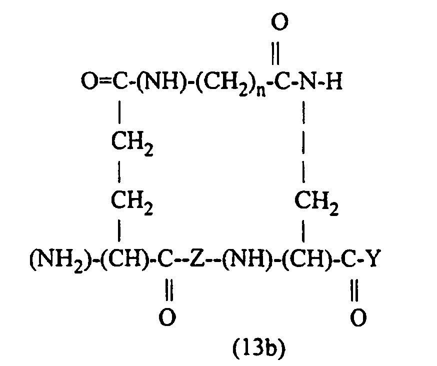
wherein Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (14b): 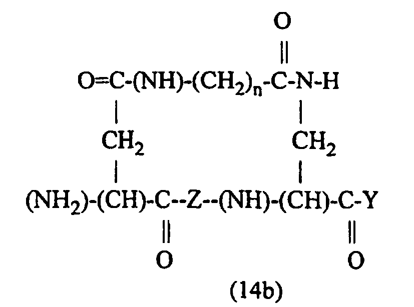
wherein Y is hydroxyl or is any amino acid or amino acid sequence; Z is any amino acid sequence consisting of six amino acids; and n is any integer from 5 to 7 inclusive.

In still another embodiment, the invention provides locked helix peptides of formula (15b): 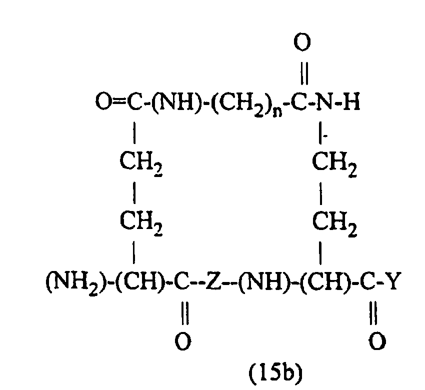
wherein Y is hydroxyl or is any amino acid or amino acid sequence; Z is any amino acid sequence consisting of six amino acids; and n is any integer from 3 to 5 inclusive.

In still another embodiment, the invention provides locked helix peptides of formula (16b): 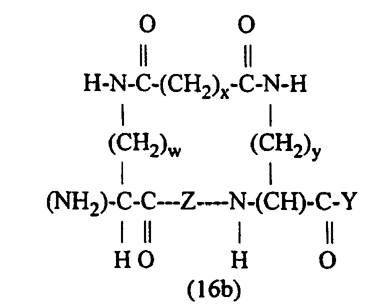
wherein Y is hydroxyl or is any amino acid or amino acid sequence; Z is any amino acid sequence consisting of six amino acids; w and y are independently selected from the integers 1 to 7 inclusive, provided that w+y is less than or equal to 8; and x is any integer in the range defined by (7-(w+y)) to (9-(w+y)) inclusive, provided that x is greater than or equal to 0.

In still another embodiment, the invention provides locked helix peptides of formula (17b): 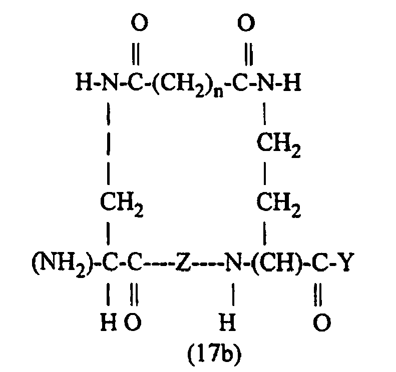
wherein Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (18b): 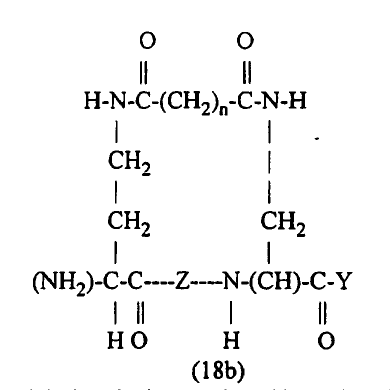
wherein Y is hydroxyl or is any amino acid or amino acid sequence; Z is any amino acid sequence consisting of six amino acids; and n is any integer from 4 to 6 inclusive.

In still another embodiment, the invention provides locked helix peptides of formula (19b): 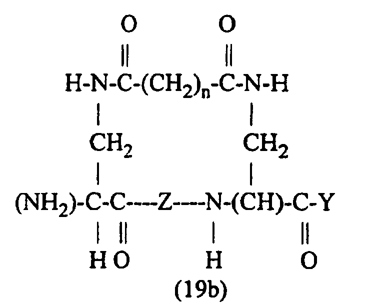
wherein Y is hydroxyl or is any amino acid or amino acid sequence; Z is any amino acid sequence consisting of six amino acids; and n is any integer from 5 to 7 inclusive.

In still another embodiment, the invention provides locked helix peptides of formula (20b): 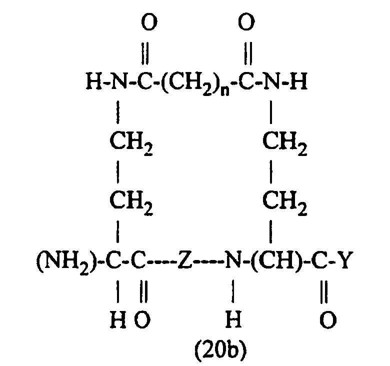
wherein Y is hydroxyl or is any amino acid or amino acid sequence; Z is any amino acid sequence consisting of six amino acids; and n is any integer from 3 to 5 inclusive.

Also provided herein are locked helix peptides of formula (1b), formula (2b), formula (3b), formula (4b), formula (5b), formula (6b), formula (7b), formula (8b), formula (9b), formula (10b), formula (11b), formula (12b), formula (13b), formula (14b), formula (15b), formula (16b), formula (17b), formula (18b), formula (19b) and formula (20b) wherein Y contains up to or about 30 amino acid residues.

Further provided herein are locked helix peptides of formula (1b), formula (2b), formula (3b), formula (4b), formula (5b), formula (6b), formula (7b), formula (8b), formula (9b), formula (10b), formula (11b), formula (12b), formula (13b), formula (14b), formula (15b), formula (16b), formula (17b), formula (18b), formula (19b) and formula (20b) wherein Y contains up to or about 25 amino acid residues.

Additionally provided herein are locked helix peptides of formula (1b), formula (2b), formula (3b), formula (4b), formula (5b), formula (6b), formula (7b), formula (8b), formula (9b), formula (10b), formula (11b), formula (12b), formula (13b), formula (14b), formula (15b), formula (16b), formula (17b), formula (18b), formula (19b) and formula (20b) wherein Y contains up to or about 20 amino acid residues.

Also encompassed herein are locked helix peptides of formula (1b), formula (2b), formula (3b), formula (4b), formula (5b), formula (6b), formula (7b), formula (8b), formula (9b), formula (10b), formula (11b), formula (12b), formula (13b), formula (14b), formula (15b), formula (16b), formula (17b), formula (18b), formula (19b) and formula (20b) wherein Y contains up to or about 15 amino acid residues.

Further encompassed herein are locked helix peptides of formula (1b), formula (2b), formula (3b), formula (4b), formula (5b), formula (6b), formula (7b), formula (8b), formula (9b), formula (10b), formula (11b), formula (12b), formula (13b), formula (14b), formula (15b), formula (16b), formula (17b), formula (18b), formula (19b) and formula (20b) wherein Y contains up to or about 10 amino acid residues.

Additionally encompassed herein are locked helix peptides of formula (1b), formula (2b), formula (3b), formula (4b), formula (5b), formula (6b), formula (7b), formula (8b), formula (9b), formula (10b), formula (11b), formula (12b), formula (13b), formula (14b), formula (15b), formula (16b), formula (17b), formula (18b), formula (19b) and formula (20b) wherein Y contains up to or about 5 amino acid residues.

Also within the scope of the invention are locked helix peptides of formula (1b), formula (2b), formula (3b), formula (4b), formula (5b), formula (6b), formula (7b), formula (8b), formula (9b), formula (10b), formula (11b), formula (12b), formula (13b), formula (14b), formula (15b), formula (16b), formula (17b), formula (18b), formula (19b) and formula (20b) wherein Y contains up to or about 3 amino acid residues.

The invention also provides locked helix peptides of formula (1c): 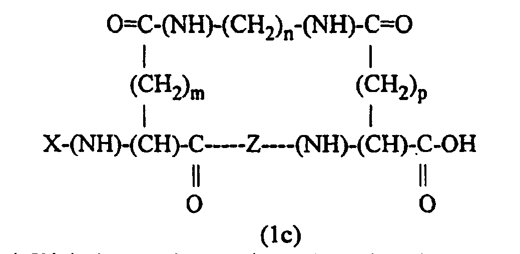
wherein X is hydrogen or is any amino acid or amino acid sequence; Z is any amino acid sequence consisting of six amino acids; m and p are independently selected from the integers 0 to 6 inclusive, provided that m+p is less than or equal to 6; and n is any integer in the range defined by (7-(m+p)) to (9-(m+p)) inclusive, provided that n is greater than 1.

In another embodiment, the invention provides locked helix peptides of formula (2c): 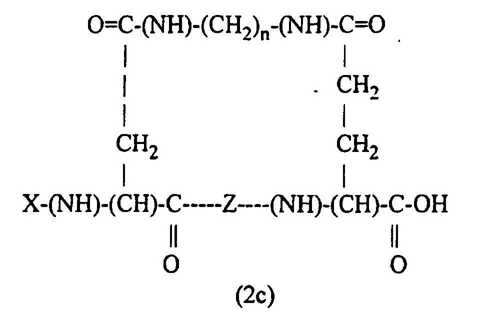
wherein X is hydrogen or is any amino acid or amino acid sequence; Z is any amino acid sequence consisting of six amino acids; and n is any integer from 4 to 6 inclusive.

In yet another embodiment, the invention provides locked helix peptides of formula (3c): 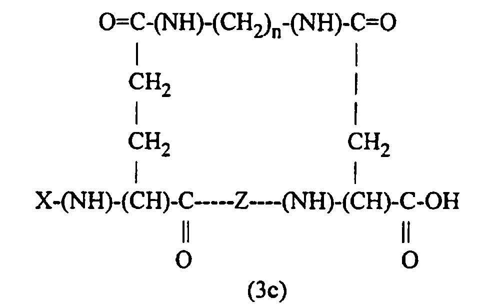
wherein X is hydrogen or is any amino acid or amino acid sequence; Z is any amino acid sequence consisting of six amino acids; and n is any integer from 4 to 6 inclusive.

In still another embodiment, the invention provides locked helix peptides of formula (4c): 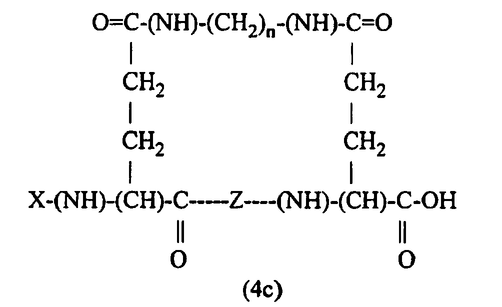
wherein X is hydrogen or is any amino acid or amino acid sequence; Z is any amino acid sequence consisting of six amino acids; and n is any integer from 3 to 5 inclusive.

In still another embodiment, the invention provides locked helix peptides of formula (5c): 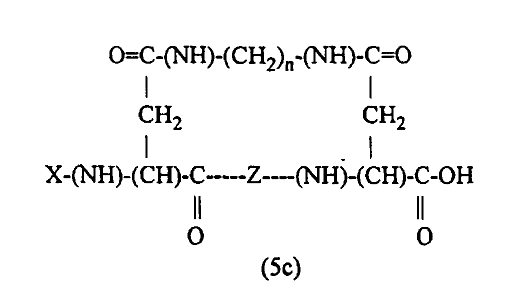
wherein X is hydrogen or is any amino acid or amino acid sequence; Z is any amino acid sequence consisting of six amino acids; and n is any integer from 5 to 7 inclusive.

In still another embodiment, the invention provides locked helix peptides of formula (6c): 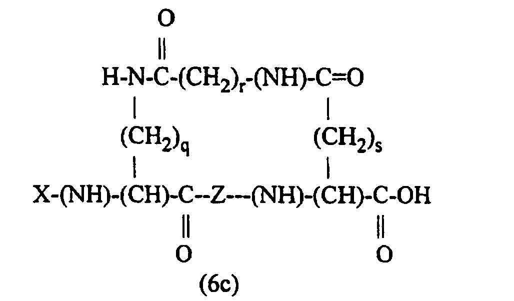
wherein X is hydrogen or is any amino acid or amino acid sequence; Z is any amino acid sequence consisting of six amino acids; q is selected from the integers 1 to 7 inclusive, and s is selected from the integers 0 to 6 inclusive, provided that q+s is less than or equal to 7; and r is any integer in the range defined by (7-(q+s)) to (9-(q+s)) inclusive, provided that r is greater than 0.

In still another embodiment, the invention provides locked helix peptides of formula (7c): 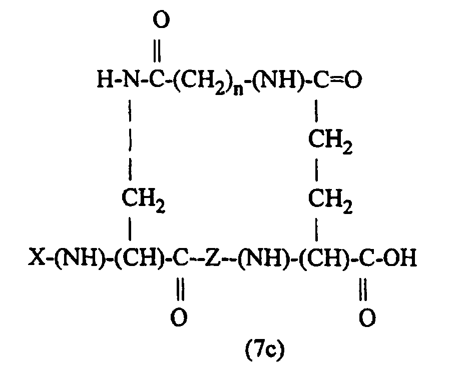
wherein X is hydrogen or is any amino acid or amino acid sequence; Z is any amino acid sequence consisting of six amino acids; and n is any integer from 4 to 6 inclusive.

In still another embodiment, the invention provides locked helix peptides of formula (8c): 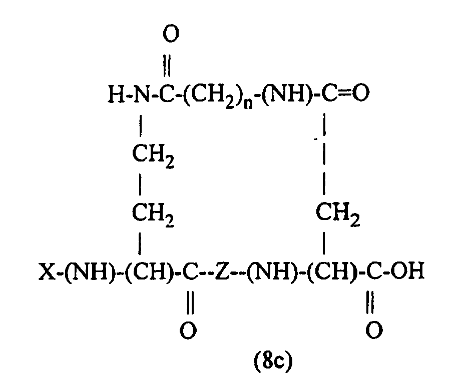
wherein X is hydrogen or is any amino acid or amino acid sequence; Z is any amino acid sequence consisting of six amino acids; and n is any integer from 4 to 6 inclusive.

In still another embodiment, the invention provides locked helix peptides of formula (9c): 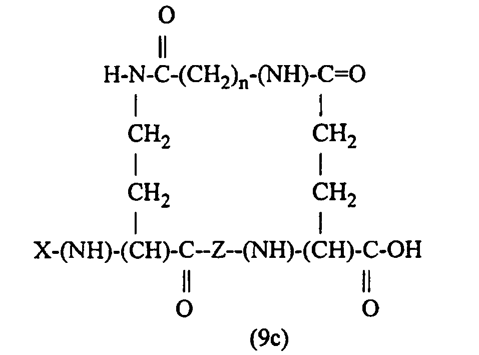
wherein X is hydrogen or is any amino acid or amino acid sequence; Z is any amino acid sequence consisting of six amino acids; and n is any integer from 3 to 5 inclusive.

In still another embodiment, the invention provides locked helix peptides of formula (10c): 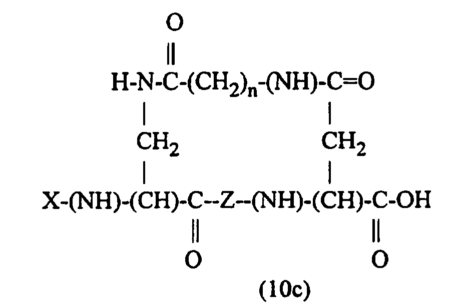
wherein X is hydrogen or is any amino acid or amino acid sequence; Z is any amino acid sequence consisting of six amino acids; and n is any integer from 5 to 7 inclusive.

In still another embodiment, the invention provides locked helix peptides of formula (11 c): 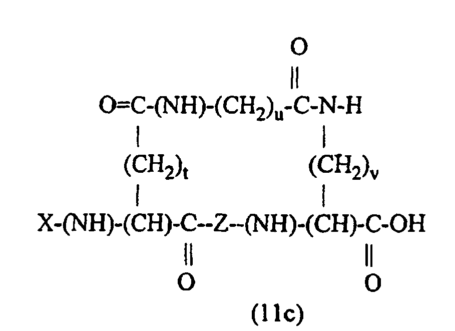
wherein X is hydrogen or is any amino acid or amino acid sequence; Z is any amino acid sequence consisting of six amino acids; t is selected from the integers 0 to 6 inclusive, and v is selected from the integers 1 to 7 inclusive, provided that t+v is less than or equal to 7; and u is any integer in the range defined by (7-(t+v)) to (9-(t+v)) inclusive, provided that u is greater than 0.

In still another embodiment, the invention provides locked helix peptides of formula (12c): 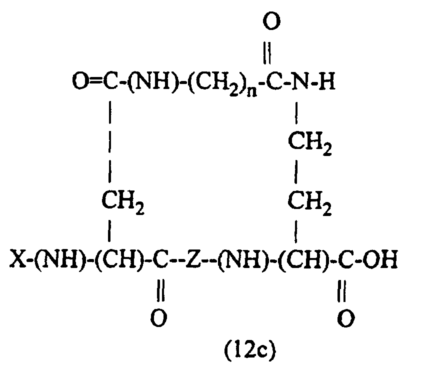
wherein X is hydrogen or is any amino acid or amino acid sequence; Z is any amino acid sequence consisting of six amino acids; and n is any integer from 4 to 6 inclusive.

In still another embodiment, the invention provides locked helix peptides of formula (13c): 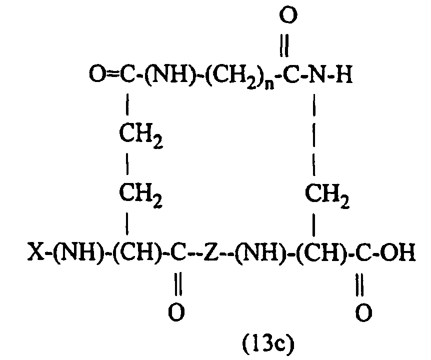
wherein X is hydrogen or is any amino acid or amino acid sequence; Z is any amino acid sequence consisting of six amino acids; and n is any integer from 4 to 6 inclusive.

In still another embodiment, the invention provides locked helix peptides of formula (14c): 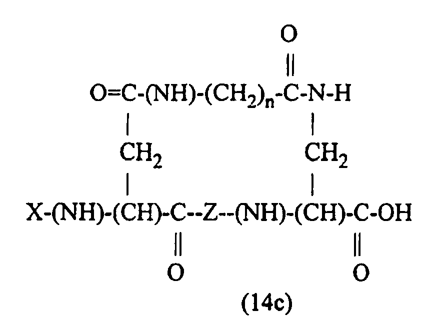
wherein X is hydrogen or is any amino acid or amino acid sequence; Z is any amino acid sequence consisting of six amino acids; and n is any integer from 5 to 7 inclusive.

In still another embodiment, the invention provides locked helix peptides of formula (15c): 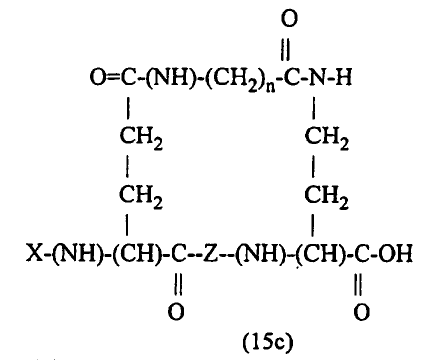
wherein X is hydrogen or is any amino acid or amino acid sequence; Z is any amino acid sequence consisting of six amino acids; and n is any integer from 3 to 5 inclusive.

In still another embodiment, the invention provides locked helix peptides of formula (16c): 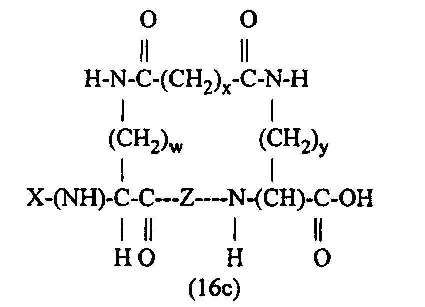
wherein X is hydrogen or is any amino acid or amino acid sequence; Z is any amino acid sequence consisting of six amino acids; w and y are independently selected from the integers I to 7 inclusive, provided that w+y is less than or equal to 8; and x is any integer in the range defined by (7-(w+y)) to (9-(w+y)) inclusive, provided that x is greater than or equal to 0.

In still another embodiment, the invention provides locked helix peptides of formula (17c): 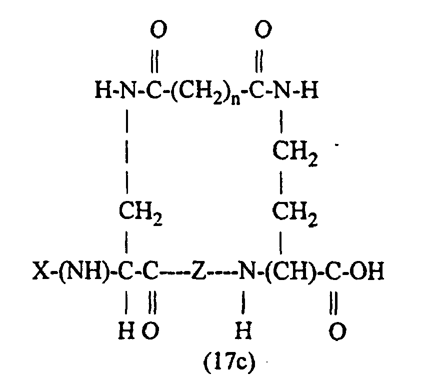
wherein X is hydrogen or is any amino acid or amino acid sequence; Z is any amino acid sequence consisting of six amino acids; and n is any integer from 4 to 6 inclusive.

In still another embodiment, the invention provides locked helix peptides of formula (18c): 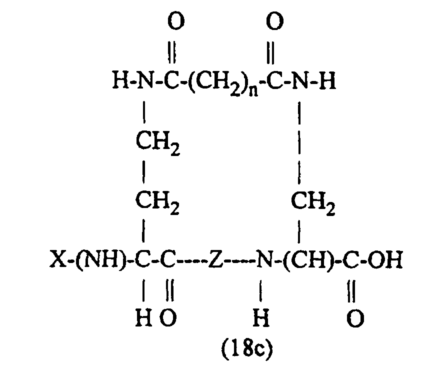
wherein X is hydrogen or is any amino acid or amino acid sequence; Z is any amino acid sequence consisting of six amino acids; and n is any integer from 4 to 6 inclusive.

In still another embodiment, the invention provides locked helix peptides of formula (19c): 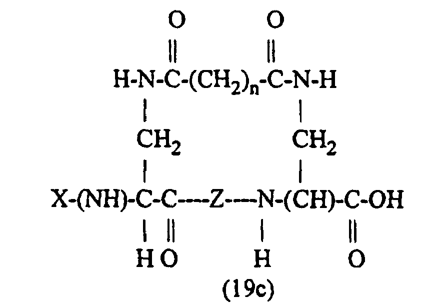
wherein X is hydrogen or is any amino acid or amino acid sequence; Z is any amino acid sequence consisting of six amino acids; and n is any integer from 5 to 7 inclusive.

In still another embodiment, the invention provides locked helix peptides of formula (20c): 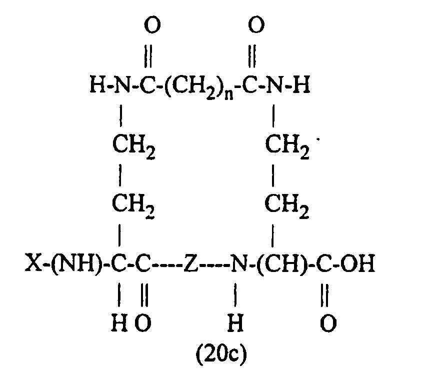 wherein X is hydrogen or is any amino acid or amino acid sequence; Z is any amino acid sequence consisting of six amino acids; and n is any integer from 3 to 5 inclusive.

Also provided herein are locked helix peptides of formula (1c), formula (2c), formula (3c), formula (4c), formula (5c), formula (6c), formula (7c), formula (8c), formula (9c), formula (10c), formula (11c), formula (12c), formula (13c), formula (14c), formula (15c), formula (16c), formula (17c), formula (18c), formula (19c) and formula (20c) wherein X contains up to or about 30 amino acid residues.

Further provided herein are locked helix peptides of formula (1c), formula (2c), formula (3c), formula (4c), formula (5c), formula (6c), formula (7c), formula (8c), formula (9c), formula (10c), formula (11c), formula (12c), formula (13c), formula (14c), formula (15c), formula (16c), formula (17c), formula (18c), formula (19c) and formula (20c) wherein X contains up to or about 25 amino acid residues.

Additionally provided herein are locked helix peptides of formula (1c), formula (2c), formula (3c), formula (4c), formula (5c), formula (6c), formula (7c), formula (8c), formula (9c), formula (10c), formula (11c), formula (12c), formula (13c), formula (14c), formula (15c), formula (16c), formula (17c), formula (18c), formula (19c) and formula (20c) wherein X contains up to or about 20 amino acid residues.

Also encompassed herein are locked helix peptides of formula (1c), formula (2c), formula (3c), formula (4c), formula (5c), formula (6c), formula (7c), formula (8c), formula (9c), formula (10c), formula (11c), formula (12c), formula (13c), formula (14c), formula (15c), formula (16c), formula (17c), formula (18c), formula (19c) and formula (20c) wherein X contains up to or about 15 amino acid residues.

Further encompassed herein are locked helix peptides of formula (1c), formula (2c), formula (3c), formula (4c), formula (5c), formula (6c), formula (7c), formula (8c), formula (9c), formula (10c), formula (11c), formula (12c), formula (13c), formula (14c), formula (15c), formula (16c), formula (17c), formula (18c), formula (19c) and formula (20c) wherein X contains up to or about 10 amino acid residues.

Additionally encompassed herein are locked helix peptides of formula (1c), formula (2c), formula (3c), formula (4c), formula (5c), formula (6c), formula (7c), formula (8c), formula (9c), formula (10c), formula (11c), formula (12c), formula (13c), formula (14c), formula (15c), formula (16c), formula (17c), formula (18c), formula (19c) and formula (20c) wherein X contains up to or about 5 amino acid residues.

Also within the scope of the invention are locked helix peptides of formula (1c), formula (2c), formula (3c), formula (4c), formula (5c), formula (6c), formula (7c), formula (8c), formula (9c), formula (10c), formula (11c), formula (12c), formula (13c), formula (14c), formula (15c), formula (16c), formula (17c), formula (18c), formula (19c) and formula (20c) wherein X contains up to or about 3 amino acid residues.

The invention also provides locked helix peptides of formula (1d): 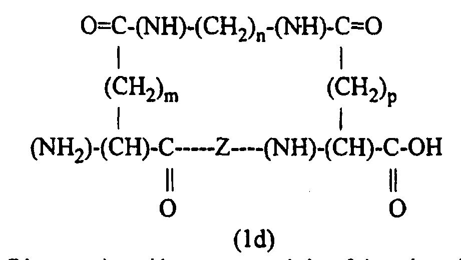
wherein Z is any amino acid sequence consisting of six amino acids; m and p are independently selected from the integers 0 to 6 inclusive, provided that m+p is less than or equal to 6; and n is any integer in the range defined by (7-(m+p)) to (9-(m+p)) inclusive, provided that n is greater than 1.

In another embodiment, the invention provides locked helix peptides of formula (2d): 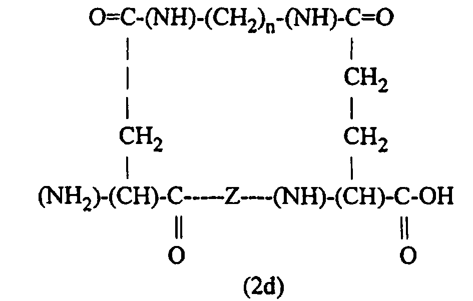
wherein Z is any amino acid sequence consisting of six amino acids; and n is any integer from 4 to 6 inclusive.

In yet another embodiment, the invention provides locked helix peptides of formula (3d): 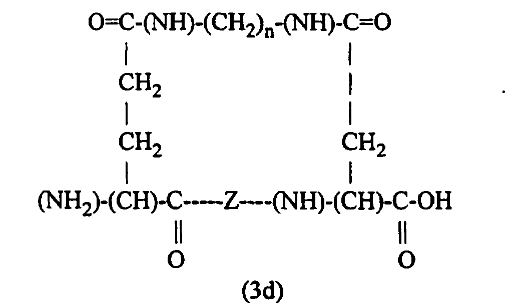
wherein Z is any amino acid sequence consisting of six amino acids; and n is any integer from 4 to 6 inclusive.

In still another embodiment, the invention provides locked helix peptides of formula (4d): 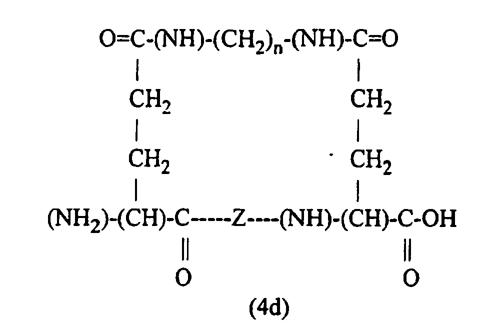
wherein Z is any amino acid sequence consisting of six amino acids; and n is any integer from 3 to 5 inclusive.

In still another embodiment, the invention provides locked helix peptides of formula (5d): 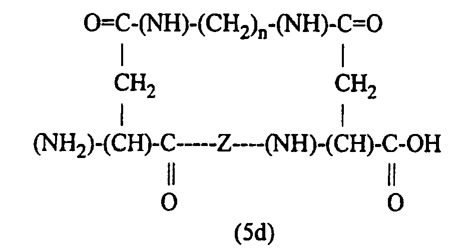
wherein Z is any amino acid sequence consistingof six amino acids; and n is any integer from 5 to 7 inclusive.

In still another embodiment, the invention provides locked helix peptides of formula (6d): 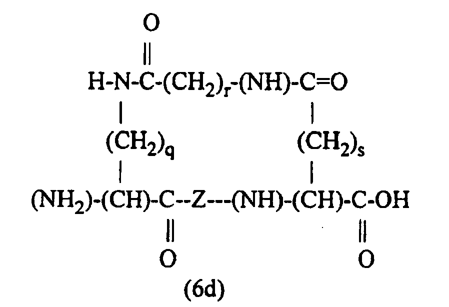
wherein Z is any amino acid sequence consisting of six amino acids; q is selected from the integers 1 to 7 inclusive, and s is selected from the integers 0 to 6 inclusive, provided that q+s is less than or equal to 7; and
r is any integer in the range defined by (7-(q+s)) to (9-(q+s)) inclusive, provided that r is greater than 0.

In still another embodiment, the invention provides locked helix peptides of formula (7d): 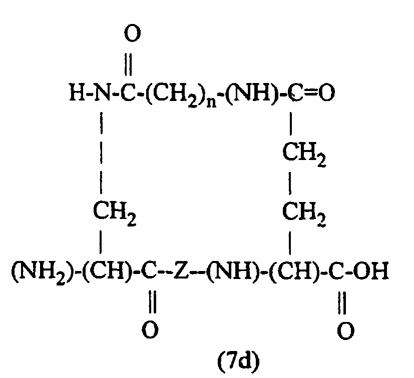
wherein Z is any amino acid sequence consisting of six amino acids; and n is any integer from 4 to 6 inclusive.

In still another embodiment, the invention provides locked helix peptides of formula (8d): 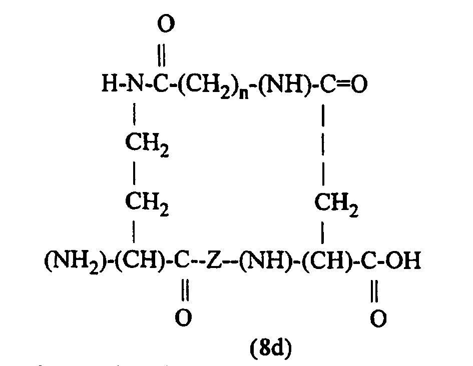
wherein Z is any amino acid sequence consistingof six amino acids; and n is any integer from 4 to 6 inclusive.

In still another embodiment, the invention provides locked helix peptides of formula (9d): 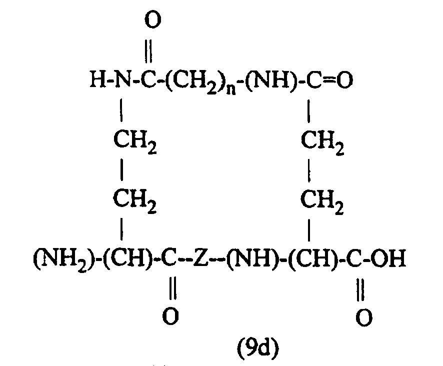
wherein Z is any amino acid sequence consistingof six amino acids; and n is any integer from 3 to 5 inclusive.

In still another embodiment, the invention provides locked helix peptides of formula (10d): 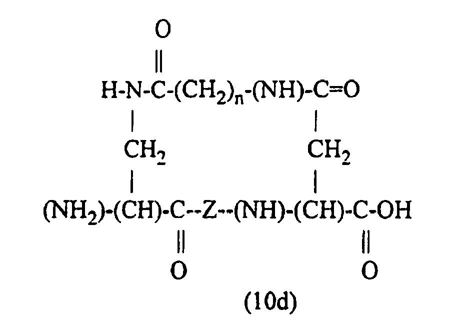
wherein Z is any amino acid sequence consisting of six amino acids; and n is any integer from 5 to 7 inclusive.

In still another embodiment, the invention provides locked helix peptides of formula (11d): 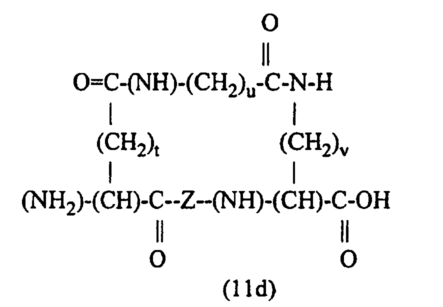
wherein Z is any amino acid sequence consisting of six amino acids; t is selected from the integers 0 to 6 inclusive, and v is selected from the integers 1 to 7 inclusive, provided that t+v is less than or equal to 7; and
u is any integer in the range defined by (7-(t+v)) to (9-(t+v)) inclusive, provided that u is greater than 0.

In still another embodiment, the invention provides locked helix peptides of formula (12d): 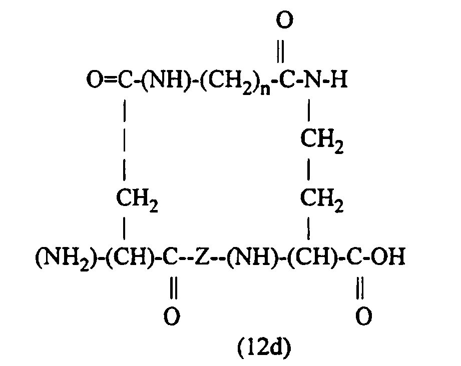
wherein Z is any amino acid sequence consisting of six amino acids; and n is any integer from 4 to 6 inclusive.

In still another embodiment, the invention provides locked helix peptides of formula (13d): 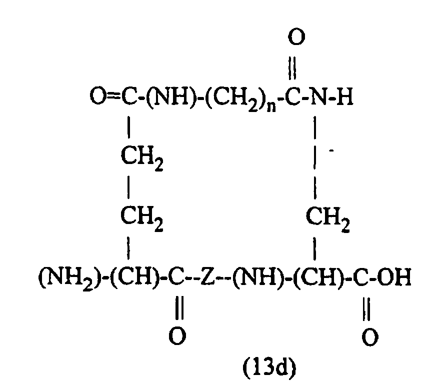
wherein Z is any amino acid sequence consistingof six amino acids; and n is any integer from 4 to 6 inclusive.

In still another embodiment, the invention provides locked helix peptides of formula (14d): 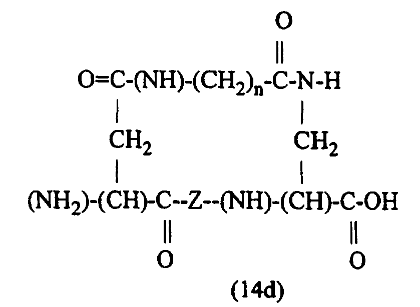
wherein Z is any amino acid sequence consisting of six amino acids; and n is any integer from 5 to 7 inclusive.

In still another embodiment, the invention provides locked helix peptides of formula (15d): 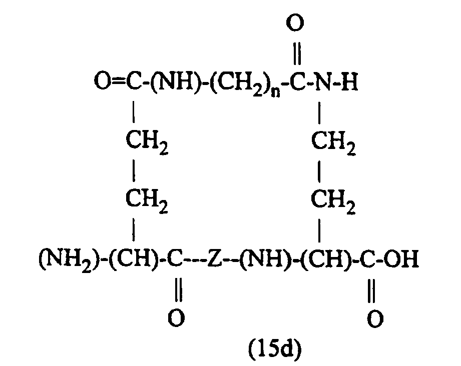
wherein Z is any amino acid sequence consisting of six amino acids; and n is any integer from 3 to 5 inclusive.

In still another embodiment, the invention provides locked helix peptides of formula (16d): 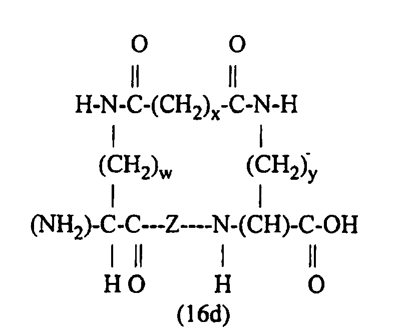
wherein Z is any amino acid sequence consisting of six amino acids; w and y are independently selected from the integers 1 to 7 inclusive, provided that w+y is less than or equal to 8; and x is any integer in the range defined by (7-(w+y)) to (9-(w+y)) inclusive, provided that x is greater than or equal to 0.

In still another embodiment, the invention provides locked helix peptides of formula (17d): 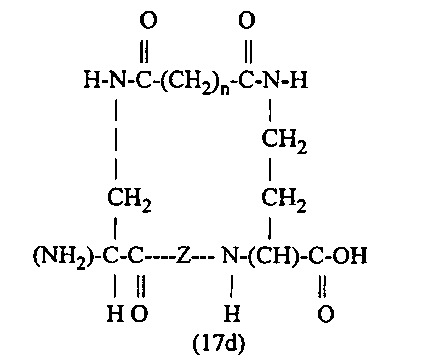
wherein Z is any amino acid sequence consistingof six amino acids; and n is any integer from 4 to 6 inclusive.

In still another embodiment, the invention provides locked helix peptides of formula (18d): 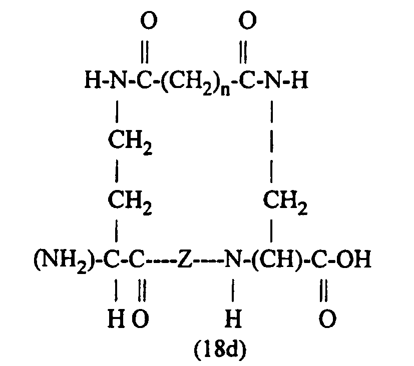
wherein Z is any amino acid sequence consisting of six amino acids; and n is any integer from 4 to 6 inclusive.

In still another embodiment, the invention provides locked helix peptides of formula (19d): 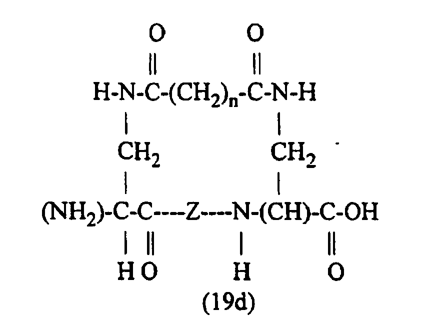
wherein Z is any amino acid sequence consisting of six amino acids; and n is any integer from 5 to 7 inclusive.

In still another embodiment, the invention provides locked helix peptides of formula (20d): 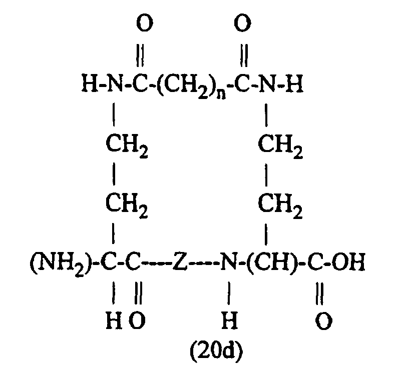
wherein Z is any amino acid sequence consisting of six amino acids; and n is any integer from 3 to 5 inclusive.

The invention also provides locked helix peptides of formula (1e): 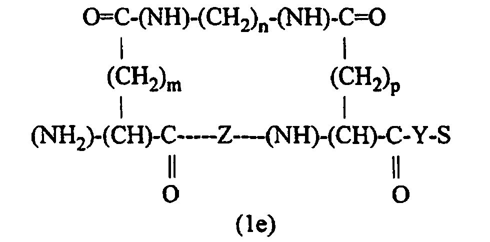
wherein S is absent or is a macromolecule; Y is absent, or is hydroxyl if S is absent, or is any amino acid or amino acid sequence;Z is any amino acid sequence consisting of six amino acids; m and p are independently selected from the integers 0 to 6 inclusive, provided that m+p is less than or equal to 6; and n is any integer in the range defined by (7-(m+p)) to (9-(m+p)) inclusive, provided that n is greater than 1.

In another embodiment, the invention provides locked helix peptides of formula (2e): 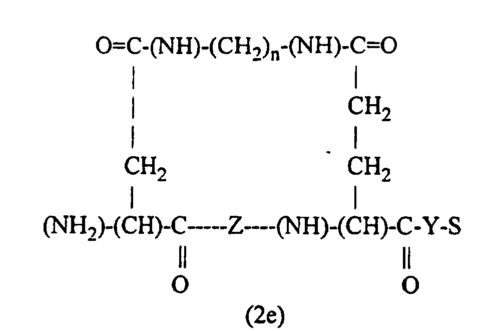
wherein S is absent or is a macromolecule; Y is absent, or is hydroxyl if S is absent, or is any amino acid or amino acid sequence; Z is any amino acid sequence consisting of six amino acids; and n is any integer from 4 to 6 inclusive.

In yet another embodiment, the invention provides locked helix peptides of formula (3e): 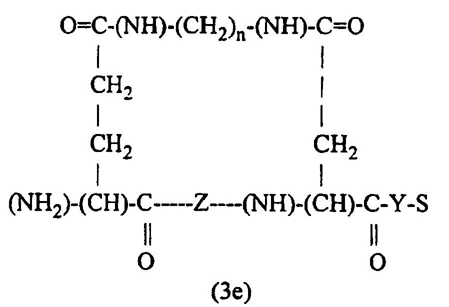
wherein S is absent or is a macromolecule; Y is absent, or is hydroxyl if S is absent, or is any amino acid or amino acid sequence; Z is any amino acid sequence consisting of six amino acids; and n is any integer from 4 to 6 inclusive.

In still another embodiment, the invention provides locked helix peptides of formula (4e): 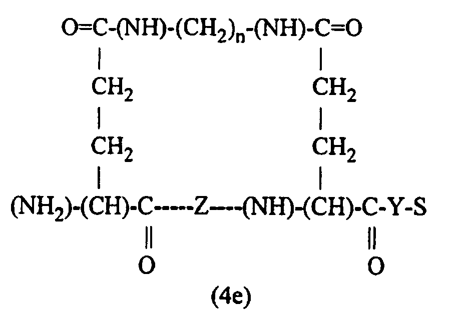
wherein S is absent or is a macromolecule; Y is absent, or is hydroxyl if S is absent, or is any amino acid or amino acid sequence; Z is any amino acid sequence consisting of six amino acids; and n is any integer from 3 to 5 inclusive.

In still another embodiment, the invention provides locked helix peptides of formula (5e): 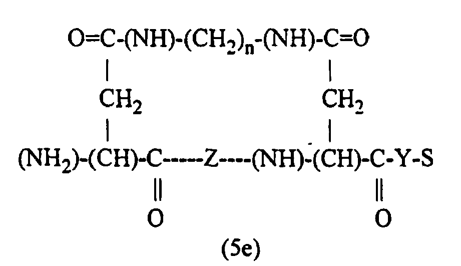
wherein S is absent or is a macromolecule; Y is absent, or is hydroxyl if S is absent, or is any amino acid or amino acid sequence; Z is any amino acid sequence consisting of six amino acids; and n is any integer from 5 to 7 inclusive.

In still another embodiment, the invention provides locked helix peptides of formula (6e): 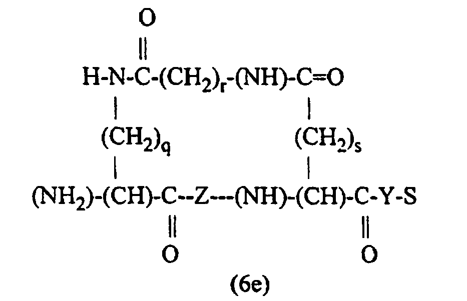
wherein S is absent or is a macromolecule; Y is absent, or is hydroxyl if S is absent, or is any amino acid or amino acid sequence; Z is any amino acid sequence consisting of six amino acids; q is selected from the integers 1 to 7 inclusive, and s is selected from the integers 0 to 6 inclusive, provided that q+s is less than or equal to 7; and r is any integer in the range defined by (7-(q+s)) to (9-(q+s)) inclusive, provided that r is greater than 0.

In still another embodiment, the invention provides locked helix peptides of formula (7e): 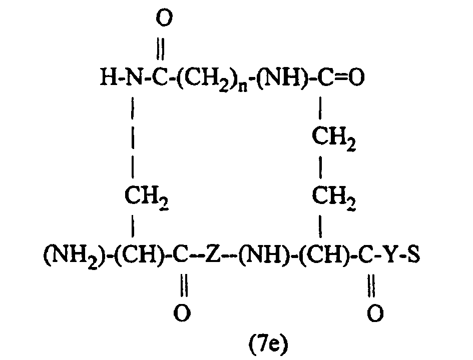
wherein S is absent or is a macromolecule; Y is absent, or is hydroxyl if S is absent, or is any amino acid or amino acid sequence; Z is any amino acid sequence consisting of six amino acids; and n is any integer from 4 to 6 inclusive.

In still another embodiment, the invention provides locked helix peptides of formula (8e): 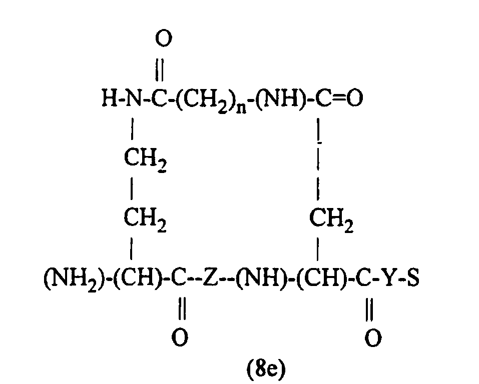
wherein S is absent or is a macromolecule; Y is absent, or is hydroxyl if S is absent, or is any amino acid or amino acid sequence; Z is any amino acid sequence consisting of six amino acids; and n is any integer from 4 to 6 inclusive.

In still another embodiment, the invention provides locked helix peptides of formula (9e): 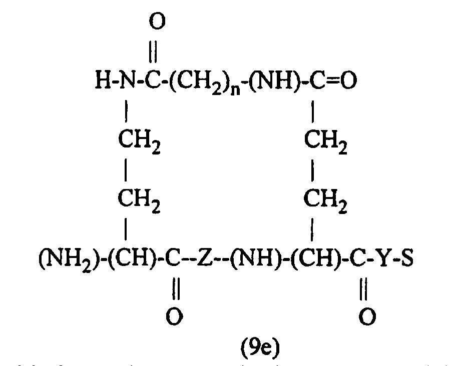
wherein S is absent or is a macromolecule; Y is absent, or is hydroxyl if S is absent, or is any amino acid or amino acid sequence; Z is any amino acid sequence consisting of six amino acids: and n is any integer from 3 to 5 inclusive.

In still another embodiment, the invention provides locked helix peptides of formula (10e): 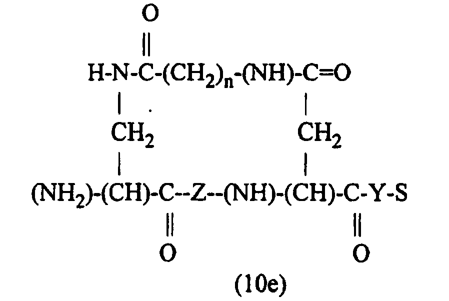
wherein S is absent or is a macromolecule; Y is absent, or is hydroxyl if S is absent, or is any amino acid or amino acid sequence; Z is any amino acid sequence consisting of six amino acids; and n is any integer from 5 to 7 inclusive.

In still another embodiment, the invention provides locked helix peptides of formula (11e): 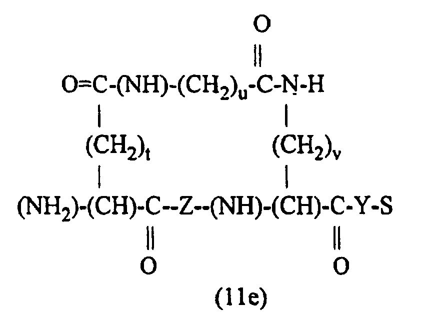
wherein S is absent or is a macromolecule; Y is absent, or is hydroxyl if S is absent, or is any amino acid or amino acid sequence; Z is any amino acid sequence consisting of six amino acids; t is selected from the integers 0 to 6 inclusive, and v is selected from the integers 1 to 7 inclusive, provided that t+v is less than or equal to 7; and u is any integer in the range defined by (7-(t+v)) to (9-(t+v)) inclusive, provided that u is greater than 0.

In still another embodiment, the invention provides locked helix peptides of formula (12e): 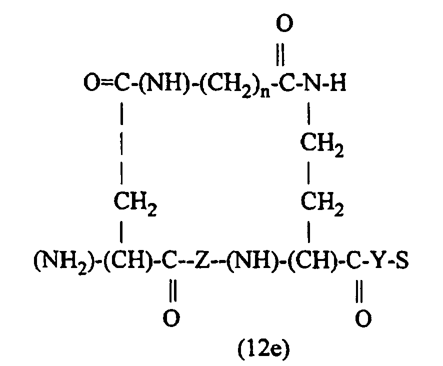
wherein S is absent or is a macromolecule; Y is absent, or is hydroxyl if S is absent, or is any amino acid or amino acid sequence; Z is any amino acid sequence consisting of six amino acids; and n is any integer from 4 to 6 inclusive.

In still another embodiment, the invention provides locked helix peptides of formula (13e): 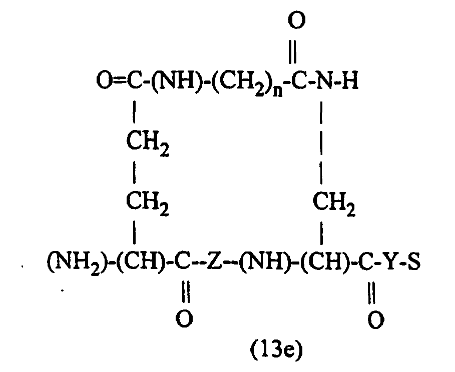
wherein S is absent or is a macromolecule: Y is absent, or is hydroxyl if S is absent, or is any amino acid or amino acid sequence: Z is any amino acid sequence consisting of six amino acids; and n is any integer from 4 to 6 inclusive.

In still another embodiment, the invention provides locked helix peptides of formula (14e): 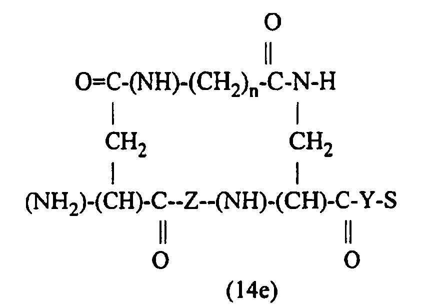
wherein S is absent or is a macromolecule; Y is absent, or is hydroxyl if S is absent, or is any amino acid or amino acid sequence: Z is any amino acid sequence consisting of six amino acids; and n is any integer from 5 to 7 inclusive.

In still another embodiment, the invention provides locked helix peptides of formula (15e): 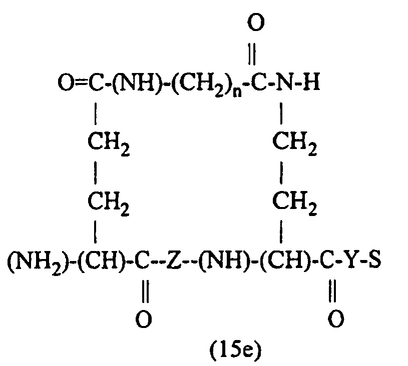
wherein S is absent or is a macromolecule; Y is absent, or is hydroxyl if S is absent, or is any amino acid or amino acid sequence; Z is any amino acid sequence consisting of six amino acids; and n is any integer from 3 to 5 inclusive.

In still another embodiment, the invention provides locked helix peptides of formula (16e): 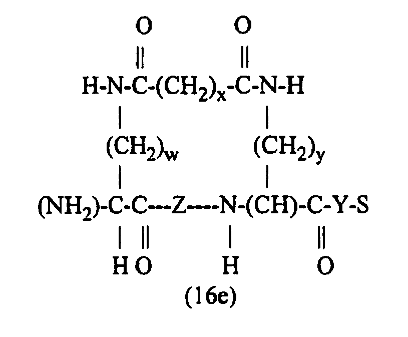
wherein S is absent or is a macromolecule; Y is absent, or is hydroxyl if S is absent, or is any amino acid or amino acid sequence; Z is any amino acid sequence consisting of six amino acids; w and y are independently selected from the integers 1 to 7 inclusive, provided that w+y is less than or equal to 8; and x is any integer in the range defined by (7-(w+y)) to (9-(w+y)) inclusive, provided that x is greater than or equal to 0.

In still another embodiment, the invention provides locked helix peptides of formula (17e): 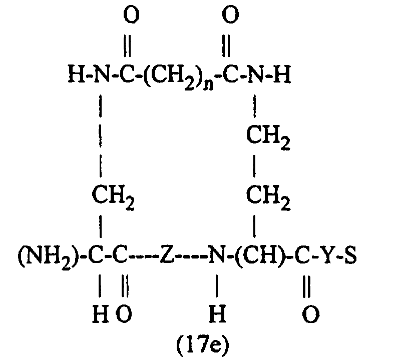
wherein S is absent or is a macromolecule; Y is absent, or is hydroxyl if S is absent, or is any amino acid or amino acid sequence; Z is any amino acid sequence consisting of six amino acids; and n is any integer from 4 to 6 inclusive.

In still another embodiment, the invention provides locked helix peptides of formula (18e): 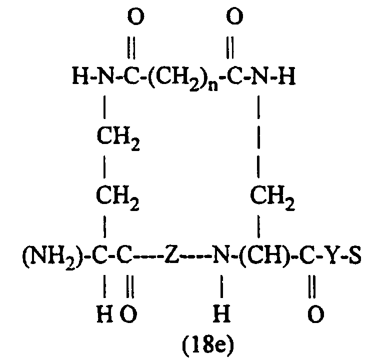
wherein S is absent or is a macromolecule; Y is absent, or is hydroxyl if S is absent, or is any amino acid or amino acid sequence; Z is any amino acid sequence consisting of six amino acids; and n is any integer from 4 to 6 inclusive.

In still another embodiment, the invention provides locked helix peptides of formula (19e): 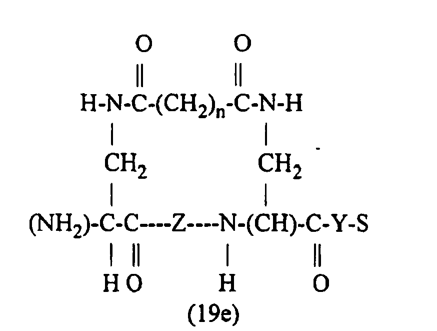
wherein S is absent or is a macromolecule; Y is absent, or is hydroxyl if S is absent, or is any amino acid or amino acid sequence; Z is any amino acid sequence consisting of six amino acids; and n is any integer from 5 to 7 inclusive.

In still another embodiment, the invention provides locked helix peptides of formula (20e): 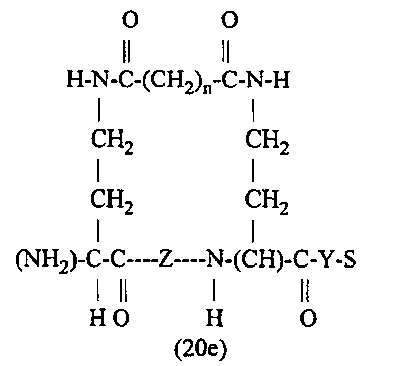
wherein S is absent or is a macromolecule; Y is absent, or is hydroxyl if S is absent, or is any amino acid or amino acid sequence; Z is any amino acid sequence consisting of six amino acids; and n is any integer from 3 to 5 inclusive.

Also provided herein are locked helix peptides of formula (1e), formula (2e), formula (3e), formula (4e), formula (5e), formula (6e), formula (7e), formula (8e), formula (9e), formula (10e), formula (11e), formula (12e), formula (13e), formula (14e), formula (15e), formula (16e), formula (17e), formula (18e), formula (19e) and formula (20e) wherein Y contains up to or about 30 amino acid residues.

Further provided herein are locked hel ix peptides of formula (1e), formula (2e), formula (3e), formula (4e), formula (5e), formula (6e), formula (7e), formula (8e), formula (9e), formula (10e), formula (11e), formula (12e), formula (13e), formula (14e), formula (15e), formula (16e), formula (17e), formula (18e), formula (19e) and formula (20e) wherein Y contains up to or about 25 amino acid residues.

Additionally provided herein are locked helix peptides of formula (1e), formula (2e), formula (3e), formula (4e), formula (5e), formula (6e), formula (7e), formula (8e), formula (9e), formula (10e), formula (11e), formula (12e), formula (13e), formula (14e), formula (15e), formula (16e), formula (17e), formula (18e), formula (19e) and formula (20e) wherein Y contains up to or about 20 amino acid residues.

Also encompassed herein are locked helix peptides of formula (1e), formula (2e), formula (3e), formula (4e), formula (5e), formula (6e), formula (7e), formula (8e), formula (9e), formula (10e), formula (11e), formula (12e), formula (13e), formula (14e), formula (15e), formula (16e), formula (17e), formula (18e), formula (19e) and formula (20e) wherein Y contains up to or about 15 amino acid residues.

Further encompassed herein are locked helix peptides of formula (1e), formula (2e), formula (3e), formula (4e), formula (5e), formula (6e), formula (7e), formula (8e), formula (9e), formula (10e), formula (11e), formula (12e), formula (13e), formula (14e), formula (15e), formula (16e), formula (17e), formula (18e), formula (19e) and formula (20e) wherein Y contains up to or about 10 amino acid residues.

Additionally encompassed herein are locked helix peptides of formula (1e), formula (2e), formula (3e), formula (4e), formula (5e), formula (6e), formula (7e), formula (8e), formula (9e), formula (10e), formula (11e), formula (12e), formula (13e), formula (14e), formula (15e), formula (16e), formula (17e), formula (18e), formula (19e) and formula (20e) wherein Y contains up to or about 5 amino acid residues.

Also within the scope of the invention are locked helix peptides of formula (1e), formula (2e), formula (3e), formula (4e), formula (5e), formula (6e), formula (7e), formula (8e), formula (9e), formula (10e), formula (11e), formula (12e), formula (13e), formula (14e), formula (15e), formula (16e), formula (17e), formula (18e), formula (19e) and formula (20e) wherein Y contains up to or about 3 amino acid residues.

The invention also provides locked helix peptides of formula (1f): 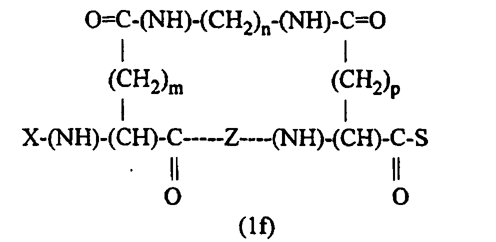
wherein S is hydroxyl or is a macromolecule; X is hydrogen or is any amino acid or amino acid sequence; Z is any amino acid sequence consisting of six amino acids; m and p are independentlyselected from the integers 0 to 6 inclusive, provided that m+p is less than or equal to 6; and n is any integer in the range defined by (7-(m+p)) to (9-(m+p)) inclusive, provided that n is greater than 1.

In another embodiment, the invention provides locked helix peptides of formula (2f): 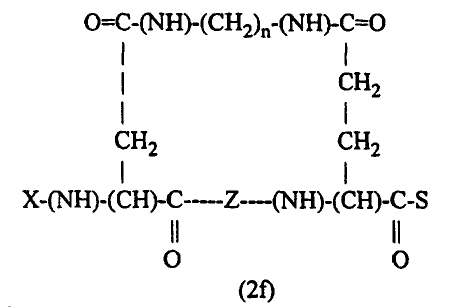
wherein S is hydroxyl or is a macromolecule; X is hydrogen or is any amino acid or amino acid sequence; Z is any amino acid sequence consisting of six amino acids; and n is any integer from 4 to 6 inclusive.

In yet another embodiment, the invention provides locked helix peptides of formula (3f): 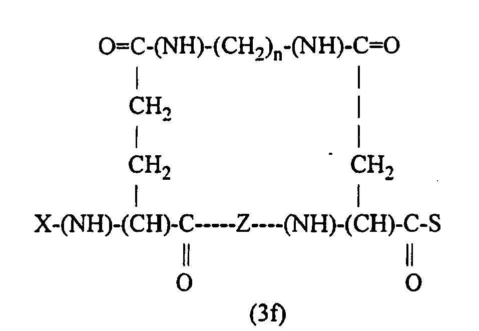
wherein S is hydroxyl or is a macromolecule; X is hydrogen or is any amino acid or amino acid sequence; Z is any amino acid sequence consisting of six amino acids; and n is any integer from 4 to 6 inclusive.

In still another embodiment, the invention provides locked helix peptides of formula (4f): 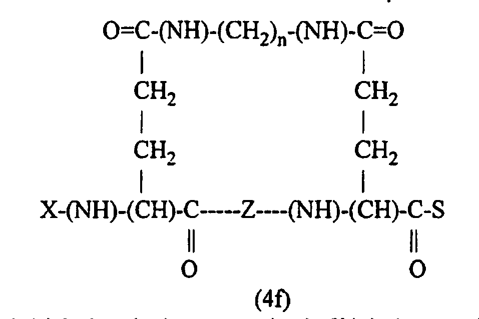
wherein S is hydroxyl or is a macromolecule; X is hydrogen or is any amino acid or amino acid sequence; Z is any amino acid sequence consisting of six amino acids; and n is any integer from 3 to 5 inclusive.

In still another embodiment, the invention provides locked helix peptides of formula (5f): 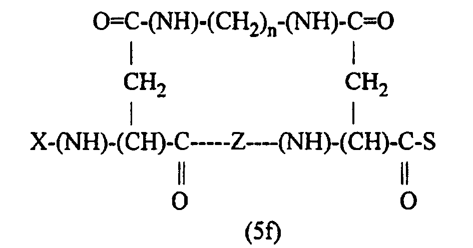
wherein S is hydroxyl or is a macromolecule; X is hydrogen or is any amino acid or amino acid sequence; Z is any amino acid sequence consisting of six amino acids; and n is any integer from 5 to 7 inclusive.

In still another embodiment, the invention provides locked helix peptides of formula (6f): 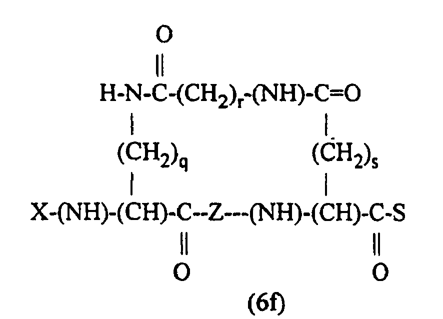
wherein S is hydroxyl or is a macromolecule; X is hydrogen or is any amino acid or amino acid sequence; Z is any amino acid sequence consisting of six amino acids; q is selected from the integers 1 to 7 inclusive, and
s is selected from the integers 0 to 6 inclusive, provided that q+s is less than or equal to 7; and r is any integer in the range defined by (7-(q+s)) to (9-(q+s)) inclusive, provided that r is greater than 0.

In still another embodiment, the invention provides locked helix peptides of formula (7f): 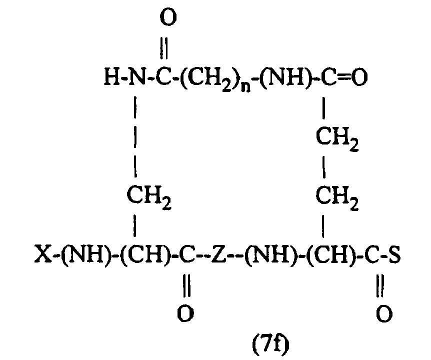
wherein S is hydroxyl or is a macromolecule; X is hydrogen or is any amino acid or amino acid sequence; Z is any amino acid sequence consisting of six amino acids; and n is any integer from 4 to 6 inclusive.

In still another embodiment, the invention provides locked helix peptides of formula (8f): 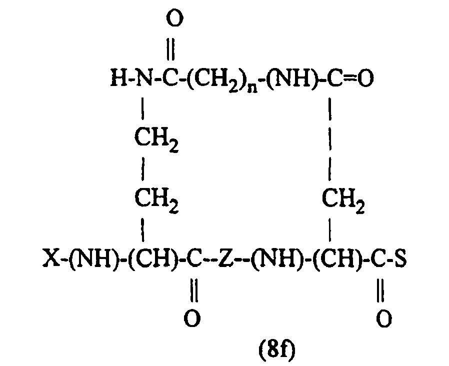
wherein S is hydroxyl or is a macromolecule; X is hydrogen or is any amino acid or amino acid sequence; Z is any amino acid sequence consisting of six amino acids; and n is any integer from 4 to 6 inclusive.

In still another embodiment, the invention provides locked helix peptides of formula (9f): 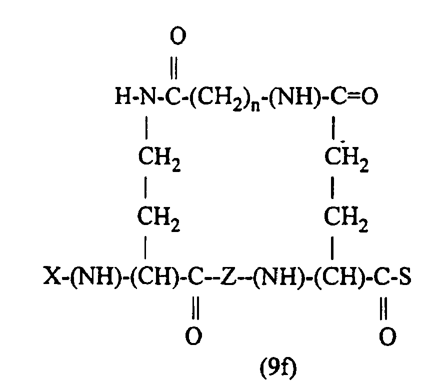
wherein S is hydroxyl or is a macromolecule; X is hydrogen or is any amino acid or amino acid sequence; Z is any amino acid sequence consisting of six amino acids; and n is any integer from 3 to 5 inclusive.

In still another embodiment, the invention provides locked helix peptides of formula (10f): 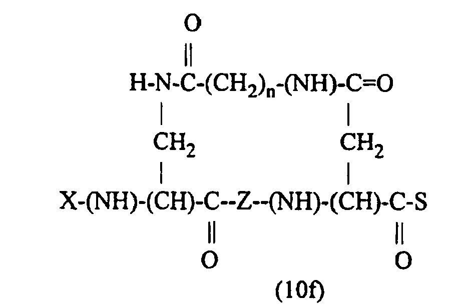
wherein S is hydroxyl or is a macromolecule; X is hydrogen or is any amino acid or amino acid sequence; Z is any amino acid sequence consisting of six amino acids; and n is any integer from 5 to 7 inclusive.

In still another embodiment, the invention provides locked helix peptides of formula (11f): 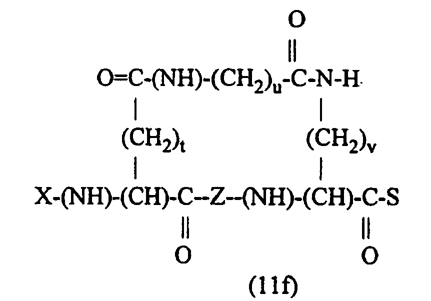
wherein S is hydroxyl or is a macromolecule; X is hydrogen or is any amino acid or amino acid sequence; Z is any amino acid sequence consisting of six amino acids; t is selected from the integers 0 to 6 inclusive, and
v is selected from the integers I to 7 inclusive, provided that t+v is less than or equal to 7; and u is any integer in the range defined by (7-(t+v)) to (9-(t+v)) inclusive, provided that u is greater than 0.

In still another embodiment, the invention provides locked helix peptides of formula (12f): 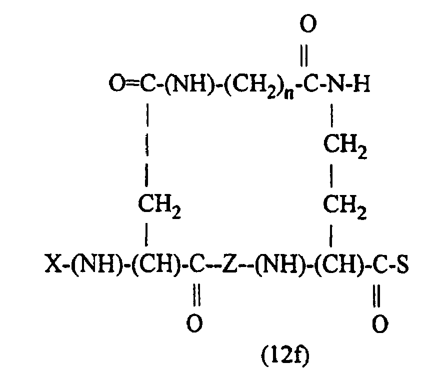
wherein S is hydroxyl or is a macromolecule; X is hydrogen or is any amino acid or amino acid sequence; Z is any amino acid sequence consisting of six amino acids; and n is any integer from 4 to 6 inclusive.

In still another embodiment, the invention provides locked helix peptides of formula (13f): 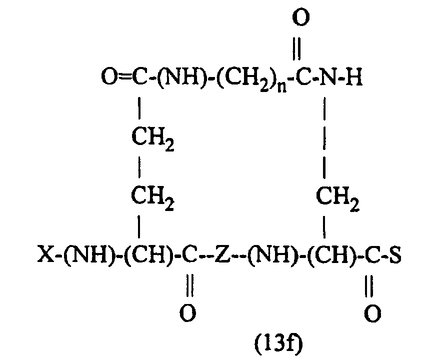
wherein S is hydroxyl or is a macromolecule; X is hydrogen or is any amino acid or amino acid sequence; Z is any amino acid sequence consisting of six amino acids; and n is any integer from 4 to 6 inclusive.

In still another embodiment, the invention provides locked helix peptides of formula (14f): 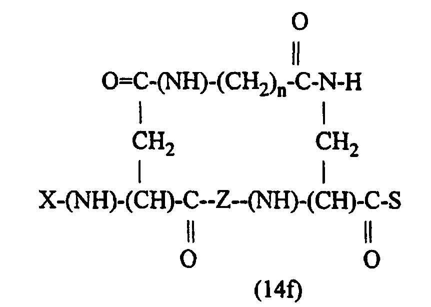
wherein S is hydroxyl or is a macromolecule; X is hydrogen or is any amino acid or amino acid sequence; Z is any amino acid sequence consisting of six amino acids; and n is any integer from 5 to 7 inclusive.

In still another embodiment, the invention provides locked helix peptides of formula (15f): 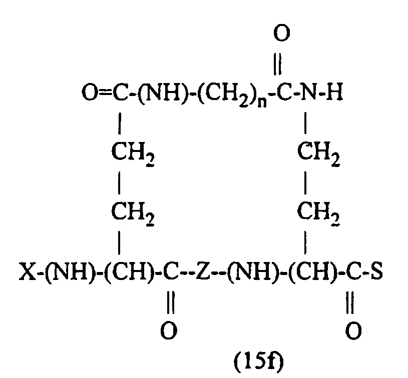
wherein S is hydroxyl or is a macromolecule; X is hydrogen or is any amino acid or amino acid sequence; Z is any amino acid sequence consisting of six amino acids; and n is any integer from 3 to 5 inclusive.

In still another embodiment, the invention provides locked helix peptides of formula (16f): 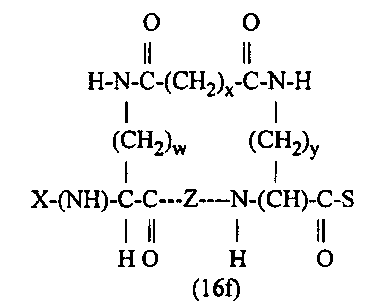
wherein S is hydroxyl or is a macromolecule; X is hydrogen or is any amino acid or amino acid sequence; Z is any amino acid sequence consisting of six amino acids; w and y are independently selected from the integers 1 to 7 inclusive, provided that w+y is less than or equal to 8; and x is any integer in the range defined by (7-(w+y)) to (9-(w+y)) inclusive, provided that x is greater than or equal to 0.

In still another embodiment, the invention provides locked helix peptides of formula (17f): 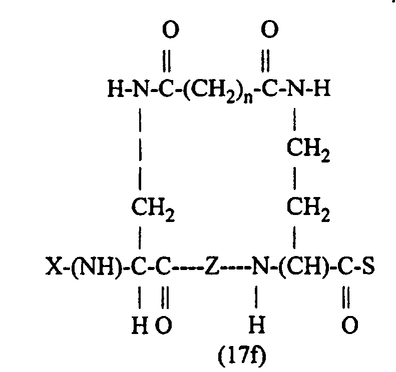
wherein S is hydroxyl or is a macromolecule; X is hydrogen or is any amino acid or amino acid sequence; Z is any amino acid sequence consisting of six amino acids; and n is any integer from 4 to 6 inclusive.

In still another embodiment, the invention provides locked helix peptides of formula (18f): 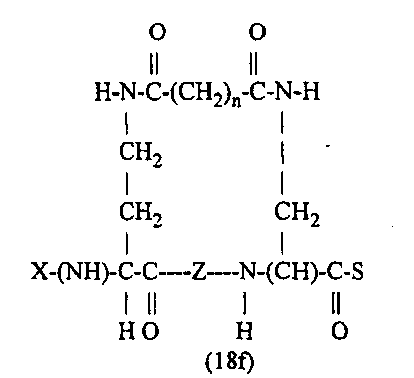
wherein S is hydroxyl or is a macromolecule; X is hydrogen or is any amino acid or amino acid sequence; Z is any amino acid sequence consisting of six amino acids; and n is any integer from 4 to 6 inclusive.

In still another embodiment, the invention provides locked helix peptides of formula (19f): 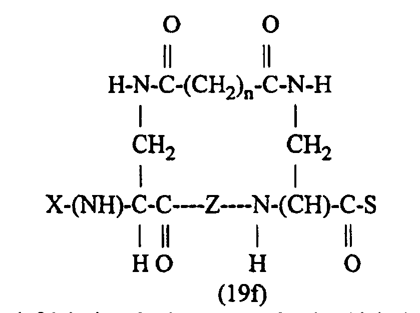
wherein S is hydroxyl or is a macromolecule; X is hydrogen or is any amino acid or amino acid sequence; Z is any amino acid sequence consisting of six amino acids; and n is any integer from 5 to 7 inclusive.

In still another embodiment, the invention provides locked helix peptides of formula (20f): 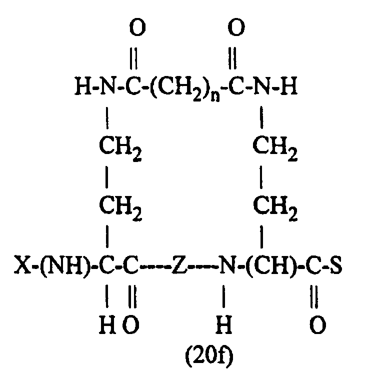
wherein S is hydroxyl or is a macromolecule; X is hydrogen or is any amino acid or amino acid sequence; Z is any amino acid sequence consisting of six amino acids; and n is any integer from 3 to 5 inclusive.

Also providedherein are locked helix peptides of formula (1f), formula (2f), formula (3f), formula (4f), formula (5f), formula (6f), formula (7f), formula (8f), formula (9f), formula (10f), formula (11f), formula (12f), formula (13f), formula (14f), formula(15f), formula (16f), formula (17f), formula (18f), formula (19f) and formula (20f) wherein X contains up to or about 30 amino acid residues.

Further provided herein are locked helix peptides of formula (1f), formula (2f), formula (3f), formula (4f), formula (5f), formula (6f), formula (7f), formula (8f), formula (9f), formula (10f), formula (11f), formula (12f), formula (13f), formula (14f), formula (15f), formula (16f), formula (17f), formula (18f), formula (19f) and formula (20f) wherein X contains up to or about 25 amino acid residues.

Additionally provided herein are locked helix peptides of formula (1f), formula (2f), formula (3f), formula (4f), formula (5f), formula (6f), formula (7f), formula (8f), formula (9f), formula (10f), formula (I 1f), formula (12f), formula (13f), formula (14f), formula (15f), formula (16f), formula (17f), formula (18f), formula (19f) and formula (20f) wherein X contains up to or about 20 amino acid residues.

Also encompassed herein are locked helix peptides of formula (1f), formula (2f), formula (3f), formula (4f), formula (5f), formula (6f), formula (7f), formula (8f), formula (9f), formula (10f), formula (11f), formula (12f), formula (13f), formula (14f), formula (15f), formula (16f), formula (17f), formula (18f), formula (19f) and formula (20f) wherein X contains up to or about 15 amino acid residues.

Further encompassed herein are locked helix peptides of formula (1f), formula (2f), formula (3f), formula(4f), formula (5f), formula (6f), formula (7f), formula (8f), formula (9f), formula (10f), formula (11f), formula (12f), formula (13f), formula (14f), formula (15f), formula (16f), formula (17f), formula (18f), formula (19f) and formula (20f) wherein X contains up to or about 10 amino acid residues.

Additionally encompassed herein are locked helix peptides of formula (1f), formula (2f), formula (3f), fonmula(4f), formula(5f), formula (6f), formula (7f), formula (8f), formula (9f), formula (10f), formula (11f), formula (12f), formula (13f), formula (14f), formula (15f), formula (16f), formula (17f), formula (18f), formula (19f) and formula (20f) wherein X contains up to or about 5 amino acid residues.

Also within the scope of the invention are locked helix peptides of formula (1f), formula (2f), formula (3f), formula (4f), formula(5f), fotmula(6f), formula (7f), formula (8f), formula (9f), formula (10f), formula (11f), formula (12f), formula (13f), formula (14f), formula (15f), formula (16f), formula (17f), formula (18f), formula (19f) and formula (20f) wherein X contains up to or about 3 amino acid residues.

The invention also provides locked helix peptides of formula (1g): 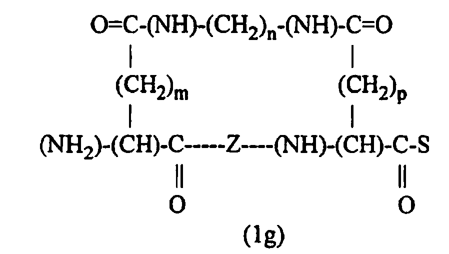
wherein S is hydroxyl or is a macromolecule; Z is any amino acid sequence consisting of six amino acids; m and p are independently selected from the integers 0 to 6 inclusive, provided that m+p is less than or equal to 6; and n is any integer in the range defined by (7-(m+p)) to (9-(m+p)) inclusive, provided that n is greater than I.

In another embodiment, the invention provides locked helix peptides of formula (2g): 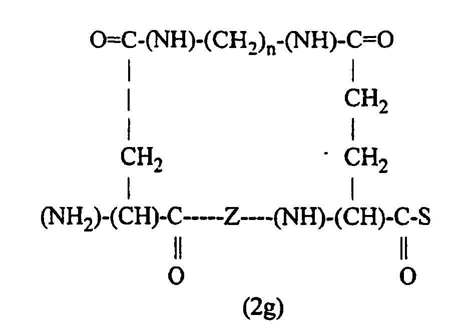
wherein S is hydroxyl or is a macromolecule;Z is any amino acid sequence consisting of six amino acids; and
n is any integer from 4 to 6 inclusive.

In yet another embodiment, the invention provides locked helix peptides of formula (3g): 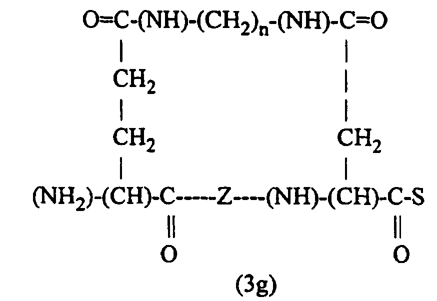
wherein S is hydroxyl or is a macromolecule;Z is any amino acid sequence consistingof six amino acids; and
n is any integer from 4 to 6 inclusive.

In still another embodiment, the invention provides locked helix peptides of formula (4g): 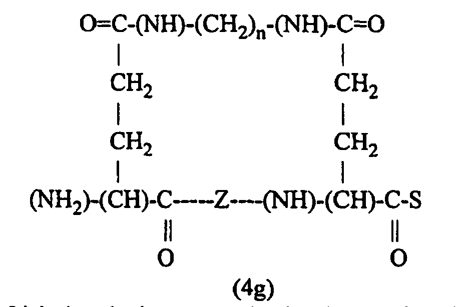
wherein S is hydroxyl or is a macromolecule;Z is any amino acid sequence consisting of six amino acids; and
n is any integer from 3 to 5 inclusive.

In still another embodiment, the invention provides locked helix peptides of formula (5g): 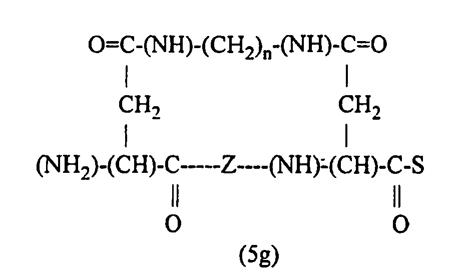
wherein S is hydroxyl or is a macromolecule; Z is any amino acid sequence consisting of six amino acids; and
n is any integer from 5 to 7 inclusive.

In still another embodiment, the invention provides locked helix peptides of formula (6g): 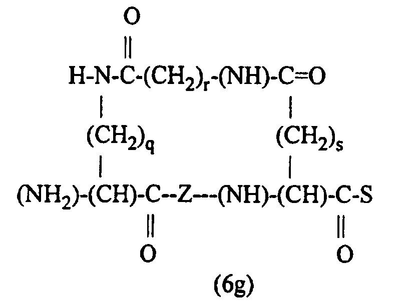
wherein S is hydroxyl or is a macromolecule; Z is any amino acid sequence consisting of six amino acids; q is selected from the integers 1 to 7 inclusive, and s is selected from the integers 0 to 6 inclusive, provided that q+s is less than or equal to 7; and r is any integer in the range defined by (7-(q+s)) to (9-(q+s)) inclusive, provided that r is greater than 0.

In still another embodiment, the invention provides locked helix peptides of formula (7g): 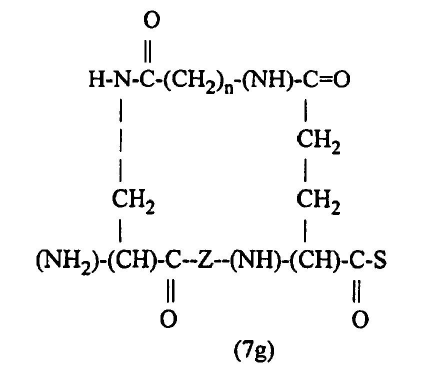
wherein S is hydroxyl or is a macromolecule;Z is any amino acid sequence consisting of six amino acids; and
n is any integer from 4 to 6 inclusive.

In still another embodiment, the invention provides locked helix peptides of formula (8g): 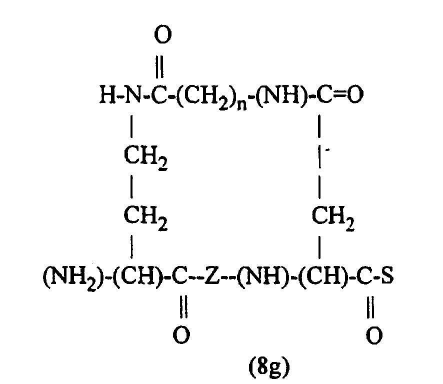
wherein S is hydroxyl or is a macromolecule;Z is any amino acid sequence consisting of six amino acids; and
n is any integer from 4 to 6 inclusive.

In still another embodiment, the invention provides locked helix peptides of formula (9g): 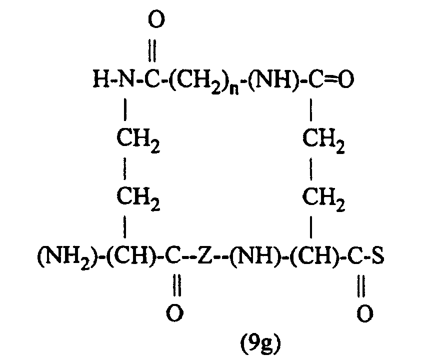
wherein S is hydroxyl or is a macromolecule;Z is any amino acid sequence consisting of six amino acids; and
n is any integer from 3 to 5 inclusive.

In still another embodiment, the invention provides locked helix peptides of formula (10g): 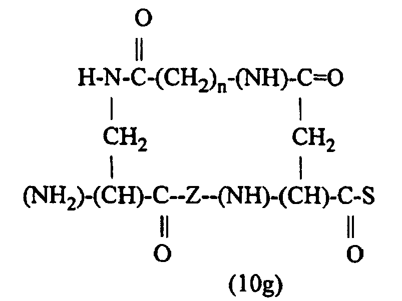
wherein S is hydroxyl or is a macromolecule;Z is any amino acid sequence consisting of six amino acids; and
n is any integer from 5 to 7 inclusive.

In still another embodiment, the invention provides locked helix peptides of formula (11g): 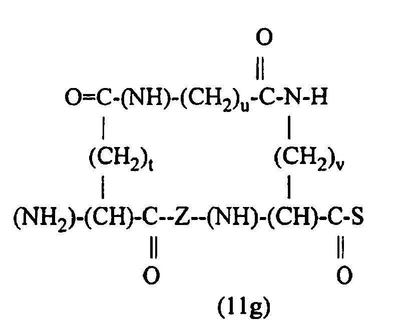
wherein S is hydroxyl or is a macromolecule; Z is any amino acid sequence consisting of six amino acids; t is selected from the integers 0 to 6 inclusive, and v is selected from the integers 1 to 7 inclusive, provided that t+v is less than or equal to 7; and u is any integer in the range defined by (7-(t+v)) to (9-(t+v)) inclusive, provided that u is greater than 0.

In still another embodiment, the invention provides locked helix peptides of formula (12g): 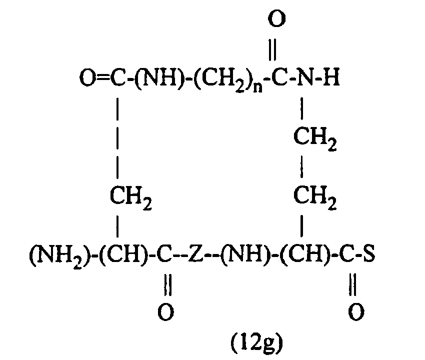
wherein S is hydroxyl or is a macromolecule;Z is any amino acid sequence consisting of six amino acids; and
n is any integer from 4 to 6 inclusive.

In still another embodiment, the invention provides locked helix peptides of formula (13g): 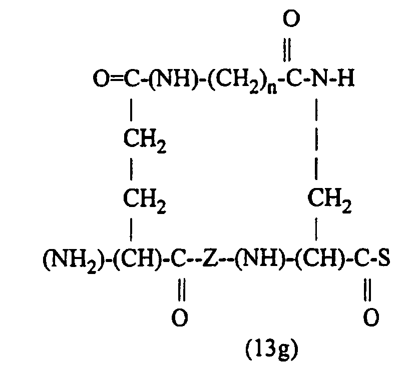
wherein S is hydroxyl or is a macromolecule;Z is any amino acid sequence consisting of six amino acids; and
n is any integer from 4 to 6 inclusive.

In still another embodiment, the invention provides locked helix peptides of formula (14g): 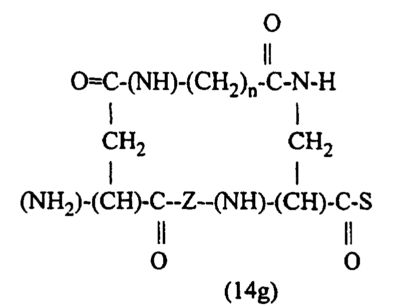
wherein S is hydroxyl or is a macromolecule;Z is any amino acid sequence consisting of six amino acids; and
n is any integer from 5 to 7 inclusive.

In still another embodiment, the invention provides locked helix peptides of formula (15g): 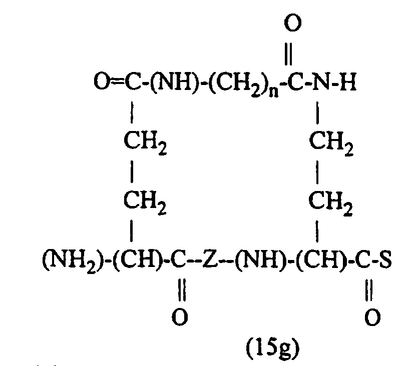
wherein S is hydroxyl or is a macromolecule; Z is any amino acid sequence consisting of six amino acids; and
n is any integer from 3 to 5 inclusive.

In still another embodiment, the invention provides locked helix peptides of formula (16g): 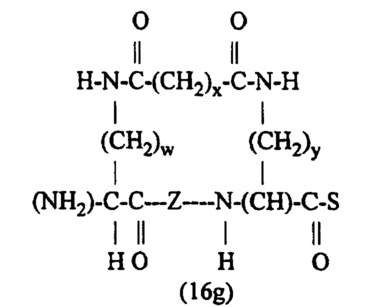
wherein S is hydroxyl or is a macromolecule; Z is any amino acid sequence consisting of six amino acids; w and y are independently selected from the integers 1 to 7 inclusive, provided that w+y is less than or equal to 8; and x is any integer in the range defined by (7-(w+y)) to (9-(w+y)) inclusive, provided that x is greater than or equal to 0.

In still another embodiment, the invention provides locked helix peptides of formula (17g): 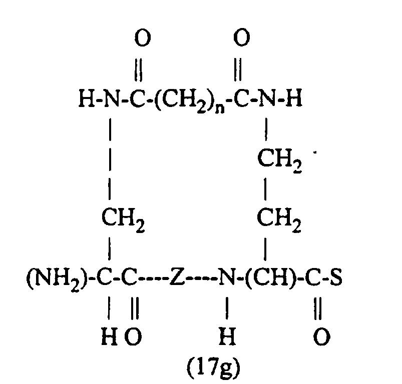
wherein S is hydroxyl or is a macromolecule;Z is any amino acid sequence consisting of six amino acids; and
n is any integer from 4 to 6 inclusive.

In still another embodiment, the invention provides locked helix peptides of formula (18g): 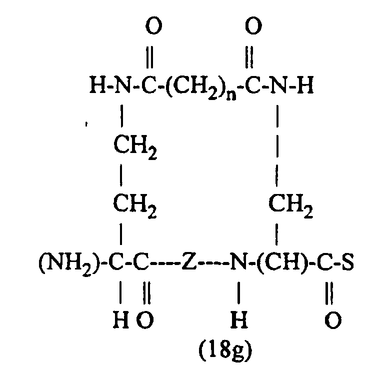
wherein S is hydroxyl or is a macromolecule;Z is any amino acid sequence consisting of six amino acids; and
n is any integer from 4 to 6 inclusive.

In still another embodiment, the invention provides locked helix peptides of formula (19g): 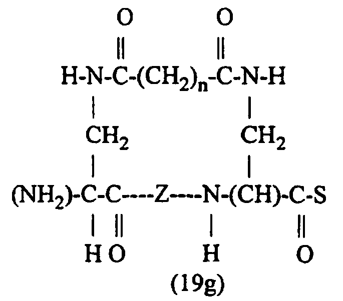
wherein S is hydroxyl or is a macromolecule;Z is any amino acid sequence consisting of six amino acids; and
n is any integer from 5 to 7 inclusive.

In still another embodiment, the invention provides locked helix peptides of formula (20g): 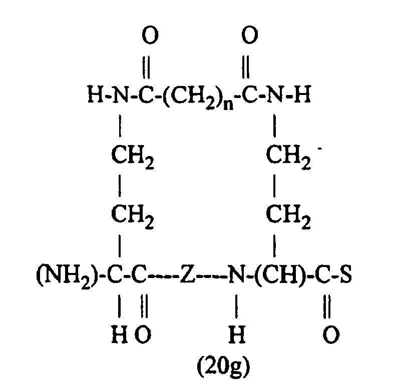
wherein S is hydroxyl or is a macromolecule;Z is any amino acid sequence consisting of six amino acids; and
n is any integer from 3 to 5 inclusive.

For locked helix peptides of formulas (1), (2), (3), (4), (5), (6), (7), (8), (9), (10), (11), (12), (13), (14), (15), (16), (17), (18), (19), (20), (1e), (2e), (3e), (4e), (5e), (6e), (7e), (8e), (9e), (10e), (11e), (12e), (13e), (14e), (15e), (16e), (17e), (18e), (19e), (20e), (1f), (2f), (3f), (4f), (5f), (6f), (7f), (8f), (9f), (10f), (11f), (12f), (13f), (14f), (15f), (16f), (17f), (18f), (19f), (20f), (1g), (2g), (3g), (4g), (5g), (6g), (7g), (8g), (9g), (10g), (11g), (12g), (13g), (14g), (15g), (16g), (17g), (18g), (19g), or (20g) bound to a macromolecule, the invention encompass es any macromolecule capable of serving as an anchor for the C-terminus of the locked helix peptide. Typically, the macromolecule functions as a solid support. In general, the solid support is an inert matrix, such as a polymeric gel, comprising a three dimensional structure, lattice or network of a material. Almost any macromolecule, synthetic or natural, can form a gel in a suitable liquid when suitably cross-linked with a difunctionalreagent. In one embodiment,the macromolecule selected is convenient for use in affinity chromatography. Most chromatographic matrices used for affinity chromatography are xerogels. Such gels shrink on drying to a compact solid comprising only the gel matrix. When the dried xerogel is resuspended in the liquid, the gel matrix imbibes liquid, swells and returns to the gel state. Xerogels suitable for use herein include polymeric gels, such as cellulose, cross-linkeddextrans (e.g. Sepharose),agarose,cross-linkedagarose, polyacrylamide gels, and polyacrylamide-agarose gels.

The locked helix peptides provided herein can be constructed according to the methods of the invention described in Sections II and III below.

In one embodiment, the peptides of the invention are designed to isolate the binding determinants from α-helical binding domains of known proteins. Such peptides have a number of uses, including the determination of whether a binding determinant in an α-helical binding domain of a known protein can serve as a structural model for the design of peptidomimetics or small molecules capable of mimicking or antagonizing the binding activity of the intact protein. In using the peptides of the invention for this purpose, the practitioner selects a binding protein with a helical domain that interacts with ligand, and then identifies a candidate binding determinant situated within a sequence of six (or more) contiguous amino acids in the helical binding domain. The candidate binding determinant can be identified by using alanine scanning mutagenesis to determine whether the candidate sequence contains one or more amino acid residues that are critical for ligand binding. Next, a constrained peptide containing the candidate sequence is designed by selecting two residues in the candidate sequence (designated *1* and *1*+7) which are separated by an intervening sequence of six amino acids and which do not interact with ligand (as determined by alanine scanning mutagenesis in the previous step) for substitution with amino acid residues having a side chain containing an amide bond-forming substitutent. The peptide is synthesized and the side chain amide bond-forming substitutent of the foreign *I* and *I*+7 residues are used to tether the peptide in an α-helical conformation according to the methods of the invention described in Section II below. Finally, the locked helix peptide's binding activity with the ligand is assayed, e.g., in a binding competition assay with the intact binding protein, and the results of the assay can be used to determine whether a peptidomimetic or small molecule antagonist could be developed using the binding determinant as a structural model.

In another embodiment, the locked helix peptides of the invention are used to replace intact binding proteins or protein binding domains in the affinity purification of ligand. For example, Protein A is commonly used for affinity chromatographicpurification of IgG molecules. The Z-domain of Protein A is a three helix bundle, 59 residues in length, which binds to the Fc portion of IgG. As described in Example 2 below, a locked helix species of the peptide Phe-Asn-Met-(1)-Gln-Gln-Arg-Arg-Phe-Tyr-(2)-Ala-Leu-His (wherein the amino acid residues at the (1) and (2) positions in the corresponding Z-domain sequence are both replaced with glutamic acid residues), corresponding to a binding determinant in helix 1 of the Z-domain can be used to bind IgG. Accordingly, the invention provides constrained helix species containing binding determinants in helix 1 of the Z-domain in Protein A, including molecules of formula (4) above wherein Z is GIn-Gln-Arg-Arg-Phe-Tyr. In one embodiment, the constrained helix species is a molecule of formula (4) wherein Z is Gln-Gln-Arg-Arg-Phe-Tyr,X is Phe-Asn-Met, and Y is Ala-Leu-His. The IgG binding molecules of the invention are conveniently synthesized using the solid phase peptide synthesis methods described in Section II below, such that the molecules are anchored to resin beads suitable for column or batch affinity chromatography.

In still another embodiment, the locked helix peptidesofthe invention are designed to mimic epitopes in proteins and are used to selectively raise polyclonal or monoclonal antibodies against such individual epitopes. Since the peptides will frequently be too small to generate an immune response, the peptides can be conjugated to carriers known to be immunogenic in the species to be immunized, e.g., keyhole limpet hemocyanin, serum albumin, bovine thyroglobulin, or soybean trypsin inhibitor using a difunctional or derivatizing agent, for example, maleimidobenzoyl sulfosuccinimide ester (conjugation through cysteine residues), N-hydroxysuccinimide(through lysine residues), glutaraldehyde,succinic anhydride, SOCl₂, or R¹N = C = NR, where R and R¹ are different alkyl groups.

The locked helix peptides of the invention are particularly useful in isolating synthetic antibody clones with a selected binding activity from phage display combinatorial libraries. The locked helix peptide provides a significant advantage over the intact protein or protein domain in that using the locked helix peptide allows the isolation of binding activities for the particular conformational epitope of interest. Without the locked helix peptides of the invention, the conformation epitope of interest would likely require structural support from other regions of the protein or protein domain whose presence in the ligand would result in the concomitant isolation of undesired clones. In addition, the synthesis of locked helix peptides anchored to polymeric resins as described in Sections II and III below would provide material that can be conveniently packed into columns for panning phage display libraries.

In another aspect, the locked helix peptides of the invention are used to provide conformationally stable variants of peptides or proteins which exhibit "floppy" or unstable α-helical secondary structure at one or more site(s) in unrestrained form under conditions of interest. In particular, the methods of the invention can solve problems presented by some antigens which relate to the instability of conformational epitopes. A conformational epitope can fail to present the desired antigenic determinant because of "floppy" or unstable α-helical secondary structure element(s) in the epitope. The restraint of such "floppy" α-helical structure(s) according to the methods of the invention would stabilize the conformational epitope and allow presentation of the desired antigenic determinant. This application of the present methods and peptides is particularly useful, for example, in vaccine design and in generating polyclonal or monoclonal antibodies from host animals or isolating antibody clones from phage display libraries.

In one embodiment the invention, where the locked helix peptides of the invention are used to provide conformationally stable variants of peptides or proteins which exhibit "floppy" or unstable α-helical secondary structure at one or more sites in unrestrained form under conditions of interest, a compound containing a constrained helical peptide that is useful as an immunogen, vaccine and diagnostic for human immunodeficiency virus (HIV) is provided. Acquired immunodeficiency syndrome (AIDS) is caused by a retrovirus identified as the human immunodeficiency virus (HIV). There have been intense efforts to develop a vaccine that induces a protective immune response based on induction of antibodies or cellular responses. Recent efforts have used subunit vaccines where an HIV protein, rather than attenuated or killed virus, is used as the immunogen in the vaccine for safety reasons.

The human immunodeficiencyvirus 1 (HIV-1) envelope glycoproteins gp 120 and gp41 mediate viral tropism to and subsequententry into target cells (Freed et al., *The Journal of Biological Chemistry* **270**, 23883-23886 (1995)). The role of gp120 is to bind to target cells by interactions with CD4 and one of several co-receptors (D'Souza et al., *Nature Medicine* **2**, 1293-1300(1996)), after which gp41 promotes the fusion of viral and cellular membranes. The mechanism by which gp41 mediates membrane fusion has recently been the subject of intensive study. Evidence suggests that the process may involve the formation of a coiled-coil trimer, which is thought to drive the transition from resting to fusogenic states, as modeled for influenza hemagglutinin (Wilson et al., *Nature* **289,** 366-373 (1981); Carr, et al., *Cell* **73**, 823-832 (1993); Bullough et al.. *Nature* **371**, 37-43 (1994)).

Two linear peptides derived from HIV- gp41 have been found to inhibit viral fusion. The first of these, DP107, represents a portion of gp41 near the N-terminal fusion peptide and has been shown to be helical in solution and oligomerize in a manner consistent with coiled-coil formation (Gallaher et al., *AIDS Res. Hum. Retroviruses* **5,** 431-440 (1989); Weissenhom et al., *Nature* **387,** 426-430 (1997)). A more potent peptide, DP178. was derived from the C-terminal region of the gp41 ectodomain (Wild, et al., PNAS 91: 9770-9774 (1994): Jiang et al., Nature, **365**:113 (1993)). Although this region of gp41 was predicted to be α-helical (Gallaheret al., *AIDS Res. Hum. Retroviruses* 5, 431-440 (1989)), DP178 itself lacks discernable structure in solution (Wild, et al., PNAS 91: 9770-9774 (1994). Attempts to explore the mechanism of action of DP 178 have been complicated by a lack of understanding of its bioactive conformation. Recently, crystallographic (Chan et al., *Cell* **89,** 263-273 (1997); Weissenhorn et al., *Nature* **387,** 426-430 (1997)) and solution (Lawless. et al., *Biochemistry* **35**, 13697-13708 (1996); Lu et al., *Nature Structural Biology* **2,** 1075-1082 (1995); Rabenstein et al., *Biochemistry* 35. 13922-13928 (1996)) studies have shown that disconnected segments of HIV-1 gp41 that overlap DP107 and DP178 associate in a tightly-packed helical bundle. The C-terminal segment, corresponding to DP178, forms an extended helix which packs in an antiparallel fashion against a groove created by an N-terminal (DP-107) coiled-coil trimer. While these data suggest one possible conformation for DP178, they do not provide conclusive information about the mechanism of peptide inhibition during viral fusion events.

The present invention provides helical constrained forms of DP178 and homologous sequences and variants, overcoming the limitations in the art concerning DP178 and providing more effective use of DP-178 like sequences. Accordingly, in one embodiment of the invention is provided a constrained helical peptide having at least its internal amino acid sequence (and preferably adjacent amino acid sequences) selected from the C-terminal region of the HIV-1 LAI isolate transmembrane protein gp41 ectodomain amino acids 633 to 678, which overlap with the sequence corresponding to peptide DP-178 (amino acid residues 643 to 673). This region is a 46-amino acid sequence (reading from the amino to carboxy terminus): NH2-WMEWEREIDNYTSLIHSLIEESQNQQEKNEQELLELDKWASLWNWF-COOH..

Peptides in an alpha-helical coiled-coil conformation interact with one another in a characteristic manner that is determined by the primary sequence of each peptide. The tertiary structure of an alpha-helix is such that 7 amino acid residues in the primary sequence correspond to approximately 2 turns of the alpha-helix. Accordingly, a primary amino acid sequence giving rise to an alpha-helical conformation may be broken down into units of 7 residues each, termed heptads (having the form **abcdefg**). The core polypeptides are comprised of a series of heptads in tandem. When the sequence of a heptad is repeated in a particular core polypeptide, the heptad may be referred to as a "heptad repeat", or simply "repeat".

According to the invention, embodiments are provided as compounds containing a constrained helical peptide that is composed of a peptide which contains a sequence of eight amino acid residues, where the sequence of eight amino acid residues has a first terminal residue and a second terminal residue, where the first terminal residue and the second terminal residue flank an internal sequence of six amino acids, wherein the first and second terminal residues have a side chain that are linked to each other forming a locking moiety to constrain the peptide to a helical form. The internal sequence of six amino acids has the form **gabcde**, **defgab**, or **cdefga** and has a sequences of six contiguous amino acids found in HIV-1 LAI strain transmembraneprotein gp41 amino acid sequence 633 to 678, in its homolog sequence from another HIV strain, in a consensus sequence of its homolog sequences from any one HIV clade, or amino acid substituted variant thereof. According to the invention, each of the amino acids in the aforementioned sequences is assigned a position of **a, b, c, d, e, f,** or **g**. The assignment is based on assigning the amino acid 633 of the HIV LAI gp41 633-678 sequence to position a of a repeating **abcdefg** heptad assignment. Subsequent amino acids in the sequence are assigned positions accordingly. Figure 18 indicates the heptad positional assignment of each amino acid in the sequence. The assignment can be readily applied to homologs and consensus sequences by aligning their amino acids to the corresponding amino acid in the representative HIV LAI sequence. The 633 amino acid or its corresponding amino acid in a homolog or consensus sequence is assigned position **a**, which begins the repeating **abcdefg** assignment pattern.

In these representative compounds and sequences shown in Figures 16-18, the locking moiety or tether is between adjacent **f** positions when the internal sequence is of the form **gabcde**, adjacent c positions when the internal sequence is of the form **defgab,** or adjacent b positions when the internal sequence is of the form **cdefga.** In the most preferred embodiments the locking occurs between adjacent **f** positions, in which case the **f** position amino acids are replaced by amino acids suitable for providing a helix locking moiety. Figure 18 provides the alignment of the repeating **abcdefg** assignment with the sequences relevant to the invention. In a preferred embodiment the internal sequence of six amino acids has the form **gabcde**. These "internal sequence" of six amino acids from gp41 can substitute for moiety "Z" in any of the compounds, formulas, and synthetic methods taught herein.

While the internal amino acid sequence is preferably from a sequence of six contiguous amino acids in HIV-1LAI strain gp41 amino acid sequence 633 to 678, in its homolog sequence from another HIV strain, or in a consensus sequence of its homolog sequences from any one HIV clade, it may be an amino acid substituted variant thereof. The sequences of the invention also include analogs of HIV gp41 sequence 633-678, truncations which may include, but are not limited to, peptides comprising the 633-678 sequence, containing one or more amino acid substitutions, insertions and/or deletions. The analogs of the sequence will exhibit antiviral activity when in constrained peptides of the invention, and may, further, possess additional advantageous features, such as, for example, increased bioavailability, and/or stability, or generate antibodies with increased HIV strain recognition.

HIV-1 and HIV-2 envelope proteins are structurally distinct, but there exists a striking amino acid conservation within the gp41 633-678 corresponding regions of HIV-1 and HIV-2. Amino acid substitutions may be of a conserved or non-conserved nature. Conserved amino acid substitutions consist of replacing one or more amino acids of the 633-678 peptide sequence with amino acids of similar charge, size, and/or hydrophobicitycharacteristics, such as, for example, a glutamic acid (E) to aspartic acid (D) amino acid substitution. Non-conserved substitutionsconsist of replacing one or more amino acids of the 633-678 peptide sequence with amino acids possessing dissimilar charge, size, and/or hydrophobicity characteristics, such as, for example, a glutamic acid (E) to valine (V) substitution.

Deletions of the 633-678 region or its homologs are also within the scope of the invention. Such deletions consist of the removal of one or more amino acids with the lower limit length of the resulting peptide sequence being 6 amino acids for use as an internal sequence of a constrained helical peptide. Preferably the deletions retain sufficient amino acids such that at least two locks may be incorporated as taught herein. Examples of such deletions are the HIV35 peptide and its constrained helix compounds of the invention that have one lock (e.g. HIV 24) and two locks (e.g. HIV 31). Most preferably, the deletions are terminal truncations, but in any event result in peptides which, when constrained along the **f-b-c** helical face, are still recognized by the coiled coil search algorithms used herein, or alternatively, retain the **a-d** helical face orientation and spatial arrangement of the parent molecule, or alternatively, can exhibit antifusogenic or antiviral activity.

Most preferred compounds are those that, when used as immunogens, generate antibodies that neutralize HIV viral fusogenic activity or infectivity.

The peptides of the invention may further include homolog sequences of the HIV LAI strain 633-678 sequence which exhibit antiviral activity when in constrained helical form. Most preferably, the constrained peptides, when used as haptens, will generate antibodies that block viral fusion events, leading to an inhibition of viral infectivity. Such homologs are peptides-whose amino acid sequences are comprised of the amino acid sequences of peptide regions of other (i.e., other than HIV-1LAI) viruses that correspond to the gp41 peptide region of 633-678. Such viruses may include, but are not limited to, other HIV-1 isolates and HIV-2 isolates. Homologs derived from the corresponding gp41 peptide region of other (i.e., non HIV-1LAI) HIV-1 isolates may include those provide in Figures 16A to 16G, or other known corresponding sequences. Particularly preferred are those derived from HIV-1SF2, HIV-1RF, and HIV-1MN, GNE6, GNE8, and Thai strain isolate A244.

In a particularly preferred embodiment, amino acids at positions a and d of the internal sequence of six amino acids are not amino acid substituted in the helical peptide, but are the amino acids in the known isolates or consensus sequences (see Figures 16A-16G and 17).

Also most preferred are embodiments where the amino acids at positions **g** and **e** of the internal sequence of six amino acids are not amino acid substituted in the helical peptide. An amino acid at any one of positions **a**, **d**, **g**, or **e** of the internal sequence of six amino acids can be conservatively substituted in the helical peptide in preferred embodiments. The a and d positions, and less directly the **g** and **e** positions, are believed to be those that are on the face of the constrained helix that interacts with the gp41 core trimer (see Figure 19). Since positions **f**, **b** and **c** are believed to not directly participate in binding, but rather serve to allow helix structure, preferred variations at positions **b** and **c** of the internal sequence of six amino acids are not amino acid substituted in the helical peptide, when not the locking (tethering) residues. Less preferred are compounds wherein an amino acid at any one of positions **b**, **c**, or **f** of the internal sequence of six amino acids is conservatively substituted or is any non-helix-breaking amino acid in the helical peptide, that does not interfere with the locking moiety. Preferred internal sequence chimeras are those in which an amino acid at any one of positions **a**, **d**, **g**, or **e** of the internal sequence of six amino acids is substituted in the helical peptide with an amino acid from the corresponding position of a different HIV virus strain.

Preferred compounds of the invention can include sequences from HIV-1 clade consensus sequences:
(clade B consensus) W m e W e r E I d n Y T ? I I y t L I e e s Q n Q Q e k N e q e L L e L d k W a s L w n W f (SEQ ID NO: 109);
(clade A consensus) W L q W d K E I s n Y T ? I I Y n L I E e S q n Q Q E k N E q d L L A L D K W a n L w n W F (SEQ ID NO: 110);
(clade C consensus) W M q W D R E I S N Y T d t I Y r L L E D S Q N Q Q E r N E K D L L A L D S W k N L W N W F (SEQ ID NO: 111);
(clade D consensus) W m e W E r E I d N Y T G I I Y s L I E e S Q I Q Q E K N E k e L L e L D K W A S L W N W F (SEQ ID NO: 112); and
(clade E consensus) W I E W e R E I S N Y T N q I Y e I L T e S Q n Q Q D R N E K D L L e L D K W A S L W n W f (SEQ ID NO: 113). The amino acids in these sequences are represented by a single letter code, wherein a lower case letter is the represented amino acid or is substituted with an amino acid from that corresponding position in a sequence within the same clade, and wherein a ? is any amino acid from that correspondingposition in a sequence from within the same clade. Most preferred are homologs or consensus sequences from Figures 16A-16G. The internal sequences are preferably found virus sequences in the group of HIV-1 clades consisting of clades A, B, C, D, E, F, G and F/B.

Compounds of the invention can have the first and second terminal residues with a side chain containing an amide bond-forming substituent that are linked to each other forming an amide bond to form a constrained helical peptide. The side chain amide bond-forming substituent of the first terminal residue and the side chain amide bond-forming substituent of the second terminal residue are independently selected from the group consisting of an amino substituent and a carboxy substituent The side chain amide bond-forming substituent of the first terminal residue is a carboxy substituent, the side chain amide bond-fonning substituent of the second terminal residue is a carboxy substituent, and the difunctional linker is a diamine wherein the first and second functional groups are amino groups. In preferred form the first terminal residue and the second terminal residue are independently selected from the group consisting of Asp and Glu, more preferred the first terminal residue and the second terminal residue are both Glu. The first terminal residues can have a D-thio-lysine side chain and the second terminal residue a L-thio-lysine that are linked to each other forming a disulfide bonded locking moiety to form a constrained helical peptide.

In another embodiment the constrained peptide is a hapten that is attached to a carrier macromolecule, preferably covalently linked to the constrained helical peptide, as discussed herein. The macromolecule can be linked to the helical peptide at the locking moiety or at amino acids at positions **f**, **b**, or **c** of the constrained helical peptide, and can be any carrier that does not interfere with the **a-d** face of the constrained helical peptide. A preferred carrier for immunogenic purposes is keyhole limpet hemocyanin, or other carriers discussed herein.

In other embodiments the compounds contain more than one constrained helical peptide. The internal sequences of a first and a second constrained helical peptide in these embodiments are preferably different. The internal sequences of the first and second constrained helical peptides are from the same HIV gp41 sequence or the same HIV clade consensus sequence, or amino acid substituted variant thereof. The internal sequences of the first and second constrained helical peptides are chosen from those that were separated by at least two helical turns (or six residues) in the HIV gp41 sequence or the same HIV clade consensus sequence, or amino acid substituted variant thereof. The compounds can further comprise a third constrained helical peptide. Again, the internal sequences of the first, second, and third constrained helical peptides are preferably different. In one example, the three sequences are present as separate constrained helical segments in a super helix of the polypeptide backbone of a 633-678 sequence as depicted in Figure 18.

In other embodiments the compounds of the invention contain I to 38, 1 to 35, or more preferably, 1 to 19 amino acids flanking either or both terminal residues of the helical peptide, The flanking amino acids preferably are the flanking amino acids for the internal sequence as found in a sequence from an HIV gp41 sequence.

In yet other embodiments, the compounds further comprising a blocking group attached at either or both of the terminal residues of the helical peptide to prevent proteolytic degradation. The blocking group can contain a D-amino acid or a non-amide bond between adjacent flanking amino acids.

Particularly preferred compounds include those in which a single lock is placed within sequence YTSLIHSLIEESQNQQEKNEQELLELD (SEQ ID NO: 2) sequence or a homolog sequence thereof, within EWDREINNYTSLIHSLIEESQNQQEKNEQE (SEQ ID NO: 107) sequence or a homolog sequence thereof, within YTSLIHSLIEESQNQQEKNEQELLELDKWASLWNF (SEQ ID NO: 108) sequence or a homolog sequence thereof, to yield a constrained helical peptide. More than one constraint, preferably two, can be placed in these sequences, with examples shown in Figure 18. Shown in Figure 18 are locations of **gabcde** form helical sections when one, two or three *i* to *i+7* locks are present in a 633-678 sequence or variant (truncated or sequence variant) thereof.. The two-lock variants (II), (III), and HIV31, and the one-lock variants HIV24, (IX) and (XI) (Figure 18) are preferred compounds demonstratingpreferred locking positions. Less preferred are the three-lock variant, the two-lock (VI) and (VII) variants, and the one lock VIII and XII variants. Particularly preferred are the truncated variants HIV24 and HIV31 and their homologs from other HIV strains or consensus sequences or substitution variants thereof. Much less preferred are *i* to *i*+4 lock to constrain a "floppy" helical segment.

In a preferred embodiment there are at least two constrained helical peptides in the compound, for example attached as different and independent haptens to KHL or a synthetic TASP or lysine network, or as two or more locked helical segments within a longer polypeptide sequence, preferably one that has a tendency to form an extended or super helical structure. The internal sequences of the first and second constrained helical peptides are preferably different, for example as multiple haptens on a single hapten carrier or two or more locked helical segments within a longer super helix polypeptide sequence. In the latter case, the internal sequences of the first and second constrained helical peptides are preferably from the same HIV gp41 sequence, the same HIV clade consensus sequence, or the same amino acid substituted variant thereof. The two helical peptides are attached to each other by a separating amino acid sequence, which can comprises from 5 to 7, 12 to 14, or 19 to 21 non helix-breaking natural or unnatural amino acids, and where preferably, the internal sequences of the first and second constrained helical peptides are from the same HIV gp41 sequence, the same HIV clade consensus sequence, or the same amino acid substituted variant thereof. The separating sequence can be a contiguous amino acid sequence selected from an intervening sequence that is located between the two internal sequences present in the same HIV gp41 sequence, the same HIV clade consensus sequence, or the same amino acid substituted variant thereof, and that excludes the two amino acids that correspond to the helical peptide locking positions immediately flanking the intervening sequence. An example is HIV31, in which the two constrained segments (internal amino acid sequences) are separated in the parent sequence (HIV35) by an eight amino acid sequence of which the amino acids at adjacent f positions used in locking are not considered part of the intervening sequence, such that the intervening sequence is a six amino acid sequence which is synthesized into the final peptide as a six amino acid separating sequence. The separating sequence is most preferably 6, 13, or 20 amino acids, in order to maintain alignment of a-d faces in between to constrained helical peptides.

The amino acids in the separating sequence retain **abcdefg** assignment positions of the intervening sequence, wherein preferably the amino acids in positions **a** and **d** in the separating sequence are identical to their corresponding intervening sequence amino acids. In addition, in preferred embodiments the amino acids in the separating sequence positions **g** and **e** also are identical to their corresponding intervening sequence amino acids. Less preferably, an amino acid at any one of positions **a, d,** g, or **e** is conservatively substituted in the separating sequence (with a sequence other than that represented in the clade at that position). Most preferably, the amino acids in the separating sequence retain **abcdefg** assignment positions of the intervening sequence and an amino acid at any one of positions **a, d, g,** or **e** is substituted in the separating sequence with a corresponding amino acid from its homolog sequence from another HIV strain, from a consensus sequence of its homolog sequences from any one HIV clade, or from an amino acid substituted variant thereof. The amino acids in the separating sequence positions **b, c,** or **f** can be any non-helix-breakingainino acid, with the preferences given in Figures 22 and 23A and B. Chimeras can be formed where an amino acid at any one of positions **a, d, g,** or **e** of the internal sequence of six amino acids is substituted in the helical peptide with an amino acid from the correspondingposition of a different HIV virus strain. Likewise substitutionsof the same nature can be made in flanking or in separating sequences. Preferred are compounds wherein the internal amino acid sequence is from any one of the peptide sequences from Figure 23A and 23B. More preferably, the compound of the invention is selected from the group consisting of constrained helical peptides of each possible sequence having any one or any combination of amino acid substitutions indicated in the constrained helical peptide series I to XII as shown in Figures 23A and 23B. In other embodiments, the compound is selected from the group consisting of constrained helical peptides of each possible sequence having any one or any combination of amino acid truncations indicated in the constrained helical peptide series I to XII as shown in Figures 23A and 23B. In yet other embodiments, the compound is selected from the group consisting of constrained helical peptides of each possible sequence having any one or any combination of amino acid substitutions indicated in the constrained helical peptide series I to XII as shown in Figures 23A and 23B in combination with any one or any combination of amino acid truncations indicated in the constrained helical peptide series I to XII as shown in Figures 23A and 23B. X in these sequences can be any non helix-breaking amino acid.

In yet another embodiment of the invention, peptides comprising the sequences described herein can be synthesized with additional chemical groups present at their amino and/or carboxy termini, such that, for example, the stability, bioavailability, and/or inhibitory activity of the peptides is enhanced. For example, hydrophobic groups such as carbobenzoxyl, dansyl, or t-butyloxycarbonylgroups, may be added to the amino termini. An acetyl group or a 9-fluorenyhnethoxy-carbonyl group may be placed at the amino termini. A hydrophobic group, t-butyloxycarbonyl, or an amido group may be added to carboxy termini. Furthermore, the peptides of the invention can be synthesized such that their steric configuration is altered. For example, the D-isomer of one or more of the amino acid residues of the peptide can be used, rather than the usual L-isomer. The compounds can contain at least one bond linking adjacent amino acids that is a non-peptide bond, and is preferably not helix breaking. N on-peptide bonds for use in flanking sequences include an imino, ester, hydrazine, semicarbazide, oxime, or azo bond. Still further, at least one of the amino acid residues of the peptides of the invention can be substituted by one of the well known non-naturally occurring amino acid residues, that is preferably not helix breaking. Most preferably the non-natural amino acid or non-amide bond linking adjacent amino acids, when present.is present outside of the internal sequence, and is, more preferably, not helix breaking. Still further, at least one of the amino acid residues of the peptides of the invention can be substituted by one of the well known non-naturallyoccurring amino acid residues. Alterations such as these can serve to increase the stability, bioavailability, immunogenicity, and/or inhibitory action of the peptides of the invention.

While not wishing to be limited by any one theory, the constrained helical peptides are believed to derive their activity by interaction of the **a-d** face of the helix. The potent anti-HIV activity of the compounds of the invention derive from the gp41 633-678 region which corresponds to a putative alpha-helix region located in the C-terminal end of the gp41 ectodomain, and which appears to associate with a distal site on gp41 whose interactive structure is influenced by the leucine zipper motif, a coiled-coil structure, referred to as DP-107. The association of these two domains may reflect a molecular linkage or "molecular clasp" intimately involved in the fusion process (see Figures 18 and 19). The DP107 region forms a core trimer complex with a groove that recognizes and binds the **a-d** face of the helical peptides of the invention.

When synthesized as peptides both DP-107 and DP-178 are potent inhibitors of HIV infection and fusion, probably by virtue of their ability to form complexes with viral gp41 and interfere with its fusogenic process; e.g., during the structural transition of the viral protein from the native structure to the fusogenic state, the DP-107 and DP-178 peptides may gain access to their respective binding sites on the viral gp41, and exert a disruptive influence.
Consequently, when more than one constrained helical peptide is present, as part of a super helix or extended helix polypeptide backbone, the positions **a** and **d** of a first constrained helical peptide are in the same plane as positions **a** and **d** of the second constrained helical peptide. In other words, the **a-d** face of the two helices are aligned in the same plane. To achieve this orientation when the helices are in a polypeptide super helix, the first and second constrainedhelical peptides are separated by either 5 to 7, 12 to 14 or 19 to 21 natural or unnatural helix-forming amino acids. Preferably, the first and second constrained helical peptides are separated by either 6, 13, or 20 natural or unnatural helix-forming amino acids. A most preferred spatial alignment of the first, second, and any additional constrained helical peptides is that found in DP107, wherein the **a-d** faces are aligned in the same plane to allow interaction with the grove in the core trimer.

When the particularly preferred tethering chemistry as taught herein is used, the compounds of the invention are selected from the group consisting of:
the compound represented by Formula (1): 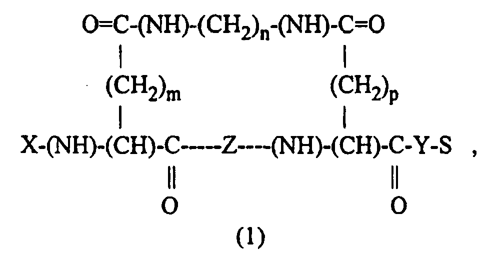
wherein S is absent or is a macromolecule,
X is hydrogen or is any amino acid or amino acid sequence,
Y is absent, or is hydroxyl if S is absent, or is any amino acid or amino acid sequence,
Z is an amino acid sequence consisting of six amino acids, wherein the internal sequence of six amino acids has the form **gabcde**, **defgab**, or **cdefga** and is selected from the group of sequences consisting of a sequence of six contiguous amino acids in HIV-1LAI strain gp41 amino acid sequence 633 to 678, in its homolog sequence from another HIV strain, in a consensus sequence of its homolog sequences from any one HIV clade, or amino acid substituted variant thereof, in which amino acid 633 or its corresponding amino acid in the homolog, consensus or variant sequence is assigned position **a** of a repeating **abcdefg** assignment;
m and p are independently selected from the integers 0 to 6 inclusive, provided that m+p is less than or equal to 6, and
n is any integer in the range defined by (7-(m+p)) to (9-(m+p)) inclusive, provided that n is greater than 1;
the compound represented by Formula (6): 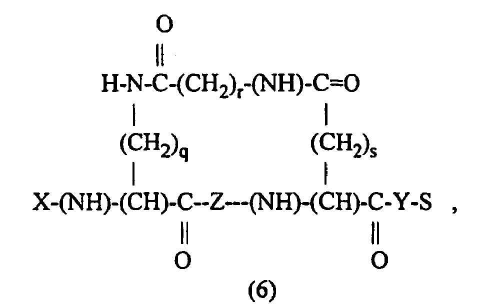 wherein S is absent or is a macromolecule, X is hydrogen or is any amino acid or amino acid sequence, Y is absent, or is hydroxyl if S is absent, or is any amino acid or amino acid sequence, Z is an amino acid sequence consisting of six amino acids, wherein the internal sequence of six amino acids has the form **gabcde**, **defgab**, or **cdefga** and is selected from the group of sequences consisting of a sequence of six contiguous amino acids in HIV-1LA1 strain gp41 amino acid sequence 633 to 678, in its homolog sequence from another HIV strain, in a consensus sequence of its homolog sequences from any one HIV clade, or amino acid substituted variant thereof, in which amino acid 633 or its corresponding amino acid in the homolog, consensus or variant sequence is assigned position a of a repeating **abcdefg**; q is selected from the integers 1 to 7 inclusive, s is selected from the integers 0 to 6 inclusive, provided that q+s is less than or equal to 7, and r is any integer in the range defined by (7-(q+s)) to (9-(q+s)) inclusive, provided that r is greater than 0;
the compound represented by Formula (11): 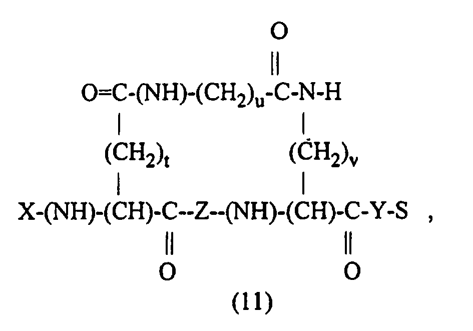
wherein S is absent or is a macromolecule, X is hydrogen or is any amino acid or amino acid sequence, Y is absent, or is hydroxyl if S is absent, or is any amino acid or amino acid sequence, Z is an amino acid sequence consistingof six amino acids, wherein the internal sequence of six amino acids has the form **gabcde, defgab, or cdefga** and is selected from the group of sequences consisting of a sequence of six contiguous amino acids in HIV-1LAI strain gp41 amino acid sequence 633 to 678, in its homolog sequence from another HIV strain, in a consensus sequence of its homolog sequences from any one HIV clade, or amino acid substituted variant thereof, in which amino acid 633 or its corresponding amino acid in the homolog, consensus or variant sequence is assigned position a of a repeating **abcdefg** assignment; t is selected from the integers 0 to 6 inclusive, and v is selected from the integers 1 to 7 inclusive, provided that t+v is less than or equal to 7; and u is any integer in the range defined by (7-(t+v)) to (9-(t+v)) inclusive, provided that u is greater than 0; and
the compound represented by Formula (16): 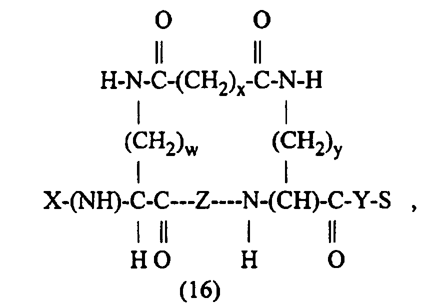
wherein S is absent or is a macromolecule, X is hydrogen or is any amino acid or amino acid sequence, Y is absent, or is hydroxyl if S is absent, or is any amino acid or amino acid sequence, Z is an amino acid sequence consisting of six amino acids, wherein the internal sequence of six amino acids has the form **gabcde, defgab**, or **cdefga** and is selected from the group of sequences consisting of a sequence of six contiguous amino acids in HIV-1LAI strain gp41 amino acid sequence 633 to 678, in its homolog sequence from another HIV strain, in a consensus sequence of its homolog sequences from any one HIV clade, or amino acid substituted variant thereof, in which amino acid 633 or its corresponding amino acid in the homolog, consensus or variant sequence is assigned position **a** of a repeating **abcdefg** assignment; w and y are independently selected from the integers 1 to 7 inclusive, provided that w+y is less than or equal to 8, and x is any integer in the range defined by (7-(w+y)) to (9-(w+y)) inclusive, provided that x is greater than or equal to 0.
These compounds can further contain S' when S is absent and X is any amino acid or amino acid sequence, wherein S' is a macromolecule attached to-X. The X or Y can contain a blocking group that prevents enzymatic degradation. Standard terminal blacking groups as known in the art are suitable. X or Y can also contain a D-amino acid or a non-amide bond between adjacent amino acids to prevent enzymatic degradation.

The compounds can be formulated with a carrier as taught herein. When the helical peptide is to be used as a hapten the carrier can be an adjuvant. Typically, compositions of the invention are sterile. Compositions can contain at least two compounds of the invention, ether free or covalently or ionically attached to one another. The peptides of the invention that have a virus fusion inhibitor activity, can be used in combination with other therapeutic agents, preferably in combination with another antiviral agent, to enhance its antiviral effect. Such antiviral agents include but are not limited to those which function on a different target molecule involved in viral replication, e.g., reverse transcriptase inhibitors, viral protease inhibitors, glycosylation inhibitors; those which act on a different target molecule involved in viral transmission;those which act on a different loci of the same molecule; and those which prevent or reduce the occurrence of viral resistance.

In treating mammals, including humans, having a viral infection, a therapeutically effective amount of the compounds of the invention, or a pharmaceutically acceptable derivative, is administered is a dose sufficient to inhibit viral replication, either alone or in combination with other virus inhibiting drugs. For example HIV31 or HIV 24 can be administered as an infusion at about 0.1 mg/kg to 1.0 mg/kg per day for about 12 weeks. A preferable dose is from 20 mg to 35 mg. Doses can be administered in intervals of from about once per day to 4 times per day and preferably from about once every two days to once per day. A preferred dose is administered to achieve peak plasma concentrations of compound of from about I mg/ml to 10 mg/ml. This may be achieved by the sterile injection of about a 2.0% solution of the administered ingredients in buffered saline (any suitable saline solutions known to those skilled in the art of chemistry may be used). Desirable blood levels may be maintained by a continuous infusion as ascertained by plasma levels measured by HPLC. Pharmaceutical compositions containing the compounds of the invention can be administeredto a human patient, by itself, or in pharmaceutical compositions where it is mixed with suitable carriers or excipient(s),as taught herein, at doses to treat a viral infection, in particular HIV infection. Suitable routes of administration include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections; transdermal, topical, vaginal and the like. Dosage forms include but are not limited to tablets, troches, dispersions, suspensions, suppositories, solutions, capsules, creams, patches, minipumps and the like.

As discussed herein the compounds of the invention are particularly suited as haptens to raise an antibody that binds to the compound, preferably the antibody specifically binds an epitope comprising an amino acid at position **a**, **d**, **e**, or **g** in the helical peptide. Preferred antibodies are monoclonal. Antibodies of the invention, not only recognize the peptides of the invention, but preferably recognize the corresponding sequence when present in the virus. They may also bind unconstrainedDP 178. More preferably, the antibody neutralizes HIV viral infectivity and/or neutralizes HIV virus membrane fusion. Thus the antibodies can recognize and bind gp41 sequence.

Described herein is a method to immunize an animal, comprising administering to the animal an immunogenic amount of a compound of the invention.

Also described herein is a method to prophylactically or therapeutically treat a mammal at risk for or infected with HIV, comprising administering a composition comprising a prophylactically or therapeutically effective amount of a compound of the invention and a carrier. While antibodies of the invention are expected to have broad viral activity, preferably, the composition comprises internal six amino acid sequences from different HIV strains or HIV clades. The compositions include a vaccine formulation. The formulations can contain one or more (multivalent) constrained helical peptides form different HIV strains, for use as a vaccine or immunogen. The composition can be administered, prophylactically or therapeutically, to a patient at risk of infection or in need of such treatment using the dosages and routes and means of administration that are readily determined. However, chronic administration may be preferred and dosages can be adjusted accordingly.

Administration of the compounds containing the constrained helical peptides of the invention as a prophylactic vaccine (or therapeutic vaccine), can comprise administering to a host a concentrationof peptides effective in raising an immune response which is sufficient to neutralize HIV, by, for example, inhibiting HIV ability to infect cells. The exact concentration will depend upon the specific peptide to be administered, but may be determined by using standard techniques for assaying the development of an immune response which are well known to those of ordinary skill in the art. The peptides to be used as vaccines are usually administered intramuscularly. The peptides may be formulated with a suitable adjuvant in order to enhance the immunological response. Such adjuvants may include, but are not limited to, mineral gels such as aluminum hydroxide; surface active substances such as lysolecithin, pluronic polyols, polyanions; other peptides; oil emulsions; and potentially useful human adjuvants such as BCG and Corynebacterium parvum. Many methods may be used to introduce the vaccine formulations described here. These methods include but are not limited to oral, intradermal, intramuscular, intraperitoneal, intravenous, subcutaneous, and intranasal routes.

A compound of this invention in a suitable carrier or excipient is used to make a vaccine. The polypeptide can be used alone, but is preferably administered in a multivalent subunit vaccine that includes internal sequences from MN strain. The vaccine usually includes constrained helices representing 3 to about 5 different strains, but 30 or more different gp41-based constrained helical polypeptides can be used to provide a more effective vaccine. Of particular interest are gp41 sequences from breakthrough isolates of HIV vaccine trials. Use of a a homolog gp41 sequence from one or more of breakthrough isolates in a subunit vaccine, usually together a sequence from a commonly present isolate like the MN sequence, can provide protection against HIV strains that are sufficiently different from the common strain (e.g., MN) that the typical single subunit vaccine does not confer protection against those strains.

Preparation of polypeptides for use in a vaccine is well known. The compound with the desired degree of purity and at a sufficient concentrationto induce antibody formation is mixed with a physiologically acceptable carrier. A physiologically acceptable carrier is nontoxic to a recipient at the dosage and concentration employed in the vaccine. Generally, the vaccine is formulated for injection, usually intramuscular or subcutaneous injection. Suitable carriers for injection include sterile water, but preferably are physiologic salt solutions, such as normal saline or buffered salt solutions such as phosphate-buffered saline or ringer's lactate. The vaccine generally contains an adjuvant. Useful adjuvants include QS21 (Quillaja saponaria, commercially available from Cambridge Biotech, Worcester, MA), which stimulates cytotoxic T-cells, and alum (aluminum hydroxide adjuvant). Formulations with different adjuvants which enhance cellular or local immunity can also be used.

Additional excipients that can be present in the vaccine include low molecular weight polypeptides (less than about 10 residues), proteins, amino acids, carbohydrates including glucose or dextrans, chelating agents such as EDTA, and other excipients that stabilize the protein or inhibit growth of microorganisms.

The vaccine can also contain other HIV proteins. In particular, gp120, or the extracellular portion of gp41 or HIV-1 core proteins such as P24, P17, and P55 can be present in the vaccine. Preferably, any gp120 present in the vaccine is from an HIV isolate sequence represented in a constrained helical peptide present in the vaccine.

Vaccine formulations generally include a total of about 10 to 5,000 ug of compound, more preferably about 100 to 1000 ug, even more preferably about 300 to 600 µg, conveniently in about 1.0 ml to 1.5 ml of carrier. The amount of compound representing any one isolate or clade present in the vaccine will vary depending on the immunogenicity of the compound. For example, a constrained helical peptide with sequences from some strains of HIV may be less immunogenic than those from the MN strain. If peptides representing two strains having different immunogenicity are used in combination, empirical titration of the amount of each virus would be performed to determine the percent of the peptide of each strain in the vaccine. For isolates having similar immunogenicity,approximately equal amounts of each isolate's peptide would be present in the vaccine. Methods of determining the relative amount of an immunogenic protein in multivalent vaccines are well known and have been used, for example, to determine relative proportions of various isolates in multivalent polio vaccines.

The vaccines are generally administered at 0, 1, and at 6, 8 or 12 months, depending on the protocol. A preferred protocol includes administration at 0, 1, 6, and 12 months. Following the immunization procedure, annual or bi-annual boosts can be administered. However, during the immunization process and thereafter, neutralizing antibody levels can be assayed and the protocol adjusted accordingly.

The vaccine is administeredto uninfected individuals. In addition, the vaccine can be administered to seropositive individuals to augment immune response to the virus.

Although the compounds described herein can be used as a vaccine as described above, the compounds can also be used alone or in combinations in the same type of formulation, for use as an immunogen, to induce antibodies that recognize the isolate(s) present in the immunogen. Immunogens are formulated in the same manner as vaccines and can include the same excipients, etc. Antibodies induced by the immunogens can be used in a diagnostic to detect the HIV strain in patient sera or body fluid samples, or to affinity purify the particular gp41 molecule or virus. The compounds also find use in diagnostic assays to detect the presence of antibodies in HIV in sera from individuals suspected of being infected.

In a further embodiment, the locked helix peptides of the invention are used to create constrained combinatorial peptide libraries. Combinatorial peptide libraries are uniquely suited to incorporate constrained peptides. The libraries are constructed with a "split synthesis" method in which a solid support (e.g. beads) is aliquoted equally and a differentamino acid is coupled separately to each portion. The portions are pooled, resplit and the process is repeated. In the "peptides-on-beads" technique, this process yields a mixture of beads, each of which is coupled to a peptide of unique sequence. The bead mixture can be used directly in a binding selection, with binding detected colorimetrically and positive beads physically removed from the mixture for microsequencing (Clackson and Wells, Tibtech, 12: 173-184 (1994)). To produce a library of peptides containing a random sequence of six (or more) amino acids locked into a helical conformation by *I* and *I+7* residues according to the invention, the split synthesis technique is modified to place *I* and *I+7* residues in set positions separated by six residues in each random amino acid sequence, and the peptides are cyclized by linking the side chain amide bond-fonning substituents of the *I* and *I*+7 residues in each peptide using any of the methods described in Section II below.

Combinatorial libraries containing the constrained peptides of the invention are a particularly powerful tool for identification of high affinity ligands in drug design. Given the prevalence of the α-helical motif in active sites of binding proteins, including DNA binding proteins, and the absence of amino acid sequence constraints in the invention's tethering system, the locked helix peptides of the invention greatly increase the utility of combinatorial peptide libraries in screening methods for specific binding activities, such as the methods of U.S. Pat. No. 5,306,619 used to screen for DNA sequence-specific binding molecules.

### II. Methods for Constructing Synthetic Locked Helix Peptides

According to the present method, an element of α-helical structure is removed from its context in a native protein by constructing a peptide with an amino acid sequence spanning the α-helical secondary structure of interest in the native protein, and constraining the short peptide into an α-helical conformation that mimics the α-helical secondary structure of interest. The present methods enable the practitioner to lock into a helical conformation any peptide that is six amino acids in length by placing an amino acid with a side chain amide bond-forming substitutent at the N-tcrminus of the peptide and placing another amino acid with a side chain amide bond-formingsubstitutentat the C-terminus of the peptide, and then joining the side chain amide bond-forming substituents of the N-terminal and C-terminal residues to form a cyclized structure which mimics the conformation of an α-helix. The present methods also enable the practitionerto lock into a helical conformation any sequence of six amino acid residues in a larger peptide by importing two residues with side chain amide bond-forming substituents into the N-terminal amino acid position and the C-terminal position amino acid position flanking the sequence (of six amino acid residues) of interest within a larger peptide, and then joining the side chain amide bond-forming substituents of the N-terminal and C-terminal flanking residues to form a cyclized structure which mimics the conformation of an α-helix.

There are at least two general methods for constructing the constrained helix peptides of the invention: (1) synthesis of a linear peptide comprising pair of residues that flank an amino acid sequence that is six residues in length, wherein the two flanking residues are independently selected from the group consisting of amino acid residues with side chain amide bond-forming substituents, followed by bridging the side chain amide bond-forming substituents of the flanking residues with a difunctional linker to cyclize the peptide: and (2) synthesis of a linear peptide comprising a pair of residues that flank an amino acid sequence that is six residues in length, wherein the two flanking residues are independently selected from the group consisting of amino acid residues with side chain amide bond-forming substituents, and wherein one of the flanking residues is added to the peptide chain carrying a difunctional linker such that one functional group of the linker is coupled to the residue's side chain amide bond-forming substitutent, followed by coupling of the linker's free functional group to the side chain amide bond-forming substitutent on the other flanking residue to cyclize the peptide.

Any amino acid that has a side chain containing a substitutent capable of forming an amide bond can be used as a flanking residue herein. Suitable flanking amino acid residues include amino acids with side chains carrying a free carboxy group, such as aminopropanedioic acid, Asp, Glu, 2-aminohexanedioic acid, and 2-aminoheptanedioic acid, and amino acids with side chains carrying a free amino group, such as 2,3-diaminopropanoicacid (2,3-diaminopropionicacid), 2,4-diaminobutanoicacid (2,4-diaminobutyricacid), 2,5-diaminopentanoic acid, and Lys.

### (1) Synthesis of Linear Peptide without Difunctional Linker-Coupled Flanking Amino Acid

### a. Peptide Synthesis

The desired peptide sequence is designed such that the sequence of six amino acid residues to be helicized extends between two flanking residues independently selected from the group consisting of amino acid residues with side chain amide bond-forming substituents. In one embodiment, the side chain amide bond-forming substituents of the N-terminal and C-terminal flanking residues are independently selected from the group consisting of a carboxy substitutent and an amino substitutent. In another embodiment, the side chain amide bond-formingsubstituents of the N-terminal and C-terminal flanking residues are both carboxy substituents. In yet another embodiment, the side chain amide bond-forming substitutent of one of the flanking residues is a carboxy substitutent and the side chain amide bond-forming substitutent of the other flanking residue is an amino substitutent. In still another embodiment, the side chain amide bond-forming substituents of the flanking residues are both amino substituents. In yet another embodiment, the flanking residues are independently selected from the group consisting of aminopropanedioic acid, Asp, Glu, 2-aminohexanedioicacid, 2-aminoheptanedioic acid, 2-aminooctanedioic acid, 2-aminononanedioic acid, 2,3-diaminopropanoic acid, 2,4-diaminobutanoicacid, 2,5-diaminopentanoicacid, Lys, 2,7-diaminoheptanoicacid, 2,8-diaminooctanoic acid, and 2,9-diaminononanoic acid.

In some embodiments, the desired peptide contains an additional amino acid or amino acids extending from the C-terminal flanking residue and/or N-terminal flanking residue.

Once the desired peptide sequence is selected, chemical synthesis can be employed to construct the constrained helix peptide of the invention. This can be accomplished by modifying any one of a number of methodologies well known in the art (see Kelley, R.F. & Winkler, M.E. in Genetic Engineering Principles and Methods, Setlow, J.K, ed., Plenum Press, N.Y., vol. 12, pp 1-19 (1990), Stewart, J.M. Young, J.D., Solid Phase Peptide Synthesis, Pierce Chemical Co., Rockford, IL (1984); see also U.S. Pat. Nos. 4,105,603; 3,972,859;3,842,067; and 3,862,925) to produce a the desired peptide.

Peptides of the invention can be conveniently prepared using solid phase peptide synthesis (Merrifield, J. Am. Chem. Soc., 85: 2149 (1964); Houghten, Proc. Natl. Acad. Sci. USA, 82: 5132 (1985). Solid phase synthesis begins at the carboxy terminus of the putative peptide by coupling a protected amino acid to an inert solid support. The inert solid support can be any macromolecule capable of serving as an anchor for the C-terminus of the initial amino acid. Typically, the macromolecular support is a cross-linked polymeric resin (e.g. a polyamide or polystyrene resin) as shown in Figures 1-1 and 1-2, on pages 2 and 4 of Stewart and Young, supra. In one embodiment, the C-terminal amino acid is coupled to a polystyrene resin to form a benzyl ester. A macromolecular support is selected such that the peptide anchor link is stable under the conditions used to deprotect the α-amino group of the blocked amino acids in peptide synthesis. If an base-labile α-protecting group is used, then it is desirable to use an acid-labile link between the peptide and the solid support. For example, an acid-labile ether resin is effective for base-labile Fmoc-amino acid peptide synthesis as described on page 16 of Stewart and Young, supra. Alternatively, a peptide anchor link and α-protecting group that are differentially labile to acidolysis can be used. For example, an aminomethyl resin such as the phenylacetamidomethyl (Pam) resin works well in conjunction with Boc-amino acid peptide synthesis as described on pages 11-12 of Stewart and Young, supra.

After the initial amino acid is coupled to an inert solid support, the α-amino protecting group of the initial amino acid is removed with, for example, trifluoroacetic acid (TFA) in methylene chloride and neutralizing in, for example, triethylamine(TEA). Following deprotection of the initial amino acid's α-amino group, the next α-amino and side chain protected amino acid in the synthesis is added. The remaining α-amino protected and, if necessary, side chain protected amino acids are then coupled sequentially in the desired order by condensation to obtain an intermediate compound connected to the solid support. Alternatively, some amino acids may be coupled to one another to form a fragment of the desired peptide followed by addition of the peptide fragment to the growing solid phase peptide chain.

The condensation reaction between two amino acids, or an amino acid and a peptide, or a peptide and a peptide can be carried out according to the usual condensation methods such as the axide method, mixed acid anhydride method, DCC (N,N'-dicyclohexylcarbodiimide) or DIC (N,N'-diisopropylcarbodiirnide) methods, active ester method, p-nitrophenyl ester method, BOP (benzotriazole-1-yl-oxy-tris [dimethylamino] phosphonium hexafluorophosphate)method, N-hydroxysuccinicacid imido ester method, etc, and Woodward reagent K method.

It is common in the chemical syntheses of peptides to protect any reactive side-chain groups of the amino acids with suitable protecting groups. Ultimately, these protecting groups are removed after the desired polypeptide chain has been sequentially assembled. Also common is the protection of the α-amino group on an amino acid or a fragment while that entity reacts at the carboxy group followed by the selective removal of the α-amino protecting group to allow subsequent reaction to take place at that location. Accordingly, it is common in polypeptide synthesis that an intermediate compound is produced which contains each of the amino acid residues located in the desired sequence in the peptide chain with various of these residues having side chain protecting groups attached. These protecting groups are then commonly removed at substantially the same time so as to produce the desired product following cleavage from the resin. Protecting groups and procedures for their use in peptide synthesis are reviewed in Protective Groups in Organic Synthesis, 2d ed., Greene. T.W. and Wuts, P.G.M., Wiley & Sons (New York: 1991).

Suitable protecting groups for α-amino and side chain amino groups are exemplified by benzyloxycarbonyl(abbreviated Z), isonicotinyloxycarbonyl (iNOC), o-chlorobenzyloxycarbonyl [Z(2CI)], p-nitrobenzyloxycarbonyl [Z(NO₂)], p-methoxybenzyloxycarbonyl [Z(OMe)], t-butoxycarbonyl (Boc), t-amyloxycarbonyl(Aoc), isobornyloxycarbonyl,adamantyloxycarbonyl,2-(4-biphenyl)-2-propyloxycarbonyl (Bpoc), 9-fluorenylmethoxycarbonyl(Fmoc), methylsulfonyethoxycarbonyl (Msc), trifluoroacetyl, phthalyl, formyl, 2-nitrophenylsulfenyl (NPS), diphenylphosphinothioyl (Ppt), and dimethylphosphinothioyl (Mpt) groups, and the like.

Protective groups for the carboxy functionalgroup are exemplified by benzyl ester, (Obz), cyclohexyl ester (Chx), 4-nitrobenzyl ester (Onb), t-butyl ester (Obut), 4-pyridylmethyl ester (Opic), and the like. It is often desirable that amino acids such as arginine, cysteine, and serine possessing a functional group other than amino and carboxy groups be protected by a suitable protecting group. For example, the guanidino group of arginine may be protected with nitro, p-toluenesulfonyl, benzyloxycarbonyl, adamantyloxycarbonyl, p-methoxybenzenesulfonyl, 4-methoxy-2, 6-dimethylbenzenesulfonyl (Nds), 1,3,5-ftimethylphenysulfonyl (Mts), and the like. The thiol group of cysteine can be protected with p-methoxybenzyl, trityl, and the like.

In one embodiment, the peptides of the invention are synthesized with the help of blocking groups that protect the side chain amide bond-forming substituents of the N-terminal and C-terminal flankingresidues. The protecting group or groups used for the side chain amide bond-formingsubstituents of the N-terminal and C-terminal flanking residues can be the same or different than the protecting group or groups used to block the side chain functional groups of other residues in the peptide. In a preferred embodiment, the protecting group or groups used to block the side chain amide bond-forining substituents is (are) differentially removable with respect to the protecting groups used for other side chain functional groups in the peptide, i.e. the side chain amide bond-forming substituents can be deprotected without deprotectingthe other side chain functional groups in the peptide, in addition to being differentially removable with respect to the α-amino protecting group used in peptide synthesis. In another preferred embodiment, the side chain amide bond-forming substituents of the flanking residues are orthogonally protected with respect to each other such that the side chain amide bond-formingsubstituent of one flanking residue can be deprotected without deprotecting the side chain amide bond-forming substituent of the other flanking residue.

Suitable protecting groups for use in orthogonally protecting the side chain amide bond-forming substituents of the flanking residues with respect to other functional groups and/or with respect to each other include pairs of differentially removable carboxy protective groups, such as a reduction-labile carboxy protective group, e.g. allyl or benzyl esters, paired with a base-labile carboxy protective group, e.g. fluorenylmethylesters, methyl or other primary alkyl esters. Fluorenylmethyl, methyl or other primary alkyl groups or other base-labile carboxy protective groups can be removed from their corresponding esters to yield a free carboxy group (without deprotecting allyl or benzyl esters or other reduction-labile esters) by saponificationof the esters with a suitable base such as piperidine and sodium hydroxide in a suitable solvent such as dimethylacetamide, or methanol and water, for a period of 10 to 120 minutes, and preferably 20 minutes, at 0 to 50°C. The allyl or benzyl or other reduction-labile esters can be removed when desired by reduction in the presence of a suitable transition metal catalyst, such as Pd(PPh₃)₄, Pd(PPh₃)₂Cl₂, Pd(OAc)₂ or Pd on carbon with a source of hydrogen, e.g. H₂ gas, in a suitable solvent such as dimethylacetamide, dimethylformamide. N-methylpyrrolidinoneor methanol for a period of 10 to 500 minutes, and preferably 100 minutes, at 0 to 50°C. For the sake of simplicity and convenience, all carboxy protective groups that are removable by Pd-catalyzed methods which result in the reduction of the protected carboxy substitutent are included in the term "reduction-labile protective groups" as used herein, even though such Pd-catalyzed deprotection methods may not result in the reduction of the protective group in question.

In embodimentswherein Pd catalysis involves the formation of intermediates of Pd derivatized with reduction-labile protecting groups, e.g. Pd-allyl derivatives, the Pd catalyst can be restored by reaction with a suitable nucleophile, such as piperidine or tributyltin hydride. When such reduction-labile groups are used to provide orthogonal protection in combination with base-labile protecting groups, it is preferable to either (1) utilize a synthetic scheme that calls for the removal of the base-labileprotecting groups before the removal of the reduction-labile protecting groups or (2) restore the Pd catalyst with a nucleophile that does not deprotect the base-labile protecting groups.

Alternatively, the carboxy substituents of the flanking residues can be orthogonally protected with respect to other functional groups and/or with respect to each other by using an acid-labile protecting group, such as a tertiary alkyl ester, e.g. t-butyl ester, in combination with a reduction-labile protecting group, such as the allyl or benzyl esters described above. The tertiary alkyl or other acid-labileester group can be removed by acidolysis, e.g. with trifluoroacetic acid in methylene chloride, and the allyl or benzyl or other reduction-labile esters can be removed by reduction in the presence of a transition metal catalyst as described above.

In another embodiment, the carboxy substituents of the flanking residues can be orthogonally protected with respect to other functional groups and/or with respect to each other by using a fluoride ion-labile protecting group, such as 2-(trimethylsilyl)ethyland silyl esters, in combination with a reduction-labile protecting group, such as the allyl or benzyl esters described above, or in combination with a base-labile protecting group, such as the fluorenylmethyl, methyl or other primary alkyl esters described above, without deprotectingthe reduction-labileor base-labile esters. The 2-(trimethylsilyl)ethyl,silyl or other fluoride-labile ester group can be removed by reaction with a suitable fluoride ion source, such as tetrabutylammonium fluoride in the presence of a suitable solvent, such as dimethylacetamide(DMA), dimethylformamide (DMF), tetrahydrofuran (THF), or acetonitrile.

Suitable protecting groups for use in orthogonally protecting the side chain amide bond-forming substituents of the flanking residues with respect to other functional groups and/or with respect to each other also include pairs of differentially removable amino protective groups, such as an allyloxycarbonyl or other reduction-labile amino protective group paired with a t-butoxycarbonyl (Boc) or other acid-labile amino protective group, and a reduction-labile amino protective group paired with a fluorenylmethoxycarbonyl (Fmoc) or other base-labile amino protective group. An allyloxycarbonyl (or other reduction-labile blocking group) protected amino group can be deprotected by reduction using a transition metal catalyst as in the procedures for removing reduction-labile carboxy protective groups described above, without deprotecting a Boc or Fmoc protected amino group. Likewise, an acid-labile amino protective group and a base-labile amino protective group can be removed by acidolysis and base saponification, respectively, without removing a reduction-labile amino protective group. For the sake of simplicity and convenience, all amino protective groups that are removable by Pd-catalyzed methods which result in the reduction of the protected amino substitutentare included in the term "reduction-labileprotective groups" as used herein, even though such Pd-catalyzed deprotection methods may not result in the reduction of the protective group in question.

In another embodiment, the amino substituents of the flanking residues can be orthogonally protected with respectto other functional groups and/or with respect to each other by using a fluoride-labile protecting group, such as 2-trimethylsilylethylcarbamate(Teoc), in combination with a reduction-labile protecting group, such as allyloxylcarbonyl, or in combination with a base-labile protecting group, such as fluorenylmethoxycarbonyl, as described above. The Teoc or other fluoride-labile group can be removed by reaction with a suitable fluoride ion source, such as tetrabutylammonium fluoride, as in the procedures for removal of fluoride-labile carboxy protective groups described above, without deprotecting an allyloxycarbonyl or fluorenylmethoxycarbonyl protected amino group. Likewise, a reduction-labile amino protective group and a base-labile amino protective group can be removed by reduction and base saponification, respectively, without removing a fluoride-labile amino protective group.

In embodiments that use a carboxy substituent as the side chain amide bond-forming substituent of one flanking residue and that use an amino substituent as the side chain amide bond-forming substituent of the other flanking residue, the carboxy substituent and the amino substituent can be orthogonally protected with respect to each other by using a reduction-labileprotecting group to block one substituent, e.g. allyl ester or allyloxycarbonyl,and a fluoride-labile,acid-labileor base-labile protecting group to block other substituent, e.g. silyl ester, t-butyl ester, fluorenylmethyl ester, Teoc, Boc, or Fmoc.

In a preferred embodiment, a reduction-labile protecting group is used to block the side chain amide bond-forming substituent of one flanking residue and the protecting group for the side chain amide bond-forming substituent of the other flanking residue is selected such that it provides orthogonal protection with respect to both the reduction-labile protecting group and the α-amino protecting group used in peptide synthesis. For example, in an embodiment using Fmoc chemistry for peptide synthesis, orthogonal protection of the side chain amide bond-forming substituents would be provided by a reduction-labile protecting group and an acid-labile protecting group. Likewise, in an embodiment using Boc chemistry for peptide synthesis, orthogonal protection of the side chain amide bond-forming substituents would be provided by a reduction-labile protecting group and a base-labile protecting group.

In yet another preferred embodiment, the side chain amide bond-formingsubstituentsof the flanking residues are orthogonally protected with respect to each other, with respect to α-amino protecting group used in peptide synthesis, and with respect to the protectinggroups used to block other side chain functional groups in the peptide chain.

In still another preferred embodiment, the side chain amide bond-forming substituents of the flanking residues are orthogonally protected with respect to each other, and with respect to α-amino protecting group used in peptide synthesis, but only one of the side chain amide bond-forming substituents is orthogonally protected with respect to the protecting groups used to block other side chain functional groups in the peptide chain. In this embodiment, it is preferable to use the side chain amide bond-forming substituent with fully orthogonal protection as the target for initial coupling of the peptide to the difunctional linker. Since the side chain amide bond-forming substituent with fully orthogonal protection can be deprotected without deprotecting other functional groups, the coupling reaction will be specific to the desired side chain amide bond-forming substituent, and will reduce the productionof unwanted peptide/difunctional linker derivatives. Although cyclization will require the deprotection of the side chain amide bond-forming substituent of the other flanking residue, and may cause concomitant deprotection of other side chain functional groups, unwanted derivatives are less likely to form given that the peptide chains are anchored to a solid support and that the linker length will regioselectively favor a coupling reaction between the unbound functional group of the linker and the side chain amide bond-forming substituent of the other flanking residue. If further peptide chain synthesis is desired after cyclization, any side chain functional groups on other amino acid residues left unprotected by the cyclization reactions can be reprotected before chain synthesis is resumed.

Many of the blocked amino acids described above can be obtained from commercial sources such as Novabiochem (San Diego, CA), Bachem CA (Torrence, CA) or Peninsula Labs (Belmont, CA).

In addition, the methods of the invention can be practiced in conjunction with solution phase peptide synthesis, for example, the solution phase peptide synthesis methods described in Principles of Peptide Synthesis, 2d ed, M. Bodanszky, Springer-Verlag (1993) or in The Practice of Peptide Synthesis, 2d ed, M. Bodanszky and A. Bodanszky, Springer-Verlag (1994). It will be appreciated that solution phase peptide synthesis methods can be easily modified to incorporate the desired flanking residues, with or without orthogonally-protected side chain amide bond-forming substituents, into the peptide chain of interest, using procedures similar to those used in the solid phase peptide synthesis methods described herein. It will be further appreciated that all references to peptide synthesis herein encompass both solid phase and solution (or liquid) phase peptide synthesis methods, unless otherwise indicated.

### b. Peptide Cyclization

After the desired amino acid sequence has been completed, the linear peptide is cyclized in order to constrain the peptide in a helical conformation. Any method of bridging the side chain amide bond-forming substituents of the flanking residues with a difunctional linker is suitable for producing the constrained helical peptides of the invention.

### (i) Selection of Difunctional Linker

Typically, the difunctional linker suitable for use herein is capable of presenting two functional groups separated by a distance of or about 5 Å to or about 30 Å, and preferably of or about 8 Å to or about 14 Å, and more preferably of or about 10 Å, such that the side chain amide bond-forming substituent of one of the flanking residues can form an amide linkage with one or either of the functional groups of the linker and the side chain amide bond-forming substituent of the other flanking residue can form an amide linkage with the remaining functional group of the linker. It will be appreciated that the nature of the molecular scaffold used to present the desired functional groups in the proper spatial relationship is not critical to the practice of the invention. Although straight chain and branched alkyl scaffolds are suitable for use herein, the invention is not so limited. For example, alkenyl, alkynyl, cycloalkyl, or other aliphatic hydrocarbon species, with or without heteroatoms, and monophenyl, biphenyl, naphthyl, and other aromatic hydrocarbon species, with or without heteroatoms, that are substituted with the desired functional groups in the proper spatial relationship (e.g. para- or meta-substitutions in ring structures such as monophenyl, biphenyl, naphthyl and the like) can be used to link the side chain amide bond-forming substituents of the flanking residues.

The functional groups used in the difunctional linker are selected such that they are capable of forming amide linkages with the side chain amide bond-forming substituents of the flanking residues used in the peptide to be cyclized. In embodiments wherein the side chain amide bond-forming substituent of each flanking residue is a carboxy substituent, the peptide can be conveniently cyclized with a diamine linker. In one example, the flanking residues and the diamine linker are selected according to Table 1 below. It will be appreciated that each of the flanking residues and linker molecules listed in Table 1 below is considered to representnot only the particular molecule correspondingto the given chemical name under IUPAC rules, but also any variant of the molecule containing additional substituents or modified substituents which do not prevent or substantially alter the functioning of the amino and/or carboxy groups contained in the molecule, which functioning is necessary for use of the molecule in the methods of the invention. Accordingly, each molecule listed will be understood to encompasses variant molecules containing alkenyl, alkynyl and other unsaturated bonds, heteroatoms, cycloalkyl substituents, aromatic substituents, or other substituents in the carbon backbone of the molecule, and/or variants containing the foregoing or other substituents or groups in place of hydrogen atoms on the carbon backbone of the molecule.

**Table 1**

| Item No. | Flanking Residue#1 | Flanking Residue#2 | Diamine Linker |
|---|---|---|---|
| 1 | aminopropanedioic acid | aminopropanedioic acid | 1,7-diaminoheptane; |
| | | | 1,8-diaminooctane; |
| | | | 1,9-diaminononane |
| | | | |
| 2 | aminopropanedioic acid | aspartic acid | 1,6-diaminohexane; |
| | | | 1,7-diaminoheptane; |
| | | | 1,8-diaminooctane |
| | | | |
| 3 | aminopropanedioic acid | glutamic acid | 1,5-diaminopentane; |
| | | | 1,6-diaminohexane; |
| | | | 1,7-diaminoheptane |
| | | | |
| 4 | aminopropanedioic acid | 2-aminohexanedioic acid | 1,4-diaminobutane; |
| | | | 1,5-diaminopentane; |
| | | | 1,6-diaminohexane |
| | | | |
| 5 | aminopropanedioic acid | 2-aminoheptanedioic acid | 1,3-diaminopropane; |
| | | | 1,4-diaminobutane; |
| | | | 1,5-diaminopentane |
| | | | |
| 6 | aminopropanedioic acid | 2-aminooctanedioic acid | 1,2-diaminoethane; |
| | | | 1,3-diaminopropane; |
| | | | 1,4-diaminobutane |
| | | | |
| 7 | aminopropanedioic acid | 2-aminononanedioic acid | 1,2-diaminoethane; |
| | | | 1,3-diaminopropane |
| | | | |
| 8 | aspartic acid | aspartic acid | 1,5-diaminopentane; |
| | | | 1,6-diaminohexane; |
| | | | 1,7-diaminoheptane |
| | | | |
| 9 | aspartic acid | glutamic acid | 1,4-diaminobutane; |
| | | | 1,5-diaminopentane; |
| | | | 1,6-diaminohexane |
| | | | |
| 10 | aspartic acid | 2-aminohexanedioic acid | 1,3-diaminopropane; |
| | | | 1,4-diaminobutane; |
| | | | 1,5-diaminopentane |
| | | | |
| 11 | aspartic acid | 2-aminoheptanedioic acid | 1,2-diaminoethane; |
| | | | 1,3-diaminopropane; |
| | | | 1,4-diaminobutane |
| | | | |
| 12 | aspartic acid | 2-aminooctanedioic acid | 1,2-diaminoethane; |
| | | | 1,3-diaminopropane; |
| | | | |
| 13 | glutamic acid | glutamic acid | 1,3-diaminopropane; |
| | | | 1,4-diaminobutane; |
| | | | 1,5-diaminopentane |
| | | | |
| 14 | glutamic acid | 2-aminohexanedioic acid | 1,2-diaminoethane; |
| | | | 1,3-diaminopropane; |
| | | | 1,4-diaminobutane |
| | | | |
| 15 | glutamic acid | 2-aminoheptanedioic acid | 1.2-diaminoethane; |
| | | | 1,3-diaminopropane |
| | | | |
| 16 | 2-aminohexanedioic | 2-aminohexanedioic acid | 1,2-diaminoethane; |
| | acid | | 1,3-diaminopropane |

In embodiments wherein the side chain amide bond-forming substituent of each flanking residue is an amino substituent, the peptide can be conveniently cyclized with a dicarboxylic acid linker. In one example, the flanking residues and the dicarboxylic acid linker are selected according to Table 2 below. It will be appreciated that each of the flanking residues and linker molecules listed in Table 2 below is considered to represent not only the particular molecule corresponding to the given chemical name under IUPAC rules, but also any variant of the molecule containing additional substituents or modified substituents which do not prevent or substantially alter the functioning of the amino and/or carboxy groups contained in the molecule, which functioning is necessary for use of the molecule in the methods of the invention. Accordingly, each molecule listed will be understood to encompasses variant molecules containing alkenyl, alkynyl and other unsaturated bonds, heteroatoms, cycloalkyl substituents, aromatic substituents, or other substituents in the carbon backbone of the molecule, and/or variants containing the foregoing or other substituents or groups in place of hydrogen atoms on the carbon backbone of the molecule.

**Table 2**

| Item No. | Flanking Residue#1 | Flanking Residue#2 | Dicarboxylic acid Linker |
|---|---|---|---|
| 1 | 2,3-diaminopropanoic acid | 2,3-diaminopropanoic acid | heptanedioic acid; |
| | | | octanedioic acid; |
| | | | nonanedioic acid |
| | | | |
| 2 | 2,3-diaminopropanoic acid | 2,4-diaminobutanoic acid | hexanedioic acid; |
| | | | heptanedioic acid; |
| | | | octanedioic acid |
| | | | |
| 3 | 2,3-diaminopropanoic acid | 2,5-diaminopentanoic acid | pentanedioic acid; |
| | | | hexanedioic acid; |
| | | | heptanedioic acid |
| | | | |
| 4 | 2,3-diaminopropanoic acid | lysine | butanedioic acid; |
| | | | pentanedioic acid; |
| | | | hexanedioic acid |
| | | | |
| 5 | 2,3-diaminopropanoic acid | 2,7-diaminoheptanoic acid | propanedioic acid; |
| | | | butanedioic acid; |
| | | | pentanedioic acid |
| | | | |
| 6 | 2,3-diaminopropanoic acid | 2,8-diaminooctanoic acid | ethanedioic acid; |
| | | | propanedioic acid; |
| | | | butanedioic acid |
| | | | |
| 7 | 2,3-diaminopropanoic acid | 2,9-diaminononanoic acid | ethanedioic acid; |
| | | | propanedioic acid |
| | | | |
| 8 | 2,4-diaminobutanoic acid | 2,4-diaminobutanoic acid | pentanedioic acid; |
| | | | hexanedioic acid; |
| | | | heptanedioic acid |
| | | | |
| 9 | 2,4-diaminobutanoic acid | 2,5-diaminopentanoic acid | butanedioic acid; |
| | | | pentanedioic acid; |
| | | | hexanedioic acid |
| | | | |
| 10 | 2,4-diaminobutanoic acid | lysine | propanedioic acid; |
| | | | butanedioic acid; |
| | | | pentanedioic acid |
| | | | |
| 11 | 2,4-diaminobutanoic acid | 2,7-diaminoheptanoic acid | ethanedioic acid; |
| | | | propanedioic acid; |
| | | | butanedioic acid |
| | | | |
| 12 | 2,4-diaminobutanoic acid | 2,8-diaminooctanoic acid | ethanedioic acid; |
| | | | propanedioic acid |
| | | | |
| 13 | 2,5-diaminopentanoic acid | 2,5-diaminopentanoic acid | propanedioic acid; |
| | | | butanedioic acid; |
| | | | pentanedioic acid |
| | | | |
| 14 | 2,5-diaminopentanoic acid | lysine | ethanedioic acid; |
| | | | propanedioic acid; |
| | | | butanedioic acid |
| | | | |
| 15 | 2,5-diaminopentanoic acid | 2,7-diaminoheptanoic acid | ethanedioic acid; |
| | | | propanedioic acid |
| | | | |
| 16 | lysine | lysine | ethanedioic acid; |
| | | | propanedioic acid |

In embodiments using an amino substituent as the side chain amide bond-forming substituent of one flanking residue and a carboxy substituent as the side chain amide bond-forming substituent of the other flanking residue, the peptide can be conveniently cyclized with an amino-substituted carboxylic acid (aminocarboxylicacid) linker. In one example, the flanking residues and the aminocarboxylic acid linker are selected according to Table 3 below. It will be appreciated that each of the flanking residues and linker molecules listed in Table 3 below is considered to represent not only the particular molecule corresponding to the given chemical name under IUPAC rules, but also any variant of the molecule containing additional substituentsor modified substituentswhich do not prevent or substantially alter the functioning of the amino and/or carboxy groups contained in the molecule, which functioning is necessary for use of the molecule in the methods of the invention. Accordingly, each molecule listed will be understood to encompasses variant molecules containing alkenyl, alkynyl and other unsaturated bonds, heteroatoms, cycloalkyl substituents, aromatic substituents, or other substituents in the carbon backbone of the molecule, and/or variants containing the foregoing or other substituents or groups in place of hydrogen atoms on the carbon backbone of the molecule.

**Table 3**

| Item No. | Flanking Residue# 1 | Flanking Residue#2 | Aminocarboxylic acid Linker |
|---|---|---|---|
| 1 | aminopropanedioic acid | 2,3-diaminopropanoic acid | 7-aminoheptanoic acid; |
| | | | 8-aminooctanoic acid; |
| | | | 9-aminononanoic acid |
| | | | |
| 2 | aminopropanedioic acid | 2,4-diaminobutanoic acid | 6-aminohexanoic acid; |
| | | | 7-aminoheptanoic acid; |
| | | | 8-aminooctanoic acid |
| | | | |
| 3 | aminopropanedioic acid | 2,5-diaminopentanoic acid | 5-aminopentanoic acid; |
| | | | 6-aminohexanoic acid; |
| | | | 7-aminoheptanoic acid |
| | | | |
| 4 | aminopropanedioic acid | 2,6-diaminohexanoic acid | 4-aminobutanoic acid; |
| | | | 5-aminopentanoic acid; |
| | | | 6-aminohexanoic acid |
| | | | |
| 5 | aminopropanedioic acid | 2,7-diaminoheptanoic acid | 3-aminopropanoic acid; |
| | | | 4-aminobutanoic acid; |
| | | | 5-aminopentanoic acid |
| | | | |
| 6 | aminopropanedioic acid | 2,8-diaminooctanoic acid | aminoethanoic acid; |
| | | | 3-aminopropanoic acid; |
| | | | 4-aminobutanoic acid |
| | | | |
| 7 | aminopropanedioic acid | 2,9-diaminononanoic acid | aminoethanoic acid; |
| | | | 3-aminopropanoic acid |
| | | | |
| 8 | aspartic acid | 2,3-diaminopropanoic acid | 6-aminohexanoic acid; |
| | | | 7-aminoheptanoic acid; |
| | | | 8-aminooctanoic acid |
| | | | |
| 9 | aspartic acid | 2,4-diaminobutanoic acid | 5-aminopentanoic acid; |
| | | | 6-aminohexanoic acid; |
| | | | 7-aminoheptanoic acid |
| | | | |
| 10 | aspartic acid | 2,5-diaminopentanoic acid | 4-aminobutanoic acid; |
| | | | 5-aminopentanoic acid; |
| | | | 6-aminohexanoic acid |
| | | | |
| 11 | aspartic acid | 2,6-diaminohexanoic acid | 3-aminopropanoic acid; |
| | | | 4-aminobutanoic acid; |
| | | | 5-aminopentanoic acid |
| | | | |
| 12 | aspartic acid | 2,7-diaminoheptanoic acid | aminoethanoic acid; |
| | | | 3-aminopropanoic acid; |
| | | | 4-aminobutanoic acid |
| | | | |
| 13 | aspartic acid | 2,8-diaminooctanoic acid | aminoethanoic acid; |
| | | | 3-aminopropanoic acid |
| | | | |
| 14 | glutamic acid | 2,3-diaminopropanoic acid | 5-aminoheptanoic acid; |
| | | | 6-aminohexanoic acid; |
| | | | 7-aminoheptanoic acid |
| | | | |
| 15 | glutamic acid | 2,4-diaminobutanoic acid | 4-aminobutanoic acid; |
| | | | 5-aminoheptanoic acid; |
| | | | 6-aminohexanoic acid |
| | | | |
| 16 | glutamic acid | 2,5-diaminopentanoic acid | 3-aminopropanoic acid; |
| | | | 4-aminobutanoic acid; |
| | | | 5-aminoheptanoic acid |
| | | | |
| 17 | glutamic acid | 2,6-diaminohexanoic acid | aminoethanoic acid; |
| | | | 3-aminopropanoic acid; |
| | | | 4-aminobutanoic acid |
| | | | |
| 18 | glutamic acid | 2,7-diaminoheptanoic acid | aminoethanoic acid; |
| | | | 3-aminopropanoic acid |
| | | | |
| 19 | 2-aminohexanedioic acid | 2,3-diaminopropanoic acid | 4-aminobutanoic acid; |
| | | | 5-aminoheptanoic acid; |
| | | | 6-aminohexanoic acid |
| | | | |
| 20 | 2-aminohexanedioic acid | 2,4-diaminobutanoic acid | 3-aminopropanoic acid; |
| | | | 4-aminobutanoic acid; |
| | | | 5-aminoheptanoic acid |
| | | | |
| 21 | 2-aminohexanedioic acid | 2,5-diaminopentanoic acid | aminoethanoic acid; |
| | | | 3-aminopropanoic acid; |
| | | | 4-aminobutanoic acid |
| | | | |
| 22 | 2-aminohexanedioic acid | lysine | aminoethanoic acid; |
| | | | 3-aminopropanoic acid |
| | | | |
| 23 | 2-aminoheptanedioic acid | 2,3-diaminopropanoic acid | 3-aminopropanoic acid; |
| | | | 4-aminobutanoic acid; |
| | | | 5-aminoheptanoic acid |
| | | | |
| 24 | 2-aminoheptanedioic acid | 2,4-diaminobutanoic acid | aminoethanoic acid; |
| | | | 3-aminopropanoic acid; |
| | | | 4-aminobutanoic acid |
| | | | |
| 25 | 2-aminoheptanedioic acid | 2,5-diaminopentanoic acid | aminoethanoic acid; |
| | | | 3-aminopropanoic acid; |
| | | | |
| 26 | 2-aminooctanedioic acid | 2,3-diaminopropanoic acid | aminoethanoic acid; |
| | | | 3-aminopropanoic acid; |
| | | | 4-aminobutanoic acid |
| | | | |
| 27 | 2-aminooctanedioic acid | 2,4-diaminobutanoic acid | aminoethanoic acid; |
| | | | 3-aminopropanoic acid |
| | | | |
| 28 | 2-aminononanedioic acid | 2,3-diaminopropanoic acid | aminoethanoic acid; |
| | | | 3-aminopropanoic acid |
| | | | |

### (ii) Cyclization Methods

Once the flanking residues and difunctional linker have been selected and the peptide chain spanning the flanking residues has been synthesized on solid phase, the difunctional linker can be used to cyclize the solid phase-boundpeptideby any convenient method. It will be appreciated that the invention encompasses methods of cyclizing a peptide after the finished peptide chain is fully synthesized, and methods of cyclizing the peptide at any point during peptide synthesis in which the peptide chain contains the flanking residues that are to be cross linked by the difunctional linker. Methods for cyclizing the peptide include (1) deprotecting the side chain amide bond-forming substituents of the flanking residues and reacting the solid phase peptide with the difunctional linker to simultaneously form amide linkages between the two functional groups of the linker and the side chain amide bond-forming substituents of both flanking residues; (2) deprotecting the side chain amide bond-forming substituent of only one of the flanking residues (without deprotectingthe side chain amide bond-forming substituent of the other flanking residue), reacting the difunctional linker with the solid phase peptide to form an amide linkage between one functional group on the linker and the side chain amide bond-forming substituent of the deprotected flanking residue, deprotectingthe side chain amide bond-forming substituent of the other flanking residue, and then intramolecularly reacting the free functional group on the linker and the side chain amide bond-forming substituent of the other flanking residue, thereby cyclizing the peptide; and (3) deprotectingthe side chain amide bond-forming substituents of both of the flanking residues, obtaining a monoprotected difunctional linker wherein only one of the linker's two amide bond-forming functional groups is capable of reacting with a counterpart side chain amide bond-forming substituent in a flanking residue, reacting the monoprotected, difunctional linker with the solid phase peptide to form an amide linkage between the free functional group on the linker and the side chain amide bond-forming substituent of one of the deprotected flanking residues, deprotecting the blocked functional group on the linker, and then intramolecularly reacting the free functional group on the linker and the side chain amide bond-forming substituent of the other flanking residue, thereby cyclizing the peptide. The orthogonal deprotection reactions, non-orthogonal deprotection reactions, and amide bond formation reactions can be performed as described in Section (B)(II)(1)(a) above.

In implementing the methods of the invention generally described as methods (2) and (3) above, it is desirable to use synthesis schemes that exploit the advantages of orthogonal protection and deprotection of functional groups to avoid formation of unwanted derivatives. It will be evident to the practitioner from the following representative synthetic schemes that the protecting groups for the side chain amide bond-forming substituents of the flanking residues, the method of peptide synthesis used, and the sequence of peptide cyclization reactions can be selected such that each of these components of the synthetic scheme increases the specificity of the reactions and improves yield of the desired product.

### (iii) Cyclization Using Diamine Linkers

In an example using carboxy substituents for the side chain amide bond-formingsubstituents of both flanking residues, a diamine linker for cyclization, and Fmoc chemistry for peptide synthesis, the carboxy substituents are orthogonally protected with respect to each other and with respect to the Fmoc-protected α-amino group of the N-terininal residue in the peptide chain by using an allyl group to protect the carboxy substituentof one flanking residue and a t-butyl ester to protect the carboxy substituent of the other flanking residue. In this example, the peptide can be cyclized by (1) using reduction to deprotect the allyl-protected carboxy substituent of one flanking residue (without deprotecting the t-butyl ester-protected carboxy substituent of the other flanking residue); (2) reacting an unprotected or monoprotected(e.g. allyloxycarbonyl-or Boc-monoprotected)diamine linker with the solid phase peptide to form an amide linkage between one of the linker's amino groups and the deprotected carboxy substituent:(3) using acidolysis to deprotect the t-butyl ester-protectedcarboxy substituent of the other flanking residue and deprotectthe Boc-protected amino group of the linker if a Boc-monoprotecteddiamine linker is used as the linker; (4) using reduction to deprotect the allyloxycarbonyl-protected amino group of the linker if an allyloxycarbonyl-monoprotected diamine linker is used as the linker; and (5) intramolecularly reacting the free carboxy substituent of the other flanking residue with the free amino group of the linker to form an amide linkage that cyclizes the peptide.

Alternatively, the peptide can be cyclized by (1) using acidolysis to deprotect the t-butyl ester-protected carboxy substituent of one flanking residue (without deprotecting the allyl-protected carboxy substituent of the other flanking residue); (2) reacting an unprotected or monoprotected (e.g. allyloxycarbonyl-or Boc-monoprotected)diamine linker with the solid phase peptide to form an amide linkage between one of the linker's amino groups and the deprotected carboxy substituent; (3) using reduction to deprotect the allyl-protected carboxy substituentof the other flankingresidue and deprotectthe allyloxycarbonyl-protectedamino group of the linker if an allyloxycarbonyl-monoprotected diamine linker is used as the linker; (4) using acidolysis to deprotect the Boc-protected amino group of the linker if a Boc-monoprotected diamine linker is used as the linker; and (5) intramolecularly reacting the free carboxy substituent of the other flanking residue with the free amino group of the linker to form an amide linkage that cyclizes the peptide.

In an example using carboxy substituents for the side chain amide bond-forming substituents of both flanking residues, a diamine linker for cyclization, and Boc chemistry for peptide synthesis, the carboxy substituents are orthogonally protected with respect to each other and with respect to the Boc-protected α-amino group of the N-terminal residue in the peptide chain by using an allyl group to protect the carboxy substituent of one flanking residue and a fluorenylmethyl (Fm) ester to protect the carboxy substituent of the other flanking residue. In this example, the peptide can be cyclized by (1) using reduction to deprotect the allyl-protected carboxy substituent of one flanking residue (without deprotecting the Fm ester-protected carboxy substituent of the other flanking residue); (2) reacting an unprotected or monoprotected (e.g. allyloxycarbonyl- or Fmoc-monoprotected) diamine linker with the solid phase peptide to form an amide linkage between one of the linker's amino groups and the deprotected carboxy substituent; (3) using base saponification to deprotect the Fm ester-protected carboxy substituent of the other flanking residue and deprotect the Fmoc-protected amino group of the linker if a Fmoc-monoprotected diamine linker is used as the linker; (4) using reduction to deprotect the allyloxycarbonyl-protected amino group of the linker if an allyloxycarbonyl-monoprotected diamine linker is used as the linker; and (5) intramolecularly reacting the free carboxy substituent of the other flanking residue with the free amino group of the linker to form an amide linkage that cyclizes the peptide.

Alternatively, the peptide can be cyclized by (1) using base saponification to deprotect the Fm ester-protected carboxy substituent of one flanking residue (without deprotecting the allyl-protected carboxy substituent of the other flanking residue); (2) reacting an unprotected or monoprotected (e.g. allyloxycarbonyl-or Fmoc-monoprotected) diamine linker with the solid phase peptide to form an amide linkage between one of the linker's amino groups and the deprotectedcarboxy substituent;(3) using reduction to deprotectthe allyl-protected carboxy substituentof the other flanking residue and deprotectthe allyloxycarbonyl-protectedamino group of the linker if an allyloxycarbonyl-monoprotected diamine linker is used as the linker; (4) using base saponification to deprotect the Fmoc-protected amino group of the linker if a Fmoc-monoprotected diamine linker is used as the linker; and (5) intramolecularly reacting the free carboxy substituent of the other flanking residue with the free amino group of the linker to form an amide linkage that cyclizes the peptide.

### (iv) Cyclization Using Dicarboxylic Acid Linkers

In an example using amino substituents for the side chain amide bond-forming substituents of both flanking residues, a dicarboxylic acid linker for cyclization, and Fmoc chemistry for peptide synthesis, the amino substituents are orthogonally protected with respect to each other and with respect to the Fmoc-protected α-amino group of the N-terminal residue in the peptide chain by using an allyloxycarbonyl group to protect the amino substituent of one flanking residue and a Boc group to protect the amino substituent of the other flanking residue. In this example, the peptide can be cyclized by (1) using reduction to deprotect the allyloxycarbonyl-protectedamino substituentof one flanking residue (without deprotecting the Boc-protected amino substituent of the other flanking residue); (2) reacting an unprotected or monoprotected (e.g. allyl- or t-butyl ester-monoprotected) dicarboxylic acid linker with the solid phase peptide to form an amide linkage between one of the linker's carboxy groups and the deprotected amino substituent; (3) using acidolysis to deprotect the Boc-protectedamino substituent of the other flanking residue, and to deprotect the t-butyl ester-protected carboxy group of the linker if a t-butyl ester-monoprotected dicarboxylic acid linker is used as the linker; (4) using reduction to deprotect the allyl-protected carboxy group of the linker if an allyl-monoprotected dicarboxylic acid linker is used as the linker; and (5) intramolecularly reacting the free amino substituent of the other flanking residue with the free carboxy group of the linker to form an amide linkage that cyclizes the peptide.

Alternatively, the peptide can be cyclized by (1) using acidolysis to deprotect the Boc-protected amino substituent of one flanking residue (without deprotecting the allyloxycarbonyl-protected amino substituentof the other flanking residue); (2) reacting an unprotected or monoprotected (e.g. allyl- or t-butyl ester-monoprotected)dicarboxylic acid linker with the solid phase peptide to form an amide linkage between one of the linker's carboxy groups and the deprotected amino substituent; (3) using reduction to deprotect the allyloxycarbonyl-protected amino substituentof the other flanking residue, and to deprotectthe allyl-protected carboxy group of the linker if an allyl-monoprotected dicarboxylic acid linker is used as the linker; (4) using acidolysis to deprotectthe t-butyl ester-protectedcarboxy group of the linker if a t-butyl ester-monoprotected dicarboxylic acid linker is used as the linker; and (5) intramolecularly reacting the free amino substituent of the other flanking residue with the free carboxy group of the linker to form an amide linkage that cyclizes the peptide.

In an example using amino substituents for the side chain amide bond-forming substituents of both flanking residues, a dicarboxylic acid linker for cyclization, and Boc chemistry for peptide synthesis, the amino substituents are orthogonally protected with respect to each other and with respect to the Boc-protected α-amino group of the N-terminal residue in the peptide chain by using an allyloxycarbonyl group to protect the amino substituentof one flanking residue and a Fmoc group to protect the amino substituent of the other flanking residue. In this example, the peptide can be cyclized by (1) using reduction to deprotect the allyloxycarbonyl-protected amino substituent of one flanking residue (without deprotecting the Fmoc-protected amino substituentof the other flanking residue); (2) reacting an unprotected or monoprotected (e.g. allyl- or Fm ester-monoprotected) dicarboxylic acid linker with the solid phase peptide to form an amide linkage between one of the linker's carboxy groups and the deprotected amino substituent: (3) using base saponification to deprotect the Fmoc-protected amino substituent of the other flanking residue, and to deprotect the Fm ester-protected carboxy group of the linker if a Fm ester-monoprotected dicarboxylic acid linker is used as the linker; (4) using reduction to deprotect the allyl-protected carboxy group of the linker if an allyl-monoprotecteddicarboxylic acid linker is used as the linker; and (5) intramolecularly reacting the free amino substituent of the other flanking residue with the free carboxy group of the linker to form an amide linkage that cyclizes the peptide.

Alternatively, the peptide can be cyclized by (1) using base saponification to deprotect the Fmoc-protected amino substituent of one flanking residue (without deprotecting the allyloxycarbonyl-protected amino substituentof the other flanking residue); (2) reacting an unprotected or monoprotected (e.g. allyl- or Fm ester-monoprotected) dicarboxylic acid linker with the solid phase peptide to form an amide linkage between one of the linker's carboxy groups and the deprotected amino substituent; (3) using reduction to deprotectthe allyloxycarbonyl-protectedamino substituentof the other flanking residue, and to deprotect the allyl-protected carboxy group of the linker if an allyl-monoprotected dicarboxylic acid linker is used as the linker; (4) using base saponificationto deprotectthe Fm ester-protectedcarboxy group of the linker if a Fmoc-monoprotected dicarboxylic acid linker is used as the linker; and (5) intramolecularly reacting the free amino substituent of the other flanking residue with the free carboxy group of the linker to form an amide linkage that cyclizes the peptide.

### (v) Cyclization Using Aminocarboxylic Acid Linkers

In an example using an amino substituent for the side chain amide bond-forming substituent of one flanking residue, a carboxy substituent for the side chain amide bond-formingsubstituent of the other flanking residue, an aminocarboxylic acid linker for cyclization, and Fmoc chemistry for peptide synthesis, the side chain amide bond-forming substituents of the flanking residues are orthogonally protected with respect to each other and with respectto the Fmoc-protected α-amino group of the N-terminal residue in the peptide chain by using an allyloxycarbonyl group to protect the amino substituent of one flanking residue and a t-butyl ester to protect the carboxy substituent of the other flanking residue. In this example, the peptide can be cyclized by (I) using reduction to deprotect the allyloxycarbonyl-protected amino substituent of one flanking residue (without deprotectingthe t-butyl ester-protectedcarboxy substituent of the other flanking residue); (2) reacting an unprotected or amino-protected (e.g. allyloxycarbonyl-protected amino or Boc-protected amino) aminocarboxylic acid linker with the solid phase peptide to form an amide linkage between the linker's carboxy group and the deprotected amino substituent; (3) using acidolysis to deprotect the t-butyl ester-protected carboxy substituent of the other flanking residue, and to deprotect the Boc-protected amino group of the linker if an aminocarboxylic acid with a Boc-protected amino group is used as the linker; (4) using reduction to deprotect the allyloxycarbonyl-protected amino group of the linker if an aminocarboxylic acid with an allyloxycarbonyl-protected amino group is used as the linker; and (5) intramolecularly reacting the free carboxy substituent of the other flanking residue and the free amino group of the aminocarboxylic acid linker to cyclize the peptide.

Alternatively, the peptide can be cyclized by (1) using acidolysis to deprotect the t-butyl ester-protected carboxy substituent of one flanking residue (without deprotecting the allyloxycarbonyl-protected amino substituent of the other flanking residue); (2) reacting an unprotected or carboxy-protected (e.g. allyl-or t-butyl ester-protectedcarboxy) aminocarboxylic acid linker with the solid phase peptide to form an amide linkage between the linker's amino group and the deprotected carboxy substituent; (3) using reduction to deprotectthe allyloxycarbonyl-protectedamino substituent of the other flanking residue, and to deprotect the allyl-protectedcarboxy group of the linker if an aminocarboxylic acid with an allyl-protected carboxy group is used as the linker; (4) using acidolysis to deprotect the t-butyl ester-protected carboxy group of the linker if an aminocarboxylic acid with a t-butyl ester-protected carboxy group is used as the linker; and (5) intramolecularlyreacting the free amino substituent of the other flanking residue and the free carboxy group of the aminocarboxylic acid linker to cyclize the peptide.

In another example using an amino substituent for the side chain amide bond-forming substituent of one flanking residue, a carboxy substituent for the side chain amide bond-forming substituent of the other flanking residue, an aminocarboxylic acid linker for cyclization, and Fmoc chemistry for peptide synthesis, the side chain amide bond-forming substituents of the flanking residues are orthogonally protected with respect to each other and with respect to the Fmoc-protected α-amino group of the N-terminal residue in the peptide chain by using a Boc group to protect the amino substituent of one flanking residue and an allyl group to protect the carboxy substituentof the other flanking residue. In this example, the peptide can be cyclized by (1) using acidolysis to deprotect the Boc-protected amino substituent of one flanking residue (without deprotectingthe allyl-protectedcarboxy substituent of the other flanking residue); (2) reacting an unprotected or amino-protected (e.g. allyloxycarbonyl-protected amino or Boc-protected amino) aminocarboxylic acid linker with the solid phase peptide to form an amide linkage between the linker's carboxy group and the deprotectedamino substituent; (3) using reduction to deprotect the allyl-protected carboxy substituent of the other flanking residue, and to deprotect the allyloxycarbonyl-protected amino group of the linker if an aminocarboxylic acid with a allyloxycarbonyl-protectedamino group is used; (4) using acidolysis to deprotect the Boc-protected amino group of the linker if an aminocarboxylic acid with an Boc-protected amino group is used as the linker; and (5) intramolecularly reacting the free carboxy substituentof the other flanking residue and the free amino group of the aminocarboxylic acid linker to cyclize the peptide.

Alternatively, the peptide can be cyclized by (1) using acidolysis to deprotect the Boc-protected amino substituent of one flanking residue (without deprotecting the allyl-protected carboxy substituent of the other flanking residue); (2) reacting an unprotected or amino-protected (e.g. allyloxycarbonyl-protected or Boc-protected amino) aminocarboxylic acid linker with the solid phase peptide to form an amide linkage between the linker's carboxy group and the deprotected amino substituent; (3) using reduction to deprotect the allyl-protected carboxy substituent of the other flanking residue, and to deprotect the allyloxycarbonyl-protected amino group of the linker if an aminocarboxylic acid with an allyloxycarbonyl-protected amino group is used as the linker; (4) using acidolysis to deprotectthe Boc-protected amino group of the linker if an aminocarboxylic acid with a Boc-protectedamino group is used as the linker; and (5) intramolecularly reacting the free carboxy substituent of the other flanking residue and the free amino group of the aminocarboxylic acid linker to cyclize the peptide.

In an example using an amino substituent for the side chain amide bond-forming substituent of one flanking residue, a carboxy substituent for the side chain amide bond-forming substituent of the other flanking residue, an aminocarboxylicacid linker for cyclization, and Boc chemistry for peptide synthesis, the side chain amide bond-forming substituents of the flanking residues are orthogonally protected with respect to each other and with respect to the Boc-protected α-amino group of the N-terminal residue in the peptide chain by using an allyloxycarbonyl group to protect the amino substituent of one flanking residue and a Fm ester to protect the carboxy substituentof the other flanking residue. In this example, the peptide can be cyclized by (1) using reduction to deprotect the allyloxycarbonyl-protected amino substituent of one flanking residue (without deprotecting the Fm ester-protected carboxy substituent of the other flanking residue); (2) reacting an unprotected or amino-protected (e.g. allyloxycarbonyl-protected amino or Fmoc-protected amino) aminocarboxylic acid linker with the solid phase peptide to form an amide linkage between the linker's carboxy group and the deprotectedamino substituent; (3) using base saponification to deprotect the Fm ester-protected carboxy substituentof the other flanking residue, and to deprotect the Fmoc-protected amino group of the linker if an aminocarboxylic acid with a Fmoc-protected amino group is used as the linker; (4) using reduction to deprotect the allyloxycarbonyl-protected amino group of the linker if an aminocarboxylic acid with an allyloxycarbonyl-protected amino group is used as the linker; and (5) intramolecularly reacting the free carboxy substituent of the other flanking residue and the free amino group of the aminocarboxylic acid linker to cyclize the peptide.

Alternatively, the peptide can be cyclized by (1) using base saponification to deprotect the Fm ester-protected carboxy substituent of one flanking residue (without deprotecting the allyloxycarbonyl-protected amino substituentof the other flanking residue); (2) reacting an unprotected or carboxy-protected (e.g. allyl-or Fm ester-protected carboxy) aminocarboxylic acid linker with the solid phase peptide to form an amide linkage between the linker's amino group and the deprotected carboxy substituent; (3) using reduction to deprotectthe allyloxycarbonyl-protectedamino substituentof the other flanking residue, and to deprotect the allyl-protectedcarboxy group of the linker if an aminocarboxylic acid with an allyl-protected carboxy group is used as the linker; (4) using base saponification to deprotect the Fm ester-protected carboxy group of the linker if an aminocarboxylic acid with a Fm ester-protected carboxy group is used as the linker; and (5) intramolecularly reacting the free amino substituent of the other flanking residue and the free carboxy group of the aminocarboxylic acid linker to cyclize the peptide.

In another example using an amino substituent for the side chain amide bond-forming substituent of one flanking residue, a carboxy substituent for the side chain amide bond-forming substituent of the other flanking residue, an aminocarboxylic acid linker for cyclization, and Boc chemistry for peptide synthesis, the side chain amide bond-forming substituents of the flanking residues are orthogonally protected with respect to each other and with respect to the Boc-protected α-amino group of the N-terminal residue in the peptide chain by using a Fmoc group to protect the amino substituent of one flanking residue and an allyl group to protect the carboxy substituentof the other flanking residue. In this example, the peptide can be cyclized by (1) using base saponification to deprotect the Fmoc-protected amino substituent of one flanking residue (without deprotecting the allyl-protected carboxy substituent of the other flanking residue); (2) reacting an unprotected or amino-protected (e.g. allyloxycarbonyl-protected amino or Fmoc-protected amino) aminocarboxylic acid linker with the solid phase peptide to form an amide linkage between the linker's carboxy group and the deprotected amino substituent; (3) using reduction to deprotect the allyl-protected carboxy substituentof the other flanking residue, and to deprotectthe allyloxycarbonyl-protectedamino group of the linker if an aminocarboxylic acid with a allyloxycarbonyl-protectedamino group is used; (4) using base saponification to deprotectthe Fmoc-protected amino group of the linker if an aminocarboxylic acid with an Fmoc-protected amino group is used as the linker; and (5) intramolecularly reacting the free carboxy substituent of the other flanking residue and the free amino group of the aminocarboxylicacid linker to cyclize the peptide.

Alternatively, the peptide can be cyclized by (1) using reduction to deprotect the allyl-protected carboxy substituent of one flanking residue (without deprotecting the Fmoc-protected amino substituent of the other flanking residue); (2) reacting an unprotected or carboxy-protected (e.g. allyl-protected or Fm ester-protected carboxy) aminocarboxylicacid linker with the solid phase peptide to form an amide linkage between the linker's amino group and the deprotected carboxy substituent; (3) using base saponification to deprotect the Fmoc-protected amino substituent of the other flanking residue, and to deprotect the Fm ester-protected carboxy group of the linker if an aminocarboxylicacid with a Fm ester-protectedcarboxy group is used as the linker; (4) using reduction to deprotect the allyl-protected carboxy group of the linker if an aminocarboxylic acid with an allyl-protected carboxy group is used as the linker; and (5) intramolecularly reacting the free amino substituent of the other flanking residue and the free carboxy group of the aminocarboxylic acid linker to cyclize the peptide.

In yet another embodiment using an amino substituent for the side chain amide bond-forming substituent of one flanking residue, a carboxy substituent for the side chain amide bond-forming substituent of the other flanking residue, an aminocarboxylic acid linker for cyclization, and Fmoc chemistry for peptide synthesis, the regioselectivity of the cyclization procedure is provided by orthogonally protecting the side chain amide bond-forming substituents of the flanking residues with respect to the Fmoc-protected α-amino group of the N-terminal residue in the peptide chain but not with respect to each other, and orthogonally protecting one of the aminocarboxylic acid linker's functional groups with respect to the Fmoc-protected α-amino group of the N-terminal residue in the peptide chain.

In an example of the foregoing embodiment using an allyloxycathonyl-protected amino substituent as the side chain amide bond-forming substituent of one flanking residue, an allyl-protected carboxy substituent as the side chain amide-bond forming substituent of the other flanking residue, a monoprotected aminocarboxylic acid linker, and Fmoc chemistry for peptide synthesis, the peptide can be cyclized by (1) using reduction to orthogonally deprotect the side chain amide bond-forming substituents of the flanking residues (without deprotecting the Fmoc-protected α-amino group of the N-terminal residue in the peptide chain); (2) reacting a carboxy-protected(e.g. allyl- or t-butyl ester protected carboxy) or amino-protected (e.g. allyloxycarbonyl-or Boc-protectedamino) aminocarboxylic acid linker with the solid phase peptide to form an amide linkage between the unprotected functional group of the linker and the corresponding side chain amide bond-forming substituent on one of the flanking residues; (3) using reduction or acidolysis, as appropriate, to deprotectthe protected functional group of the linker; and (4) intramolecularly reacting the free side chain amide bond-forming substituent of the other flanking residue and the free functional group of the linker to cyclize the peptide.

In an example of the foregoing embodimentusing a Boc-protectedamino substituent as the side chain amide bond-forming substituent of one flanking residue, a t-butyl ester-protected carboxy substituent as the side chain amide-bond forming substituent of the other flanking residue, a monoprotected aminocarboxylic acid linker, and Fmoc chemistry for peptide synthesis, the peptide can be cyclized by (1) using acidolysis to orthogonally deprotect the side chain amide bond-forming substituents of the flanking residues (without deprotectingthe Fmoc-protected α-amino group of the N-terminal residue in the peptide chain); (2) reacting a carboxy-protected(e.g. allyl or t-butyl ester-protected carboxy) or amino-protected (e.g. allyloxycarbonyl-or Boc-protected amino) aminocarboxylic acid linker with the solid phase peptide to form an amide linkage between the unprotected functional group of the linker and the corresponding side chain amide bond-forming substituent on one of the flanking residues; (3) using reduction or acidolysis, as appropriate, to deprotect the protected functional group of the linker; and (4) intramolecularly reacting the free side chain amide bond-forming substituent of the other flanking residue and the free functional group of the linker to cyclize the peptide.

In still another embodiment using an amino substituent for the side chain amide bond-forming substituentof one flanking residue, a carboxy substituent for the side chain amide bond-forming substituent of the other flanking residue, an aminocarboxylic acid linker for cyclization, and Boc chemistry for peptide synthesis, the regioselectivity of the cyclization procedure is provided by orthogonally protecting the side chain amide bond-forming substituents of the flanking residues with respect to the Boc-protected α-amino group of the N-terminal residue in the peptide chain but not with respect to each other, and orthogonally protecting one of the aminocarboxylic acid linker's functional groups with respect to the Boc-protected α-amino group of the N-terminal residue in the peptide chain.

In an example of the foregoing embodiment using an allyloxycarbonyl-protected amino substituent as the side chain amide bond-forming substituent of one flanking residue, an allyl-protected carboxy substituent as the side chain amide-bond forming substituentof the other flanking residue, an aminocarboxylic acid linker, and Boc chemistry for peptide synthesis, the peptide can be cyclized by (I) using reduction to orthogonal ly deprotect the side chain amide bond-forming substituents of the flanking residues (without deprotecting the Boc-protectedα-amino group of the N-terminal residue in the peptide chain); (2) reacting a carboxy-protected (e.g. allyl- or Fm ester-protected carboxy) or amino-protected (e.g. allyloxycarbonyl- or Fmoc-protected amino) aminocarboxylic acid linker with the solid phase peptide to form an amide linkage between the unprotected functional group of the linker and the corresponding side chain amide bond-forming substituent on one of the flanking residues; (3) using reduction or base saponification, as appropriate, to deprotect the protected functional group of the linker; and (4) intramolecularly reacting the free side chain amide bond-forming substituent of the other flanking residue and the free functional group of the linker to cyclize the peptide.

In an example of the foregoing embodiment using a Fmoc-protected amino substituent as the side chain amide bond-forming substituent of one flanking residue, a Fm ester-protectedcarboxy substituent as the side chain amide-bond forming substituentof the other flanking residue, an aminocarboxylic acid linker, and Boc chemistry for peptide synthesis, the peptide can be cyclized by (1) using base saponification to orthogonally deprotect the side chain amide bond-forming substituents of the flanking residues (without deprotectingthe Boc-protected α-amino group of the N-terminal residue in the peptide chain); (2) reacting a carboxy-protected (e.g. allyl- or Fm ester-protected carboxy) or amino-protected (e.g. allyloxycarbonyl- or Fmoc-protected amino) aminocarboxylic acid linker with the solid phase peptide to form an amide linkage between the unprotected functional group of the linker and the corresponding side chain amide bond-forming substituent on one of the flanking residues; (3) using reduction or base saponification, as appropriate, to deprotect the protected functional group of the linker; and (4) intramolecularly reacting the free side chain amide bond-forming substituent of the other flanking residue and the free functional group of the linker to cyclize the peptide.

Following cyclization, the helix-constrained peptide is optionally cleaved away from the solid support, recovered and purified. The peptide can be removed from the solid support by a reagent capable of disrupting the peptide-solid phase link, and optionally deprotecting blocked side chain functional groups on the peptide. In one embodiment, the peptide is cleaved away from the solid phase by acidolysis with liquid hydrofluoric acid (HF), which also removes any remaining side chain protective groups. Preferably, in order to avoid alkylation of residues in the peptide (for example, alkylation of methionine, cysteine, and tyrosine residues), the acidolysis reaction mixture contains thio-cresoland cresol scavengers. Following HF cleavage, the resin is washed with ether, and the free peptide is extracted from the resin with sequential washes of acetic acid solutions. The combined washes are lyophilized, and the residue is purified.

### c. Liquid Phase Cyclization

Alternatively, the peptide can be cleaved away from the solid support prior to the cyclization step. In one embodiment, after the difunctional linker is coupled to the side chain amide bond-forming substituent of the first flanking residue in the peptide, the peptide is cleaved away from the solid support. The peptide is recovered, deblocked at the side chain amide bond-forming substituent of the second flanking residue (if necessary), and then cyclized at low concentration in a reaction mixture to maximize intramolecular amide bond formation. Typically, a maximum level of intramolecularamide bond formation can be achieved under conditions in which the concentration of the peptide provides an intramolecular concentration of free carboxy and amino groups that exceeds the intermolecular concentration of free carboxy and amino groups in the reaction mixture. In one embodiment, a peptide concentration of 1 nM to 1 M, and preferably 1 µM to 1 mM, and more preferably 1 µM to 100 µM, is used to maximize cyclization. The cyclization of free peptide can be conducted with any of the amino acid coupling reactions used to helicize peptide bound to a solid support described above.

### d. Synthetic Schemes

In one embodiment, any helix constrained compound of formulas (1), (1a), (1b), (1c), (1d), (1e), (1f), and (1g) is made by utilizing (in peptide synthesis as described in Section (B)(II)(I)(a) above) the particular combination of flanking residues and diamine linker shown in Table 1 above that provides the values of n, m and p characterizing the compound of interest, and cyclizing the resulting peptide according to the methods described in Section (B)(II)(l)(b)(ii) or (iii) above. For example, any compound of formulas (1), (1a), (1b), (1c), (1d), (1e), (1f), and (1g) characterizedby m=0, p=0, and n=7, 8, or 9 can be made by utilizing (in peptide synthesis as described in Section (B)(II)(1)(a) above) the flanking residues and any diamine linker listed in Item No. 1 in Table 1 above, and cyclizing the resulting peptide according to the methods described in Section (B)(II)(1)(b)(ii)or (iii) above. In another example, any compound of formulas (1), (1a), (1b), (1c), (1d), (1e), (1f), and (1g) characterized by m=0, p=6, and n=2 or 3, or characterized by m=6, p=0, and n=2 or 3, can be made by utilizing (in peptide synthesis as described in Section (B)(II)(1)(a) above) the flanking residues and any diamine linker listed in Item No. 7 in Table 1 above, and cyclizing the resulting peptide according to the methods described in Section (B)(II)(1)(b)(ii) or (iii) above. In yet another example, any compound of formulas (1), (1a), (1b), (1c), (1d), (1e), (1f), and (1g) characterized by m=3, p=3, and n=2 or 3, can be made by utilizing (in peptide synthesis as described in Section (B)(II)(1)(a) above) the flanking residues and any diamine linker listed in Item No. 16 in Table 1 above, and cyclizing the resulting peptide according to the methods described in Section (B)(II)(1)(b)(ii) or (iii) above.

In another embodiment, any helix constrained compound of formulas (2), (2a), (2b), (2c), (2d), (2e), (2f), (2g), (3), (3a), (3b), (3c), (3d), (3e), (3f), and (3g) is made by utilizing (in peptide synthesis as described in Section (B)(11)(1)(a) above) the flanking residues and any diamine linker listed in Item No. 9 in Table 1 above, and cyclizing the resulting peptide according to the methods described in Section (B)(II)(1)(b)(ii) or (iii) above.

In another embodiment, any helix constrained compound of formulas (4), (4a), (4b), (4c), (4d), (4e), (4f), and (4g) is made by utilizing (in peptide synthesis as described in Section (B)(II)(1)(a) above) the flanking residues and any diamine linker listed in Item No. 13 in Table 1 above, and cyclizing the resulting peptide according to the methods described in Section (B)(II)(1)(b)(ii) or (iii) above.

In another embodiment, any helix constrained compound of formulas (5), (5a), (5b), (5c), (5d), (5e), (5f), and (5g) is made by utilizing (in peptide synthesis as described in Section (B)(II)(1)(a) above) the flanking residues and any diamine linker listed in Item No. 8 in Table 1 above, and cyclizing the resulting peptide according to the methods described in Section (B)(II)(1)(b)(ii) or (iii) above.

In another embodiment, any helix constrained compound of formulas (6), (6a), (6b), (6c), (6d), (6e), (6f), (6g), (11), (11a), (11b), (11c), (11d), (11e), (11f), and (11g) is made by utilizing (in peptide synthesis as described in Section (B)(II)(1)(a)above) the particular combination of flanking residues and aminocarboxylic acid linker shown in Table 3 above that provides the values of q, r and s characterizing the compound of interest, or the values oft, u and v characterizing the compound of interest; as appropriate, and cyclizing the resulting peptide according to the methods described in Section (B)(II)(1)(b)(ii) or (v) above. For example, any compound of formulas (6), (6a), (6b), (6c), (6d), (6e), (6f), (6g), (11), (11a), (11b), (11c), (11d), (11e), (11f), and (11g) characterized by q=1, s=0, and r=6,7, or 8, or characterized by t=0, v=1, and u=6, 7, or 8, as appropriate, can be made by utilizing (in peptide synthesis as described in Section (B)(II)(1)(a) above) the flanking residues and any aminocarboxylic acid linker listed in Item No. 1 in Table 3 above, and cyclizing the resulting peptide according to the methods described in Section (B)(II)(11)(b)(ii) or (v) above.

In another example, any compound of formulas (6), (6a), (6b), (6c), (6d), (6e), (6f), (6g), (11), (11a), (11b), (11c), (11d), (11e), (11f), and (11g) characterized by q=1, s=6, and r=tor 2, or characterized by t=6, v=1, and u=1 or 2. as appropriate, can be made by utilizing (in peptide synthesis as described in Section (B)(II)(1)(a)above) the flanking residues and any aminocarboxylic acid linker listed in Item No. 28 in Table 3 above, and cyclizing the resulting peptideaccordingto the methods described in Section (B)(II)(1)(b)(ii) or (v) above.

In another example, any compound of formulas (6), (6a), (6b), (6c), (6d), (6e), (6f), (6g), (11), (11a), (11b), (11c), (11d), (11e), (11f), and (11g) characterized by q=7, s=0, and r=lor 2, or characterized by t=0, v=7, and u=1 or 2, as appropriate, can be made by utilizing (in peptide synthesis as described in Section (B)(II)(1)(a) above) the flanking residues and any aminocarboxylic acid linker listed in Item No. 7 in Table 3 above, and cyclizing the resulting peptide according to the methods described in Section (B)(II)(1)(b)(ii) or (v) above.

In another example, any compound of formulas (6), (6a), (6b), (6c), (6d), (6e), (6f), (6g), (11), (11a), (11b), (11c), (11d), (11e), (11f), and (11g) characterized by q=3, s=4, and r=1 or 2, or characterized by t=4, v=3, and u=1 or 2, as appropriate, can be made by utilizing (in peptide synthesis as described in Section (B)(II)(1)(a)above) the flanking residues and any aminocarboxylic acid linker listed in Item No. 25 in Table 3 above, and cyclizing the resulting peptide according to the methods described in Section (B)(II)(l)(b)(ii) or (v) above.

In another embodiment, any helix constrained compound of formulas (7), (7a), (7b), (7c), (7d), (7e), (7f), (7g), (13), (13a), (13b), (13c), (13d), (13e), (13f), and (13g) is made by utilizing (in peptide synthesis as described in Section (B)(II)(1)(a) above) the flanking residues and any aminocarboxylic acid linker listed in Item No. 14 in Table 3 above, and cyclizing the resulting peptide according to the methods described in Section (B)(II)(1)(b)(ii) or (v) above.

In another embodiment, any helix constrained compound of formulas (8), (8a), (8b), (8c), (8d), (8e), (8f), (8g), (12), (12a), (12b), (12c), (12d), (12e), (12f), and (12g) is made by utilizing (in peptide synthesis as described in Section (B)(II)(1)(a) above) the flanking residues and any aminocarboxylic acid linker listed in Item No. 9 in Table 3 above, and cyclizing the resulting peptide according to the methods described in Section (B)(II)(1)(b)(ii) or (v) above.

In another embodiment, any helix constrained compound of formulas (9), (9a), (9b), (9c), (9d), (9e), (9f), (9g), (15), (15a), (15b), (15c), (15d), (15e), (15f), and (15g) is made by utilizing (in peptide synthesis as described in Section (B)(II)(1)(a) above) the flanking residues and any aminocarboxylic acid linker listed in Item No. 15 in Table 3 above, and cyclizing the resulting peptide according to the methods described in Section (BxII)(1)(b)(ii) or (v) above.

In another embodiment, any helix constrained compound of formulas (10), (10a), (10b), (10c), (10d), (10e), (10f), (10g), (14), (14a), (14b), (14c), (14d), (14e), (14f), and (14g) is made by utilizing (in peptide synthesis as described in Section (BxII)(1)(a) above) the flanking residues and any aminocarboxylic acid linker listed in Item No. 8 in Table 3 above, and cyclizing the resulting peptide according to the methods described in Section (B)(II)(1)(b)(ii) or (v) above.

In one embodiment, any helix constrained compound of formulas (16), (16a), (16b), (16c), (16d), (16e), (16f), and (16g) is made by utilizing (in peptide synthesis as described in Section (B)(II)(1)(a) above) the particular combination of flanking residues and dicarboxylic acid linker shown in Table 2 above that provides the values of w, x and y characterizing the compound of interest, and cyclizing the resulting peptide according to the methods described in Section (B)(II)(1)(b)(ii)or (iv) above. For example, any compound of formulas (16), (16a), (16b), (16c), (16d), (16e), (16f), and (16g) characterized by w=1, y=1, and x=5, 6, or 7 can be made by utilizing (in peptide synthesis as described in Section (B)(11)(1)(a) above) the flanking residues and any dicarboxylic acid linker listed in Item No. 1 in Table 2 above, and cyclizing the resulting peptide according to the methods described in Section (B)(II)(1)(b)(ii) or (iv) above. In another example, any compound of formulas (16),(16a), (16b), (16c), (16d), (16e), (16f), and (16g) characterized by w=1, y=7, and x=0 or 1, or characterized by w=7, y=1, and x=0 or 1, can be made by utilizing (in peptide synthesis as described in Section (B)(II)(1)(a)above) the flanking residues and any dicarboxylic acid linker listed in Item No. 7 in Table 2 above, and cyclizing the resulting peptide according to the methods described in Section (B)(II)(1)(b)(ii) or (iv) above. In yet another example, any compound of formulas (16), (16a), (16b), (16c), (16d), (16e), (16f), and (16g) characterized by w=4, y=4, and x=0 or 1, can be made by utilizing (in peptide synthesis as described in Section (B)(II)(1)(a) above) the flanking residues and any dicarboxylic acid linker listed in Item No. 16 in Table 2 above, and cyclizing the resulting peptide according to the methods described in Section (B)(II)(1)(b)(ii) or (iv) above.

In another embodiment, any helix constrained compound of formulas (17), (17a), (17b), (17c), (17d), (17e), (17f), (17g), (18), (18a), (18b), (18c), (18d), (18e), (18f), and (18g) is made by utilizing (in peptide synthesis as described in Section (B)(II)(1)(a) above) the flanking residues and any dicarboxylic acid linker listed in Item No. 2 in Table 2 above, and cyclizing the resulting peptide according to the methods described in Section (B)(II)(1)(b)(ii) or (iv) above.

In another embodiment, any helix constrained compound of formulas (19), (19a), (19b), (19c), (19d), (19e), (19f), and (19g) is made by utilizing (in peptide synthesis as described in Section (B)(II)(1)(a) above) the flanking residues and any dicarboxylic acid linker listed in Item No. 1 in Table 2 above, and cyclizing the resulting peptide according to the methods described in Section (B)(II)(1)(b)(ii) or (iv) above.

In another embodiment, any helix constrained compound of formulas (20), (20a), (20b), (20c), (20d), (20e). (20f), and (20g) is made by utilizing (in peptide synthesis as described in Section (B)(II)(1)(a) above) the flanking residues and any dicarboxylic acid linker listed in Item No. 8 in Table 2 above, and cyclizing the resulting peptide according to the methods described in Section (B)(II)(1)(b)(ii) or (iv) above.

### (2) Synthesis of Linear Peptide with Difunctional Linker-Coupled Flanking Amino Acid

The peptide is designed such that the sequence to be helicized comprises an amino acid sequence that is six residues in length that extends between flanking residues as described in Section (B)(II)(1)(a) above. The peptide can be constructed using a modification of the solid phase synthesis methods described in Section (B)(II)(1)(a)above wherein one of the flanking residues is coupled to a difunctional linker before addition to the peptide chain. This allows the linker to be incorporated into the peptide as part of a standard amino acid.

The flanking residue can be coupled to the difunctional linker by any convenient means. Typically, the side chain amide bond-forming substituent of the flanking residue is used to form an amide linkage with one of the functional groups on the linker. In one embodiment designed for use in conjunction with Fmoc chemistry, the linker-derivatizedflanking residue is created by obtaining from a commercial source an amino acid residue with an Fmoc-protected α-amino substituent, a t-butyl ester-protected α-carboxy substituent, and an unprotected side chain amino substituent, and then reacting the α-substituent protected amino acid with a difunctional linker having a free carboxy group to form an amide linkage between the linker's free carboxy group and the unprotected side chain amino substituent of the amino acid using any of the condensation methods described in Section (B)(II)(1)(a) above. The t-butyl ester-protected α-carboxy substituent of the derivatizedamino acid residue is then removed by acidolysis to permit incorporationof the derivatized amino acid into the peptide chain.

In another embodiment designed for use in conjunction with Fmoc chemistry, the linker-derivatized flanking residue is created by obtaining from a commercial source an amino acid residue with an Fmoc-protected α-amino substituent, an allyl-protectedα-carboxy substituent, and an unprotected side chain amino substituent,and then reacting the α-substituent protected amino acid with a difunctional linker having a free carboxy group to form an amide linkage between the linker's free carboxy group and the unprotected side chain amino substituent of the amino acid using any of the condensation methods described in Section (B)(II)(1)(a) above. The allyl-protected α-carboxy substituent of the derivatized amino acid residue is then removed by reduction to permit incorporation of the derivatized amino acid into the peptide chain.

In one embodiment designed for use in conjunction with Boc chemistry, the linker-derivatized flanking residue is created by obtaining from a commercial source an amino acid residue with an Boc-protected α-amino substituent, a Fm ester-protected α-carboxy substituent, and an unprotected side chain amino substituent, and then reacting the α-substituent protected amino acid with a difunctional linker having a free carboxy group to form an amide linkage between the linker's free carboxy group and the unprotected side chain amino substituent of the amino acid using any of the condensation methods described in Section (B)(II)(1)(a) above. The Fm ester-protected α-carboxy substituent of the derivatized amino acid residue is then removed by base saponification to permit incorporation of the derivatized amino acid into the peptide chain.

In another embodiment designed for use in conjunction with Boc chemistry, the linker-derivatized flanking residue is created by obtaining from a commercial source an amino acid residue with an Boc-protected α-amino substituent, an allyl-protectedα-carboxy substituent, and an unprotected side chain amino substituent, and then reacting the α-substituent protected amino acid with a difunctional linker having a free carboxy group to form an amide linkage between the linker's free carboxy group and the unprotected side chain amino substituent of the amino acid using any of the condensation methods described in Section (B)(II)(1)(a) above. The allyl-protected α-carboxy substituent of the derivatized amino acid residue is then removed by reduction to permit incorporation of the derivatized amino acid into the peptide chain.

In one embodiment designed for use in conjunction with Fmoc chemistry, the linker-derivatized flanking residue is created by obtaining from a commercial source an amino acid residue with an Fmoc-protected α-amino substituent, a t-butyl ester-protected α-carboxy substituent, and an unprotected side chain carboxy substituent, and then reacting the α-substituentprotected amino acid with a difunctional linker having a free amino group to form an amide linkage between the linker's free amino group and the unprotected side chain carboxy substituent of the amino acid using any of the condensation methods described in Section (B)(II)(1)(a) above. The t-butyl ester-protected α-carboxy substituent of the derivatized amino acid residue is then removed by acidolysis to permit incorporation of the derivatized amino acid into the peptide chain.

In another embodiment designed for use in conjunction with Fmoc chemistry, the linker-derivatized flanking residue is created by obtaining from a commercial source an amino acid residue with an Fmoc-protected α-amino substituent, an allyl-protected α-carboxy substituent, and an unprotectedside chain carboxy substituent, and then reacting the α-substituent protected amino acid with a difunctional linker having a free amino group to form an amide linkage between the linker's free amino group and the unprotected side chain carboxy substituentof the amino acid using any of the condensation methods described in Section (B)(II)(l)(a) above. The allyl-protected α-carboxy substituent of the derivatized amino acid residue is then removed by reduction to permit incorporation of the derivatized amino acid into the peptide chain.

In one embodiment designed for use in conjunction with Boc chemistry, the linker-derivatized flanking residue is created by obtaining from a commercial source an amino acid residue with an Boc-protected α-amino substituent, a Fm ester-protected α-carboxy substituent, and an unprotected side chain carboxy substituent, and then reacting the α-substituent protected amino acid with a difunctional linker having a free amino group to form an amide linkage between the linker's free amino group and the unprotected side chain carboxy substituent of the amino acid using any of the condensation methods described in Section (B)(II)(1)(a) above. The Fm ester-protected α-carboxy substituent of the derivatized amino acid residue is then removed by base saponification to permit incorporation of the derivatized amino acid into the peptide chain.

In another embodiment designed for use in conjunction with Boc chemistry, the linker-derivatized flanking residue is created by obtaining from a commercial source an amino acid residue with an Boc-protected α-amino substituent, an allyl-protected α-carboxy substituent, and an unprotectedside chain carboxy substituent, and then reacting the α-substituent protected amino acid with a difunctional linker having a free amino group to form an amide linkage between the linker's free amino group and the unprotected side chain carboxy substituentof the amino acid using any of the condensation methods described in Section (B)(II)(1)(a) above. The allyl-protected α-carboxy substituent of the derivatized amino acid residue is then removed by reduction to permit incorporation of the derivatized amino acid into the peptide chain.

It is desirable to protect one of the functional groups on the difunctional linker either before the linker is coupled to the flanking residue that is selected to carry the linker or after the coupling but before the addition of the linker-coupled flanking residue to the peptide chain. This improves yield by avoiding unwanted reaction of the free functional group on the flanking residue-coupledlinker during peptide synthesis. The free functional group on the linker can be blocked with any of the amino or carboxy protective groups described in Section (B)(II)(1)(a) above. In one embodiment, the free functional group on the linker and the α-amino groups are orthogonally protected such that the α-amino groups can be deprotected in peptide synthesis without deprotectingthe free functional group on the linker. It will be appreciated that any of the foregoing procedures for coupling difunctional linkers to flanking residues can be easily modified to derivatize a particular flanking residue with a selected orthogonally monoprotected difunctional linker.

In one embodiment designed for use in conjunction with Fmoc chemistry, an orthogonally monoprotected difunctional linker-derivatized flanking residue is created by obtaining from a commercial source an amino acid residue with an Fmoc-protected α-amino substituent, a t-butyl ester-protected α-carboxy substituent, and an unprotected side chain amino substituent, and then reacting the α-substituent protected amino acid with a difunctional linker carrying a free carboxy group and either an allyl-protectedcarboxy group or an allyloxycarbonyl-protected amino group to form an amide linkage between the linker's free carboxy group and the unprotected side chain amino substituent of the amino acid using any of the condensation methods described in Section (B)(II)(1)(a) above. The t-butyl ester-protected α-carboxy substituent of the derivatized amino acid residue is then removed by acidolysis to permit incorporation of the derivatized amino acid into the peptide chain.

In another embodiment designed for use in conjunction with Fmoc chemistry, an orthogonally monoprotec ted difunctional linker-derivatized flanking residue is created by obtaining from a commercial source an amino acid residue with an Fmoc-protected α-amino substituent, an allyl-protected α-carboxy substituent, and an unprotected side chain amino substituent, and then reacting the α-substituent protected amino acid with a difunctional linker carrying a free carboxy group and either a Boc-protected amino group or a t-butyl ester-protectedcarboxy group to form an amide linkage between the linker's free carboxy group and the unprotected side chain amino substituent of the amino acid using any of the condensation methods described in Section (B)(II)(1)(a)above. The allyl-protected α-carboxy substituent of the derivatized amino acid residue is then removed by reduction to permit incorporation of the derivatized amino acid into the peptide chain.

In one embodiment designed for use in conjunction with Boc chemistry, an orthogonally monoprotec ted difunctional linker-derivatized flanking residue is created by obtaining from a commercial source an amino acid residue with an Boc-protected α-amino substituent, a Fm ester-protected α-carboxy substituent, and an unprotected side chain amino substituent, and then reacting the α-substituent protected amino acid with a difunctional linker carrying a free carboxy group and either an allyloxycarbonyl-protected amino group or an allyl-protectedcarboxy group to form an amide linkage between the linker's free carboxy group and the unprotected side chain amino substituent of the amino acid using any of the condensation methods described in Section (B)(II)(1)(a) above. The Fm ester-protected α-carboxy substituent of the derivatized amino acid residue is then removed by base saponification to permit incorporation of the derivatized amino acid into the peptide chain.

In another embodiment designed for use in conjunction with Boc chemistry, an orthogonally monoprotected difunctional linker-derivatized flanking residue is created by obtaining from a commercial source an amino acid residue with an Boc-protected α-amino substituent, an allyl-protected α-carboxy substituent, and an unprotected side chain amino substituent, and then reacting the α-substituent protected amino acid with a difunctional linker carrying a free carboxy group and either a Fmoc-protected amino group or a Fm ester-protectedcarboxy group to form an amide linkage between the linker's free carboxy group and the unprotected side chain amino substituent of the amino acid using any of the condensation methods described in Section (B)(II)(1)(a) above. The allyl-protected α-carboxy substituent of the derivatized amino acid residue is then removed by reduction to permit incorporation of the derivatized amino acid into the peptide chain.

In one embodiment designed for use in conjunction with Fmoc chemistry, an orthogonally monoprotected difunctional linker-derivatized flanking residue is created by obtaining from a commercial source an amino acid residue with an Fmoc-protected α-amino substituent, a t-butyl ester-protectedα-carboxy substituent, and an unprotected side chain carboxy substituent, and then reacting the α-substituent protected amino acid with a difunctional linker carrying a free amino group and either an allyloxycarbonyl-protected amino group or an allyl-protected carboxy group to form an amide linkage between the linker's free amino group and the unprotected side chain carboxy substituent of the amino acid using any of the condensation methods described in Section (B)(II)(1)(a) above. The t-butyl ester-protected α-carboxy substituent of the derivatizedamino acid residue is then removed-by acidolysis to permit incorporation of the derivatized amino acid into the peptide chain.

In another embodiment designed for use in conjunction with Fmoc chemistry, an orthogonally monoprotected difunctional linker-derivatized flanking residue is created by obtaining from a commercial source an amino acid residue with an Fmoc-protected α-amino substituent, an allyl-protected α-carboxy substituent, and an unprotected side chain carboxy substituent, and then reacting the α-substituent protected amino acid with a difunctional linker carrying a free amino group and either a Boc-protected amino group or a t-butyl ester-protected carboxy group to form an amide linkage between the linker's free amino group and the unprotected side chain carboxy substituent of the amino acid using any of the condensation methods described in Section (B)(II)(1)(a)above. The allyl-protected α-carboxy substituent of the derivatized amino acid residue is then removed by reduction to permit incorporation of the derivatized amino acid into the peptide chain.

In one embodiment designed for use in conjunction with Boc chemistry, an orthogonally monoprotected difunctional linker-derivatized flanking residue is created by obtaining from a commercial source an amino acid residue with an Boc-protected α-amino substituent, a Fm ester-protected α-carboxy substituent, and an unprotected side chain carboxy substituent, and then reacting the α-substituent protected amino acid with a difunctional linker carrying a free amino group and either an allyloxycarbonyl-protected amino group or an allyl-protected carboxy group to form an amide linkage between the linker's free amino group and the unprotected side chain carboxy substituent of the amino acid using any of the condensation methods described in Section (B)(II)(1)(a) above. The Fm ester-protected α-carboxy substituent of the derivatized amino acid residue is then removed by base saponification to permit incorporation of the derivatized amino acid into the peptide chain.

In another embodiment designed for use in conjunction with Boc chemistry, an orthogonally monoprotected difunctional linker-derivatized flanking residue is created by obtaining from a commercial source an amino acid residue with an Boc-protected α-amino substituent, an allyl-protected α-carboxy substituent, and an unprotected side chain carboxy substituent, and then reacting the α-substituent protected amino acid with a difunctional linker carrying a free amino group and either a Fmoc-protected amino group or a Fm ester-protected carboxy group to form an amide linkage between the linker's free amino group and the unprotected side chain carboxy substituent of the amino acid using any of the condensation methods described in Section (B)(II)(1)(a)above. The allyl-protected α-carboxy substituent of the derivatized amino acid residue is then removed by reduction to permit incorporation of the derivatized amino acid into the peptide chain.

In another aspect, the foregoing embodiments utilizing a difunctional linker-derivatized flanking residue can be modified by orthogonally protecting the side chain amide bond-forming substituent of the underivatized(not pre-coupled to difunctional linker) flanking residue with respect to the α-amino protection chemistry used in peptide synthesis and with respect to any or all of the amide bond-forming substituents found in the side chains of other amino acid residues in the peptide. In this aspect, the side chain amide bond-forming substituent of the underivatized, flanking residue can be selectively deblocked, yielding a peptide that can be cyclized by a condensation reaction that is specificallytargeted to be between the deprotected side chain amide bond-forming substituent of the underivatized, flanking residue and the free functional group of the difunctional linker. Suitable methods for orthogonal protection of side chain amide bond-forming substituents are described in Section (B)(II)(1)(a) above.

Following completion of solid phase peptide synthesis, the peptide can be cyclized by a coupling reaction between the free functional group of the difunctional linker and the side chain amide bond-forming substituentof the underivatized, flanking residue as described in Section (B)(II)(1)(b) above. Any blocking group(s) protecting the underivatized, flanking residue's side chain amide bond-forming substituent and/or the free functional group of the difunctional linker is (are) removed, and the deprotected groups are coupled to form an amide linkage using any of the condensation methods described in Section (B)(II)(1)(a) above. Optionally, the resulting cyclized (constrained helix) peptide is cleaved away from the solid support, recovered and purified.

Alternatively, the peptide can be cleaved away from the solid support prior to the cyclization step. In one embodiment, after synthesis of the linear peptide chain is complete, the peptide is cleaved away from the solid support. The peptide is recovered, deblocked at the side chain amide bond-forming substituent of the underivatized, flanking residue and/or the free functional group of the difunctional linker, and then cyclized at low concentration in a reaction mixture in order to maximize intramolecularamide bond formation. Typically, a maximum level of intramolecular amide bond formation can be achieved under conditions in which the concentration of the peptide provides an intramolecular concentration of free amide bond-forming substituents or groups that exceeds the intermolecular concentration of free amide bond-forming substituents or groups in the reaction mixture. In one embodiment, a peptide concentration of 1 nM to 1 M, and preferably 1 µM to 1 mM, and more preferably 1 µM to 100 µM, is used to maximize cyclization. The cyclization of free peptide can be conducted with any of the condensationreactions used to helicize solid phase peptide described above.

### III. Methods for Constructing Semisynthetic Locked Helix Proteins

Also provided herein are semisynthetic proteins comprising locked helix peptides attached onto or incorporated in between one or more larger, recombinantly synthesized protein molecules. The semisynthetic, locked helix peptides of the invention can be made by any convenientmethod, including ligation of the locked helix peptides synthesized as described in Section (B)(II) above to one or more recombinantly synthesized protein sequences. For example, protein ligases such as the "subtiligases" can be used to concatenate the locked helix peptides made as described herein to larger, recombinantly synthesized protein fragments.

In one embodiment, the methods of the invention are modified in order to produce a locked helix peptide that functions as "first ligation substrate" in the subtiligase catalyzed peptide ligation methods described in International Patent Application No. PCT/US 91/05480 (WO 92/02615 published 20 February 1992) or as "donor ester", "donor peptide", and "Pₙ...P₄-P₃-P₂-P₁-glc-F-amide ester", respectively, in the subtiligase catalyzed peptide ligation methods described in Abrahmsen et al., Biochem., 30: 4151-4159(1991), Jackson et al., Science, 266: 243-247 (1994), and Chang et al., Proc. Natl. Acad. Sci. USA, 91: 12544-12548 (1994). The locked helix peptide can be synthesized such that the C-terminal amino acid residue of the cyclized peptide is in an ester linkage with the 2-hydroxyl group of a 2-hydroxycarboxylic acid, such as glycolic acid or lactic acid, to form a leaving group favored by the particular subtiligase of interest, i.e. such that the 2-hydroxycarboxylic acid ester, shown as the X residue of the "first ligation substrate" in Fig. 2B of WO 92/02615,resemblesthe first residue positioned on the N-terminal side of the hydrolyzable amide bond in the normal peptide substrate of subtilisin, shown as residue P₁'of the "hydrolysis substrate" in Fig. 2B.

In another embodiment, the leaving group comprises a 2-hydroxycarboxylic acid and another amino acid residue, shown as the R₂" residue of the first ligation substrate in Fig. 2B of WO 92/02615, wherein the carboxy group of the 2-hydroxycarboxylicacid residue is in an amide linkage with the α-amino group of the additional amino acid residue. In such embodiments, the amino acid residue in the leaving group can be selected to resemble the second residue positioned on the N-terminal side of the hydrolyzable amide bond in the normal peptide substrate of subtilisin, shown as residue P₂' of the "hydrolysis substrate" in Fig. 2B of WO 92/02615. In a preferred embodiment, the leaving group is a glycolate-phenylalanyl (glc-F) moiety such as the glycolate-phenylaianyl-amide (glc-F-NH₂) moiety described in Example 2 of WO 92/02615.

In one aspect, the glc-F leaving group is placed in its proper position at the C-tetininus of the locked helix peptide by obtaining a Boc- or Fmoc-α-amino protected phenylalanine, linking the α-amino protected phenylalanine to solid phase resin with an α-carboxy ester or amide linkage, deprotecting the protected α-amino group, adding a glycolic acid residue in the form of a t-butyl ether to form an amide linkage between the carboxy group of the glycolic acid and the free α-amino group of the solid phase phenylalanine, removing the t-butyl ether group from the glycolic acid residue with acid and forming an ester linkage between the free hydroxyl of the glycolic acid residue and the α-carboxy of the next amino acid residue in the C-terminal sequence desired for the locked helix peptide. Subsequentamino acids can be added and the resulting peptide can be helicized according to any of the above described methods which utilize standard Boc or Fmoc chemistry for peptide synthesis. In one embodiment, a glc-F-NH₂ leaving group is incorporated into the desired peptide chain essentially as described in Example 2 of WO 92/02615 or as described in Jackson et al., Science, 266: 243-247 (1994).

In yet another embodiment, the "donor peptide" includes a flexible linker sequence between the C-terminal residue of the locked helix peptide sequence and the leaving group sequence, such as a di- or tri-glycine linker, to promote flexibility and accessibility of the donor peptide's leaving group to subtiligase.

After the donor peptide (with the helix locking tether in place) is obtained, a subtiligase can be used to ligate a peptide or protein fragment (produced by recombinant or other synthetic methods), designated the "second ligation substrate" in Fig. 2C of WO 92/02615, the "acceptor peptide" in Fig. 1 on page 244 of Jackson et al., Science, 266:243-247 (1994), and the "Nucleophile" peptide in the synthetic scheme on page 12545 of Chang et al., Proc. Natl. Acad. Sci. USA, 91: 12544-12548 (1994), to the C-terminus of the donor peptide by displacement of the leaving group according to any of the subtiligase-catalyzed peptide ligation methods described above. In embodimentsusing acceptorpeptides or proteins having a relatively inaccessible N-terininus due to higher order protein structure, ligation efficiency can be improved by altering the design of the acceptor peptide to incorporate a flexible linker sequence, such as a di- or tri-glycine sequence, at the N-terminus to promote flexibility and accessibility of the acceptor peptide N-terminus in the peptide ligation reaction. Alternatively,the accessibility of the acceptor peptide N-terminus and/or donor peptide C-terminus to subtiligase can be improved by conducting the ligation reaction under denaturing conditions which eliminates unfavorable structural conformations that may be assumed by the peptide substrates. In such embodiments, it is preferable to use a denaturation-stable subtiligase, such as the "stabiligase" described in Chang et al., supra (capable of retaining nearly 50% of catalytic activity in 4 M guanidine hydrochloride).

It will be appreciated that additional peptides can be synthesized with a suitable leaving group at the C-terminus and successively ligated to the N-tetminus of the semisyntheticpeptide containing the locked helix moiety by repeating the foregoing procedures until a completed peptide with the desired N-terminus is obtained.

In the event that the completed, semisynthetic, locked helix protein is obtained in a denatured, incorrectly folded, or otherwise inactive form as a result of the synthetic procedures used, the inactive species can be refolded into the native or active conformation by renaturation techniques that are well known in the art. Typical renaturation procedures use a chaotrope, such as urea at high pH or guanidine hydrochloride, to unfold inactive material followed by dilution of the denaturant to permit refolding to occur, while preventing the formation of random disulfide bonds prior to the assumption of the biologically active conformation through non-covaient, intramolecular interactions (see, U.S. Pat. Nos. 4,512,922; 4,518,256; 4,511,502; and 4,511,503). Reversed micelles or ion exchange chromatography are used to assist refolding of denatured proteins by enclosing a single protein molecule within micelles or isolating proteins on a resin and then removing the denaturant (Hagen et al., Biotechnol Bioeng., 35: 966-975 (1990); Creighton in Protein Structure Folding and Design, Oxender, D.L., ed., Alan R. Liss, Inc. (New York: 1985), pp. 249-251. In addition, conformation-specific refolding can be performed with ligands and antibodies to the native structure of the protein (Cleland and Wang in Biotechnology, Rehm, H.-J., and Reed, G., eds, VCH (New York), pp. 528-555. Since they are more likely to interact with the protein in its native conformation, these binding molecules can be used to guide the folding reactions towards native state protein. The foregoing recovery methods are regarded as being universally applicable, with minor modifications, to the recovery of biologically active recombinant proteins from inclusion bodies, and are equally applicable to the recovery of biologically active proteins from the semisynthetic methods of the invention.

### IV. Methods for Constructing Macromolecule-Bound Locked Helix Peptides

In one embodiment, the constrained, helical peptides of the invention bound to a macromolecular solid support can be obtained by constructing the locked helix peptides with the solid phase synthesis techniques described in Section (II) above and recovering the intact, solid support-peptide conjugate. Alternatively, the cyclized peptide can be cleaved away from solid phase following synthesis and then attached to the macromolecule of choice by any convenient method known in the art. For example, a commonly employed technique for attaching peptide ligands to polysaccharidematrices, e.g. agarose, dextran or cellulose, involves activation of the carrier with cyanogen halides and subsequent coupling of the peptide's primary aliphatic or aromatic amines to the activated matrix. The activation of polysaccharides with cyanogen bromide (CNBr) at alkaline pH was introduced to affinity chromatography by Axen *et al., Nature.* **214:** 1302 (1967). In one aspect of the invention, the activation of polysaccharide matrices, particularly agarose matrices, is performed according to the titration-activation method. In this procedure, for example, 20 g of exhaustively washed moist agarose cake is added to 20 ml of water in a 100 ml beaker equipped with a 0-100°C thermometer, a pH meter and a 25 mm magnetic stirring bar. The suspension is stirred slowly, the temperature lowered to about 10-15°C by the addition of crushed ice and the pH adjusted to 10.8±0.1 by the addition of 1-2 drops of 4 N NaOH. The activation procedure is initiated by the addition of the CNBr and the pH of the reaction maintained at 10.8±0.1 by manual titration with the 4 N NaOH. The CNBr (100 g/mg moist weight gel) can be added as a crystalline solid, a crushed solid, an aqueous solution or by adding an aliquot of a stock solution. The latter can be prepared by dissolving CNBr in acetonitrile (1 g/ml) and storing in a tightly stoppered vial at -20°C. The temperature is subsequently allowed to rise to 18-20°C.

Despite the relative simplicity of the titration method, it may be preferable to use the faster and technically simplified method of March *et al., Anal. Biochem.,* **60:** 149 (1974). The activation procedure is performed in concentrated carbonate buffer. The required amount of washed gel is suspended in an equal volume of 2 M NaHCO₃-NaCO₃ buffer (pH 10.9) in a beaker equipped with a thermometer and magnetic stirring bar. The slurry is cooled to approximately 4-5°C, the activated gel is transferred to a sintered funnel and washed.

The concentration of CNBr recommended in the procedures described above is satisfactory for moderate levels of peptide substitution. When lower or higher levels of activation are required, 50 mg and 200-300 mg CNBr/g moist weight gel respectively can be employed together with 2 M and 8 M NaOH for the titration.

It is generally recognized that the CNBr-activated intermediate functional groups of polysaccharide gels display limited stability and therefore it is preferable that the gel be washed as rapidly as possible prior to transferring the gel to the coupling-reaction medium. At the end of the activation step, the gel is rapidly cooled by the addition of crushed ice and poured into a large sintered glass funnel which has been pre-cooled with crushed ice. The suspension is rapidly filtered into a Buchner flask (2 liter) containing solid ferrous sulfate to remove unreacted CNBr and cyanides as harmless ferrocyanide. The gel is subsequently washed under suction with 1 liter ice-cold distilled water and I liter of the buffer to be used in the coupling stage, typically ice-cold 0.1 M NaHCO₃-NaCO₃ buffer (pH 8.5-9.5).

CNBr-activated Sepharose 4B is available commercially from Pharmaciaand obviates the hazardous manipulation of CNBr. The activated gel is freeze dried in the presence of dextran and lactose to preserve the beaded form and supplied in 15 g air-tight packs. The required amount of freeze-dried powder is swollen in 1 mM HCl on a glass filter and washed with at least 200 ml of the same solution per gram of powder. 1 g of freeze-dried material is roughly equivalent to 3.5 ml final gel volume. The peptide ligand-binding capacity of the gel is conserved more effectively by washing with solutions of low pH than with solutions of pH greater than 7. The gel is then ready to couple peptide ligand as soon as the washing is completed.

Pharmacia also markets CNBr-activatedSepharose 6 MB for use in cell biology and immunology for the separationof"functionallyhomogeneouscell populations". It is produced by activation of Sepharose 6MB macrobeads(diameter200-300 µm) with cyanogen bromide and is handled in a manner analogous to CNBr-activated Sepharose 4B.

The peptide to be coupled is suspended in a volume of the cold buffer equal to the volume of the packed gel, added to the moist, washed gel, and then the suspension is immediately mixed (in a Buchner funnel) with a glass stirring rod. The entire procedure of washing, adding the peptide solution, and mixing preferably consumes less than 90 seconds. The suspension is transferred from the Buchner funnel to a beaker containing a magnetic mixing bar and is gently stirred at 4°C. Although the reaction is essentially complete in 2 to 3 hours, the mixture is allowed to stand at 4°C for 16 to 20 hours to insure complete loss of reactive polysaccharide groups. The peptide-linked gel is then washed with large volumes of water until it is established that peptide is no longer being removed.

The quantity of peptide coupled to the polysaccharidegel can in part be controlled by the amount of peptide added to the activated matrix. When highly substituted polysaccharidegel derivatives are desired, the amount of peptide added should be 20 to 30 times greater than that which is desired in the final product. For ordinary procedures, 100 to 150 mg of cyanogen bromide are used per ml of packed polysaccharide gel, but much higher coupling yields can be obtained if this amount is increased to 250 to 300 mg. The pH at which the coupling reaction is performed also affects the degree of coupling, since it is only, the unprotonated form of a peptide's amino groups that reacts with CNBr-activated polysaccharides. Preferably, the N-terminal α-amino group of the peptide ligand is used for coupling with the activated polysaccharide matrix. α-amino groups will couple optimally at a pH of about 9.5 to 10.0. If coupling at the ε-amino group(s) of the selected peptide ligand (such as the ε-amino groups of the lysinyl residues) is desired, the coupling reaction should be conducted at a pH value of about 10.0, and a large excess of peptide should be added. If coupling at the aromatic amino groups in the histidyl or tryptophanyl residues of the selected peptide is desired, very high coupling efficiency can be obtained at pH values between 8 and 9.

Further details of the invention can be found in the following examples, which further define the scope of the invention.

### EXAMPLES

### EXAMPLE 1

### Experimental Section

### Computational Methods

All calculations were performed with the DISCOVER program (Biosym Technologies, San Diego) using the all-atom AMBER force field (Weiner, S. J.; Kollman, P. A.; Case, D. A.; Singh, U. C.; Ghio, C.; Alagona, G.; Profeta, S., Jr.; Weiner, P. *J. Am. Chem.* Soc. **1984**, *106,* 765-784; Weiner, S. J.; Kollman, P. A.; Nguyen, D. T.; Case, D. A. *J. Comp. Chem.* **1986**, 7, 230-252) with a distance dependent dielectric constant (ε=4*r*).

### Synthesis

### Materials and Methods

Peptideswere synthesized using standard solid phase synthesis techniques (Merrifield, R.B. *J. Am. Chem. Soc.* **1963,** *85,* 2149-2154; Kaiser, E.; Colescot, R.L.; Bossinger, C.D.; Cook, P.I. *Anal. Biochem.* **1970,** *34,* 595-598). Organic chemicals were purchased from Aldrich (Milwaukee WI) or Fluka (Ronkonkoma, NY). Protected amino acids were purchased from Bachem CA (Torrance CA) or Peninsula Labs (Belmont CA). BOP (benzotriazole-1-yl-oxy-tris [dimethylamino] phosphonium hexafluorophosphate) was purchased from Richelieu Biotechnologies(Montreal). Solvents were purchased from Baxter (McGaw Park IL), Baker (Phillipsburg NJ), or Mallinckrodt (Paris KY). Polystyrene supports were purchased from Advanced ChemTech (Louisville KY).

Mono-t-butyloxycarbonyl (BOC) 1,3-propanediamine was prepared as follows. 2-(*tert-*butoxycarbonyloxyimino)-2-phenylacetonitrile (18 g, 73 mmol) was added portionwise over 10 minutes to a solution of 1,3-diaminopropane(12.5 g, 184 mmol) in 100 mL of tetrahydrofuran cooled to 0° C. After four hours at 0° C the reaction was allowed to warm to 25° C for two hours. The reaction was diluted with 150 mL of ethyl acetate and washed twice with 100 mL of saturated aqueous sodium chloride. The organic phase was extracted with three 100 mL volumes of 10% aqueous citric acid, the combined aqueous portions were then washed twice with 100 mL of ethyl acetate. The aqueous phase was cooled in an ice bath and the pH was adjusted to approximately 13 with 50 % sodium hydroxide. The basic aqueous phase was then extracted with three 100 mL volumes of dichloromethane.The organic portion was then dried with potassium carbonate and filtered. Solvent was removed by rotary evaporation to yield mono-*tert*-butyloxycarbonyl-1,3-diaminopropane.

Peptides were purified by reverse-phase HPLC on a Vydac C-18 column, eluted with acetonitrile-water gradients containing 0. 1% v/v trifluoroacetic acid (TFA). Peptides were characterized by electrospray MS on a PE SCIEX API III+ triple quadrupole mass spectrometerand by quantitative amino acid analysis on a Beckmann 6300 automated amino acid analyzer. Organic intermediates were analyzed by ¹H and ¹³C nuclear magnetic resonance (NMR) on a Varian VXR-300S and by high-resolution mass spectrometry (MS) on a JEOL JMS-HX110HF/HX110HF tandem mass spectrometer.
**AcTNE(OFm)DLAARRE(OAllyl)QQnh-MBHA-polystyrene (1a):**

Linear peptide **1a** with the sequence shown was synthesized on p-MBHA resin (4.25 grams (g), 0.57 milliequivalents/gram (meq/g), 2.42 millimoles (mmol)) using standard coupling cycles with three molar equivalents of BOC-amino acid, 3.3 molar equivalents of BOP and 3.3 molar equivalents of N-methylmorpholinein dichloromethane(CH₂Cl₂) and dimethyl acetamide (DMA) if needed for solubility, for one hour at room temperature. The N-acetyl cap was attached by treatment with 5 milliliters (mL) of acetic anhydride in 3% triethylamine(TEA) in CH₂Cl₂ for 20 minutes at room temperature. The resin was dried and weighed (4.41 g, estimated at 0.22 meq/g).
**AcTNE(OFm)DLAARRE(OFm)QQnh-MBHA-polystyrene (1f):**
**AcAEE(OFm)AAAKFLE(OAllyl)AHAnh-MBHA-polystyrene (2a):**

Linear peptides **1f** and **2a** as shown were synthesized as described above for **1a.**
**AcTNQ(γ-NHCH₃)DLAARRQ(γ-NHCH₃)QQoh₂ (1b):**

Linear resin-bound peptide 1f (0.60g, 0.17 mmol) was doubly deprotected with 20% piperidine/DMA for 20 minutes. The free carboxylic acids were coupled to methylamine (CH₃NH₃Cl, 0.26 g, 3.85 mmol) with BOP (1.57 g. 3.55 mmol) and N-methylmorpholine(0.90 mL, 8.2 mmol) in CH₂Cl₂/DMA for 1.5 hours. The resin was washed and dried, and the peptide-resin bond was cleaved with anhydrous hydrofluoric acid (HF) (10mL) at 0°C for one hour with anisole (1 mL) and ethylmethylsulfide (EtSMe) (0.5 mL) as scavengers. The resin was washed twice with ether, once with ethyl acetate, and again with ether. The free peptide was then extracted from the resin with sequential washes of 10% acetic acid, glacial acetic acid, acetonitrile, 10% acetic acid, and water. The combined solutions were lyophilized and the residue was purified.

**_CH₂CH₂CH₂_**

**cyclo-AcTNQ(γ-NH)DLAARRQ(γ-NH)QQnh₂ (1c):**

### 1. Using unprotected propanediamine:

Linear peptide 1a on the resin (0.51 g, 0.11 mmol) was deprotected at the fluorenylmethyl ester with 20% piperidine/DMA for 20 minutes and the resulting piperidine salt was neutralized by washing twice with 1% TFA in CH₂Cl₂. The free carboxylic acid was coupled to 1,3-propanediamine(0.12 mL, 1.44 mmol) with BOP (0.40 g, 0.90 mmol) and diisopropylethylamine(DIPEA) (0.17 mL, 0.98 mmol) in CH₂Cl₂ for one hour, followed by addition of DMA and continued coupling for an additional 45 minutes. The glutamic acid allyl ester was deprotected with tetrakis (triphenylphosphine)palladium(0)(Pd(PPh₃)₄) (0.21 g, 0.18 mmol) in 20% piperidine/DMA for 1.5 hours and the piperidine was removed by washing twice with 1% TFA in CH₂Cl₂. The resulting amino acid was cyclized with BOP (0.32 g, 0.72 mmol) and DIPEA (0.13 mL, 0.75 mmol) in CH₂Cl₂ for 3.5 hours. A Kaiser test gave a noticeable purple color, so the cyclization was repeated with BOP (0.44 g, 0.99 mmol) and DIPEA (0.19 mL, 1.09 mmol) in CH₂Cl₂ for three days. The peptide was cleaved from the resin as described above for 1b.

### 2. Using mono-BQC propanediamine:

Linear peptide 1a on the resin (0.57 g, 0.13 mmol) was deprotected at the fluorenylmethyl ester with 20% piperidine/DMAfor 0.5 hour and the resulting piperidine salt was neutralized by washing twice with 1 % TFA in CH₂Cl₂. The free carboxylic acid was coupled to mono-*tert*-butyloxycarbonyl-1,3-propanediamine (0.23 g, 1.32 mmol) with BOP (0.52 g, 1.18 mmol) and DIPEA (0.25 mL, 1.44 mmol) in CH₂Cl₂/DMA for one hour. The glutamic acid allyl ester was deprotected with Pd(PPh₃)₄ (0.21 g, 0.18 mmol) in 20% piperidine/DMA for 1.5 hours and the piperidine was removed by washing twice with 1% TFA in CH₂Cl₂; the Kaiser test was negative at this point. The BOC group was removed with TFA/CH₂Cl₂/anisole/1,2-ethanedithiol (45:45:5:5 vol/vol); the free amine then gave a positive Kaiser test. The resulting amino acid was cyclized with BOP (0.58 g, 1.31 mmol) and DIPEA (0.30 mL, 1.72 mmol) in CH₂Cl₂ for two hours, whereupon the Kaiser test gave only a faint blue-green color. The peptide was cleaved from the resin as described above for **1b.**
**AcAEQ(γ-NHCH₃)AAAKFLQ(γ-NHCH₃)AHAnh₂ (2b):**

Linear peptide 2a on the resin (0.60 g, 0.19 mmol) was deprotected at both the allyl and the fluorenylmethyl esters with Pd(PPh₃)₄ (0.1 g, 0.09 mmol) in 20% piperidine/DMA for 30 minutes and the resulting piperidine salt was neutralized by washing with 50% TFA in CH₂Cl₂ containing anisole and 1,2-ethanedithiol. The free carboxylic acids were coupled to methylamine (40% aqueous, 0.16 mL, 1.86 mmol) with BOP (0.32 g, 0.72 mmol) and DIPEA (0.35 mL, 2.01 mmol) in CH₂Cl₂/DMA for 1 hour. The peptide was cleaved from the resin as described above for **1b.**
**1d, 1e, 2c, 2d, 2e:**

**1d** and **1e** were prepared from **1a** and **2c-2e** were prepared from **2a** by the same procedures as described above for **1c** using unprotected 1,3-propanediamine, coupling with 1,4-butanediamine for **1d** and **2d** and with 1,5-pentanediamine for **1e** and **2e.**
**AcTNk(S-Acm)DLAARRK(S-Acm)QQnh₂ (3a):**
**AcAEk(S-Acm)AAAKFLK(S-Acm)AHAnh₂ (4a):**

Linear peptides **3a** and **4a** were synthesized by standard Merrifield techniques using FMOC chemistry (Atherton, E.; Sheppard. R.C. *J. Chem. Soc., Chem. Commun.* **1985,** 165-166). Fmoc-D-Thiolys(Acm)-OH (7, k(S-Acm)) and Fmoc-L-Thiolys(Acm)-OH (10, k(S-Acm)) were prepared as described below.
**AcTNk(S)DLAARRK(S)QQnh₂ (3b):**
**AcAEk(S)AAAKFLK(S)AHAnh₂ (4b):**

Cyclic peptides **3b** and **4b** were prepared from **3a** and **4a** respectively by simultaneous deprotection and oxidation. Approximately 16 mg of Acm-protectedpeptide was dissolved in 1.5 mL of water containing 10% acetic acid and then diluted to 50 mL total volume with trifluoroethanol to give a final concentration of approximately200 micromoles/liter (µM). A total of 12 mL of a 6 millimoles/liter (mM) solution of iodine (80 milligrams (mg) dissolved in 3 mL of acetic acid and diluted to 50 mL with trifluoroethanol) was added in 1 mL portions over the course of 10 hours while the reaction progress was monitored by HPLC. When the starting material had been consumed, the reaction was diluted with water and lyophilized and the crude oxidized material was purified.

### (2S,5R)-2,5-dihydro-3,6-diethoxy-2-isopropyl-5-(4-bromobutyl)pyrazine (5):

n-butyllithium(13.3 mL of a 1.6 M solution in hexanes, 21.3 mmol) was added to a solution of (2S)-2,5-dihydro-3,6-diethoxy-2-isopropyl pyrazine (Schöllkopf reagent)(4.30g, 20.3 mmol)in tetrahydrofuran (THF) over the course of five minutes. The solution was maintained at -78°C for 15 minutes after which 1,4-dibromobutane (9.75 mL, 81.2 mmol) was added in a single portion. After 2.5 hours at -78°C the reaction was allowed to warm to room temperature and diluted with diethyl ether (100 mL). The organic phase was washed with water (100 mL), brine (100 mL) and then dried with magnesium sulfate (MgSO₄). Following filtration and concentration most of the residual 1,4-dibromobutane was removed under high vacuum. The remaining oil was purified by silica gel chromatography (2% ethyl acetate in hexanes) to provide 5 (3.7 g, 57%) as a colorless liquid;[α]²⁵_{D}-1.13 (c=4.5,CHCl₃); IR (thin film) 2957, 1689, 1456, 1364, 1304, 1230, 1144, 1038 cm⁻¹; ¹H NMR (300 MHz, CDCl₃) δ 4.04-4.18 (m, 4H) 3.94-4.02 (m, 1H) 3.90 (t, J=3.9, 1H) 3.39 (t, J=6.9, 2H) 2.21-2.32 (m, 1H) 1.68-1.92 (m, 4H) 1.33-1.47 (m, 2H) 1.274 (t, J=7.2. 3H) 1.268 (t, J=7.2,3H) 1.03 (d, J=6.9, 3H) 0.70 (d, J=6.9, 3H); ¹³C NMR (100.6 MHz, CDCl₃) d 163.12, 163.05, 60.72, 60.48, 55.09, 33.60, 33.11, 32.66, 31.80, 23.22, 19.03, 16.6, 14.34, 14.31; Mass Spectrum (FAB+) 347.1 (MH+).

### (2S,5R)-2,5-dihydro-3,6-diethoxy-2-isopropyl-5-(4-4-methoxybenzyl)-thiobutyl) pyrazine (6):

Potassium *tert*-butoxide (11.8 mL of a 1 M solution in THF) was added over five minutes to a solution of 4-methoxy-α-toluenethiol (1.85 mL of 90%, 11.8 mmol) in THF (20 mL) at 25°C, generating a thick white precipitate which was stirred for 25 minutes. A solution of 5 (3.7 g, 10.7 mmol) in THF (20 mL) was added and stirring continued for three hours. The reaction was concentrated by rotary evaporation and then partitioned between water (50 mL) and diethyl ether (100 mL). The organic portion was washed with brine, dried(MgSO₄), and concentrated. The residue was purified by silica gel chromatography (1% increasing to 2.5% ethyl acetate in hexanes) to provide 6 (2.90 g, 91%) as a colorless oil; [α]²⁵_{D} -3.77° (c=3.5, CHCl₃); IR (thin film) 2957, 1689, 1510, 1238, 1038 cm⁻¹, ¹H NMR (300 MHz, CDCl₃) δ 7.21 (d, J=8.7, 2H) 6.84 (d, J=8.4, 2H) 4.02-4.20 (m, 4H) 3.93-3.99 (m, 1H) 3.88 (t, J=3.3, 1H) 3.80 (s, 3H) 3.65 (s, 2H) 2.39 (t, J=7.5, 2H) 2.24-2.32 (m, 1H) 1.64-1.82 (m, 2H) 1.50-1.62 (m, 2H) 1.22-1.38 (m, 2H) 1.27 (t, J=7.2, 6H) 1.03 (d, J=6.9, 3H) 0.70 (d, J=6.9, 3H); ¹³C NMR (100.6 MHz CDCl₃) δ 163.20, 163.00, 158.50, 130.57, 129.80, 113.80, 60.67, 60.45, 60.40, 55.24, 55.20, 35.57, 33.68, 31.74, 31.14, 29.19, 23.92, 19.05, 16.60, 14.36. 14.32; Mass Spectrum (FAB+) 421.2 (MH+).

### (2R) 2-(9-Fluorenylmetboxycarbonyl)amino-6-acetamidomethylthiohexanoic acid (D-Thio(Acm)iysine) (7):

Water (30 mL) and 3N HCl (6.5 mL) was added to a solution of 6 (2.90 g, 9.32 mmol) in THF (50 mL). The mixture was stirred at 25°C for 12 hours and then the THF was removed by rotary evaporation. The solution was adjusted to pH 10 with aqueous potassium carbonate (K₂CO₃) and then extracted twice with ethyl acetate (75 mL). The organic extracts were dried (MgSO₄), concentrated and the residue was purified by silica gel chromatography (1:1 increasing to 2:1 ethyl acetate/hexanes with 0.5% triethylamine) to yield crude S-methoxybenzylthiolysineethyl ester (2.65 g, 91%) as a colorless oil which was carried on directly to the next step. This ester (3.02 g, 9.71 mmol) was dissolved in a mixture of trifluoroacetic acid (50 mL) and anisole (1 mL) at 0°C, and mercuric acetate (3.09 g, 9.71 mmol) was added to give a clear solution. After 15 minutes the trifluoroaceticacid was removed by rotary evaporation and the residue was first diluted with water (75 mL), then washed with diethyl ether (75 mL). The aqueous portion was treated with hydrogen sulfide (H₂S) (bubbled through the solution) for 30 minutes and the resulting black precipitate was removed by filtration through a bed of celite. The filtrate was concentrated by rotary evaporation, redissolved in water (20 mL) and filtered through a 0.45 micrometer (µm) nylon filter. The solution was again concentrated and the residual foam was dried under high vacuum overnight. The residue was dissolved in trifluoroacetic acid (15 mL) and acetamidomethanol (Fluka, 0.95 g, 10.7 mmol) was added. After 45 minutes the reaction was concentrated by rotary evaporation and then dried under high vacuum overnight. The residue was dissolved in THF (20 mL) and cooled to 0°C. A solution of lithium hydroxide (1.22 g, 29.1 mmol) in water (20 mL) was added in two portions at 15 minute intervals. After two hours the reaction was allowed to warm to room temperature and the pH was adjusted to 7.0 with I mole/liter (M) aqueous citric acid. The solvent was removed by rotary evaporation, the residue was dissolved in dioxane (80 mL), and Fmoc N-hydroxy succinimide (3.3 g, 9.71 mmol) was added followed by saturated aqueous sodium bicarbonate (NaHCO₃) (15 mL). After one hour, the solvent was removed by rotary evaporation and the residue was partitioned between water (50 mL) and ethyl acetate (50 mL). The aqueous portion was adjusted to pH 2.5 with 1 M aqueous citric acid and then extracted three times with ethyl acetate (75 mL each). The combined organic phases were dried (MgSO₄) and concentrated. The residue was purified by silica gel chromatography (first with 2:1 ethyl acetate/hexanes with 0.5% acetic acid, then ethyl acetate with 0.5% acetic acid, then 5% methanol in ethyl acetate with 0.5% acetic acid), product containing fractions were concentrated from toluene (150 mL, three times) prior to dissolution in water with acetonitrile and lyophilization to provide 7 (3.16 g, 71% over four steps) as a white powder; [α]²⁵ _{D} -1.8° (c=2, EtOH); IR (thin film) 2800-3400, 1709, 1536, 1260, 759, 740 cm⁻¹; ¹H NMR(300 MHZ, DMSO-d6) δ 8.42 (t, J=6.0, 1H) 7.88 (d, J=7.3, 2H) 7.72(d, J=7.5, 2H) 7.58 (d, J=8.1. 1H) 7.407 (t, J=7.5, 2H) 7.32 (t. J=7.5, 2H) 4.16-4.30 (m, 5H) 3.91 (m, 1H) 2.53 (t, J=7.2, 2H) 1.82 (s, 3H) 1.33-1.76 (m, 6H); ¹³C NMR (100.6 MHZ, DMSO-d6) δ 174.00, 169.16, 156.08, 143.86, 143.80, 140.71, 127.61, 127.05, 125.28, 120.08, 65.57, 53.90, 46.68, 30.54, 30.01, 28.79, 24.91, 22.54; High Resolution Mass Spectrum (FAB+) 457.1785, Err[ppm/mmu] -2.7/-1.2.

### (2R,5S)-2,5-dihydro-3,6-dimethoxy-2-isopropyl-5-(4-bromobutane) pyrazine (8):

[α]²⁵_{D}+1.73° (c=4.4, CHCl₃); IR (thin film) 2959, 1696, 1458, 1434, 1237, 1196, 1007 cm⁻¹; ¹H NMR (300 MHZ, CDCl₃) δ 3.98-4.07 (m, 1H) 3.95 (t, J=3.6, 1H) 3.70 (s, 3H) 3.68 (s, 3H)3.40 (t, J=6.9, 2H) 2.19-2.32 (m, 1H) 1.66-1.94 (m, 4H) 1.34-1.48 (m, 2H) 1.05 (d, J=6.9, 3H) 0.69 (d, J=6.9, 3H); ¹³C NMR (100.6 MHZ, CDCl₃) δ 163.62, 163.60, 60.77, 55.11, 52.31, 33.54, 33.10, 32.61, 31.73, 23.26, 19.02, 16.56; Mass Spectrum (FAB+) 319.1 (MH+).

### (2R,5S)-2,5-dihydro-3,6-dimethoxy-2-isopropyl-5-(4-(4-methoxybenzyl)-thiobutyl) pyrazine (9):

[α]²⁵_{D}+3.96° (c=3.63, CHCl₃); 1R (thin film) 2944, 1696, 1510, 1238 cm⁻¹; ¹H NMR (300 MHZ, CDCl₃) δ 7.21 (d, J=8.7, 2H) 6.83 (d, J=8.4, 2H) 3.97-4.25 (m, 1H) 3.93 (t, J=3.3, 1H) 3.79 (s, 3H) 3.68 (s, 3H) 3.67 (s, 3H) 3.65 (s, 2H) 2.39 (t, J=7.5, 2H) 2.18-2.30 (m, 1H) 1.60-1.84 (m, 2H) 1.49-1.62 (m, 2H) 1.23-1.38 (m, 2H) 1.27 (t, J=7.2, 6H) 1.04 (d, J=6.9, 3H) 0.68 (d, J=6.9, 3H); ¹³C NMR (100.6 MHZ, CDCl₃) δ 163.72, 163.47, 158.48, 130.55, 129.78, 113.78, 60.68, 55.22, 55.18, 52.27, 35.58, 33.63, 31.64, 31.12, 29.13, 23.88, 19.02, 16.51; Mass Spectrum (FAB+) 393.2 (MH+).

### (2S) 2-(9-Fluorenylmethoxycarbonyl)amino-6-acetamidomethylthiobexanoimcid: (L-Thio(Acm)lysine) (10):

[α]²⁵_{D} +1.3° (c=2, EtOH); IR (thin film) 2800-3400, 1709, 1536, 1260, 759, 740 cm⁻¹; ¹H NMR (300 MHZ, DMSO-d6) δ 8.42 (t, J=6.0, 1H) 7.88 (d, J=7.3, 2H) 7.72 (d, J=7.5, 2H) 7.58 (d, J=8.1, 1H) 7.407 (t, J=7.5, 2H) 7.32 (t, J=7.5, 2H) 4.16-4.30 (m, 5H) 3.91 (m, 1H) 2.53 (t, J=7.2, 2H) 1.82 (s, 3H) 1.33-1.76 (m, 6H); ¹³C NMR (100.6 MHZ, DMSO-d6) δ 174.00, 169.16, 156.08, 143.86, 143.80, 140.71, 127.61, 127.05, 125.28, 120.08, 65.57, 53.90, 46.68, 30.54, 30.01, 28.79, 24.91, 22.54; High Resolution Mass Spectrum (FAB+) 457.1776, Err[ppm/mmu] -4.6/-2.1.

These materials were prepared in the same manner as 5, 6, and 7 starting from (2R)-2,5-dihydro-3,6-dimethoxy-2-isopropyl pyrazine (Merck).

### NMR Spectroscopy

For each peptide, 2-4 mg of purified material was dissolved in 440 microliters (µl) of 25 mM d₃-sodium acetate containing 5% deuterium oxide (D₂O) and 0.1 millimoles/liter (mM) sodium azide yielding a total peptide concentration of 1-6 mM; the pH was adjusted to 4.5 by microliter additions of 1M sodium hydroxide (NaOH). All spectra were acquired at 5°C or 10°C on a Bruker AMX-500 spectrometer. Two dimensional COSY (Aue, W. P., Bartholdi, E. & Ernst, R. R. *J. Chem. Phys.* **1976**, *64,* 2229-2246), ROESY (Bothner-By, A. A., Stephens, R. L., Lee, J.-m., Warren, C. D. & Jeanloz, R. W. J. *Am. Chem. Soc.* **1984***,106,* 811-813; Rance, M. *J. Magn. Reson.* **1987,** *74,* 557-564) and TOCSY (Braunschweiler, L. & Ernst, R. R. *J. Magn. Reson.* **1983,** *53,* 521-528; Bax, A. & Davis, D. G. *J. Magn. Reson.* **1985.** *65,* 355-360) spectra were acquired with phase discrimination in ω₁ achieved with TPPI (Marion, D. & Wüthrich. K. *Biochem. Biophys. Res. Commun.* **1983,** *113,* 967-974). Total acquisition times were approximately 2, 4, and 12 hours for COSY. TOCSY and ROESY spectra, respectively. Water suppression was achieved by coherent low power irradiation of the water resonance for the 1.5 second (s) recycle delay. ROESY and TOCSY spectra were acquired as described by Akke, M., Skelton; N. J., Kördel, J. & Chazin, W. J. In *Techniques in Protein Chemistry II;* Villafranca. J. J., Ed.; Academic Press, Inc.: Boca Raton, FL, 1991; pp. 401-408; in addition, first-orderphase corrections were avoided by acquisition in a sine-modulated fashion in ω₁. TOCSY mixing was achieved with a clean DIPSI-2rc sequence applied for 90 milliseconds (ms) (Cavanagh, J. & Rance, M. *J. Magn. Reson.* **1992**, 96, 670-678). The ROESY spectra were collected with a 4.0 kilohertz (kHz) spin-lock pulse of 200 ms duration. The spectra were processed and analyzed using the Felix software package (Biosym Technologies, San Diego, CA). ³J_{HN-Hα} were obtained by fitting an antiphase pair of Lorentzian lines to ω₂ slices of high digital resolution COSY spectra.

Amide proton exchange rates with solvent were measured for 1b and 1c by lyophilizing the peptide from H₂O and acquiring a series of one dimensional (1D) NMR spectra immediately after dissolving the peptide in D₂O. Exchange rate constants were determined by performing a three parameter exponential fit to the decaying amide signals. Protection factors were calculated as the ratio of exchange rate in the cross-linked and uncross-linked peptide.

### Structure Calculation

NOESY (Kumar, A., Ernst, R. R. & Wüthrich, K. *Biochem. Biophys. Res. Commun.* **1980,** *95,* 1-6; Bodenhausen, G., Kogler, H. & Ernst, R. R. *J. Magn. Reson.* **1984,** 58, 370-388) and ROESY data were collected with mixing times of 300 ms and 200 ms, respectively, from water (H₂O) and D₂O using a sample of 1c (approximately 8 mM). Total acquisition times were 24 hours per experiment. Distance restraints were generated from these data by categorizing cross-peaks as strong, medium, weak or very weak according to the integrated peak volume, and assigning an upper bound of 2.9, 3.5, 4.6, or 5.6 angstroms (Å), respectively, to the corresponding interproton distance. The dihedral angle φ was restrained between -90° and -40° for residues in which ³J_{HN-Hα} less than 6.0 hertz (Hz). Values of ³J_{Hα-Hβ} were determined from a COSY spectrum acquired in D₂O solution with a 35° mixing pulse. χ₁ restraints and H_{β} stereospecific assignments were obtained for four side-chains on the basis of these coupling constants and the results of initial structure calculations (Skelton, N. J., Garcia, K. C., Goeddel, D. V., Quan, C. & Burnier, J. P. *Biochemistry* **1994,** *33,* 13581-13592).

Structures were calculated using the program DGII using the CVFF force field parameters (Biosym Technologies, San Diego, CA). Input restraints consisted of 141 interproton distances, 9 φ dihedral angle restraints and 5χ₁ dihedral angle restraints. Explicit hydrogen bonds were not included. Structures were generated with triangle and tetrangle smoothing prior to perspective embedding of all atoms. The embedded structures were annealed for 10,000 steps in four-dimensional space while cooling from 200 degrees kelvin (K) with all atom masses set to 1000. The DG structures were refined by rMD using the AMBER force field within the DISCOVER program (Biosym Technologies). Structures were annealed at 600 K for 3 picoseconds (ps), cooled to 0 K over 1.8 ps and finally subjected to 200 cycles of rEM. Charges on Glu, Asp, Arg, C-terminal and N-terminal residues were reduced to 0.2 e and a distance dependent dielectric pf 1/4r was employed. Restraints were employed as square well potentials with force constraints of 25 kilocalories/mole/angstrom² (kcal·mol⁻¹Å⁻²) and 100 kilocalorieslmole/radian² (kcal·mol⁻¹rad⁻²) for distances and dihedral angles, respectively. In the final round of calculation, 60 structures were embedded in DGII and refined by rMD.

### Circular Dichroism

CD spectra were acquired on an Aviv 62 spectrometer with a 0.1 centimeter (cm) path length temperature-controlled cell. Solutions for analytical spectra were prepared by dilution of NMR samples to approximately 100 micromoles/liter (µM) with additional NMR buffer. Points were taken every 0.2 nanometers (nm) with 0.2 nm bandwidth and 2 seconds (s) averaging time. The shortest wavelength attainable was limited by absorption of the acetate buffer. Curves shown are smoothed with standard parameters (10-point smoothing).

### RESULTS AND DISCUSSION

### Design Considerations

Given synthetic and geometric considerations, it was determined that amide chemistry should be used to link the *I* and *I+7* side-chains. Disulfide bonds, while synthetically feasible, introduced an unwanted 90° twist into the linkage. In order to exploit the ability of simple alkyl chain linkers to avoid steric crowding in the region near the *I+3* and *I+4* residues, linkage methods for bridging either Gln or Asn at *I* and *I+7* with an alkanediyl chain were considered. Gln was chosen because its greater length allows use of the minimum size tether to link these side-chains. A representative set of protein crystal structures from the Brookhaven Database (Bernstein, F. C.; Koetzle, T. F.; Williams, G. J. B.; Meyer, E. F.; Brice, M. D.; Rodgers, J. R.; Kennard, O.; Shimanouchi, T.; Tasumi, M. *J. Mol. Biol.* **1977**, 112, 535-542) was searched for all occurrences of glutamine in an α helical context (with φ = -60° ± 30° and ψ = -45° ± 30°). The resulting data set was used to determine the side-chain rotamer distributions of naturally occurring helical glutamine residues. In general, amino acid residues in an α helical context have χ₁ ≈ -60°, a conformation suitable for the *I*+7 position of a side chain linker. Glutamine has a relatively high population (14.6%) of the χ₁ = 180° rotamer, representing a significant natural conformation that points the side chain towards the C terminal end of the helix. Rotamer combinations were identified that minimized the Nε2 - Nε2 distance between the *I* and *1+7* side-chains in a model helical peptide. Depending on χ₃ values, distances ranging from 5.3Å to 7Å were found if the *I* glutamine assumes χ₁ and χ₂ angles of 180° and 60° and the *I+7* glutamine assumes χ₁ and χ₂ values of -60° and 180°, respectively. Model building indicated that a 4-methylene "bridge" could efficiently link these two glutamine side-chains without incurring unfavorable torsional interactions. Models of 3-, 4-, and 5-methylene-bridgedhelical peptides were constructedusing distance geometry methods (Quantum Chemistry Program Exchange, Program #590, entitled DGEOM by Blaney *et al.)* followed by energy minimization. All residues except the linked glutamines were alanine. The conformational stabilities of helical peptides were assessed using 1 nanosecond (ns) of unconstrained molecular dynamics at 298 K following an initial 100 picoseconds (ps) equilibration period during which harmonic restraints (25 kilocalories/mole/angstrom (kcal-mol⁻¹Å⁻¹)) were applied to maintain helicity. As a control, a polyalanine helix was calculated for 1 ns in the presence of identical restraints.

Peptides containing a 3-methylene bridge maintained a consistent helical conformation but showed significant "bending" of the helix axis. Peptides containing a 4-methylene bridge maintained helicity with little distortion, having comparable backbone dihedral angles to the control peptide; χ₁ and χ₂ angles of the tethered glutamines did not change during the simulation. Peptides based on a 5-methylene bridge transiently escaped out of a helical conformation into nested turns centered around the I+5 residue. Multiple side-chain rotamers were also observed in the *I+7* residue. Based on these observations, it was determined that the 4-methylene bridge would provide the preferred tether length.

### Synthesis and Characterization

Amino acid sequences for trial peptides were based on the C-terminal helix of apamin (Habermann, E. and Reiz, K.G., *Biochem. Z* **1965,** 343, 192-203; Callewaert, G.L., Shipolini, R., and Vernon, C.A., *FEBS Lett.* **1968,** *1,* 111-113; Shipolini,R., Bradbury, A.F., Callewaert, G.L., and Vernon, C.A., *Chem. Commun.* **1967,** 679-680) and on S peptide derived from the C-peptide from RNAse A (Brown, J.E.; Klee, W.A. *Biochemistry* **1971,** *10,* 470-476). The sequences of these peptides are shown in Table 1 below.

**Table 1. Structures of peptides 1-4**

| **Peptide** | **Sequence** | **Side Chain Protection** |
|---|---|---|
| 1 | **Ac T N X D L A A R R Z Q Q** NH₂ | **a**: protected, on resin, X=Glu(OAll), Z=Glu(OFm) |
| | | **b:** X=Z=Gln(NMe) |
| **2** | **Ac A E X A A A K F L Z A H A NH₂** | **c:** X-Z=Gln(N(CH₂)₃N)Gln |
| | | **d:** X-Z=Gln(N(CH₂)₄N)Gln |
| | | **e:** X-Z=Gln(N(CH₂)₅N)Gln |
| | | **f:** protected, on resin, X=Z=Glu(OFm) |
| | | |
| **3** | **Ac T N X D L A A R R Z Q Q** NH₂ | **a:** X=D-Thiolys(Acm), Z=L-Thiolys(Acm) |
| | | **b:** X-Z=D-Thiolys-S-S-L-Thiolys |
| 4 | **Ac A E X A A A K F L Z A H A** NH₂ | |

Linear protected peptides **1a**, **1f**, and **2a** were synthesized by standard Merrifield methods using t-butyloxycarbonyl (BOC) chemistry. Control peptides **1b** and **2b** were elaborated from **1f** and **2a** by simultaneous deprotection of both glutamate residues followed by coupling with methylamine (Figure 1). Synthesis of **1d** from **1f** by double deprotection and coupling with 1,4-butanediamine was achieved in low yield/purity. Constrained peptides **1c-e** and **2c-e** were elaborated from **1a** and **2a** by removal of the fluorenylmethylester from Glu3, coupling with the appropriate alkanediamine, removal of the allyl ester from Glu10, and cyclization (Figure 1). Yields were improved by the use of mono-BOC protected alkanediamine in the first coupling step and by the use of a polystyrene resin with 2% divinylbenzene (DVB) crosslinker. The completed peptides were cleaved from the resin with hydrofluoric acid (HF) and purified by preparative high performance liquid chromatography (HPLC). Installation of the tether on the solid phase allowed the completion of the synthesis with only a single purification.

Thiolysine based peptides **3a** and **4a** were synthesized in the linear acetamidomethyl-protected form using standard Merrifield methods and FMOC chemistry, followed by cleavage from the resin with trifluoroaceticacid/triethylsilane(9:1 v/v) and purification by preparative HPLC. These were converted into the disulfide forms **3b** and **4b** in solution by simultaneous deprotection and oxidation with acetic acid and molecular iodine in trifluoroethanol.

Peptides 1-4 were characterized by mass spectrometryand by quantitative amino acid analysis. All peptides gave results consistent with the intended structures.

Protected D- (**7**) and L-Thiolysine(**10**) were prepared as shown in Figure 2. The Schollkopf reagent (Schöllkpf, U.; Groth, U.; Deng, C. *Angew. Chem. Int. Ed Engl.* **1981,** 20, 798-799) was treated with n-butyllithium followed by 1,4-dibromobutaneto give the known bromobutyl pyrazine **5.** The bromide was displaced with the potassium salt of methoxytoluenethiol to give **6**. The pyrazine was hydrolyzed with aqueous hydrochloric acid (HCl) and the thiol was deprotected with mercuric acetate (Hg(OAc)₂) in TFA followed by H₂S. The crude thiolysine ethyl ester was then reprotected with acetamidomethanol in TFA. The ester was hydrolyzed with lithium hydroxide (LiOH) and the free S-protectedamino acid was N-pmtected with Fmoc N-hydroxysuccinimide in dioxane to give 7. The same procedures were used for the synthesis of 10.

### Proton NMR

Peptides 1-4 were studied by 2D ¹H NMR. Resonance positions were obtained by standard sequential assignment methods (Wüthrich, K. (1986) *NMR of Proreins and Nucleic Acids.,* Wiley, New York), and are listed in Table 2 below.

**Table 2. Chemical Shifts^{a} (backbone coupling constants^{b}) of the apamin-sequence peptides 1 and 3**

| **Residue** | **1b** | **1c** | **1d** | **1e** | **3a** | **3b** |
|---|---|---|---|---|---|---|
| Acetyl | | | | | | |
| CH₃ | 2.02 | 2.01 | 2.02 | 2.02 | 2.02 | 2.02 |

| Thrl | | | | | | |
|---|---|---|---|---|---|---|
| H^{N} | 8.34 | 8.47 | 8.46 | 8.46 | 8.33 | 8.34 |
| H^{α}(³J_{HN-Hα}) | 4.22(7.2) | 4.22(6.9) | 4.21(6.9) | 4.22 (6.9) | 4.22 (7.5) | 4.22 (7.3) |
| H^{β} | 4.17 | 4.30 | 4.28 | 4.30 | 4.15 | 4.16 |
| H^{γ} | 1.13 | 1.18 | 1.17 | 1.19 | 1.12 | 1.14 |

| Asn2 | | | | | | |
|---|---|---|---|---|---|---|
| H^{N} | 8.69 | 8.78 | 8.78 | 8.79 | 8.55 | 8.6 |
| H^{α}(³J_{HN-Hα}) | 4.54(6.6) | 4.47(5.0) | 4.49(5.4) | 4.47 (5.0) | 4.60(6.9) | 4.54 (6.3) |
| H^{β} | 2.75* | 2.77* | 2.77* | 2.78* | 2.73* | 2.73* |

| Gln3 (D-Thiolysine) | | | | | | |
|---|---|---|---|---|---|---|
| H^{N} | 8.49 | 8.53 | 8.56 | 8.46 | 8.28 | 8.42 |
| H^{α}(³J_{HN-Hα}) | 4.12(6.5) | 4.11(4.9) | 3.93(4.7) | 4.03 (4.9) | 4.11(6.5) | 4.01 (6.4) |
| H^{β} | 2.01,1.87 | 2.11,1.75 | 2.11,1.81 | 2.09, 1.83 | 1.72,1.64 | 1.84* |
| H^{γ} | 2.22* | 2.42,2.21 | 2.33,2.26 | 2.33, 2.24 | 1.32* | 1.29* |
| H^{δ} | n.a. | n.a. | n.a. | n.a. | 1.52* | 1.63, 1.47 |
| H^{ε} | ??? | 8.01 | ??? | 7.99 | 2.52* | 2.53, 2.59 |

| Asp4 | | | | | | |
|---|---|---|---|---|---|---|
| H^{N} | 8.32 | 8.05 | 8.09 | 8.03 | 8.29 | 8.07 |
| H^{α}(³J_{HN-Hα}) | 4.46(6.6) | 4.31(4.8) | 4.32(4.7) | 4.32 (4.8) | 4.50 (6.7) | 4.37 (5.5) |
| H^{β} | 2.65* | 2.77,2.61 | 2.76,2.60 | 2.76,2.61 | 2.71,2.60 | 2.67* |

| Leu5 | | | | | | |
|---|---|---|---|---|---|---|
| H^{N} | 8.20 | 7.92 | 7.93 | 8.03 | 8.24 | 8.15 |
| H^{α}(³J_{HN-Hα}) | 4.05(5.7) | 3.95(5.3) | 3.97(5.1) | 3.94 (4.8) | 4.07 (5.5) | 4.06 (5.5) |
| H^{β} | 1.60* | 1.69,1.50 | 1.67,1.50 | 1.66, 1.50 | 1.61, 1.51 | 1.64, 1.51 |
| H^{γ} | 1.50 | 1.61 | 1.60 | 1.59 | 1.58 | 1.61 |
| H^{δ} | 0.84,0.76 | 0.83,0.79 | 0.83,0.79 | 0.83, 0.79 | 0.85,0.77 | 0.85, 0.79 |

| Ala6 | | | | | | |
|---|---|---|---|---|---|---|
| H^{N} | 8.14 | 8.01 | 7.93 | 8.02 | 8.18 | 8.12 |
| H^{α}(³J_{HN-Hα}) | 4.07(5.1) | 3.96(4.6) | 3.97(nd) | 3.96 (5.0) | 4.07 (5.2) | 4.11 (5.8) |
| H^{β} | 1.33 | 1.37 | 1.33 | 1.36 | 1.33 | 1.38 |

| Ala7 | | | | | | |
|---|---|---|---|---|---|---|
| H^{N} | 7.94 | 8.67 | 8.59 | 8.35 | 8.00 | 8.21 |
| H^{α}(³J_{HN-Hα}) | 4.13(5.4) | 3.77(4.4) | 3.94(4.8) | 3.87 (4.5) | 4.11 (5.2) | 4.09 (5.5) |
| H^{β} | 1.34 | 1.46 | 1.47 | 1.46 | 1.33 | 1.40 |

| Arg8 | | | | | | |
|---|---|---|---|---|---|---|
| H^{N} | 8.01 | 7.65 | 7.91 | 7.82 | 8.08 | 7.89 |
| H^{α}(³J_{HN-Hα}) | 4.14(6.3) | 4.01(4.9) | 4.03(4.6) | 4.02 (4.9) | 4.14(6.3) | 4.17 (6.1) |
| H^{β} | 1.77* | 1.86* | 1.85* | 1.86* | 1.75* | 1.83, 1.75 |
| H^{γ} | 1.64,1.56 | 1.76,1.60 | 1.73,1.59 | 1.77, 1.59 | 1.62, 1.55 | 1.54, 1.49 |
| H^{δ} | 3.11* | 3.15,3.08 | 3.14,3.05 | 3.15,3.06 | 3.11* | 3.10* |
| H^{ε} | ??? | 7.21 | 7.23 | 7.23 | ??? | ??? |

| Arg9 | | | | | | |
|---|---|---|---|---|---|---|
| H^{N} | 8.17 | 7.78 | 7.89 | 7.94 | 8.19 | 7.97 |
| H^{α}(³J_{HN-Hα}) | 4.17(6.1) | 4.10(5.5) | 4.11(5.2) | 4.08 (5.1) | 4.18 (6.3) | 4.21 (6.6) |
| H^{β} | 1.75* | 1.87* | 1.83* | 1.83* | 1.74* | 1.85, 1.77 |
| H^{γ} | 1.61,1.54 | 1.71,1.56 | 1.71,1.57 | 1.72, 1.56 | 1.61, 1.54 | 1.64, 1.56 |
| H^{δ} | 3.11* | 3.13* | 3.12* | 3.11* | 3.11* | 3.11* |
| H^{ε} | ??? | 7.24 | 7.23 | 7.23 | ??? | ??? |

| Glu10 (L-Thiolysine) | | | | | | |
|---|---|---|---|---|---|---|
| H^{N} | 8.30 | 7.92 | 7.71 | 7.73 | 8.18 | 8.09 |
| H^{α}(³J_{HN-Hα}) | 4.14(6.3) | 4.02(5.4) | 4.17(6.3) | 4.07 (5.6) | 4.14 (6.4) | 4.16 (6.3) |
| H^{β} | 2.03,1.95 | 2.11* | 2.08* | 2.12, 2.06 | 1.71* | 1.75, 1.65 |
| H^{γ} | 2.26* | 2.47,2.41 | 2.49,2.36 | 2.40, 2.17 | 1.34* | 1.32* |
| H^{δ} | n.a. | n.a. | n.a. | n.a. | 1.64* | 1.54, 1.49 |
| H^{ε} | ??? | 7.71 | ??? | 7.85 | 2.53* | ??? |

| Gln11 | | | | | | |
|---|---|---|---|---|---|---|
| H^{N} | 8.36 | 7.79 | 7.82 | 7.87 | 8.39 | 8.19 |
| H^{α}(³J_{HN-Hα}) | 4.18(6.9) | 4.15(6.1) | 4.17(6.3) | 4.15 (6.2) | 4.19 (6.6) | 4.20 (6.6) |
| H^{β} | 2.04,1.95 | 2.13,2.04 | 2.09,2.04 | 2.12.2.05 | 2.04.1.95 | 2.05, 1.96 |
| H^{γ} | 2.32* | 2.44,2.38 | 2.38* | 2.39* | 2.32* | 2.31* |

| Gln12 | | | | | | |
|---|---|---|---|---|---|---|
| H^{N} | 8.40 | 7.97 | 8.11 | 7.87 | 8.34 | 8.31 |
| H^{α}(³J_{HN-Hα}) | 4.16(7.0) | 4.15(6.7) | 4.17(6.7) | 4.15 (6.7) | 4.18(7.0) | 4.18 (6.9) |
| H^{β} | 2.04,1.93 | 2.08,1.99 | 2.07,1.96 | 2.08, 1.98 | 2.05,1.94 | 2.06, 1.93 |
| H^{γ} | 2.32* | 2.41,2.37 | 2.36* | 2.39,2.35 | 2.31* | 2.32* |

| | | | | | | |
|---|---|---|---|---|---|---|
| ^{a} Chemical shifts obtained at pH 4.5 and 5°C. Shifts are relative to the internal H₂O resonance at 4.96 parts per million (p.p.m.), and are accurate to ± 0.02 p.p.m. ^{b 3}J_{HN-Hα} are listed in parentheses in units of hertz (Hz). | | | | | | |

Representative TOCSY and ROESY spectra of a diamide-constrained peptide (1c) are shown in Figures 10 and 3. A summary of the H^{N}-H^{N} and H^{α}-H^{N} ROEs between neighboring residues used to make these assignments are depicted in Figure 4 for **1b** and **1c.** The degree of helicity of each peptide was judged from these spectra by the presence of intense sequential H^{N}-H^{N} ROE cross-peaks, the presence of *I, I+3* H^{α}-H^{N} or H^{α}-H^{β} ROE cross-peaks and ³J_{HN-Hα} less than 6.0 Hz. The data summarized in Figure 4 indicate that peptide **1c** is helical between residues Asn2 and Gln10. Beyond Gln10, ³J_{HN-Hα} rises above 6.0 Hz but some medium range ROEs are still present, indicating partial or transient helical character. Such fraying at helix termini is commonly observed in NMR studies of peptides and proteins. The ¹H chemical shifts of **1c** change by less than 0.02 ppm over the concentration range 8.0-0.06 mM; this indicated that the helical conformation was not stabilized by a self-association event.

The incorporation of the diamide cross-link in peptides **1** and **2** clearly reduced the mean value of ³J_{HN-Hα} in the restrained region, increased the number of observable *(I, I+3*) ROEs and increased the percent helicity observed by circular dichroism (CD) as shown in Table 3 below. Thus, peptides **1c-1e** and 2c-2e were significantlymore helical than the control peptides **1b** and **2b**. The results for peptide 4 indicated that formation of the disulfide bond constrained the peptide to be helical. However, a number of medium-range ROEs could not be observed and ³J_{HN-Hα} values were greater than 6.0 Hz for the two thiolysine residues and Leu9 in 4b; this indicated a distortion from an ideal helical structure in the region of the D-thiolysine residue, as expected from simple structural considerations. The data in Table 3 below indicated that incorporation of the disulfide bond in peptide 3b did not impart helical character, suggesting that the thiolysine method may have a dependence on primary sequence and is therefore not generally applicable.

**Table 3. Evaluation of peptide helicity.**

| **Peptide** | **Description** | **Mean ³J_{NH-Nα} in constrained region** | **Fraction of medium-range ROEs obs.** | **Percent Helicity by CD** |
|---|---|---|---|---|
| **1b** | Apamin, N-methyl Gln Control | 6.00 | 0.14 | 20 |
| **1c** | Apamin, 3 carbon linker | 4.98 | 0.69 | 84 |
| **1d** | Apamin, 4 carbon linker | 5.18 | 0.56 | 63 |
| **1e** | Apamin, 5 carbon linker | 4.96 | 0.69 | 100 |
| **2b** | C-tide, N-methyl Gln Control | 5.89 | 0.08 | 32 |
| **2c** | C-tide, 3 carbon linker | 4.81 | 0.75 | 60 |
| **2d** | C-tide, 4 carbon linker | 4.83 | 0.43 | 82 |
| **2e** | C-tide, 5 carbon linker | 4.90 | 0.80 | 63 |
| **3a** | Apamin, S-Acm thiolys control | 6.01 | 0.03 | 10 |
| **3b** | Apamin, thiolys disulfide | 5.96 | 0.08 | 35 |
| **4a** | C-tide S-Acm thiolys control | 5.96 | 0.05 | 19 |
| **4b** | C-tide, thiolys disulfide | 5.65 | 0.48 | 27 |

Values below 6 for the mean 3-bond NH-Na coupling constant indicate helicity. Medium range *I*-*I*+*3* ROEs are expressed as the observed fraction of the total number of such ROEs possible, with very weak ROEs counted as one half. Percent helicity as determined by CD is derived as by Lyu *et al.,;* Sherman, J.C.; Chen, A.; *Kallenbach,N.R., Proc. Natl. Acad Sci. U.S.A.* **88:**5317-5320 (1991), and Johnson, W.C.; Tinoco Jr., I., *J. Am Chem. Soc.,* **94:** 4389-4390 (1972)

Peptide 1c was chosen for a more detailed analysis by NMR. ROESY spectra with higher sensitivity (increased total acquisition time and peptide concentration) and NOESY spectra were acquired and analyzed to provide input restraints to structure calculations. In addition to the ROEs described above, H^{α}-H^{N} (*I,I+4*) interactions were observed, indicating that the helical conformation adopted is not of the 3₁₀ type, but rather is of the regular α helical variety (Wüthrich, K. (1986) *NMR of Proteins and Nucleic Acids.,* Wiley, New York). Interproton distance restraints were generated from the ROESY and NOESY data, and used as a basis for calculating a structure for 1c using distance geometry (DG) and restrained molecular dynamics (rMD). Nearly half (66) of the 141 restraints were between amino acids two to four residues apart in the primary sequence, as expected for a helical conformation. Dihedral angle restraints, based on observed ³J_{HN-Hα} and ³J_{Hα-Hβ}, were also used in these calculations, but explicit hydrogen bond restraints were not utilized.

The final ensemble of 20 structures is depicted in Figure 5. The structures agreed with the input data very well, with no distance restraint violations above 0.1 angstroms (Å), no dihedral angle violations above 1.0°, and a mean restraint violation energy term of 0.10 ± 0.09 kilocalories/mole(kcal·mol⁻¹). The available NMR data define well the backbone atoms of residues Thr1 to Gln10 (average root mean squared deviation from the mean structure = 0.38 ± 0.08 Å), but the two C-terminal glutamine residues are not well defined. The side chains of Thr1, Gln3, Asp4, Leu6 and Gln10 have well defined χ₁ values, but only Gln10 has a consistent value of χ₂ in all structures.

H^{N}(*I*)*-*O(*I-4*) hydrogen bonds are were to the amide protons of Leu5, Ala6, and Gln10 in greater than 90% of the structures, indicating that these residues adopted a predominantly α-helical conformation. Although (*i*,*i*-4) hydrogen bonds were observed to the amide protons of Ala7, Arg8 and Arg9 in approximately 50% of the structures, H^{N}(*I*) - O(*I-3*) hydrogen bonds were present in 25-35% of the structures, indicating that there was a slight distortion of the helix in this region. The data presented in Table 4 below indicated that the amide hydrogens of Leu5 to Gln10 were all protected from exchange with solvent in peptide 1c compared to the control peptide 1b by factors of up to 25. This observation is also consistent with the amide hydrogens of these residues participating in hydrogen bonds. Interestingly, hydrogen bonds from Asp4 H^{N} to Thr 1 O^{γ2} were present in 80% of the structures, indicating that an N-cap hydrogen bonding interaction (Harper, E.T.; Rose, G.D. *Biochemistry* **1993,** *32:*7605-7609) was present even in this short peptide. However, the amide proton of Asp4 was not noticeably protected from exchange (Table 4), hence this hydrogen bond may be more transient.

**Table 4. Amide hydrogen exchange rates constants^{a} and protection factors^{b} for peptide 1b and 1c**

| **Residue** | **log *k* (1b)** | **log *k* (1c)** | **Protection Factor** |
|---|---|---|---|
| Thr1 | -2.44 | -2.48 | approx. 1 |
| Asn2 | n.d. | n.d. | - |
| Gln3 | n.d. | n.d. | - |
| Asp4 | -2.72 | -2.84 | 1.3 |
| Leu5 | -2.69 | -3.51 | 6.7 |
| Ala6 | -2.73 | -3.77 | 10.9 |
| Ala7 | -2.51 | -3.55 | 11.1 |
| Arg8 | n.d. | -3.33 | >26 |
| Arg9 | n.d. | -3.21 | >20 |
| Gln10 | n.d. | -3.29 | >25 |
| Gln11 | n.d. | -1.83 | approx. 1.0 |
| Gln12 | n.d. | n.d. | - |

| | | | |
|---|---|---|---|
| ^{a} The rate constants are expressed in units of seconds⁻¹ (s⁻¹). n.d. indicates that the exchange was sufficiently fast that no peak was observed in the NMR spectrum acquired 300 seconds (s) after addition of D₂O. In these cases, assuming that greater than 90% of the hydrogens have exchanged in 300 s allows a lower limit of 0.013 s⁻¹ (log *k* = -1.89) to be calculated for the rate constant. ^{b} Protection factors are calculated as the rate constant for peptide **1c** divided by that of peptide **1b.** | | | |

With the exception of the ψ angles of Ala6 and Gln10, the backbone dihedral angles throughout the tethered region were close to those expected for an ideal α helix (mean φ = -63°±8°, mean ψ = -42°±8°) indicating that any deviation from ideality was very slight. The ψ of Ala6 is was 15 ° lower than expected for an α helix and was more similar to that expected for a ³10 helix; the higher value of ψ for Gin 10 0 reflected the fraying beyond the tethered region. The slight distortion at Ala6 could be the result of the short tether present in this peptide (only three methylene groups). Although the diamide linkage was not well defined by the NMR data, the side-chains of Gln3 and Gln10 adopted conformations close to those predicted by the modeling experiments described above (Gln3 χ₁ = -173°±17°, χ₂ = 34°±47°; Gln10 χ₁ = -71°±7°, χ₂= 174°±22°). The overall conclusion was that in solution, 1c adopted an α helical structure from Asp2 to Gln10 with an N-terminal capping box and a very slight distortion in the central turn of the helix.

### Circular Dichroism

CD spectra were acquired on aqueous solutions of 1-4 between 20 and 120 micromoles/liter (µM) at 280K, pH 5. Spectra of peptides **11** and **3** (apamin sequence) are shown in Figure 8 and those of peptides 2 and 4 (C-peptide sequence) in Figure 8. Numerical values for percent helicity, calculated from the per-residue molar ellipticity of the peptides at 222 nanometers (nm), are shown above in Table 3.

The CD data supported the conclusions derived from the NMR studies. Both tethering methods substantially enhanced the helicity of the C-peptide sequence (Figure 8). However, only the diamide method was capable of rendering the apamin sequence helical under the conditions used; the thiolysine-constrained peptide **3**b did not appear to be helical (Figure 7). The CD spectrum of **4b**, in spite of substantial negative ellipticityat 222 nm, showed several features which indicated a lesser degree of helicity than those of **2c-2e:** the short-wavelengthminimum in **4b** was shifted from 208 nm (a typical value for an α helix) to **204** nm, and the observable shoulder of the 190 nm maximum was much smaller than those of **2c-2e.**

Thermal denaturation experiments were performed on the apamin-basedpeptides **1b-1e.** In the initial experiment, CD spectra of peptides **1b-1e** were taken at 10 °C intervals from 7 °C (280 K) to 57 °C (330 K). Given that **1c-1e** showed good retention of helicity in this temperature range, spectra of **1c** were taken up to 97 °C (370 K), where some loss of helicity was observed (Figure 6). The molar ellipticity of **1c** at 97 °C and 222 nm was still substantially more negative than that of the non-helical control peptide **1b** at 7 °C and 222 nm.

Experiments to examine the effects of heating and recooling the peptides were complicated by several factors: the CD spectrometer showed a baseline drift over long experiments; the concentration of the samples changed because of evaporation at higher operating temperatures; and there appeared to be some variation in sample behavior depending on the rates of heating and cooling. A set of CD spectra of **1c** was acquired before, during, and after heating at 87 °C for one day. The effect of baseline drift was reduced by linear normalization of the spectra based on Θ₂₄₅. The effect of concentration change due to sample evaporation was corrected by normalizing the post-heating spectrum to the same amplitude as the pre-heating spectrum at wavelength (λ) of 204 nm. This wavelength was chosen as the point where an α helix and a random coil have equal contributionsto the ellipticity, and hence interconversion of a peptide between these conformations will not affect the magnitude of the ellipticity. The resulting spectra are shown in Figure 9. The close match in curve shape between the pre- and post-heating spectra indicated that most or all of the helical structure was regained on cooling after the partial denaturation induced by heating at 87 °C. The small difference in overall amplitude could be due to a small amount of permanent denaturation or could be an artifact of the normalization procedure. This experiment demonstrated that the α helix of **1c** was stable to relatively harsh conditions, a feature which improves its general utility.

### Conclusion

A new method for constraining small peptides to an α helical conformation has been devised. This *I* to *I+7* amide-based tether is successful as a general method for inducing α helicity in small peptides and possesses several desirable features. First, it allows the maximum possible sequence variability. Any residue except the two tethering residues themselves may be changed. Second, the helicity induced by this method approaches 100% in aqueous solution at room temperature (RT). The comparison of helical peptides **1c-1e** with non-helical peptide 1 b shows that the helicity is achieved by introduction of the linker rather than being a property of the primary sequence. Third, these tethered peptides are synthesized by standard solid-phase (Merrifield) chemistry and require only inexpensive,commercially available reagents. Fourth, the method can be used for peptides as short as eight residues. Fifth, it poses no chemical requirementsas to environment and has been shown to induce good helicity despite changes in temperature and buffer conditions. This method is generally useful for studies of biologically active helical regions of proteins, for the experimental study of helix formation, propagation, and stability, and for physical organic experiments on the interactions of helical peptides with their environments.

### EXAMPLE 2

The peptide cyclized peptide FNM(S)QQRRFY**(6)**ALH (Fig. 11) was synthesized using Fmoc chemistry with standard solid phase protocols in which Fmoc-glutamic acid, δ-(5-allyloxycarbonyl-1,5-diaminopentane)(5) (synthesized as described below) and Fmoc glutamic acid δ-allyl ester **(6)** (commercially available from Millipore) are incorporated as standard amino acids in peptide synthesis, followed by cyclization as shown in Fig. 11. Fmoc-glutamic acid, δ-(5-allyloxycarbonyl-1,5-diaminopentane) **(5)** was synthesized as shown in Scheme 1 below.

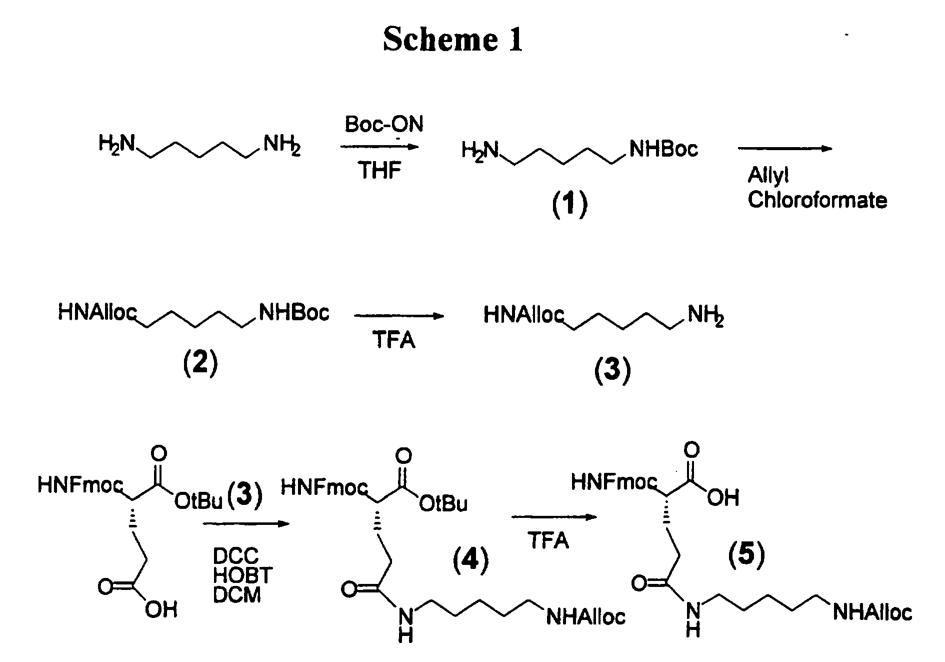

Mono-t-butyloxycarbonyl (BOC) 1,5-pentanediamine was synthesized by using 1,5-diaminopentane (12.5 g, 122 mmol) in place of 1,3-diaminopropane in the synthesis of mono-allyloxycarbonyl-1,3-diaminopropanedescribed in Example 1 above, yielding 10 g (49 mmol) of mono-*tert-*butyloxycarbonyl-1.5-diaminopentane**(1)**. The mono-*tert*-butyloxycarbonyl-1,5-diaminopentane **(1)** (5.8 g, 28.7 mmol) was dissolved in 75 mL of dichloromethane with 7.5 mL of diisopropylethylamine and cooled to 0° C. A solution of allyl chloroformate (3.3 mL) in dichloromethane (25 mL) was added over five minutes. The reaction was allowed to warm to room temperature for one hour and then solvent was removed by rotary evaporation. The residue was dissolved in 100 mL of ethyl acetate and washed with three 100 mL portions of 10% citric acid, once with 100 mL saturated aqueous sodium bicarbonate and once with 100 mL of saturated aqueous sodium chloride. The organic phase was dried over magnesium sulfate and solvent was removed by rotary evaporation. The resulting oil (2) was treated with 25 mL of trifluoroacetic acid for 30 minutes. The trifluoroacetic acid was removed by rotary evaporation and the resulting reside was twice dissolved in dichloromethane and then evaporated to remove residual solvent. The residue was dissolved in 50 mL of 3N hydrochloric acid and washed with two 50 mL portions of dichloromethane. The aqueous phase was cooled in an ice bath and the pH was adjusted to approximately 13 with 50% aqueous sodium hydroxide. The basic aqueous phase was extracted with three 100 mL portions of dichloromethane, the combined organics were washed with 100 mL of saturated aqueous sodium chloride and then dried over potassium carbonate. The mixture was filtered, the solvent removed first by rotary evaporation and then by high vacuum to yield 3.95 g of mono-allyloxycarbonyl-1,5-diaminopentane (3) as a colorless oil.

Fmoc-Glutamicacid, α-*tert*-butyl ester, 9.0 g (21.1 mmol, Bachem California) was dissolved in 100 mL of dichloromethane.Dicyclohexylcarbodiimide(4.4 g, 21.3 mol) and N-hydroxybenzotriazole (0.3 g, 2.1 mmol) was added to this solution, followed by the mono-allyloxycarbonyl-1,5-diaminopentane (3) (3.95 g, 21.2 mmol). The reaction was stirred at 25° C for 14 hours, then cooled to 0° C for one hour. Insoluble material was removed by filtration, and the filtrate was concentrated by rotary evaporation. The residue was dissolved in 150 mL of ethyl acetate and washed twice with 100 mL of 10% aqueous citric acid, twice with 100 mL of saturated aqueous sodium bicarbonate and once with 100 mL brine. After drying over magnesium sulfate and filtering the solvent was removed by rotary evaporation. The residue was dissolved in approximately 75 mL of ethyl acetate with heating and 2:1 hexanes:ethyl acetate was added until the solution became cloudy. After standing for several hours the crystalline precipitate was removed by filtration, the white crystals were washed with 2:1 hexanes:ethyl acetate and dried under vacuum to yield 11.4 g of (**4**) (90%).

The *tert* -butyl ester (**4**), 11 g, 18.5 mmol) was dissolved in 50 mL of trifluoroacetic acid with stirring. After 45 minutes, the trifluoroaceticacid was removed by rotary evaporation;residual trifluoroacetic acid was removed by evaporation from 50 mL of dichloromethane three times. The residue was dissolved in 75 mL of ethyl acetate with heating, filtered through celite, and 3:1 hexanes:ethyl acetate was added until a haze developed. Crystals were allowed to grow at 25° C for three hours, then cooled to 0° C for one hour. The crystals were isolated by filtration and washed with 3:1 hexanes:ethyl acetate, then dried under vacuum to yield 9.5 g (95%) of (**5**) as off white crystals.

Followingpeptide synthesis of FNM(5)QQRRFY(6)ALH, the N-terminus of the solid phase peptide was coupled to mono *tert*-butyl-succinicacid the allyl and allyloxycarbonyl protecting groups were removed using 500 mg Pd(PPh₃)₂Cl₂ in 20 mL of 20% piperidine in dimethyl acetamide for 1.5 hours at room temperature. The resin was then washed with 20% piperidine in dimethyl acetamide, dimethyl acetamide, dichloromethane and finally with 0.5% trifluoroacetic acid in dichloromethane. The resin was suspended in dichloromethane and 1.5 equivalents of HATU with 3 eq N,N-diisopropylethylamine in 5 mL of dimethyl acetamide was added. After two hours the resin was checked for free amines by ninhydrin test and found to be negative. The peptide was cleaved from the resin with 95% trifluoroacetic acid 5% triethylsilane and purified using reverse phase HPLC.

The helical structure of the cyclized peptide shown in Fig. 11 was confirmed by circular dichroism (CD) and nuclear magnetic resonance (NMR). Both of these methods indicated that the locked helix peptide displayed predominantly α-helical character. The locked helix peptide was determined to bind IgG with an affinity (Kd) of approximately 100 µM both by microcalorimetry and surface plasmon resonance. A control peptide lacking the locking portion of the molecule did not exhibit IgG binding detectable by microcalorimetry.

### EXAMPLE 3

To confirm that the covalent locking mechanism is fully functional and that peptides constrained by this technique are able to bind ligand with high affinity, a 33 amino acid peptide based on helix 1 of the Z domain of protein A was synthesized with the i to i + 7 linkage as shown in Scheme 2 below: 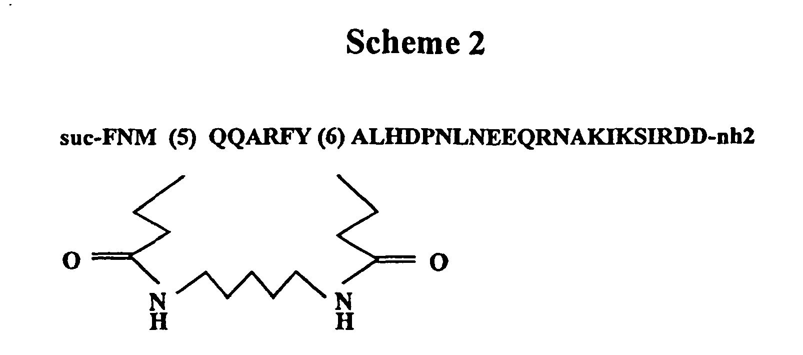 where (5) and (6) are the allyloxycarbonyl and allyl protected amino acids described in Example 2 above. The peptide was synthesized and cyclized as described in Example 2 above. The helicity of the peptide was verified by CD and NMR, and thermal denaturation of the peptide as monitored by CD indicated that the peptide only partly unfolds at 90° C, consistent with the stability of the covalent linkage. The IgG binding affinity (Kd) of this peptide (as measured by surface plasmon resonance) was determined to be approximately 20 nM.

### EXAMPLE 4

Linear peptides derived from the ectodomain of the HIV-1 envelope protein gp41 are known to inhibit viral fusion events. The most potent of these (DP178) corresponds to a membrane proximal region of gp41, which is predicted to be α-helical. However, DP178 itself lacks discernable structure in solution, rendering mechanistic interpretation of its activity difficult. By applying the helix locking chemistry taught herin, constrained versions of DP178 were made to determine whether helicity is necessary or sufficient for its infectivity inhibition activty and to define a likely mode of action for this molecule in primary infection (as measured using viral infectivity assays).

By constraining DP178 analogs into a helical conformation we show that helicity is necessary, but not sufficient, for inhibitory potency. The correct face of the helix must also be exposed. Two recent crystal structures of gp41 indicate that this face is buried in a groove formed by a coiled-coil trimer. Taken together, these results indicate that DP178 inhibits infectivity by blocking this groove, and that the conformation of gp41 observed by crystallography represents the fusogenic state.

A series of analogs of DP178 in which segments of the amide backbone were constrained to be helical (Figure 12) were prepared. Because short α-helices are usually unstructured in solution (Marqusee et al. *Proc. Natl. Acad Sci. USA* 86:5286-5290 (1989)), a covalent crosslink between amino acid side chains at positions *i* and *i+7* of the polypeptide chain as taught herein (see also Phelan et al., *J. Am. Chem. Soc.* 119:455-460 (1997), which lock the intervening residues into a stable α-helical conformation.

A truncated form of DP178, designated HIV35 (Figure 12) was used as a reference. In the absence of detailed information regarding the associaton of DP178 with DP107, the coiled-coil propensities (Lupas et al., *Science* 252:1162-1164 (1991)) for 29 distinct gp160 sequences were computed in order to determine whether the region corresponding to DP178 scored as a coiled-coil. The N-terminal 27 residues of DP178, selected for the reference peptide HIV35, maintained a high overall score with a consistent heptad register. The "a-d" face predicted by the scoring algorithm corresponded to the face seen to pack against the trimer core. This corresponding region is entirely helical in the x-ray structures, and packs against the trimer core using a 4-3 heptad repeat akin to that found in coiled-coils. Using the helical locking chemistry and methods taught herin we enforced the exposure of this repeat (positions "a" and "d" of the heptad) by introducing crosslinks between pairs of adjacent residues on the opposite face of the helix (position "f"). Thus, "f" to "f" (tethers) locks were made to constrain the potential helix. Analogs of HIV35 (Figure 12) were prepared containing either one (HIV24) or two (HIV31) tethers to impart increasing helicity. A control peptide (HIV30) was prepared in which a tether was introduced between successive "d" residues to stabilize helicity while blocking potential binding interactions across the "a-d" face.

Linear peptides were synthesized according to standard solid phase techniques using Fmoc chemistry (Fields et al., *Int. J. Peptide Protein Res.* 35:161-214 (1990)) as taught herein. In particular, helix dipole effects were minimized by blocking the C-termini as amides and the N-termini as succinate groups. After formation of the lactam bridges as taught herein (see also Phelan et al., *J. Am. Chem. Soc.* 119:455-460 (1997)), the peptides were cleaved from the resin and purified to homogeneity using preparative reversed phase HPLC with water/acetonitrile/0.1% TFA gradients in the mobile phase. The identity of each peptide was confirmed by electrospray mass spectrometry: HIV 24, calculated mass 3396.8, observed, 3396.0; HIV 30, calculated mass 3413.7, observed, 3413.8; HIV 31, calculated mass 3520.0, observed, 3520.7; HIV 35, calculated mass 3330.8, observed, 3330.5.

Circular dichroism analysis (Figure 13) confirms that the locking strategy markedly increases the helicity of the DP178 truncations. CD spectra were recorded on an AVIV 62DS CD spectrometer using 0.05 cm pathlength cuvettes. Spectra were gathered by averaging data from three runs spanning 250 nm to 190 nm in 1.0 nm increments, with 2 second averaging time at each wavelength. Peptide concentrations were approximately 200 µM in a solution of 10 mM Tris HCl pH 7.5 with 6% acetonitrile (v/v). For conversion of raw data to molar ellipticity values, precise concentrations were determined by measuring A₂₇₆ and A₂₈₀ (Edelhoch, *Biochemistry* 6:1948-1954 (1967)); these values were confirmed by quantitative amino acid analysis.

The unconstrained peptide HIV35 has an almost featureless spectrum, similar to that reported for DP178 (Lawless, et al., *Biochemistry* 35:13697-13708 (1996)). The CD spectra of peptides containing a single constraint (HIV24 and HIV30) display minima at 209 and 222 nm characteristic of α-helices. The intensity ratios of these two regions are skewed from ideality, suggesting that regions of the peptide backbone outside the constrained segment are disordered. By constrast, the doubly-constrained analog HIV31 appears to be largely helical by CD, giving the shape and intensity profile of a typical α-helix.

Viral infectivity assays were used to characterize the locked-helix constructs. Normal human peripheral blood mononuclear cells (PBMCs) were stimulated with phytohemagglutinin (PHA) in RPMI 1640 medium containing interleukin 2 for 24 hours. The PHA medium was removed and the cells grown overnight in RPMI 1640 with glutamine, 20% heat inactivated fetal calf serum, and gentamicin. At the start of the assay, pre-titered virus stocks were equilibrated with peptides for one hour before adding to the PBMCs (2.5 x 10⁵ cells per well). Cells were grown for three days, rinsed to remove extracellular virus and peptides, then supplemented with fresh medium and grown for an additional four days. After seven days the cells were lysed and p24 antigen was determined by ELISA. Peptides were run in triplicate at each concentration. Viral titers were determined in duplicate for each run. Each assay also included the following controls, in triplicate: Uninfected cells as a negative control, infected cells without peptide as a positive control, and virus innoculum without cells to establish a baseline p24 level. Peptides were tested for cytotoxicity by incubating them at the highest assay concentration (approximately 100 µM) with uninfected cells and then growing the cells as described above. After 7 days the cell counts were estimated by microscopy and compared to an identical batch of cells which were not treated with the peptides; none of the peptides inhibited normal cell growth under these conditions.

When tested in viral infectivity assays, the peptides displayed a striking pattern of relative potency that extended across both syncitium inducing (SI) and non-syncitium inducing (NSI) strains of HIV-1 (Zhang et al., *Nature* 383:768 (1996)). As shown in Figures 14A and 14B, truncating the hydrophobic C-terminus of DP178 (HIV35) caused a dramatic drop in activity, which was partially restored when a single restraint, i.e. constrained helical peptide, (and partial α-helical character) was introduced (HIV24). Adding a second restraint (HIV31) imparted strong helical character and enhanced the potency of the peptide to levels comparable to DP178. Thus, the additional stabilization afforded by preorganizing HIV31 into an active helical conformation offset the loss of binding energy caused by deleting the C-terminus. By contrast, a single restraint that induced helicity while blocking the "**a-d**" face (HIV30) completely ablated activity.

A series of shorter constrained peptides spanning positions 631-644, 643-656, 649-662, 656-669, and 663-678 of HIV-1_{LAI}, tethered between adjacent residues at the "f' positions of the heptad, were prepared to determine whether a subset of HIV35 or its N- and C-terminal flanking regions was sufficient to block infectivity. All peptides, whether constrained or unconstrained, failed to show significant activity. Peptide 631-644 contains the hydrophobic cluster observed in the x-ray structure to pack into a cavity in the trimer core (Chan et al., *Cell* 89:263-273 (1997)).

The relative activities of HIV35, HIV24, and HIV31 demonstrate a clear correlation between helicity and inhibitory potency. The widely disparate activities of HIV30 and HIV24 indicate that peptide inhibition also requires exposure of the face of the helix seen by crystallography to pack against the N-terminal trimer core of gp41.

The data presented herin, combined with prior model studies on isolated peptides and the recently published crystal structures, strongly support the hypothesis that the peptides inhibit viral infectivity by binding to the resting state of gp41 and preventing formation of the fusogenic state. Peptide HIV31 is conformationally constrained to be largely helical, and is likely to interact as such with an accessible cognate surface in the resting state of gp41. Because x-ray analysis shows that the face of HIV31 required for inhibiting viral fusion is buried in the groove formed by the N-terminal trimer core, we believe (without being bound to any particular theory) that this groove represents the cognate surface for the peptides.

Figure 15 outlines schematically a current model for assembly of the fusgenic state of gp41, and the mechanism by which the constrained helices inhibit this process. The model is presented without meaning to be limiting to the invention and without binding the inventors to any particular theory of operation of the invention. The resting state of gp41 (upper left) is presumed to be constitutively trimerized, featuring a coiled-coil bundle near the N-terminal fusion peptide (arrow). The region corresponding to the C-terminus of the ectodomain (dark lines) is not initially bound to the trimer bundle, and has an unknown conformation. A conformational shift resulting from the binding of gp120 to either CD4, a co-receptor, or both, may then allow association of the C-terminal portion of gp41 with the N-terminal bundle. The resulting antiparallel helical array (top right) observed in the x-ray structures is presumably the fusogenic state of gp41. Rearrangement to this state can be blocked if the trimer grooves are occupied by inhibitory peptides (bottom left). Once blocked in this manner, a subsequent conformational shift in the gp41 cluster would sequester the protein off-pathway (bottom right).

Peptides DP178 and HIV24 effectively inhibit the infectivity of genetically distant and phenotypically distinct subtypes of HIV-1 (Gao et al., *Journal of Virology* 70:1651-1667 (1996)). Moreover, the surface to which they are proposed to bind is one of the most highly conserved regions in the HIV-1 genome. We have assayed DP178 against other strains and found it to have similar inhibitory potency against the laboratory-adapted strain MN/H9 and primary isolates 301660 and Th009. Strain Th009 is from subtype E and is genetically distant from the predominant North American subtype B (e.g. JRCSF) (Zhang et al., *Nature* 383:768 (1996)). These results are in accord with observations from other labs (Wild et al., *Proc. Natl. Acad. Sci. USA* **89:**10537-10541 (1992); Wild et al., *Proc. Natl. Acad. Sci. USA* 91: 9770-9774 (1994); Jiang et al., *Nature* 365:113 (1993)). In addition to JRCSF and BZ167, we tested HIV24 against Th009 and found it to have comparable potency, suggesting that the membrane fusion mechanism proposed extends to widely disparate strains of HIV-1.

Other agents, such as antibodies, which target this surface may thus hold promise for the therapeutic treatment of AIDS.

### EXAMPLE 5

To prepare a vaccine that would be effective against HIV infection, either as a prophylactic or post-infection therapeutic (optionally in combination with anti-HIV drugs or other subunit vaccines), constrained α-helical peptides from the 633-678 region of gp41 were prepared and used as immunogens.

Variants of HIV 24 were prepared with the sequence "Gly Gly Cys" at the C-terminus or "Cys Gly Gly" at the N-terminus. These peptides were conjugated to KLH using a heterobifunctional crosslinker such as 4-(N-Maleimidomethyl)-cyclohexane-1-carboxylic acid 3-sulfo-N-hydroxysuccinimide ester, available from Sigma, or its equivalent (e.g. "Sulfo-MBS" from Pierce). Immunizations were performed as described below.

Polyclonal antibodies were generated in female guinea pigs (Hartley Strain from Simonson Labs) against KLH-conjugated HIV peptides. Fifty µg peptide in 250 µL PBS was emulsified with 250 µL Freund's adjuvant (complete adjuvant for the primary injection and incomplete adjuvant for all boosts). Injections of 70-100 µg peptide/kg body weight were administered with a combination of subcutaneous and intramuscular sites in a three-week cycle. Bleeds were taken on the second and third weeks following each boost.

Sera from immunized animals was loaded on a Protein A column to provide, on elution, purified total Ig. Antibodies selective for the locked helices were obtained by passing the total Ig pool over an affinity column containing support loaded with immobilized locked helices. This support was prepared by first reacting the cysteine-containing peptides described above (HIV 26, 27, 28, and 29) with biotin-maleimide (also from Sigma; N-biotinyl-N'-[6-maleimidohexanoyl]-hydrazide) to afford peptides biotinylated at either terminus. These peptides were loaded onto a resin pre-loaded with streptavidin (Pierce, "Ultralink Avidin") to provide the affinity gel described.

The total Ig pool from the protein A column was passed over the appropriate affinity column (i.e. the one with the matching hapten immobilized). Nonspecific antibodies were eluted in the flow-through and saved as negative controls. Specific antibodies were eluted as from the Protein A column, dialyzed into assay buffer, and stored.

Surprisingly, the antibody titers observed were quite high for gp41 subunit peptides. This is particularly surprising since this region of gp41 (633-678) is not known in the art to generate HIV neutralizing antibodies.

The affinity purified polyclonal antibodies are tested in the viral infectivity assays used to evaluate the peptides. The haptens used to generate polyclonal antibody preparations that inhibit infectivity are desirable immunogenic agents for use in a vaccine. Most preferred are candidates that elicit broadly cross-reactive antibodies able to neutralize a variety of diverse HIV-1 isolates *in vitro.*

Candidate HIV-1 vaccines can be tested in available animal models, for example, in chimpanzees as described by Berman et al., *J. Virol.* 7:4464-9 (1992); Haigwood et al., *J. Virol.* 66:172-82 (1992) and Salmon-Ceron et al., *AIDS Res. and Human Retroviruses* 11:1479-86 (1995) for gp120 subunit vaccines. Most preferred are candidates that elicit broadly cross-reactive antibodies able to neutralize a variety of diverse HIV-1 isolates in these animal studies, providing protection from challenge by homologous and heterologous strains of HIV-1. Successful protection of chimpanzees is encouraging and has historically proved to be a reliable indicator of vaccine efficacy.

## Claims

1. A method of constructing a constrained helical peptide, comprising the steps of:
(a) synthesizing a peptide, wherein the peptide comprises a sequence of eight amino acid residues, wherein the sequence of eight amino acid residues has a first terminal residue and a second terminal residue, wherein the first terminal residue and the second terminal residue flank an internal sequence of six amino acid residues, and wherein the first and second terminal residues have a side chain containing an amide bond-forming substituent;
(b) providing a difunctional linker having a first functional group capable of forming an amide linkage with the side chain amide bond-forming substituent of the first terminal residue and having a second functional group capable of forming an amide linkage with the side chain amide bond-forming substituent of the second terminal residue; and
(c) cyclizing the peptide by reacting the side chain amide bond-forming substituent of the first terminal residue with the first functional group of the difunctional linker to form an amide linkage and reacting the side chain amide bond-forming substituent of the second terminal residue with the second functional group of the difunctional linker to form an amide linkage, yielding a constrained helical peptide.

2. The method of claim 1 wherein in step (a) the side chain amide bond-forming substituent of the first terminal residue is protected with a first protecting group and the side chain amide bond-forming substituent of the second terminal residue is protected with a second protecting group, wherein the first protecting group and the second protecting group are differentially removable, and wherein in step (c) the first protecting group is removed such that the side chain amide bond-forming substituent of the first terminal residue is deprotected and the side chain amide bond-forming substituent of the second terminal residue is not deprotected before the peptide is reacted with the difunctional linker, and thereafter the peptide is reacted with the difunctional linker to form an amide linkage between the side chain amide bond-forming substituent of the first terminal residue and the first functional group of the difunctional linker, and thereafter the second protecting group is removed from the side chain amide bond-forming substituent of the second terminal residue and the peptide is cyclized by intramolecularly reacting the side chain amide bond-forming substituent of the second terminal residue with the second functional group of the difunctional linker to form an amide linkage.

3. A method of constructing a constrained helical peptide, comprising the steps of:
(a) synthesizing a peptide, wherein the peptide comprises a sequence of eight amino acid residues, wherein the sequence of eight amino acid residues has a first terminal residue and a second terminal residue, wherein the first terminal residue and the second terminal residue flank an internal sequence of six amino acid residues, wherein the first and second terminal residues have a side chain containing an amide bond-forming substituent, wherein the first terminal residue is coupled to a difunctional linker having a first functional group and a second functional group, wherein the first functional group is in an amide linkage with the side chain amide bond-forming substituent of the first terminal residue, and wherein the second functional group of the difunctional linker is capable of forming an amide linkage with the side chain amide bond-forming substituent of the second terminal residue; and
(b) cyclizing the peptide by intramolecularly reacting the side chain amide bond-forming substituent of the second terminal residue with the second functional group of the difunctional linker to form an amide linkage and yield a constrained helical peptide.

4. A compound selected from the group consisting of:
the compound represented by Formula (1): 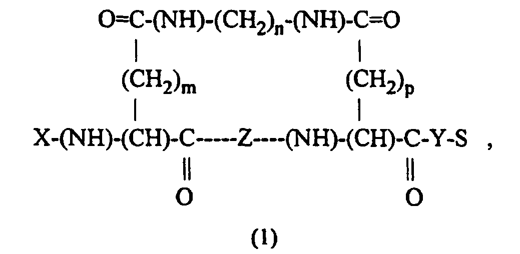
wherein S is absent or is a macromolecule, X is hydrogen or is any amino acid or amino acid sequence, Y is absent, or is hydroxyl if S is absent, or is any amino acid or amino acid sequence, Z is any amino acid sequence consisting of six amino acids; m and p are independently selected from the integers 0 to 6 inclusive, provided that m+p is less than or equal to 6, and n is any integer in the range defined by (7-(m+p)) to (9-(m+p)) inclusive, provided that n is greater than 1;
the compound represented by Formula (6): 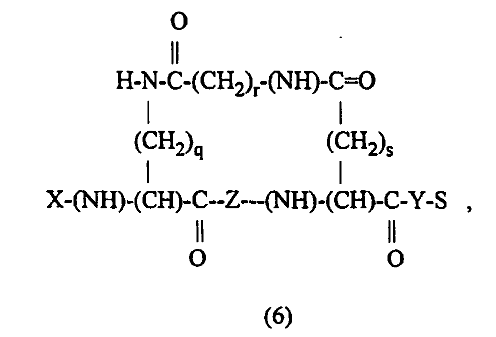
wherein S is absent or is a macromolecule. X is hydrogen or is any amino acid or amino acid sequence. Y is absent, or is hydroxyl if S is absent, or is any amino acid or amino acid sequence, Z is any amino acid sequence consisting of six amino acids, q is selected from the integers I to 7 inclusive, s is selected from the integers 0 to 6 inclusive, provided that q+s is less than or equal to 7, and r is any integer in the range defined by (7-(q+s)) to (9-(q+s)) inclusive, provided that r is greater than 0;
the compound represented by Formula (11): 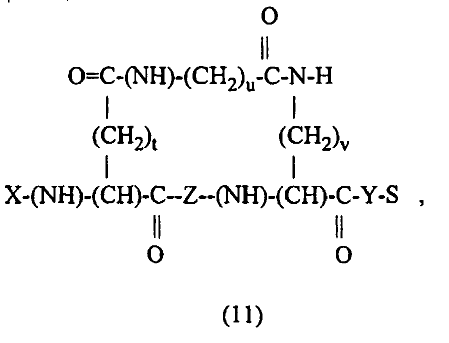
wherein S is absent or is a macromolecule, X is hydrogen or is any amino acid or amino acid sequence, Y is absent, or is hydroxyl if S is absent, or is any amino acid or amino acid sequence, Z is any amino acid sequence consisting of six amino acids; t is selected from the integers 0 to 6 inclusive, and v is selected from the integers 1 to 7 inclusive, provided that t+v is less than or equal to 7; and u is any integer in the range defined by (7-(t+v)) to (9-(t+v)) inclusive, provided that u is greater than 0; and
the compound represented by Formula (16): 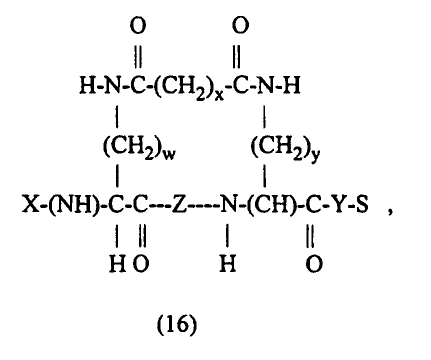
wherein S is absent or is a macromolecule, X is hydrogen or is any amino acid or amino acid sequence, Y is absent, or is hydroxyl if S is absent, or is any amino acid or amino acid sequence, Z is any amino acid sequence consisting of six amino acids; w and y are independently selected from the integers 1 to 7 inclusive, provided that w+y is less than or equal to 8, and x is any integer in the range defined by (7-(w+y)) to (9-(w+y)) inclusive, provided that x is greater than or equal to 0.

5. The compound of claim 4 that is the compound of Formula (1), wherein Z is Gln-Gln-Arg-Arg-Phe-Tyr.

6. A compound according to claim 4, wherein Z is an amino acid sequence consisting of six amino acids, wherein the internal sequence of six amino acids has the form **gabcde, defgab,** or **cdefga** and is selected from the group of sequences consisting of a sequence of six contiguous amino acids in HIV-1LAI strain gp41 amino acid sequence 633 to 678, in its homolog sequence from another HTV strain, in a consensus sequence of its homolog sequences from any one HIV clade, or amino acid substituted variant thereof, in which amino acid 633 or its corresponding amino acid in the homolog, consensus or variant sequence is assigned position a of a repeating **abcdefg** assignment.

7. The compound of claim 6, further comprising S' when S is absent and X is any amino acid or amino acid sequence, wherein S' is a macromolecule attached to X.

8. The compound of claims 6, wherein the homolog or consensus sequence is shown in Figures 16A-16M.

9. A compound of claims 6, further comprising a second constrained helical peptide.

10. An antibody that binds to a compound of claim 6, wherein the antibody specifically binds an epitope comprising an amino acid at position **a**, **d**, **e**, or **g** in the helical peptide.

11. The use of a compound of claim 6 for the manufacture of a composition for the prophylactic or therapeutic treatment of a mammal at risk of or infected with HIV.

12. The use of claim 11, wherein the composition comprises internal six amino acid sequences from different HIV strains or HIV clades.

13. A vaccine comprising at least one compound of claims 6.

14. A compound of claim 6 for use in the prophylactic or therapeutic treatment of a mammal at risk of or infected with HTV.

## Patentansprüche

1. Verfahren zur Konstruktion eines eingeschränkten helikalen Peptids, folgende Schritte umfassend:
(a) das Synthetisieren eines Peptids, worin das Peptid eine Sequenz aus acht Aminosäureresten umfasst, worin die Sequenz aus acht Aminosäureresten einen ersten terminalen Rest und einen zweiten terminalen Rest aufweist, worin der erste terminale Rest und der zweite terminale Rest eine interne Sequenz aus sechs Aminosäureresten flankieren und worin der erste und der zweite terminale Rest eine Seitenkette aufweisen, die einen eine Amidbindung bildenden Substituenten enthält;
(b) das Bereitstellen eines difunktionellen Linkers mit einer ersten funktionellen Gruppe, die zur Bildung einer Amidbindung mit dem eine Amidbindung bildenden Seitenketten-Substituenten des ersten terminalen Rests in der Lage ist, und einer zweiten funktionellen Gruppe, die zur Bildung einer Amidbindung mit dem eine Amidbindung bildenden Seitenketten-Substituenten des zweiten terminalen Restes in der Lage ist; und
(c) das Cyclisieren des Peptids durch Reaktion des eine Amidbindung bildenden Seitenketten-Substituenten des ersten terminalen Rests mit der ersten funktionellen Gruppe des difunktionellen Linkers, um eine Amidbindung zu bilden, und das Umsetzen des eine Amidbindung bildenden Seitenketten-Substituenten des zweiten terminalen Rests mit der zweiten funktionellen Gruppe des difunktionellen Linkers, um eine Amidbindung zu bilden, was ein eingeschränktes helikales Peptid ergibt.

2. Verfahren nach Anspruch 1, worin in Schritt (a) der eine Amidbindung bildende Seitenketten-Substituent des ersten terminalen Rests mit einer ersten Schutzgruppe geschützt wird und der eine Amidbindung bildende Seitenketten-Substituent des zweiten terminalen Rests mit einer zweiten Schutzgruppe geschützt wird, worin die erste Schutzgruppe und die zweite Schutzgruppe unterschiedlich entfernbar sind, und worin in Schritt (c) die erste Schutzgruppe entfernt wird, sodass der eine Amidbindung bildende Seitenketten-Substituent des ersten terminalen Rests entschützt wird und der eine Amidbindung bildende Seitenketten-Substituent des zweiten terminalen Rests nicht entschützt wird, bevor das Peptid mit dem difunktionellen Linker umgesetzt wurde, wonach das Peptid mit dem difunktionellen Linker umgesetzt wird, um eine Amidbindung zwischen dem eine Amidbindung bildenden Seitenketten-Substituenten des ersten terminalen Rests und der ersten funktionellen Gruppe des difunktionellen Linkers zu bilden, wonach die zweite Schutzgruppe von dem eine Amidbindung bildenden Seitenketten-Substituenten des zweiten terminalen Rests entfernt wird und das Peptid cyclisiert wird, indem der eine Amidbindung bildende Seitenketten-Substituent des zweiten terminalen Rests mit der zweiten funktionellen Gruppe des difunktionellen Linkers intramolekular umgesetzt wird, um eine Amidbindung zu bilden.

3. Verfahren zur Konstruktion eines eingeschränkten helikalen Peptids, folgende Schritte umfassend:
(a) das Synthetisieren eines Peptids, worin das Peptid eine Sequenz aus acht Aminosäureresten umfasst, worin die Sequenz aus acht Aminosäureresten einen ersten terminalen Rest und einen zweiten terminalen Rest aufweist, worin der erste terminale Rest und der zweite terminale Rest eine interne Sequenz aus sechs Aminosäureresten flankieren, worin der erste und der zweite terminale Rest eine Seitenkette aufweisen, die einen eine Amidbindung bildenden Substituenten enthält, worin der erste terminale Rest an einen difunktionellen Linker gebunden ist, der eine erste funktionelle Gruppe und eine zweite funktionelle Gruppe aufweist, worin die erste funktionelle Gruppe in einer Amidbindung mit dem eine Amidbindung bildenden Seitenketten-Substituenten des ersten terminalen Rests vorliegt und worin die zweite funktionelle Gruppe des difunktionellen Linkers zur Bildung einer Amidbindung mit dem eine Amidbindung bildenden Seitenketten-Substituenten des zweiten terminalen Rests in der Lage ist; und
(b) das Cyclisieren des Peptids durch intramolekulares Umsetzen des eine Amidbindung bildenden Seitenketten-Substituenten des zweiten terminalen Rests mit der zweiten funktionellen Gruppe des difunktionellen Linkers, um eine Amidbindung zu bilden und ein eingeschränktes helikales Peptid zu erhalten.

4. Verbindung, ausgewählt aus der aus Folgendem bestehenden Gruppe:
Verbindung der Formel (1): 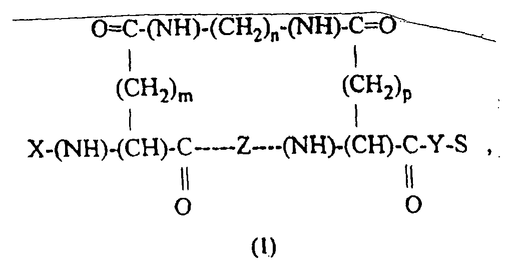
worin S nicht vorhanden ist oder ein Makromolekül ist, X Wasserstoff oder eine beliebige Aminosäure oder Aminosäuresequenz ist, Y nicht vorhanden ist oder Hydroxyl ist, wenn S nicht vorhanden ist, oder eine beliebige Aminosäure oder Aminosäuresequenz ist, Z eine beliebige Aminosäuresequenz bestehend aus sechs Aminosäuren ist; m und p unabhängig voneinander aus den ganzen Zahlen von 0 bis 6, Grenzen eingeschlossen, ausgewählt sind, mit der Maßgabe, dass m+p kleiner als oder gleich 6 ist, und n eine beliebige ganze Zahl im Bereich von (7-(m+p)) bis (9-(m+p)), Grenzen eingeschlossen, ist, mit der Maßgabe, dass n größer als 1 ist;
Verbindung der Formel (6): 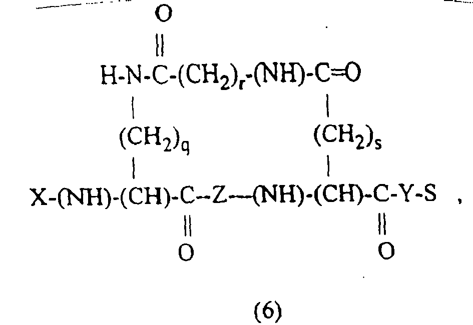
worin S nicht vorhanden ist oder ein Makromolekül ist, X Wasserstoff oder eine beliebige Aminosäure oder Aminosäuresequenz ist, Y nicht vorhanden ist oder Hydroxyl ist, wenn S nicht vorhanden ist, oder eine beliebige Aminosäure oder Aminosäuresequenz ist, Z eine beliebige Aminosäuresequenz bestehend aus sechs Aminosäuren ist; q aus den ganzen Zahlen von 1 bis 7, Grenzen eingeschlossen, ausgewählt ist, s aus den ganzen Zahlen von 0 bis 6, Grenzen eingeschlossen, ausgewählt ist, mit der Maßgabe, dass q+s kleiner als oder gleich 7 ist, und r eine beliebige ganze Zahl im Bereich von (7-(q+s)) bis (9-(q+s)), Grenzen eingeschlossen, ist, mit der Maßgabe, dass r größer als 0 ist;
Verbindung der Formel (11): 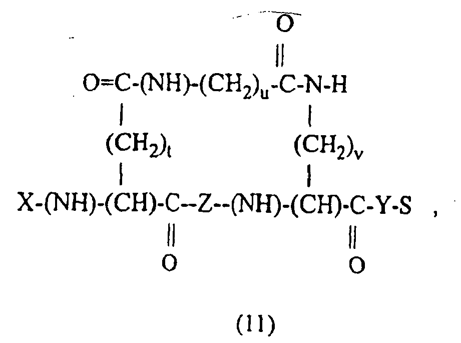
worin S nicht vorhanden ist oder ein Makromolekül ist, X Wasserstoff oder eine beliebige Aminosäure oder Aminosäuresequenz ist, Y nicht vorhanden ist oder Hydroxyl ist, wenn S nicht vorhanden ist, oder eine beliebige Aminosäure oder Aminosäuresequenz ist, Z eine beliebige Aminosäuresequenz bestehend aus sechs Aminosäuren ist; t aus den ganzen Zahlen von 0 bis 6, Grenzen eingeschlossen, ausgewählt ist und v aus den ganzen Zahlen von 1 bis 7, Grenzen eingeschlossen, ausgewählt ist, mit der Maßgabe, dass t+v kleiner als oder gleich 7 ist, und u eine beliebige ganze Zahl im Bereich von (7-(t+v)) bis (9-(t+v)), Grenzen eingeschlossen, ist, mit der Maßgabe, dass u größer als 0 ist; und
Verbindung der Formel (16): 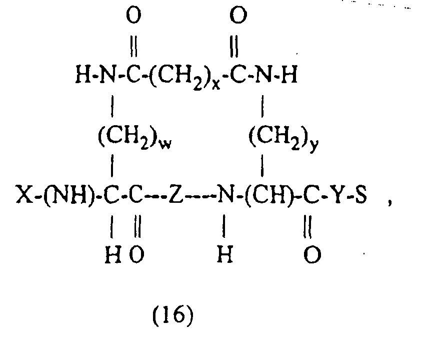
worin S nicht vorhanden ist oder ein Makromolekül ist, X Wasserstoff oder eine beliebige Aminosäure oder Aminosäuresequenz ist, Y nicht vorhanden ist oder Hydroxyl ist, wenn S nicht vorhanden ist, oder eine beliebige Aminosäure oder Aminosäuresequenz ist, Z eine beliebige Aminosäuresequenz bestehend aus sechs Aminosäuren ist; w und y unabhängig voneinander aus den ganzen Zahlen von 1 bis 7, Grenzen eingeschlossen, ausgewählt sind, mit der Maßgabe, dass w+y kleiner als oder gleich 8 ist, und x eine beliebige ganze Zahl im Bereich von (7-(w+y)) bis (9-(w+y)), Grenzen eingeschlossen, ist, mit der Maßgabe, dass x größer als oder gleich 0 ist.

5. Verbindung nach Anspruch 4, die der Verbindung der Formel (1) entspricht, worin Z Gln-Gln-Arg-Arg-Phe-Tyr ist.

6. Verbindung nach Anspruch 4, worin Z eine Aminosäuresequenz bestehend aus sechs Aminosäuren ist, worin die interne Sequenz aus sechs Aminosäuren die Form gabcde, defgab oder cdefga aufweist und aus einer Gruppe von Sequenzen ausgewählt ist, die eine Sequenz aus sechs zusammenhängenden Aminosäuren in der HIV-1LAI-Stamm-gp41-Aminosäuresequenz 633 bis 678, in seiner homologen Sequenz von einem anderen HIV-Stamm, in einer Consensus-Sequenz seiner homologen Sequenz von einer beliebigen HIV-Clade oder einer Aminosäure-substituierten Variante davon umfasst, worin der Aminosäure 633 oder ihrer entsprechenden Aminosäure in der homologen Sequenz, Consensus-Sequenz oder Sequenzvariante die Position a einer Grundaufstellung abcdefg zugeordnet ist.

7. Verbindung nach Anspruch 6, weiters S' umfassend, wenn S nicht vorhanden ist und X eine beliebige Aminosäure oder Aminosäuresequenz ist, worin S' ein an X gebundenes Makromolekül ist.

8. Verbindung nach Anspruch 6, worin die homologe oder Consensus-Sequenz in Fig. 16A-16M dargestellt ist.

9. Verbindung nach Anspruch 6, weiters ein zweites eingeschränktes helikales Peptid umfassend.

10. Antikörper, der an eine Verbindung nach Anspruch 6 bindet, worin der Antikörper spezifisch an ein Epitop bindet, das eine Aminosäure an Position a, d, e oder g im helikalen Peptid umfasst.

11. Verwendung einer Verbindung nach Anspruch 6 zur Herstellung einer Zusammensetzung zur prophylaktischen oder therapeutischen Behandlung eines Säugetiers, das HIV-gefährdet ist oder mit HIV infiziert ist.

12. Verwendung nach Anspruch 11, worin die Zusammensetzung interne sechs Aminosäuren umfassende Sequenzen von unterschiedlichen HIV-Stämmen oder HIV-Claden umfasst.

13. Vakzine, zumindest eine Verbindung nach Anspruch 6 umfassend.

14. Verbindung nach Anspruch 6 zur Verwendung in der prophylaktischen oder therapeutischen Behandlung eines Säugetiers, das HIV-gefährdet oder mit HIV infiziert ist.

## Revendications

1. Procédé pour construire un peptide à structure hélicoïdale sous contrainte, comprenant les étapes consistant à :
(a) synthétiser un peptide, le peptide comprenant une séquence de huit résidus d'aminoacides, la séquence de huit résidus d'aminoacides comprenant un premier résidu terminal et un second résidu terminal, le premier résidu terminal et le second résidu terminal étant adjacents à une séquence interne de six résidus d'aminoacides, et les premier et second résidus terminaux ayant une chaîne latérale contenant un substituant de formation de liaison amide ;
(b) fournir un segment de liaison difonctionnel comprenant un premier groupe fonctionnel capable de former une liaison amide avec le substituant de formation de liaison amide de chaîne latérale du premier résidu terminal et ayant un second groupe fonctionnel capable de former une liaison amide avec le substituant de formation de liaison amide de chaîne latérale du second résidu terminal ; et
(c) cycliser le peptide en faisant réagir le substituant de formation de liaison amide de chaîne latérale du premier résidu terminal avec le premier groupe fonctionnel du segment de liaison difonctionnel pour former une liaison amide et en faisant réagir le substituant de formation de liaison amide de chaîne latérale du second résidu terminal avec le second groupe fonctionnel du segment de liaison difonctionnel pour former une liaison amide, ce qui donne un peptide à structure hélicoïdale sous contrainte.

2. Procédé suivant la revendication 1, dans lequel, dans l'étape (a), le substituant de formation de liaison amide de chaîne latérale du premier résidu terminal est protégé avec un premier groupe protecteur et le substituant de formation de liaison amide de chaîne latérale du second résidu terminal est protégé avec un second groupe protecteur, le premier groupe protecteur et le second groupe protecteur pouvant être éliminés de manière différentielle, et dans lequel, dans l'étape (c), le premier groupe protecteur est éliminé de sorte que le substituant de formation de liaison amide de chaîne latérale du premier résidu terminal est débarrassé de sa protection et le substituant de formation de liaison amide de chaîne latérale du second résidu terminal n'est pas débarrassé de sa protection avant la réaction du peptide avec le segment de liaison difonctionnel, puis le peptide est amené à réagir avec le segment de liaison difonctionnel pour former une liaison amide entre le substituant de formation de liaison amide de chaîne latérale du premier résidu terminal et le premier groupe fonctionnel du segment de liaison difonctionnel, puis le second groupe protecteur est éliminé du substituant de formation de liaison amide de chaîne latérale du second résidu terminal et le peptide est cyclisé par réaction intramoléculaire du substituant de formation de liaison amide de chaîne latérale du second résidu terminal avec le second groupe fonctionnel du segment de liaison difonctionnel pour former une liaison amide.

3. Procédé pour construire un peptide à structure hélicoïdale sous contrainte, comprenant les étapes consistant à :
(a) synthétiser un peptide, le peptide comprenant une séquence de huit résidus d'aminoacides, la séquence de huit résidus d'aminoacides comprenant un premier résidu terminal et un second résidu terminal, le premier résidu terminal et le second résidu terminal étant adjacents à une séquence interne de six résidus d'aminoacides, les premier et second résidus terminaux ayant une chaîne latérale contenant un substituant de formation de liaison amide, le premier résidu terminal étant couplé à un segment de liaison difonctionnel comprenant un premier groupe fonctionnel et un second groupe fonctionnel, le premier groupe fonctionnel étant en liaison amide avec le substituant de formation de liaison amide de chaîne latérale du premier résidu terminal, et le second groupe fonctionnel du segment de liaison difonctionnel étant capable de former une liaison amide avec le substituant de formation de liaison amide de chaîne latérale du second résidu terminal ; et
(b) cycliser le peptide par réaction intramoléculaire du substituant de formation de liaison amide de chaîne latérale du second résidu terminal avec le second groupe fonctionnel du segment de liaison difonctionnel pour former une liaison amide et obtenir un peptide à structure hélicoïdale sous contrainte.

4. Composé choisi dans le groupe consistant en :
le composé représenté par la formule (1) : 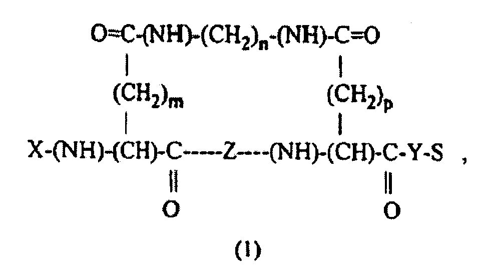
dans laquelle S est absent ou représente une macromolécule, X représente un atome d'hydrogène ou n'importe quel aminoacide ou n'importe quelle séquence d'aminoacides, Y est absent ou représente un groupe hydroxyle si S est absent, ou représente n'importe quel aminoacide ou séquence d'aminoacides, Z représente n'importe quelle séquence d'aminoacides consistant en six aminoacides ; m et p sont choisis indépendamment parmi les nombres entiers 0 à 6 inclus, pourvu que la somme m+p soit inférieure ou égale à 6, et n représente n'importe quel nombre entier dans l'intervalle défini par (7-(m+p)) à (9-(m+p)) inclus, pourvu que n soit supérieur à 1 ;
le composé représenté par la formule (6) : 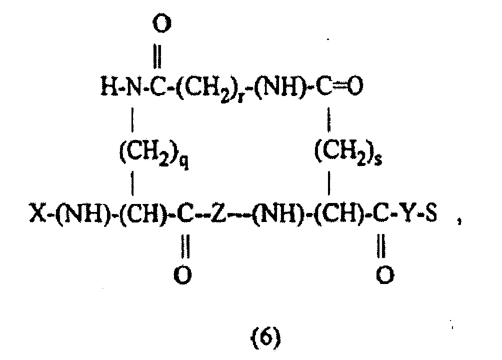
dans laquelle S est absent ou représente une macromolécule, X représente un atome d'hydrogène ou n'importe quel aminoacide ou séquence d'aminoacides, Y est absent ou représente un groupe hydroxyle si S est absent, ou représente n'importe quel aminoacide ou séquence d'aminoacides, Z représente n'importe quelle séquence d'aminoacides consistant en six aminoacides, q est choisi parmi les nombres entiers 1 à 7 inclus, s est choisi parmi les nombres entiers 0 à 6 inclus, pourvu que la somme q+s soit inférieure ou égale à 7, et r représente n'importe quel nombre entier dans l'intervalle défini par (7-(q+s)) à (9-(q+s)) inclus, pourvu que r soit supérieur à 0 ;
le composé représenté par la formule (11) : 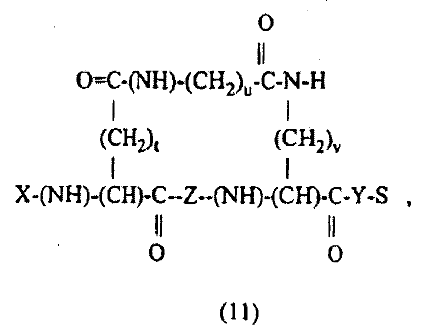
dans laquelle S est absent ou représente une macromolécule, X représente un atome d'hydrogène ou n'importe quel aminoacide ou n'importe quelle séquence d'aminoacides, Y est absent ou représente un groupe hydroxyle si S est absent, ou représente n'importe quel aminoacide ou séquence d'aminoacides, Z représente n'importe quelle séquence d'aminoacides consistant à 6 aminoacides ; t est choisi parmi les nombres entiers 0 à 6 inclus et v est choisi parmi les nombres entiers 1 à 7 inclus, pourvu que la somme t+v soit inférieure ou égale à 7 ; et u représente n'importe quel nombre entier dans l'intervalle défini par (7-(t+v)) à (9-(t+v)) inclus, pourvu que u soit supérieur à 0 ; et
le composé représenté par la formule (16) : 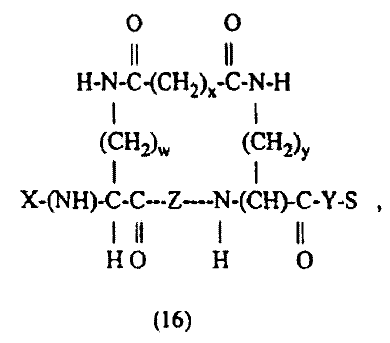
dans laquelle S est absent ou représente une macromolécule, X représente un atome d'hydrogène ou n'importe quel aminoacide ou n'importe quelle séquence d'aminoacides, Y est absent ou représente un groupe hydroxyle si S est absent, ou représente n'importe quel aminoacide ou séquence d'aminoacides, Z représente n'importe quelle séquence d'aminoacides consistant à 6 aminoacides ; w et y sont choisis indépendamment parmi les nombres entiers 1 à 7 inclus, pourvu que la somme w+y soit inférieure ou égale à 8, et x représente n'importe quel nombre entier dans l'intervalle défini par (7-(w+y)) à (9-(w+y)) inclus, pourvu que x soit supérieur à 0.

5. Composé suivant la revendication 4, qui est le composé de formule (1), dans laquelle Z représente Gln-Gln-Arg-Arg-Phe-Tyr.

6. Composé suivant la revendication 4, dans lequel Z représente une séquence d'aminoacides consistant en six aminoacides, la séquence interne de six aminoacides répondant à la forme **gabcde, defgab** ou **cdefga** et étant choisie dans le groupe de séquences consistant en une séquence de six aminoacides contigus dans la séquence d'aminoacides 633 à 678 de la souche gp41 du HIV-ILAI, dans sa séquence homologue provenant d'une autre souche du HIV, dans une séquence consensus de ses séquences homologues provenant de n'importe quelle autre clade du HIV, ou de ses variants de substitution d'aminoacides, dans lequel il est attribué à l'aminoacide 633 ou à son aminoacide correspondant dans la séquence homologue, consensus ou variante la position **a** d'une attribution de séquence **abcdefg** répétée.

7. Composé suivant la revendication 6, comprenant en outre S' lorsque S est absent et X représente n'importe quel aminoacide ou n'importe quelle séquence d'aminoacides, S' représentant une macromolécule fixée à X.

8. Composé suivant la revendication 6, dans lequel la séquence homologue ou consensus est représentée sur les figures 16A à 16M.

9. Composé suivant la revendication 6, comprenant en outre un second peptide à structure hélicoïdale sous contrainte.

10. Anticorps qui se lie à un composé de la revendication 6, l'anticorps se liant spécifiquement à un épitope comprenant un aminoacide en position **a**, **d**, **e** ou **g** dans le peptide à structure hélicoïdale.

11. Utilisation d'un composé de la revendication 6 pour la production d'une composition destinée au traitement prophylactique ou thérapeutique d'un mammifère présentant un risque d'être infecté, ou infecté, par le HIV.

12. Utilisation suivant la revendication 11, dans laquelle la composition comprend des séquences internes de six aminoacides provenant de différentes souches du HIV ou de différents clades du HIV.

13. Vaccin comprenant au moins un composé de la revendication 6.

14. Composé de la revendication 6 destiné à être utilisé dans le traitement prophylactique ou thérapeutique d'un mammifère présentant un risque d'être infecté, ou infecté, par le HIV.


### SEQUENCE LISTING

(1) GENERAL INFORMATION:
   (i) APPLICANT: Genentech, Inc.
   (ii) TITLE OF INVENTION: Constrained Helical Peptides and Methods of Making Same
   (iii) NUMBER OF SEQUENCES: 113
   (iv) CORRESPONDENCE ADDRESS:
      (A) ADDRESSEE: Genentech, Inc.
      (B) STREET: 1 DNA Way
      (C) CITY: South San Francisco
      (D) STATE: California
      (E) COUNTRY: USA
      (F) ZIP: 94080
   (v) COMPUTER READABLE FORM:
      (A) MEDIUM TYPE: 3.5 inch, 1.44 Mb floppy disk
      (B) COMPUTER: IBM PC compatible
      (C) OPERATING SYSTEM: PC-DOS/MS-DOS
      (D) SOFTWARE: WinPatin (Genentech)
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/876698
      (B) FILING DATE: 6/16/97
   (vii) PRIOR APPLICATION DATA:
      (A) APPLICATION NUMBER: 08/743698
      (B) FILING DATE: 11/6/96
   (viii) ATTORNEY/AGENT INFORMATION:
      (A) NAME: Torchia, PhD., Timothy E.
      (B) REGISTRATION NUMBER: 36,700
      (C) REFERENCE/DOCKET NUMBER: P1005R2PCT
   (ix) TELECOMMUNICATION INFORMATION:
      (A) TELEPHONE: 650/225-8674
      (B) TELEFAX: 650/952-9881
(2) INFORMATION FOR SEQ ID NO:1:
   (i) SEQUENCE CHARACTERISTICS:
      (A) LENGTH: 36 amino acids
      (B) TYPE: Amino Acid
      (D) TOPOLOGY: Linear
   (ii) MOLECULE TYPE: DP178
   (xi) SEQUENCE DESCRIPTION: SEQ ID NO:1:
   Tyr Thr Ser Leu Ile His Ser Leu Ile Glu Glu Ser Gln Asn Gln
   1 5 10 15
   Gln Glu Lys Asn Glu Gln Glu Leu Leu Glu Leu Asp Lys Trp Ala
   20 25 30
   Ser Leu Trp Asn Trp Phe
   35 36
(2) INFORMATION FOR SEQ ID NO:2:
   (i) SEQUENCE CHARACTERISTICS:
      (A) LENGTH: 27 amino acids
      (B) TYPE: Amino Acid
      (D) TOPOLOGY: Linear
   (xi) SEQUENCE DESCRIPTION: SEQ ID NO:2:
   Tyr Thr Ser Leu Ile His Ser Leu Ile Glu Glu Ser Gln Asn Gln
   1 5 10 15
   Gln Glu Lys Asn Glu Gln Glu Leu Leu Glu Leu Asp
   20 25 27
(2) INFORMATION FOR SEQ ID NO:3:
   (i) SEQUENCE CHARACTERISTICS:
      (A) LENGTH: 27 amino acids
      (B) TYPE: Amino Acid
      (D) TOPOLOGY: Linear
   (xi) SEQUENCE DESCRIPTION: SEQ ID NO:3:
   Tyr Thr Ser Leu Ile His Ser Leu Ile Xaa Glu Ser Gln Asn Gln
   1 5 10 15
   Gln Xaa Lys Asn Glu Gln Glu Leu Leu Glu Leu Asp
   20 25 27
(2) INFORMATION FOR SEQ ID NO:4:
   (i) SEQUENCE CHARACTERISTICS:
      (A) LENGTH: 27 amino acids
      (B) TYPE: Amino Acid
      (D) TOPOLOGY: Linear
   (xi) SEQUENCE DESCRIPTION: SEQ ID NO:4:
   Tyr Thr Xaa Leu Ile His Ser Leu Ile Xaa Glu Ser Gln Asn Gln
    1               5                  10                  15
   Gln Xaa Lys Asn Glu Gln Glu Leu Xaa Glu Leu Asp
                   20                  25      27
(2) INFORMATION FOR SEQ ID NO:5:
   (i) SEQUENCE CHARACTERISTICS:
      (A) LENGTH: 27 amino acids
      (B) TYPE: Amino Acid
      (D) TOPOLOGY: Linear
   (xi) SEQUENCE DESCRIPTION: SEQ ID NO:5:
   Tyr Thr Ser Leu Ile His Ser Xaa Ile Glu Glu Ser Gln Asn Xaa
    1               5                  10                  15
   Gln Glu Lys Asn Glu Gln Glu Leu Leu Glu Leu Asp
                   20                  25      27
(2) INFORMATION FOR SEQ ID NO:6:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:6:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Glu Lys Arg Ala Val Gly Xaa
   35 40 45
   Ile Gly Ala Met Phe Leu Gly Phe Leu Gly Ala Ala Gly Ser Thr
   50 55 60
   Met Gly Ala Ala Ser Met Thr Leu Thr Val Gln Ala Arg Gln Leu
   65 70 75
   Leu Ser Gly Ile Val Gln Gln Gln Asn Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Lys Asp
   110 115 120
   Gln Gln Leu Leu Gly Ile Trp Gly Cys Ser Gly Lys Leu Ile Cys
   125 130 135
   Thr Thr Ala Val Pro Trp Asn Ala Ser Trp Ser Asn Lys Ser Leu
   140 145 150
   Xaa Xaa Ile Trp Xaa Asn Met Thr Trp Met Glu Trp Glu Arg Glu
   155 160 165
   Ile Asp Asn Tyr Thr Xaa Leu Ile Tyr Thr Leu Ile Glu Glu Ser
   170 175 180
   Gln Asn Gln Gln Glu Lys Asn Glu Gln Glu Leu Leu Glu Leu Asp
   185 190 195
   Lys Trp Ala Ser Leu Trp Asn Trp Phe Xaa Ile Thr Asn Trp Leu
   200 205 210
   Trp Tyr Ile Lys Ile Phe Ile Met Ile Val Gly Gly Leu Val Gly
   215 220 225
   Leu Arg Ile Val Phe Ala Val Leu Ser Ile Val Asn Arg Val Arg
   230 235 240
   Gln Gly Tyr Ser Pro Leu Ser Phe Gln Thr Xaa Leu Pro Ala Pro
   245 250 255
   Arg Gly Pro Asp Arg Pro Glu Gly Ile Glu Glu Glu Gly Gly
   260 265 269
(2) INFORMATION FOR SEQ ID NO:7:
   (i) SEQUENCE CHARACTERISTICS:
      (A) LENGTH: 268 amino acids
      (B) TYPE: Amino Acid
      (D) TOPOLOGY: Linear
   (ii) MOLECULE TYPE: HIV-JRCSF
   (xi) SEQUENCE DESCRIPTION: SEQ ID NO:7:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Ile
                   35                  40                  45
   Gly Ala Leu Phe Leu Gly Phe Leu Gly Ala Ala Gly Ser Thr Met
                   50                  55                  60
   Gly Ala Arg Ser Met Thr Leu Thr Val Gln Ala Arg Gln Leu Leu
                   65                  70                  75
   Ser Gly Ile Val Gln Gln Gln Asn Asn Leu Leu Arg Ala Ile Glu
                   80                  85                  90
   Ala Gln Gln His Met Leu Gln Leu Thr Val Trp Gly Ile Lys Gln
                   95                 100                 105
   Leu Gln Ala Arg Val Leu Ala Val Glu Arg Tyr Leu Lys Asp Gln
                  110                 115                 120
   Gln Leu Met Gly Ile Trp Gly Cys Ser Gly Lys Leu Ile Cys Thr
                  125                 130                 135
   Thr Ala Val Pro Trp Asn Thr Ser Trp Ser Asn Lys Ser Leu Asp
                  140                 145                 150
   Ser Ile Trp Asn Asn Met Thr Trp Met Glu Trp Glu Lys Glu Ile
                  155                 160                 165
   Glu Asn Tyr Thr Asn Thr Ile Tyr Thr Leu Ile Glu Glu Ser Gln
                  170                 175                 180
   Ile Gln Gln Glu Lys Asn Glu Gln Glu Leu Leu Glu Leu Asp Lys
                   185                 190                 195
   Trp Ala Ser Leu Trp Asn Trp Phe Gly Ile Thr Lys Trp Leu Trp
                   200                 205                 210
   Tyr Ile Lys Ile Phe Ile Met Ile Val Gly Gly Leu Ile Gly Leu
                   215                 220                 225
   Arg Ile Val Phe Ser Val Leu Ser Ile Val Asn Arg Val Arg Gln
                   230                 235                 240
   Gly Tyr Ser Pro Leu Ser Phe Gln Thr Leu Leu Pro Ala Thr Arg
                   245                 250                 255
   Gly Pro Asp Arg Pro Glu Gly Ile Glu Glu Glu Gly Gly
                   260                 265         268
(2) INFORMATION FOR SEQ ID NO:8:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:8:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Glu Lys Arg Ala Val Gly Ile
   35 40 45
   Gly Ala Val Phe Leu Gly Phe Leu Gly Ala Ala Gly Ser Thr Met
   50 55 60
   Gly Ala Ala Ser Met Thr Leu Thr Val Gln Ala Arg Leu Leu Leu
   65 70 75
   Ser Gly Ile Val Gln Gln Gln Asn Asn Leu Leu Arg Ala Ile Glu
   80 85 90
   Ala Gln Gln Arg Met Leu Gln Leu Thr Val Trp Gly Ile Lys Gln
   95 100 105
   Leu Gln Ala Arg Val Leu Ala Val Glu Arg Tyr Leu Gly Asp Gln
   110 115 120
   Gln Leu Leu Gly Ile Trp Gly Cys Ser Gly Lys Leu Ile Cys Thr
   125 130 135
   Thr Ala Val Pro Trp Asn Ala Ser Trp Ser Asn Lys Ser Leu Asp
   140 145 150
   Arg Ile Trp Asn Asn Met Thr Trp Met Glu Trp Glu Arg Glu Ile
   155 160 165
   Asp Asn Tyr Thr Ser Glu Ile Tyr Thr Leu Ile Glu Glu Ser Gln
   170 175 180
   Asn Gln Gln Glu Lys Asn Glu Gln Glu Leu Leu Glu Leu Asp Lys
   185 190 195
   Trp Ala Ser Leu Trp Asn Trp Phe Asp Ile Thr Lys Trp Leu Trp
   200 205 210
   Tyr Ile Lys Ile Phe Ile Met Ile Val Gly Gly Leu Val Gly Leu
   215 220 225
   Arg Leu Val Phe Thr Val Leu Ser Ile Val Asn Arg Val Arg Gln
   230 235 240
   Gly Tyr Ser Pro Leu Ser Phe Gln Thr Leu Leu Pro Ala Pro Arg
   245 250 255
   Gly Pro Asp Arg Pro Glu Gly Ile Glu Glu Glu Gly Gly
   260 265 268
(2) INFORMATION FOR SEQ ID NO:9:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:9:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Arg
                   20                  25                  30
   Ala Lys Arg Arg Val Val Gln Arg Glu Lys Arg Ala Val Gly Leu
                   35                  40                  45
   Gly Ala Leu Phe Leu Gly Phe Leu Gly Ala Ala Gly Ser Thr Met
                   50                  55                  60
   Gly Ala Arg Ser Met Thr Leu Thr Val Gln Ala Arg Gln Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                 100                 105
   Leu Gln Ala Arg Val Leu Ala Val Glu Arg Tyr Leu Arg Asp Gln
                  110                 115                 120
   Gln Leu Leu Glu Ile Trp Gly Cys Ser Gly Lys Leu Ile Cys Thr
                  125                 130                 135
   Thr Thr Val Pro Trp Asn Ala Ser Trp Ser Asn Lys Ser Leu Asn
   140 145 150
   Gln Ile Trp Asp Asn Met Thr Trp Met Glu Trp Glu Arg Glu Ile
   155 160 165
   Asp Asn Tyr Thr Ser Leu Ile Tyr Thr Leu Ile Glu Glu Ser Gln
   170 175 180
   Asn Gln Gln Glu Lys Asn Glu Gln Glu Leu Leu Glu Leu Asp Lys
   185 190 195
   Trp Ala Ser Leu Trp Asn Trp Phe Asn Ile Thr Asn Trp Leu Trp
   200 205 210
   Tyr Ile Lys Ile Phe Ile Met Ile Val Gly Gly Leu Val Gly Leu
   215 220 225
   Arg Ile Val Phe Ser Val Leu Ser Ile Val Asn Arg Val Arg Gln
   230 235 240
   Gly Tyr Ser Pro Leu Ser Phe Gln Thr Arg Leu Pro Ala Arg Arg
   245 250 255
   Glu Pro Asp Arg Pro Glu Gly Ile Glu Glu Glu Gly Gly
   260 265 268
(2) INFORMATION FOR SEQ ID NO:10:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:10:
   Gly Gly Gly Asn Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Leu
                   35                  40                  45
   Gly Ala Leu Phe Leu Gly Phe Leu Gly Ala Ala Gly Ser Thr Met
                   50                  55                  60
   Gly Ala Ala Ser Leu Thr Leu Thr Val Gln Ala Arg Leu Leu Leu
                   65                  70                  75
   Ser Gly Ile Val Gln Gln Gln Asn Asn Leu Leu Met Ala Ile Glu
                   80                  85                  90
   Ala Gln Gln His Met Leu Glu Leu Thr Val Trp Gly Ile Lys Gln
                   95                  100                 105
   Leu Gln Ala Arg Val Leu Ala Val Glu Arg Tyr Leu Lys Asp Gln
                       110                 115                 120
   Gln Leu Leu Gly Ile Trp Gly Cys Ser Gly Lys Leu Ile Cys Thr
                   125                 130                 135
   Thr Ala Val Pro Trp Asn Ala Ser Trp Ser Asn Lys Ser Leu Ser
                   140                 145                 150
   Asp Ile Trp Asp Asn Met Thr Trp Met Glu Trp Glu Arg Glu Ile
                   155                 160                 165
   Asp Asn Tyr Thr Asn Leu Ile Tyr Ser Leu Ile Glu Asp Ser Gln
                   170                 175                 180
   Ile Gln Gln Glu Lys Asn Glu Lys Glu Leu Leu Glu Leu Asp Lys
                   185                 190                 195
   Trp Ala Ser Leu Trp Asn Trp Phe Asn Ile Thr Asn Trp Leu Trp
                   200                 205                 210
   Tyr Ile Lys Ile Phe Ile Met Ile Val Gly Gly Leu Ile Gly Leu
                   215                 220                 225
   Arg Ile Val Phe Ala Val Leu Ser Ile Val Asn Arg Val Arg Gln
                   230                 235                 240
   Gly Tyr Ser Pro Leu Ser Phe Gln Thr Arg Leu Pro Gly Arg Arg
                   245                 250                 255
   Gly Pro Asp Arg Pro Glu Gly Ile Glu Glu Glu Gly Gly
                   260                 265         268
(2) INFORMATION FOR SEQ ID NO:11:
   (i) SEQUENCE CHARACTERISTICS:
      (A) LENGTH: 267 amino acids
      (B) TYPE: Amino Acid
      (D) TOPOLOGY: Linear
   (xi) SEQUENCE DESCRIPTION: SEQ ID NO:11:
   Gly Gly Gly Asn Met Arg Asp Asn Trp Arg Ser Glu Leu Tyr Lys
   1 5 10 15
   Tyr Lys Val Val Lys Ile Glu Leu Leu Gly Val Ala Pro Thr Lys
   20 25 30
   Ala Lys Arg Arg Val Val Gln Arg Glu Lys Arg Ala Val Gly Ile
   35 40 45
   Gly Ala Val Phe Leu Gly Phe Leu Gly Ala Ala Gly Ser Thr Met
   50 55 60
   Gly Ala Ser Met Thr Leu Thr Val Gln Ala Arg Leu Leu Leu Ser
   65 70 75
   Gly Ile Val Gln Gln Gln Asn Asn Leu Leu Arg Ala Ile Glu Gly
   80 85 90
   Gln Gln His Leu Leu Gln Leu Thr Val Trp Gly Ile Lys Gln Leu
   95 100 105
   Gln Ala Arg Ile Leu Ala Val Glu Arg Tyr Leu Lys Asp Gln Gln
   110 115 120
   Leu Leu Gly Ile Trp Gly Cys Ser Gly Lys Leu Ile Cys Thr Thr
   125 130 135
   Ala Val Pro Trp Asn Ala Ser Trp Ser Asn Lys Ser Leu Glu Glu
   140 145 150
   Ile Trp Asp Asn Met Thr Trp Met Glu Trp Glu Arg Glu Ile Asp
   155 160 165
   Asn Tyr Thr Ser Leu Ile Tyr Thr Leu Ile Glu Glu Ser Gln Asn
   170 175 180
   Gln Gln Glu Lys Asn Glu Gln Glu Leu Leu Gly Leu Asp Lys Trp
   185 190 195
   Ala Ser Leu Trp Asn Trp Phe Thr Ile Thr Asn Trp Leu Trp Tyr
   200 205 210
   Ile Arg Ile Phe Ile Met Ile Val Gly Gly Leu Val Gly Leu Arg
   215 220 225
   Ile Val Phe Thr Val Leu Ser Ile Val Asn Arg Val Arg Gln Gly
   230 235 240
   Tyr Ser Pro Leu Ser Phe Gln Thr Arg Leu Pro Ala Pro Arg Gly
   245 250 255
   Pro Asp Arg Pro Glu Gly Ile Glu Glu Glu Gly Gly
   260 265 267
(2) INFORMATION FOR SEQ ID NO:12:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:12:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Thr Lys
                    20                  25                  30
   Ala Lys Arg Arg Val Val Gln Arg Glu Lys Arg Ala Val Gly Thr
                    35                  40                  45
   Ile Gly Ala Met Phe Leu Gly Phe Leu Gly Ala Ala Gly Ser Thr
                    50                  55                  60
   Met Gly Ala Thr Ser Met Thr Leu Thr Val Gln Ala Arg Leu Leu
                                   65                  70                  75
   Leu Ser Gly Ile Val Gln Gln Gln Asn Asn Leu Leu Arg Ala Ile
                   80                  85                  90
   Glu Ala Gln Gln His Leu Leu Gln Leu Thr Val Trp Gly Ile Lys
                   95                  100                 105
   Gln Leu Gln Ala Arg Val Leu Ala Val Glu Arg Tyr Leu Arg Asp
                   110                 115                 120
   Gln Gln Leu Leu Gly Ile Trp Gly Cys Ser Gly Lys Leu Ile Cys
                   125                 130                 135
   Thr Thr Thr Val Pro Trp Asn Thr Ser Trp Ser Asn Lys Ser Leu
                   140                 145                 150
   Asp Lys Ile Trp Gly Asn Met Thr Trp Met Glu Trp Glu Arg Glu
                   155                 160                 165
   Ile Asp Asn Tyr Thr Ser Leu Ile Tyr Thr Leu Ile Glu Glu Ser
                   170                 175                 180
   Gln Asn Gln Gln Glu Lys Asn Glu Gln Glu Leu Leu Glu Leu Asp
                   185                 190                 195
   Lys Trp Ala Ser Leu Trp Asn Trp Phe Asn Ile Thr Asn Trp Leu
                   200                 205                 210
   Trp Tyr Ile Lys Ile Phe Ile Met Ile Val Gly Gly Leu Val Gly
                   215                 220                 225
   Leu Arg Ile Val Phe Thr Val Leu Ser Ile Val Asn Arg Val Arg
                   230                 235                 240
   Gln Gly Tyr Ser Pro Leu Ser Phe Gln Thr Arg Leu Pro Ser Gln
                   245                 250                 255
   Arg Gly Pro Asp Arg Pro Glu Gly Ile Glu Glu Glu Gly Gly
                   260                 265             269
(2) INFORMATION FOR SEQ ID NO:13:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:13:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Glu Lys Arg Ala Val Gly Ile
   35 40 45
   Gly Ala Val Phe Leu Gly Phe Leu Gly Ala Ala Gly Ser Thr Met
   50 55 60
   Gly Ala Ala Ala Met Thr Leu Thr Val Gln Ala Arg Leu Leu Leu
   65 70 75
   Ser Gly Ile Val Gln Gln Gln Asn Asn Leu Leu Arg Ala Ile Glu
   80 85 90
   Ala Gln Gln His Leu Leu Gln Leu Thr Val Trp Gly Ile Lys Gln
   95 100 105
   Leu Gln Ala Arg Val Leu Ala Val Glu Arg Tyr Leu Arg Asp Gln
   110 115 120
   Gln Leu Leu Gly Ile Trp Gly Cys Ser Gly Lys Leu Ile Cys Thr
   125 130 135
   Thr Ala Val Pro Trp Asn Ala Ser Trp Ser Asn Lys Ser Leu Asn
   140 145 150
   Lys Ile Trp Asp Asn Met Thr Trp Ile Glu Trp Asp Arg Glu Ile
   155 160 165
   Asn Asn Tyr Thr Ser Ile Ile Tyr Ser Leu Ile Glu Glu Ser Gln
   170 175 180
   Asn Gln Gln Glu Lys Asn Glu Gln Glu Leu Leu Glu Leu Asp Lys
   185 190 195
   Trp Ala Ser Leu Trp Asn Trp Phe Asp Ile Thr Lys Trp Leu Trp
   200 205 210
   Tyr Ile Lys Ile Phe Ile Met Ile Val Gly Gly Leu Ile Gly Leu
   215 220 225
   Arg Ile Val Phe Ser Val Leu Ser Ile Val Asn Arg Val Arg Gln
   230 235 240
   Gly Tyr Ser Pro Leu Ser Phe Gln Thr His Leu Pro Ser Ser Arg
   245 250 255
   Gly Pro Asp Arg Pro Gly Gly Ile Glu Glu Glu Gly Gly
   260 265 268
(2) INFORMATION FOR SEQ ID NO:14:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:14:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
                      20                      25                      30
   Ala Lys Arg Arg Val Val Gln Arg Glu Lys Arg Ala Val Gly Leu
                   35                      40                      45
   Gly Ala Leu Phe Leu Gly Phe Leu Gly Ala Ala Gly Ser Thr Met
                   50                      55                      60
   Gly Ala Ala Ser Ile Thr Leu Thr Val Gln Ala Arg Gln Leu Leu
                   65                      70                      75
   Ser Gly Ile Val Gln Gln Gln Asn Asn Leu Leu Arg Ala Ile Glu
                   80                      85                      90
   Ala Gln Gln His Leu Leu Gln Leu Thr Val Trp Gly Ile Lys Gln
                   95                      100                     105
   Leu Gln Ala Arg Val Leu Ala Val Glu Arg Tyr Leu Arg Asp Gln
                   110                     115                     120
   Gln Leu Leu Gly Ile Trp Gly Cys Ser Gly Lys Leu Ile Cys Thr
                   125                     130                     135
   Thr Thr Val Pro Trp Asn Thr Ser Trp Ser Asn Lys Ser Leu Asn
                   140                     145                     150
   Glu Ile Trp Asp Asn Met Thr Trp Met Lys Trp Glu Arg Glu Ile
                   155                     160                     165
   Asp Asn Tyr Thr His Ile Ile Tyr Ser Leu Ile Glu Gln Ser Gln
                   170                     175                     180
   Asn Gln Gln Glu Lys Asn Glu Gln Glu Leu Leu Ala Leu Asp Lys
                   185                     190                     195
   Trp Ala Ser Leu Trp Asn Trp Phe Asp Ile Thr Lys Trp Leu Trp
                   200                     205                     210
   Tyr Ile Lys Ile Phe Ile Met Ile Val Gly Gly Leu Ile Gly Leu
                   215                     220                     225
   Arg Ile Val Phe Val Val Leu Ser Ile Val Asn Arg Val Arg Gln
                   230                     235                     240
   Gly Tyr Ser Pro Leu Ser Phe Gln Thr His Leu Pro Ala Gln Arg
                   245                     250                     255
   Gly Pro Asp Arg Pro Asp Gly Ile Glu Glu Glu Gly Gly
                   260                     265         268
(2) INFORMATION FOR SEQ ID NO:15:
   (i) SEQUENCE CHARACTERISTICS:
      (A) LENGTH: 267 amino acids
      (B) TYPE: Amino Acid
      (D) TOPOLOGY: Linear
   (xi) SEQUENCE DESCRIPTION: SEQ ID NO:15:
   Gly Gly Gly Asp Met Arg Asp Asn Trp Arg Ser Glu Leu Tyr Lys
    1               5                  10                  15
   Tyr Lys Val Val Thr Ile Glu Pro Leu Gly Val Ala Pro Thr Lys
                   20                  25                  30
   Ala Lys Arg Arg Val Val Gln Arg Glu Lys Arg Ala Ala Ile Gly
                   35                  40                  45
   Ala Leu Phe Leu Gly Phe Leu Gly Ala Ala Gly Ser Thr Met Gly
                   50                  55                  60
   Ala Ala Ser Val Thr Leu Thr Val Gln Ala Arg Leu Leu Leu Ser
                   65                  70                  75
   Gly Ile Val Gln Gln Gln Asn Asn Leu Leu Arg Ala Ile Glu Ala
                   80                  85                  90
   Gln Gln His Met Leu Gln Leu Thr Val Trp Gly Ile Lys Gln Leu
                   95                  100                 105
   Gln Ala Arg Val Leu Ala Val Glu Arg Tyr Leu Lys Asp Gln Gln
                   110                 115                 120
   Leu Leu Gly Phe Trp Gly Cys Ser Gly Lys Leu Ile Cys Thr Thr
                   125                 130                 135
   Thr Val Pro Trp Asn Ala Ser Trp Ser Asn Lys Ser Leu Asp Asp
                   140                 145                 150
   Ile Trp Asn Asn Met Thr Trp Met Gln Trp Glu Arg Glu Ile Asp
                   155                 160                 165
   Asn Tyr Thr Ser Leu Ile Tyr Ser Leu Leu Glu Lys Ser Gln Thr
                   170                 175                 180
   Gln Gln Glu Lys Asn Glu Gln Glu Leu Leu Glu Leu Asp Lys Trp
                   185                 190                 195
   Ala Ser Leu Trp Asn Trp Phe Asp Ile Thr Asn Trp Leu Trp Tyr
                   200                 205                 210
   Ile Lys Ile Phe Ile Met Ile Val Gly Gly Leu Val Gly Leu Arg
                   215                 220                 225
   Ile Val Phe Ala Val Leu Ser Ile Val Asn Arg Val Arg Gln Gly
                   230                 235                 240
   Tyr Ser Pro Leu Ser Leu Gln Thr Arg Pro Pro Val Pro Arg Gly
                   245                 250                 255
   Pro Asp Arg Pro Glu Gly Ile Glu Glu Glu Gly Gly
                   260                 265     267
(2) INFORMATION FOR SEQ ID NO:16:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:16:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Thr Lys
                    20                  25                  30
   Ala Lys Arg Arg Val Val Gln Arg Glu Lys Arg Ala Val Gly Ile
                    35                  40                  45
   Gly Ala Leu Phe Leu Gly Phe Leu Gly Ala Ala Gly Ser Thr Met
                    50                  55                  60
   Gly Ala Ala Ser Met Thr Leu Thr Val Gln Ala Arg Gln Leu Leu
                    65                  70                  75
   Ser Gly Ile Val Gln Gln Gln Asn Asn Leu Leu Arg Ala Ile Glu
                    80                  85                  90
   Ala Gln Gln His Leu Leu Gln Leu Thr Val Trp Gly Ile Lys Gln
                    95                 100                 105
   Leu Gln Ala Arg Ile Leu Ala Val Glu Arg Tyr Leu Lys Asp Gln
                   110                 115                 120
   Gln Leu Leu Gly Ile Trp Gly Cys Ser Gly Lys Leu Ile Cys Thr
                   125                 130                 135
   Thr Ala Val Pro Trp Asn Ala Ser Trp Ser Asn Lys Ser Leu Glu
                   140                 145                 150
   Gln Ile Trp Asn His Thr Thr Trp Met Glu Trp Asp Arg Glu Ile
                   155                 160                 165
   Asn Asn Tyr Thr Ser Leu Ile His Ser Leu Ile Glu Glu Ser Gln
                   170                 175                 180
   Asn Gln Gln Glu Lys Asn Glu Gln Glu Leu Leu Glu Leu Asp Lys
                   185                 190                 195
   Trp Ala Ser Leu Trp Asn Trp Phe Asn Ile Thr Asn Trp Leu Trp
                   200                 205                 210
   Tyr Ile Lys Leu Phe Ile Met Ile Val Gly Gly Leu Val Gly Leu
                   215                 220                 225
   Arg Ile Val Phe Ala Val Leu Ser Ile Val Asn Arg Val Arg Gln
                   230                 235                 240
   Gly Tyr Ser Pro Leu Ser Phe Gln Thr His Leu Pro Thr Pro Arg
                   245                 250                 255
   Gly Pro Asp Arg Pro Glu Gly Ile Glu Glu Glu Gly Gly
                   260                 265         268
(2) INFORMATION FOR SEQ ID NO:17:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:17:
   Gly Gly Gly Asp Met Arg Asp Asn Trp Arg Ser Glu Leu Tyr Lys
   1               5                   10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Ile
                   35                  40                  45
   Gly Ala Leu Phe Leu Gly Phe Leu Gly Ala Ala Gly Ser Thr Met
                   50                  55                  60
   Gly Ala Arg Ser Met Thr Leu Thr Val Gln Ala Arg Gln Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                  100                 105
   Leu Gln Ala Arg Ile Leu Ala Val Glu Arg Tyr Leu Lys Asp Gln
                   110                 115                 120
   Gln Leu Leu Gly Ile Trp Gly Cys Ser Gly Lys Leu Ile Cys Thr
                   125                 130                 135
   Thr Ala Val Pro Trp Asn Ala Ser Trp Ser Asn Lys Ser Leu Glu
                   140                 145                 150
   Gln Ile Trp Asn Asn Met Thr Trp Met Glu Trp Asp Arg Glu Ile
                   155                 160                 165
   Asn Asn Tyr Thr Ser Leu Ile His Ser Leu Ile Glu Glu Ser Gln
                   170                 175                 180
   Asn Gln Gln Glu Lys Asn Glu Gln Glu Leu Leu Glu Leu Asp Lys
                   185                 190                 195
   Trp Ala Ser Leu Trp Asn Trp Phe Asn Ile Thr Asn Trp Leu Trp
                   200                 205                 210
   Tyr Ile Lys Ile Phe Ile Met Ile Val Gly Gly Leu Val Gly Leu
                   215                 220                 225
   Arg Ile Val Phe Ala Val Leu Ser Ile Val Asn Arg Val Arg Gln
                   230                 235                 240
   Gly Tyr Ser Pro Leu Ser Phe Gln Thr His Leu Pro Thr Pro Arg
                   245                 250                 255
   Gly Pro Asp Arg Pro Glu Gly Ile Glu Glu Glu Gly Gly
                   260                 265         268
(2) INFORMATION FOR SEQ ID NO:18:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:18:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Ile
                   35                  40                  45
   Gly Ala Leu Phe Leu Gly Phe Leu Gly Ala Ala Gly Ser Thr Met
                   50                  55                  60
   Gly Cys Thr Ser Met Thr Leu Thr Val Gln Ala Arg Gln Leu Leu
                   65                  70                  75
   Ser Asp Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                 100                 105
   Leu Gln Ala Arg Ile Leu Ala Val Glu Arg Tyr Leu Lys Asp Gln
                  110                 115                 120
   Gln Leu Leu Gly Ile Trp Gly Cys Ser Gly Lys Leu Ile Cys Thr
                  125                 130                 135
   Thr Ala Val Pro Trp Asn Ala Ser Trp Ser Asn Lys Ser Leu Glu
                  140                 145                 150
   Gln Ile Trp Asn Asn Met Thr Trp Met Glu Trp Asp Arg Glu Ile
                  155                 160                 165
   Asn Asn Tyr Thr Ser Leu Ile His Ser Leu Ile Glu Glu Ser Gln
                  170                 175                 180
   Asn Gln Gln Glu Lys Asn Glu Gln Glu Leu Leu Glu Leu Asp Lys
                  185                 190                 195
   Trp Ala Ser Leu Trp Asn Trp Phe Asn Ile Thr Asn Trp Leu Trp
                  200                 205                 210
   Tyr Ile Lys Leu Phe Ile Met Ile Val Gly Gly Leu Val Gly Leu
                  215                 220                 225
   Arg Ile Val Phe Ala Val Leu Ser Ile Val Asn Arg Val Arg Gln
                   230                 235                 240
   Gly Tyr Ser Pro Leu Ser Phe Gln Thr His Leu Pro Ile Pro Arg
                   245                 250                 255
   Gly Pro Asp Arg Pro Glu Gly Ile Glu Glu Glu Gly Gly
                   260                 265         268
(2) INFORMATION FOR SEQ ID NO:19:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:19:
   Gly Gly Gly Asp Met Arg Asp Asn Trp Arg Ser Glu Leu Tyr Lys
   1 5 10 15
   Tyr Lys Val Val Lys Ile Glu Thr Leu Gly Val Ala Pro Thr Lys
   20 25 30
   Ala Lys Arg Arg Val Val Gln Arg Glu Lys Arg Ala Val Gly Ile
   35 40 45
   Gly Ala Leu Phe Leu Gly Phe Leu Gly Ala Ala Gly Ser Thr Met
   50 55 60
   Gly Ala Ala Ser Met Thr Leu Thr Val Gln Ala Arg Gln Leu Leu
   65 70 75
   Ser Gly Ile Val Gln Gln Gln Asn Asn Leu Leu Arg Ala Ile Glu
   80 85 90
   Ala Gln Gln His Leu Leu Gln Leu Thr Val Trp Gly Ile Lys Gln
   95 100 105
   Leu Gln Ala Arg Ile Leu Ala Val Glu Arg Tyr Leu Lys Asp Gln
   110 115 120
   Gln Leu Leu Gly Ile Trp Gly Cys Ser Gly Lys Leu Ile Cys Thr
   125 130 135
   Thr Ala Val Pro Trp Asn Ala Ser Trp Ser Asn Lys Ser Leu Glu
   140 145 150
   Gln Phe Trp Asn Asn Met Thr Trp Met Glu Trp Asp Arg Glu Ile
   155 160 165
   Asn Asn Tyr Thr Ser Leu Ile His Ser Leu Ile Asp Glu Ser Gln
   170 175 180
   Asn Gln Gln Glu Lys Asn Glu Gln Glu Leu Leu Glu Leu Asp Lys
   185 190 195
   Trp Ala Ser Leu Trp Asn Trp Phe Asn Ile Thr Asn Trp Leu Trp
   200 205 210
   Tyr Ile Lys Ile Phe Ile Met Ile Val Gly Gly Leu Val Gly Leu
   215 220 225
   Arg Ile Val Phe Ala Val Leu Ser Ile Val Asn Arg Val Arg Gln
   230 235 240
   Gly Tyr Ser Pro Leu Ser Phe Gln Thr His Leu Pro Asn Arg Gly
   245 250 255
   Gly Pro Asp Arg Pro Glu Gly Ile Glu Glu Glu Gly Gly
   260 265 268
(2) INFORMATION FOR SEQ ID NO:20:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:20:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Ala
                   35                  40                  45
   Ile Gly Ala Leu Phe Leu Gly Phe Leu Gly Ala Ala Gly Ser Thr
                   50                  55                  60
   Met Gly Ala Val Ala Leu Thr Leu Thr Val Gln Thr Arg Gln Leu
                   65                  70                  75
   Leu Ser Gly Ile Val Gln Gln Gln Asn Asn Leu Leu Arg Ala Ile
                   80                  85                  90
   Glu Ala Gln Gln His Leu Leu Gln Leu Thr Val Trp Gly Ile Lys
                   95                 100                 105
   Gln Leu Gln Ala Arg Val Leu Ala Val Glu Arg Tyr Leu Lys Asp
                  110                 115                 120
   Gln Gln Leu Leu Gly Ile Trp Gly Cys Ser Gly Lys Leu Ile Cys
                  125                 130                 135
   Thr Thr Ala Val Pro Trp Asn Ala Ser Trp Ser Asn Lys Ser Leu
                  140                 145                 150
   Asp Lys Ile Trp Asn Asn Met Thr Trp Met Glu Trp Glu Arg Glu
                  155                 160                 165
   Ile Asp Asn Tyr Thr Asn Leu Ile Tyr Thr Leu Ile Glu Glu Ser
                  170                 175                 180
   Gln Asn Gln Gln Glu Lys Asn Glu Lys Asp Leu Leu Glu Leu Asp
                   185                 190                 195
   Thr Trp Ala Ser Leu Trp Asn Trp Phe Asp Ile Thr Asn Trp Leu
                   200                 205                 210
   Trp Tyr Ile Lys Ile Phe Ile Met Ile Ile Gly Gly Leu Ile Gly
                   215                 220                 225
   Leu Arg Ile Val Phe Thr Ile Leu Ser Leu Val Asn Arg Val Arg
                   230                 235                 240
   Gln Gly Tyr Ser Pro Leu Ser Phe Gln Thr Arg Phe Pro Val Pro
                   245                 250                 255
   Arg Gly Pro Asp Arg Pro Glu Gly Ile Glu Glu Glu Gly Gly
                   260                 265             269
(2) INFORMATION FOR SEQ ID NO:21:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:21:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Glu Lys Arg Ala Val Gly Ala
   35 40 45
   Leu Gly Ala Leu Phe Leu Gly Phe Leu Gly Ala Ala Gly Ser Thr
   50 55 60
   Met Gly Ala Ala Ser Met Ala Leu Thr Val Gln Thr Arg Gln Leu
   65 70 75
   Met Ser Gly Ile Val Gln Gln Gln Asn Asn Leu Leu Lys Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Lys Asp
   110 115 120
   Gln Gln Leu Leu Arg Ile Trp Gly Cys Ser Gly Lys Leu Ile Cys
   125 130 135
   Thr Thr Thr Val Pro Trp Asn Ala Ser Trp Ser Asn Lys Ser Leu
   140 145 150
   Asp Lys Ile Trp Asp Asn Met Thr Trp Met Glu Trp Glu Arg Glu
   155 160 165
   Ile Asp Asn Tyr Thr Gly Leu Ile Tyr Thr Leu Ile Glu Glu Ser
   170 175 180
   Gln Ile Gln Gln Glu Lys Asn Glu Gln Glu Leu Leu Glu Leu Asp
   185 190 195
   Lys Trp Ala Ser Leu Trp Asn Trp Phe Asp Ile Thr Lys Trp Leu
   200 205 210
   Trp Tyr Ile Lys Ile Phe Ile Met Ile Val Gly Gly Leu Ile Gly
   215 220 225
   Leu Arg Ile Val Phe Thr Val Leu Ser Ile Val Asn Arg Val Arg
   230 235 240
   Gln Gly Tyr Ser Pro Leu Ser Phe Gln Thr Arg Leu Pro Ala Gln
   245 250 255
   Arg Gly Pro Asp Arg Pro Glu Gly Ile Glu Glu Glu Gly Gly
   260 265 269
(2) INFORMATION FOR SEQ ID NO:22:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:22:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Thr
                   35                  40                  45
   Ile Gly Ala Met Phe Leu Gly Phe Leu Gly Ala Ala Gly Ser Thr
                   50                  55                  60
   Met Gly Ala Ala Ser Ile Thr Leu Thr Val Gln Ala Arg Leu Leu
                   65                  70                  75
   Leu Ser Gly Ile Val Gln Gln Gln Asn Asn Leu Leu Arg Ala Ile
                   80                  85                  90
   Glu Ala Gln Gln His Leu Leu Gln Leu Thr Val Trp Gly Ile Lys
                   95                 100                 105
   Gln Leu Gln Ala Arg Val Leu Ala Leu Glu Arg Tyr Leu Arg Asp
                  110                 115                 120
   Gln Gln Leu Leu Gly Ile Trp Gly Cys Ser Gly Lys Leu Ile Cys
                  125                 130                 135
   Thr Thr Ala Val Pro Trp Asn Ala Ser Trp Ser Asn Lys Thr Leu
                   140                 145                 150
   Asp Met Ile Trp Asp Asn Met Thr Trp Met Glu Trp Glu Arg Glu
                   155                 160                 165
   Ile Glu Asn Tyr Thr Gly Leu Ile Tyr Thr Leu Ile Glu Glu Ser
                   170                 175                 180
   Gln Asn Gln Gln Glu Lys Asn Glu Gln Asp Leu Leu Ala Leu Asp
                   185                 190                 195
   Lys Trp Ala Ser Leu Trp Asn Trp Phe Asp Ile Ser Asn Trp Leu
                   200                 205                 210
   Trp Tyr Ile Lys Ile Phe Ile Met Ile Val Gly Gly Leu Ile Gly
                   215                 220                 225
   Leu Arg Ile Val Phe Thr Val Leu Ser Ile Val Asn Arg Val Arg
                   230                 235                 240
   Gln Gly Tyr Ser Pro Leu Ser Phe Gln Thr His Leu Pro Ala Pro
                   245                 250                 255
   Arg Gly Pro Asp Arg Pro Glu Gly Ile Glu Glu Glu Gly Gly
                   260                 265             269
(2) INFORMATION FOR SEQ ID NO:23:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:23:
   Gly Gly Gly Asn Met Arg Asp Asn Trp Arg Ser Lys Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Lys Lys Arg Ala Val Gly Thr
   35 40 45
   Ile Gly Ala Met Phe Leu Gly Phe Leu Gly Ala Ala Gly Ser Thr
   50 55 60
   Met Gly Ala Ala Ser Met Thr Leu Thr Val Gln Ala Arg Leu Leu
   65 70 75
   Leu Ser Gly Ile Val Gln Gln Gln Asn Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Gln Asp
   110 115 120
   Gln Gln Leu Leu Gly Ile Trp Gly Cys Ser Gly Lys Leu Ile Cys
   125 130 135
   Thr Thr Thr Val Pro Trp Asn Ala Ser Trp Ser Asn Lys Ser Leu
   140 145 150
   Asp Glu Ile Xaa Asn Asn Met Thr Trp Met Gln Trp Glu Arg Glu
   155 160 165
   Ile Ser Asn Tyr Thr Ser Leu Ile Tyr Thr Leu Ile Glu Glu Ser
   170 175 180
   Gln Asn Gln Gln Glu Lys Asn Glu Leu Glu Leu Leu Glu Leu Asp
   185 190 195
   Lys Trp Ala Ser Leu Xaa Asn Trp Phe Asp Ile Thr Asn Trp Leu
   200 205 210
   Trp Ser Ile Lys Ile Phe Ile Met Ile Val Ala Gly Leu Val Gly
   215 220 225
   Leu Arg Ile Val Phe Ala Val Leu Ser Ile Val Asn Arg Val Arg
   230 235 240
   Gln Gly Tyr Ser Pro Leu Ser Phe Gln Thr Arg Leu Pro Thr Pro
   245 250 255
   Arg Gly Pro Asp Arg Pro Gly Gly Ile Glu Glu Glu Gly Gly
   260 265 269
(2) INFORMATION FOR SEQ ID NO:24:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:24:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Leu
                   35                  40                  45
   Ile Gly Ala Leu Phe Leu Gly Phe Leu Gly Ala Ala Gly Ser Thr
                   50                  55                  60
   Met Gly Ala Ala Ser Met Thr Leu Thr Val Gln Ala Arg Gln Leu
                   65                  70                  75
   Leu Ser Gly Ile Val Gln Gln Gln Ser Asn Leu Leu Arg Ala Ile
                   80                  85                  90
   Glu Ala Gln Gln His Leu Leu Gln Leu Thr Val Trp Gly Ile Lys
                   95                  100                 105
   Gln Leu Gln Ala Arg Val Leu Ala Val Glu Arg Tyr Leu Lys Asp
                   110                 115                 120
   Gln Gln Leu Leu Gly Ile Trp Gly Cys Ser Gly Lys Leu Val Cys
                   125                 130                 135
   Thr Thr Thr Val Pro Trp Asn Ala Ser Trp Ser Asn Lys Ser Leu
                   140                 145                 150
   Asn Gln Ile Trp Asp Asn Met Thr Trp Met Glu Trp Glu Arg Glu
                   155                 160                 165
   Ile Asp Asn Tyr Thr Gly Leu Ile Tyr Arg Leu Ile Glu Glu Ser
                   170                 175                 180
   Gln Asn Gln Gln Glu Gln Asn Glu Gln Asp Leu Leu Lys Leu Asp
                   185                 190                 195
   Thr Trp Ala Ser Leu Trp Asn Trp Phe Ser Ile Thr Lys Trp Leu
                   200                 205                 210
   Trp Tyr Ile Lys Ile Phe Ile Met Ile Val Gly Gly Leu Ile Gly
                   215                 220                 225
   Leu Arg Ile Ile Phe Ala Val Leu Ser Ile Val Asn Arg Val Arg
                   230                 235                 240
   Gln Gly Tyr Ser Pro Leu Ser Phe Gln Thr Leu Leu Pro Ala Pro
                   245                 250                 255
   Arg Gly Pro Asp Arg Pro Glu Gly Ile Glu Glu Glu Gly Gly
                   260                 265             269
(2) INFORMATION FOR SEQ ID NO:25:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:25:
   Gly Gly Gly Asn Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Leu
                   35                  40                  45
   Leu Gly Ala Val Phe Leu Gly Phe Leu Gly Ala Ala Gly Ser Thr
                   50                  55                  60
   Met Gly Ala Arg Ser Met Ala Leu Thr Val Gln Ala Arg Gln Leu
                       65                  70                  75
   Leu Ser Gly Ile Val Gln Gln Gln Asn Asn Leu Leu Arg Ala Ile
                       80                  85                  90
   Glu Ala Gln Gln His Leu Leu Gln Leu Thr Val Trp Gly Ile Lys
                       95                 100                 105
   Gln Leu Gln Ala Arg Val Leu Ala Val Glu Arg Tyr Leu Arg Asp
                      110                 115                 120
   Gln Gln Leu Leu Gly Ile Trp Gly Cys Ser Gly Lys Leu Ile Cys
                      125                 130                 135
   Thr Thr Ala Val Pro Trp Asn Ala Ser Trp Ser Asn Lys Ser Met
                      140                 145                 150
   Asp Met Ile Trp Asn Asn Met Thr Trp Met Glu Trp Glu Arg Glu
                      155                 160                 165
   Ile Asp Asn Tyr Thr Ser Leu Ile Tyr Thr Leu Ile Glu Glu Ser
                      170                 175                 180
   Gln Asn Gln Gln Glu Lys Asn Glu Gln Glu Leu Leu Glu Leu Asn
                      185                 190                 195
   Lys Trp Glu Asn Leu Trp Ser Trp Phe Asp Ile Ser Asn Trp Leu
                      200                 205                 210
   Trp Tyr Ile Lys Ile Phe Ile Met Ile Val Gly Gly Leu Val Gly
                      215                 220                 225
   Leu Arg Ile Val Phe Ala Val Leu Ser Ile Val Asn Ser Val Arg
                      230                 235                 240
   Gln Gly Tyr Ser Pro Leu Ser Phe Gln Thr Arg Leu Pro Ala Pro
                      245                 250                 255
   Arg Gly Pro Asp Arg Pro Glu Gly Ile Glu Glu Glu Gly Gly
                      260                 265             269
(2) INFORMATION FOR SEQ ID NO:26:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:26:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Thr Leu
                   35                  40                  45
   Gly Ala Met Phe Leu Gly Phe Leu Gly Ala Ala Gly Ser Thr Met
                   50                  55                  60
   Gly Ala Arg Ser Leu Thr Leu Thr Val Gln Ala Arg Gln Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                  100                 105
   Leu Gln Ala Arg Val Leu Ala Val Glu Arg Tyr Leu Lys Asp Gln
                   110                 115                 120
   Gln Leu Leu Gly Ile Trp Gly Cys Ser Gly Lys Leu Ile Cys Thr
                   125                 130                 135
   Thr Ala Val Pro Trp Asn Ala Ser Trp Ser Asn Lys Ser Leu Asp
                   140                 145                 150
   Gln Ile Trp Asn Asn Met Thr Trp Met Glu Trp Glu Arg Glu Ile
                   155                 160                 165
   Asp Asn Tyr Thr Asn Leu Ile Tyr Thr Leu Ile Glu Glu Ser Gln
                   170                 175                 180
   Asn Gln Gln Glu Lys Asn Glu Gln Glu Leu Leu Glu Leu Asp Lys
                   185                 190                 195
   Trp Ala Ser Leu Trp Asn Trp Phe Asp Ile Ser Lys Trp Leu Trp
                   200                 205                 210
   Tyr Ile Lys Ile Phe Ile Met Ile Val Gly Gly Leu Val Gly Leu
                   215                 220                 225
   Arg Ile Val Phe Thr Val Leu Ser Ile Val Asn Arg Val Arg Gln
                   230                 235                 240
   Gly Tyr Ser Pro Leu Ser Phe Gln Thr Arg Phe Pro Ala Pro Arg
                   245                 250                 255
   Gly Pro Asp Arg Pro Glu Gly Ile Glu Glu Glu Gly Gly
                   260                 265         268
(2) INFORMATION FOR SEQ ID NO:27:
   (i) SEQUENCE CHARACTERISTICS:
      (A) LENGTH: 267 amino acids
      (B) TYPE: Amino Acid
      (D) TOPOLOGY: Linear
   (xi) SEQUENCE DESCRIPTION: SEQ ID NO:27:
   Ala Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                       20                      25                      30
   Ala Arg Arg Arg Val Val Gln Arg Glu Lys Arg Ala Val Gly Met
                   35                  40                  45
   Leu Gly Ala Met Phe Leu Gly Phe Leu Gly Ala Ala Gly Ser Thr
                   50                  55                  60
   Met Gly Ala Arg Ser Met Thr Leu Thr Val Gln Ala Arg Gln Leu
                   65                  70                  75
   Leu Ser Gly Ile Val Gln Gln Gln Asn Asn Leu Leu Arg Ala Ile
                   80                  85                  90
   Glu Ala Gln Gln His Leu Leu Gln Leu Thr Val Trp Gly Ile Lys
                   95                  100                 105
   Gln Leu Gln Ala Arg Val Leu Ala Val Glu Arg Tyr Leu Lys Asp
                   110                 115                 120
   Gln Gln Leu Leu Gly Ile Trp Gly Cys Ser Gly Lys Leu Ile Cys
                   125                 130                 135
   Thr Thr Thr Val Pro Trp Asn Ala Ser Trp Ser Asn Lys Ser Leu
                   140                 145                 150
   Asn Glu Ile Trp Asp Asn Met Thr Trp Met Gln Trp Glu Arg Glu
                   155                 160                 165
   Ile Asp Asn Tyr Thr His Leu Ile Tyr Thr Leu Ile Glu Glu Ser
                   170                 175                 180
   Gln Asn Gln Gln Glu Lys Asn Glu Gln Glu Leu Leu Glu Leu Asp
                   185                 190                 195
   Lys Trp Leu Trp Ser Trp Phe Ser Ile Thr Asn Trp Leu Trp Tyr
                   200                 205                 210
   Ile Arg Ile Phe Ile Ile Ile Val Gly Gly Leu Val Gly Leu Arg
                   215                 220                 225
   Ile Val Phe Ala Val Leu Ser Ile Val Asn Arg Val Arg Gln Gly
                   230                 235                 240
   Tyr Ser Pro Leu Ser Phe Gln Thr Arg Leu Pro Thr Gln Arg Gly
                   245                 250                 255
   Pro Asp Arg Pro Glu Gly Ile Glu Glu Glu Gly Gly
                   260                 265     267
(2) INFORMATION FOR SEQ ID NO:28:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:28:
   Ala Gly Gly Asn Met Lys Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Val
                   35                  40                  45
   Ile Gly Ala Met Phe Leu Gly Phe Leu Gly Ala Ala Gly Ser Thr
                   50                  55                  60
   Met Gly Ala Ala Ser Ile Thr Leu Thr Val Gln Ala Arg Lys Leu
                   65                  70                  75
   Leu Ser Gly Ile Val Gln Gln Gln Asn Asn Leu Leu Arg Ala Ile
                   80                  85                  90
   Glu Ala Gln Gln His Leu Leu Gln Leu Thr Val Trp Gly Ile Lys
                   95                 100                 105
   Gln Leu Gln Ala Arg Val Leu Ala Val Glu Arg Tyr Leu Arg Asp
                  110                 115                 120
   Gln Gln Leu Leu Gly Ile Trp Gly Cys Ser Gly Lys Leu Ile Cys
                  125                 130                 135
   Thr Thr Thr Val Pro Trp Asn Thr Ser Trp Ser Asn Lys Ser Leu
                  140                 145                 150
   Asp Lys Ile Trp Asn Asn Met Thr Trp Met Glu Trp Glu Arg Glu
                  155                 160                 165
   Ile Asp Asn Tyr Thr Ser Leu Ile Tyr Thr Leu Leu Glu Glu Ser
                  170                 175                 180
   Gln Asn Gln Gln Glu Lys Asn Glu Gln Glu Leu Leu Glu Leu Asp
                  185                 190                 195
   Lys Trp Ala Ser Leu Trp Asn Trp Phe Ser Ile Thr Asn Trp Leu
                  200                 205                 210
   Trp Tyr Ile Arg Ile Phe Ile Met Ile Val Gly Gly Leu Ile Gly
                  215                 220                 225
   Leu Arg Ile Ile Phe Ala Val Leu Ser Ile Val Asn Arg Val Arg
                  230                 235                 240
   Gln Gly Tyr Ser Pro Leu Ser Phe Gln Thr Leu Ile Pro Ala Gln
                  245                 250                 255
   Arg Gly Pro Asp Arg Pro Glu Gly Ile Glu Glu Gly Gly Gly
                  260                 265             269
(2) INFORMATION FOR SEQ ID NO:29:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:29:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Glu Lys Arg Ala Val Gly Met
   35 40 45
   Leu Gly Ala Met Phe Leu Gly Phe Leu Gly Ala Ala Gly Ser Thr
   50 55 60
   Met Gly Ala Thr Ser Met Ala Leu Thr Val Gln Ala Arg Gln Leu
   65 70 75
   Leu Ser Gly Ile Val Gln Gln Gln Asn Asn Leu Leu Arg Ala Ile
   80 85 90
   Lys Ala Gln Gln His Leu Leu Gln Leu Thr Val Trp Gly Ile Lys
   95 100 105
   Gln Leu Gln Ala Arg Ile Leu Ala Val Glu Arg Tyr Leu Lys Asp
   110 115 120
   Gln Gln Leu Leu Gly Phe Trp Gly Cys Ser Gly Lys Leu Ile Cys
   125 130 135
   Thr Thr Ala Val Pro Trp Asn Ala Ser Trp Ser Asn Lys Thr Leu
   140 145 150
   Asp Gln Ile Trp Asn Asn Met Thr Trp Met Glu Trp Asp Arg Glu
   155 160 165
   Ile Asp Asn Tyr Thr His Leu Ile Tyr Thr Leu Ile Glu Glu Ser
   170 175 180
   Gln Asn Gln Gln Glu Lys Asn Gln Gln Glu Leu Leu Gln Leu Asp
   185 190 195
   Lys Trp Ala Ser Leu Trp Thr Trp Ser Asp Ile Thr Lys Trp Leu
   200 205 210
   Trp Tyr Ile Lys Ile Phe Ile Met Ile Val Gly Gly Leu Ile Gly
   215 220 225
   Leu Arg Ile Val Phe Ala Val Leu Ser Ile Val Asn Arg Val Arg
   230 235 240
   Gln Gly Tyr Ser Pro Leu Ser Phe Gln Thr Leu Leu Pro Asn Pro
   245 250 255
   Arg Gly Pro Asp Arg Pro Glu Gly Thr Glu Glu Gly Gly Gly
   260 265 269
(2) INFORMATION FOR SEQ ID NO:30:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:30:
   Gly Gly Gly Asp Met Arg Asp Asn Trp Arg Ser Glu Leu Tyr Lys
   1 5 10 15
   Tyr Lys Val Ile Lys Ile Glu Pro Leu Gly Ile Ala Pro Thr Lys
   20 25 30
   Ala Lys Arg Arg Val Val Gln Arg Glu Lys Arg Ala Val Gly Ile
   35 40 45
   Val Gly Ala Met Phe Leu Gly Phe Leu Gly Ala Ala Gly Ser Thr
   50 55 60
   Met Gly Ala Val Ser Leu Thr Leu Thr Val Gln Ala Arg Gln Leu
   65 70 75
   Leu Ser Gly Ile Val Gln Gln Gln Asn Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Arg Asp
   110 115 120
   Gln Gln Leu Leu Gly Ile Trp Gly Cys Ser Gly Lys Leu Ile Cys
   125 130 135
   Thr Thr Ala Val Pro Trp Asn Ala Ser Trp Ser Asn Lys Ser Leu
   140 145 150
   Glu Asp Ile Trp Asp Asn Met Thr Trp Met Gln Trp Glu Arg Glu
   155 160 165
   Ile Asp Asn Tyr Thr Asn Thr Ile Tyr Thr Leu Leu Glu Glu Ser
   170 175 180
   Gln Asn Gln Gln Glu Lys Asn Glu Gln Glu Leu Leu Glu Leu Asp
   185 190 195
   Lys Trp Ala Ser Leu Trp Asn Trp Phe Ser Ile Thr Asn Trp Leu
   200 205 210
   Trp Tyr Ile Lys Ile Phe Ile Met Ile Val Gly Gly Leu Val Gly
   215 220 225
   Leu Arg Ile Val Phe Ala Val Leu Ser Ile Val Asn Arg Val Arg
   230 235 240
   Gln Gly Tyr Ser Pro Leu Ser Phe Gln Thr Arg Leu Pro Val Pro
   245 250 255
   Arg Gly Pro Asp Arg Pro Asp Gly Ile Glu Glu Glu Gly Gly
   260 265 269
(2) INFORMATION FOR SEQ ID NO:31:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:31:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Ile Ala Pro Thr Lys
                   20                  25                  30
   Ala Lys Arg Arg Val Val Gln Arg Glu Lys Arg Ala Val Gly Ile
                   35                  40                  45
   Ile Gly Ala Met Phe Leu Gly Phe Leu Gly Ala Ala Gly Ser Thr
                   50                  55                  60
   Met Gly Ala Arg Ser Met Thr Leu Thr Val Gln Ala Arg Lys Leu
                   65                  70                  75
   Leu Ser Gly Ile Val Gln Gln Gln Asn Asn Leu Leu Arg Ala Ile
                   80                  85                  90
   Glu Ala Gln Gln His Leu Leu Gln Leu Thr Val Trp Gly Ile Lys
                   95                 100                 105
   Gln Leu Gln Ala Arg Val Leu Ala Val Glu Arg Tyr Leu Arg Asp
                  110                 115                 120
   Gln Gln Leu Leu Gly Ile Trp Gly Cys Ser Gly Lys Leu Ile Cys
                  125                 130                 135
   Thr Thr Ala Val Pro Trp Asn Thr Ser Trp Ser Asn Lys Ser Met
                  140                 145                 150
   Glu Asp Ile Trp Asp Asn Met Thr Trp Met Gln Trp Glu Lys Glu
                  155                 160                 165
   Ile Asp Asn Tyr Thr Asn Thr Ile Tyr Thr Leu Leu Glu Glu Ser
                  170                 175                 180
   Gln Asn Gln Gln Glu Lys Asn Glu Gln Glu Leu Leu Glu Leu Asp
                  185                 190                 195
   Lys Trp Ala Ser Leu Trp Asn Trp Phe Asn Ile Thr Asn Trp Leu
                  200                 205                 210
   Trp Tyr Ile Lys Ile Phe Ile Met Ile Val Gly Gly Leu Val Gly
                  215                 220                 225
   Leu Arg Ile Val Phe Ala Val Leu Ser Val Val Asn Arg Val Arg
                   230                 235                 240
   Gln Gly Tyr Ser Pro Leu Ser Phe Gln Thr Arg Leu Pro Thr Pro
                   245                 250                 255
   Arg Gly Pro Asp Arg Pro Asp Gly Ile Glu Glu Glu Gly Gly
                   260                 265             269
(2) INFORMATION FOR SEQ ID NO:32:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:32:
   Gly Gly Gly Asn Met Arg Asp Asn Trp Arg Asn Glu Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Glu Lys Arg Ala Val Gly Met
   35 40 45
   Leu Gly Ala Met Phe Leu Gly Phe Leu Gly Ala Ala Gly Ser Thr
   50 55 60
   Met Gly Ala Arg Ser Leu Thr Leu Thr Val Gln Ala Arg Gln Leu
   65 70 75
   Leu Ser Gly Ile Val Gln Gln Gln Asn Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Arg Asp
   110 115 120
   Gln Gln Leu Leu Gly Ile Trp Gly Cys Ser Gly Lys Leu Ile Cys
   125 130 135
   Thr Thr Thr Val Pro Trp Asn Ala Ser Trp Ser Asn Lys Thr Leu
   140 145 150
   Asp Gln Ile Trp Asn Asn Met Thr Trp Met Glu Trp Glu Arg Glu
   155 160 165
   Ile Asp Asn Tyr Thr Ser Leu Ile Tyr Thr Leu Ile Glu Gln Ser
   170 175 180
   Gln Asn Gln Gln Glu Lys Asn Glu Gln Glu Leu Leu Glu Leu Asp
   185 190 195
   Lys Trp Ala Ser Leu Trp Ser Trp Tyr Asp Ile Ser Asn Trp Leu
   200 205 210
   Trp Tyr Ile Lys Ile Phe Ile Met Ile Val Gly Gly Leu Ile Gly
   215 220 225
   Leu Arg Ile Val Phe Ala Val Leu Ser Ile Val Asn Arg Val Arg
   230 235 240
   Gln Gly Tyr Ser Pro Leu Ser Phe Gln Thr Leu Leu Pro Ala Thr
   245 250 255
   Arg Gly Pro Arg Gln Pro Glu Glu Ile Glu Glu Glu Gly Gly
   260 265 269
(2) INFORMATION FOR SEQ ID NO:33:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:33:
   Gly Gly Gly Asn Met Arg Asp Asn Trp Arg Ser Glu Leu Tyr Lys
   1 5 10 15
   Tyr Lys Val Val Arg Ile Glu Pro Leu Gly Val Ala Pro Thr Arg
   20 25 30
   Ala Lys Arg Arg Val Val Gln Arg Glu Lys Arg Ala Val Gly Thr
   35 40 45
   Ile Gly Ala Met Phe Leu Gly Phe Leu Gly Ala Ala Gly Ser Thr
   50 55 60
   Met Gly Ala Gly Ser Ile Thr Leu Thr Val Gln Ala Arg His Leu
   65 70 75
   Leu Ser Gly Ile Val Gln Gln Gln Asn Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Arg Asp
   110 115 120
   Gln Gln Leu Leu Gly Ile Trp Gly Cys Ser Gly Lys Leu Ile Cys
   125 130 135
   Thr Thr Thr Val Pro Trp Asn Ala Ser Trp Ser Asn Lys Ser Leu
   140 145 150
   Asn Met Ile Trp Asn Asn Met Thr Trp Met Gln Trp Glu Arg Glu
   155 160 165
   Ile Asp Asn Tyr Thr Gly Ile Ile Tyr Asn Leu Leu Glu Glu Ser
   170 175 180
   Gln Asn Gln Gln Glu Lys Asn Glu Gln Glu Leu Leu Glu Leu Asp
   185 190 195
   Lys Trp Ala Asn Leu Trp Asn Trp Phe Asp Ile Thr Gln Trp Leu
   200 205 210
   Trp Tyr Ile Arg Ile Phe Ile Met Ile Val Gly Gly Leu Val Gly
   215 220 225
   Leu Lys Ile Val Phe Ala Val Leu Ser Ile Val Asn Arg Val Arg
   230 235 240
   Gln Gly Tyr Ser Pro Leu Ser Phe Gln Thr His Leu Pro Ala Pro
   245 250 255
   Arg Gly Pro Asp Arg Pro Glu Gly Ile Glu Gly Glu Gly Gly
   260 265 269
(2) INFORMATION FOR SEQ ID NO:34:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:34:
   Gly Gly Gly Asn Met Arg Asp Asn Trp Arg Ser Glu Leu Tyr Lys
    1               5                  10                  15
   Tyr Lys Val Val Arg Ile Glu Pro Leu Gly Val Ala Pro Thr Lys
                   20                  25                  30
   Ala Lys Arg Arg Val Val Gln Arg Glu Lys Arg Ala Val Gly Thr
                   35                  40                  45
   Ile Gly Ala Met Phe Leu Gly Phe Leu Gly Ala Ala Gly Ser Thr
                   50                  55                  60
   Met Gly Ala Gly Ser Leu Thr Leu Thr Val Gln Ala Arg Gln Leu
                   65                  70                  75
   Leu Ser Gly Ile Val Gln Gln Gln Asn Asn Leu Leu Arg Ala Ile
                   80                  85                  90
   Asp Ala Gln Gln His Leu Leu Gln Leu Thr Val Trp Gly Ile Lys
                   95                 100                 105
   Gln Leu Gln Ala Arg Val Leu Ala Val Glu Arg Tyr Leu Arg Asp
                  110                 115                 120
   Gln Gln Leu Leu Gly Ile Trp Gly Cys Ser Gly Lys Leu Ile Cys
                  125                 130                 135
   Thr Thr Thr Val Pro Trp Asn Ala Ser Trp Ser Asn Lys Ser Met
                  140                 145                 150
   Asn Gln Ile Trp Asp Asn Leu Thr Trp Met Glu Trp Glu Arg Glu
                   155                 160                 165
   Ile Asp Asn Tyr Thr Ser Ile Ile Tyr Ser Leu Ile Glu Glu Ser
                   170                 175                 180
   Gln Asn Gln Gln Gly Lys Asn Glu Gln Glu Leu Leu Glu Leu Asp
                   185                 190                 195
   Lys Trp Ala Ser Leu Trp Asn Trp Phe Asp Ile Thr Asn Trp Leu
                   200                 205                 210
   Trp Tyr Ile Lys Ile Phe Ile Met Ile Val Gly Gly Leu Ile Gly
                   215                 220                 225
   Leu Arg Ile Val Phe Thr Val Leu Ser Ile Val Asn Arg Val Arg
                   230                 235                 240
   Gln Gly Tyr Ser Pro Leu Ser Phe Gln Thr His Leu Pro Thr Pro
                   245                 250                 255
   Arg Gly Pro Asp Arg Pro Glu Gly Ile Glu Glu Glu Gly Gly
                   260                 265             269
(2) INFORMATION FOR SEQ ID NO:35:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:35:
   Gly Gly Gly Asn Met Arg Asp Asn Trp Arg Ser Glu Leu Tyr Lys
   1               5                   10                  15
   Tyr Lys Val Val Lys Ile Glu Pro Leu Gly Val Ala Pro Thr Xaa
               20                  25                  30
   Ala Lys Arg Arg Val Val Gln Arg Glu Lys Arg Ala Val Gly Ile
           35                  40                  45
   Gly Ala Ala Ser Pro Gly Phe Leu Gly Ala Ala Gly Ser Thr Met
       50                  55                  60
   Xaa Ala Ala Pro Thr Thr Leu Thr Val Gln Pro Arg Gln Leu Leu
   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
               95                  100                 105
   Leu Gln Ala Arg Val Leu Ala Val Glu Arg Tyr Leu Lys Asp Gln
           110                 115                 120
   Gln Leu Leu Gly Ile Trp Gly Cys Ser Gly Lys Leu Ile Cys Thr
       125                 130                 135
   Thr Thr Val Pro Trp Asn Ala Ser Trp Ser Asn Lys Ser Leu Asp
   140                 145                 150
   Glu Ile Trp Asn Asn Met Thr Trp Met Glu Trp Glu Arg Glu Ile
                   155                 160                 165
   Asn Asn Tyr Thr Gly Leu Ile Tyr Thr Leu Ile Glu Glu Ser Gln
               170                 175                 180
   Xaa Gln Gln Glu Lys Asn Glu Leu Asp Leu Leu Glu Leu Asp Lys
           185                 190                 195
   Trp Ala Ser Leu Trp Asn Trp Phe Asp Ile Thr Asn Xaa Leu Trp
       200                 205                 210
   Tyr Ile Lys Ile Phe Ile Met Ile Val Gly Gly Leu Val Gly Leu
   215                 220                 225
   Arg Ile Ile Phe Thr Val Leu Ser Ile Val Asn Arg Val Arg Gln
                   230                 235                 240
   Gly Tyr Ser Pro Leu Ser Phe Gln Thr His Leu Pro Xaa Pro Arg
               245                 250                 255
   Gly Pro Asp Arg Pro Gly Gly Ile Glu Glu Glu Gly Gly
           260                 265             268
(2) INFORMATION FOR SEQ ID NO:36:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:36:
   Gly Gly Gly Asp Met Arg Asp Asn Trp Arg Ser Glu Leu Tyr Lys
   1               5                   10                  15
   Tyr Lys Val Val Arg Ile Glu Pro Leu Gly Ile Ala Pro Thr Arg
                   20                  25                  30
   Ala Lys Arg Arg Val Val Gln Arg Glu Lys Arg Ala Val Gly Thr
                   35                  40                  45
   Leu Gly Ala Met Phe Leu Gly Phe Leu Gly Ala Ala Gly Ser Thr
                   50                  55                  60
   Met Gly Ala Ala Ser Val Ala Leu Thr Val Gln Ala Arg Gln Leu
                   65                  70                  75
   Leu Ser Gly Ile Val Gln Gln Gln Asn Asn Leu Leu Arg Ala Ile
                   80                  85                  90
   Glu Ala Gln Gln His Met Leu Gln Leu Thr Val Trp Gly Ile Lys
                   95                  100                 105
   Gln Leu Gln Ala Arg Val Leu Ala Val Glu Arg Tyr Leu Gly Asp
   110 115 120
   Gln Gln Leu Leu Gly Ile Trp Gly Cys Ser Gly Lys Leu Ile Cys
   125 130 135
   Thr Thr Thr Val Pro Trp Asn Thr Ser Trp Ser Asn Lys Ser Leu
   140 145 150
   Asp Asp Ile Trp Thr Asn Met Thr Trp Met Glu Trp Lys Arg Glu
   155 160 165
   Ile Asp Asn Tyr Thr Ser Leu Ile Tyr Thr Leu Ile Glu Glu Ser
   170 175 180
   Gln Arg Gln Gln Glu Lys Asn Glu Gln Glu Leu Leu Glu Leu Asp
   185 190 195
   Lys Trp Asp Ser Leu Trp Asn Trp Phe Thr Ile Ser Lys Trp Leu
   200 205 210
   Trp Tyr Ile Lys Ile Phe Ile Met Ile Val Ala Gly Leu Val Gly
   215 220 225
   Leu Arg Ile Val Phe Ala Val Leu Ser Ile Val Asn Lys Val Arg
   230 235 240
   Gln Gly Tyr Ser Pro Val Ser Phe Gln Thr Arg Leu Pro Ala Gln
   245 250 255
   Arg Gly Pro Asp Arg Pro Glu Glu Ile Glu Glu Glu Gly Gly
   260 265 269
(2) INFORMATION FOR SEQ ID NO:37:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:37:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
   20 25 30
   Pro Lys Arg Arg Val Val Gln Arg Glu Lys Arg Ala Val Gly Thr
   35 40 45
   Ile Gly Ala Met Phe Leu Gly Phe Leu Gly Thr Ala Gly Ser Thr
   50 55 60
   Met Gly Ala Ala Ser Ile Thr Leu Thr Val Gln Ala Arg Gln Leu
   65 70 75
   Leu Ser Gly Ile Val Gln Gln Gln Arg Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Lys Asp
   110 115 120
   Gln Gln Leu Leu Gly Ile Trp Gly Cys Ser Gly Lys Leu Ile Cys
   125 130 135
   Thr Thr Ala Val Pro Trp Asn Ala Ser Trp Ser Asn Lys Ser Leu
   140 145 150
   Asp Lys Ile Trp Asn Asn Met Thr Trp Met Glu Trp Glu Arg Glu
   155 160 165
   Ile Asp Asn Tyr Thr Arg Glu Ile Tyr Thr Leu Ile Glu Glu Ser
   170 175 180
   Gln Asn Gln Gln Glu Lys Asn Glu Leu Glu Leu Leu Glu Leu Asp
   185 190 195
   Lys Trp Ala Ser Leu Trp Asn Trp Phe Asp Ile Thr Lys Trp Leu
   200 205 210
   Trp Tyr Ile Lys Ile Phe Ile Met Ile Val Gly Gly Leu Val Gly
   215 220 225
   Leu Arg Ile Val Phe Ala Val Leu Ser Ile Val Asn Arg Val Arg
   230 235 240
   Gln Gly Tyr Ser Pro Leu Ser Leu Gln Thr Arg Phe Pro Ala Gln
   245 250 255
   Arg Gly Pro Gly Gly Pro Glu Gly Ile Glu Glu Glu Gly Gly
   260 265 269
(2) INFORMATION FOR SEQ ID NO:38:
   (i) SEQUENCE CHARACTERISTICS:
      (A) LENGTH: 267 amino acids
      (B) TYPE: Amino Acid
      (D) TOPOLOGY: Linear
   (xi) SEQUENCE DESCRIPTION: SEQ ID NO:38:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Ile Ala Pro Thr Lys
                   20                  25                  30
   Ala Lys Arg Arg Val Val Gln Arg Glu Lys Arg Ala Val Gly Ile
                   35                  40                  45
   Gly Ala Val Phe Leu Gly Phe Leu Gly Ala Ala Gly Ser Thr Met
                   50                  55                  60
   Gly Ala Ala Ala Met Thr Leu Thr Val Gln Ala Arg Leu Leu Leu
                       65                  70                  75
   Thr Gly Ile Val Gln Gln Gln Asn Asn Leu Leu Lys Ala Ile Glu
                       80                  85                  90
   Ala Gln Gln His Leu Leu Gln Leu Thr Val Trp Gly Ile Lys Gln
                       95                  100                 105
   Leu Gln Ala Arg Val Leu Ala Val Glu Arg Tyr Leu Lys Asp Gln
                       110                 115                 120
   Gln Leu Leu Gly Ile Trp Gly Cys Ser Gly Lys Leu Ile Cys Thr
                       125                 130                 135
   Thr Thr Val Pro Trp Asn Thr Ser Trp Ser Asn Lys Ser Leu Asp
                       140                 145                 150
   Lys Ile Trp Gly Asn Met Thr Trp Met Glu Trp Glu Arg Glu Ile
                       155                 160                 165
   Asp Asn Tyr Thr Gly Leu Ile Tyr Thr Leu Ile Glu Glu Ser Gln
                       170                 175                 180
   Asn Gln Gln Glu Lys Asn Glu Gln Glu Leu Leu Glu Leu Asp Lys
                       185                 190                 195
   Trp Ala Ser Leu Trp Asn Trp Phe Thr Ile Thr Asn Trp Leu Trp
                       200                 205                 210
   Tyr Ile Lys Ile Phe Ile Met Ile Val Gly Gly Leu Ile Gly Leu
                       215                 220                 225
   Arg Ile Val Phe Ala Val Leu Ser Ile Val Asn Arg Val Arg Gln
                       230                 235                 240
   Gly Tyr Ser Pro Leu Ser Phe Gln Thr Arg Leu Pro Ala Pro Arg
                       245                 250                 255
   Gly Pro Asp Arg Pro Glu Gly Ile Glu Glu Glu Gly
                       260                 265     267
(2) INFORMATION FOR SEQ ID NO:39:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:39:
   Gly Gly Gly Asp Met Arg Glu Asn Trp Arg Ser Glu Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Glu Lys Arg Ala Val Gly Phe
   35 40 45
   Gly Ala Met Phe Leu Gly Phe Leu Gly Ala Ala Gly Ser Thr Met
   50 55 60
   Gly Ala Ala Ser Met Thr Leu Thr Val Gln Ala Arg Leu Leu Leu
   65 70 75
   Ser Gly Ile Val Gln Gln Gln Ser Asn Leu Leu Arg Ala Ile Glu
   80 85 90
   Ala Gln Gln His Leu Leu Gln Leu Thr Val Trp Gly Ile Lys Gln
   95 100 105
   Leu Gln Ala Arg Val Leu Ala Val Glu Arg Tyr Leu Lys Asp Gln
   110 115 120
   Gln Leu Leu Gly Ile Trp Gly Cys Ser Gly Lys Leu Ile Cys Thr
   125 130 135
   Thr Asn Val Pro Trp Asn Lys Thr Trp Ser Asn Lys Ser Leu Asp
   140 145 150
   Gln Ile Trp Gln Asn Met Thr Trp Met Gln Trp Glu Arg Glu Ile
   155 160 165
   Asp Lys Tyr Thr Asp Val Ile Tyr Thr Leu Ile Gly Glu Ser Gln
   170 175 180
   Asn Gln Gln Glu Lys Asn Glu Gln Glu Leu Leu Glu Leu Asp Lys
   185 190 195
   Trp Ala Ser Leu Trp Asn Trp Phe Asp Ile Thr Gln Trp Leu Trp
   200 205 210
   Tyr Ile Lys Ile Phe Ile Met Ile Val Gly Gly Leu Val Gly Leu
   215 220 225
   Arg Ile Val Phe Ser Val Leu Ser Ile Val Asn Arg Val Arg Gln
   230 235 240
   Gly Tyr Ser Pro Leu Ser Phe Gln Thr Arg Leu Pro Ala Ala Arg
   245 250 255
   Gly Pro Asp Arg Pro Glu Gly Ile Glu Glu Glu Gly Gly
   260 265 268
(2) INFORMATION FOR SEQ ID NO:40:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:40:
   Gly Gly Gly Asn Met Lys Asp Asn Trp Arg Ser Glu Leu Tyr Lys
     1               5                  10                  15
   Tyr Lys Val Val Arg Ile Glu Pro Leu Gly Ile Ala Pro Thr Arg
                       20                  25                  30
   Ala Lys Arg Arg Val Val Gln Arg Glu Lys Arg Ala Ala Gly Leu
                       35                  40                  45
   Gly Val Met Phe Leu Gly Phe Leu Gly Ala Ala Gly Ser Thr Met
                       50                  55                  60
   Gly Ala Ala Ser Ile Ala Leu Thr Val Gln Ala Arg Gln Leu Leu
                       65                  70                  75
   Ser Gly Ile Val Gln Gln Gln Asn Asn Leu Leu Arg Ala Ile Glu
                       80                  85                  90
   Ala Gln Gln His Met Leu Gln Leu Thr Val Trp Gly Ile Lys Gln
                       95                  100                 105
   Leu Gln Ala Arg Val Leu Ala Val Glu Arg Tyr Leu Arg Asp Gln
                       110                 115                 120
   Gln Leu Leu Gly Ile Trp Gly Cys Ser Gly Lys Leu Ile Cys Thr
                       125                 130                 135
   Thr Thr Val Pro Trp Asn Ala Ser Trp Ser Asn Lys Ser His Asp
                       140                 145                 150
   Gln Ile Trp Gln Asn Met Thr Trp Met Gln Trp Glu Lys Glu Ile
                       155                 160                 165
   Asp Asn Tyr Thr Ser Leu Ile Tyr Asn Leu Ile Glu Val Ser Gln
                       170                 175                 180
   Asn Gln Gln Glu Lys Asn Glu Gln Glu Leu Leu Glu Leu Asp Lys
                       185                 190                 195
   Trp Ala Ser Leu Trp Asn Trp Phe Asp Ile Thr Asn Trp Leu Trp
                       200                 205                 210
   Tyr Ile Lys Ile Phe Ile Met Ile Val Gly Gly Leu Ile Gly Leu
                       215                 220                 225
   Arg Ile Val Phe Ile Val Leu Ser Ile Val Asn Arg Val Arg Gln
                       230                 235                 240
   Gly Tyr Ser Pro Leu Ser Phe Gln Thr His Leu Pro Ala Arg Arg
                       245                 250                 255
   Gly Pro Asp Arg Pro Glu Gly Ile Glu Glu Glu Gly Gly
                       260                 265         268
(2) INFORMATION FOR SEQ ID NO:41:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:41:
   Gly Gly Gly Asn Met Arg Asp Asn Trp Arg Ser Glu Leu Tyr Lys
    1               5                  10                  15
   Tyr Lys Val Val Lys Ile Glu Pro Leu Gly Val Ala Pro Thr Arg
                   20                  25                  30
   Ala Lys Arg Arg Val Val Gln Arg Glu Lys Arg Ala Val Gly Ile
                   35                  40                  45
   Gly Ala Met Phe Leu Gly Phe Leu Gly Ala Ala Gly Ser Thr Met
                   50                  55                  60
   Gly Ala Ala Ser Leu Thr Leu Thr Val Gln Ala Arg Leu Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                 100                 105
   Leu Gln Ala Arg Val Leu Ala Val Glu Arg Tyr Leu Lys Asp Gln
                  110                 115                 120
   Gln Leu Leu Gly Ile Trp Gly Cys Ser Gly Lys Leu Ile Cys Thr
                  125                 130                 135
   Thr Thr Val Pro Trp Asn Thr Ser Trp Ser Asn Lys Ser Leu Asp
                  140                 145                 150
   Gln Ile Trp Gly Asn Met Thr Trp Met Gln Trp Glu Arg Glu Ile
                  155                 160                 165
   Asp Asn Tyr Thr Gly Leu Ile Tyr Thr Leu Ile Glu Glu Ser Gln
                  170                 175                 180
   Asn Gln Gln Glu Lys Asn Glu Gln Glu Leu Leu Glu Leu Asp Lys
                  185                 190                 195
   Trp Ala Ser Leu Trp Asn Trp Phe Asn Ile Thr Asn Trp Leu Trp
                  200                 205                 210
   Tyr Ile Lys Ile Phe Ile Met Ile Val Ala Gly Leu Val Gly Leu
                  215                 220                 225
   Arg Val Val Phe Ile Val Leu Ser Ile Val Asn Arg Val Arg Gln
                  230                 235                 240
   Gly Tyr Ser Pro Leu Ser Phe Gln Thr His His Pro Ala Leu Arg
                  245                 250                 255
   Gly Pro Asp Arg Pro Glu Gly Ile Glu Glu Glu Gly Gly
                  260                 265         268
(2) INFORMATION FOR SEQ ID NO:42:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:42:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Glu Lys Arg Ala Val Gly Ile
   35 40 45
   Val Gly Ala Met Phe Leu Gly Phe Leu Gly Ala Ala Gly Ser Thr
   50 55 60
   Met Gly Ala Ala Ser Met Thr Leu Thr Val Gln Ala Arg Leu Leu
   65 70 75
   Leu Ser Gly Ile Val Gln Gln Gln Asn Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Lys Asp
   110 115 120
   Gln Gln Leu Leu Gly Ile Trp Gly Cys Ser Gly Lys Leu Ile Cys
   125 130 135
   Thr Thr Thr Val Pro Trp Asn Thr Ser Trp Ser Asn Lys Ser Leu
   140 145 150
   Ser Glu Ile Trp Asp Asn Met Thr Trp Met Gln Trp Glu Arg Glu
   155 160 165
   Ile Asp Asn Tyr Thr Ser Leu Ile Tyr Thr Leu Ile Glu Glu Ser
   170 175 180
   Gln Asn Gln Gln Glu Lys Asn Glu Gln Glu Leu Leu Glu Leu Asp
   185 190 195
   Lys Trp Ala Gly Leu Trp Asn Trp Phe Glu Ile Thr Asn Trp Leu
   200 205 210
   Trp Tyr Ile Lys Ile Phe Ile Met Ile Val Gly Gly Leu Val Gly
   215 220 225
   Leu Arg Ile Val Phe Ala Val Leu Ser Ile Val Asn Arg Val Arg
   230 235 240
   Gln Gly Tyr Ser Pro Val Ser Phe Gln Thr His Leu Pro Ala Pro
   245 250 255
   Arg Gly Pro Asp Arg Pro Glu Gly Ile Glu Glu Glu Gly Gly
   260 265 269
(2) INFORMATION FOR SEQ ID NO:43:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:43:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Arg
   20 25 30
   Ala Lys Arg Arg Val Val Gln Arg Glu Lys Arg Ala Val Gly Ala
   35 40 45
   Leu Gly Ala Met Phe Leu Gly Phe Leu Gly Ala Ala Gly Ser Thr
   50 55 60
   Met Gly Ala Ala Ser Met Thr Leu Thr Val Gln Ala Arg Gln Leu
   65 70 75
   Leu Ser Gly Ile Val Gln Gln Gln Asn Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Ile Leu Ala Val Glu Arg Tyr Leu Lys Asp
   110 115 120
   Gln Gln Leu Leu Gly Ile Trp Gly Cys Ser Gly Lys Leu Ile Cys
   125 130 135
   Thr Thr Ala Val Pro Trp Asn Thr Ser Trp Ser Asn Lys Ser Leu
   140 145 150
   Glu Lys Ile Trp Asn Asn Met Thr Trp Met Glu Trp Glu Arg Glu
   155 160 165
   Ile Asp Asn Tyr Thr Gly Leu Ile Tyr Ser Leu Ile Glu Glu Ser
   170 175 180
   Gln Asn Gln Gln Glu Lys Asn Glu Gln Asp Leu Leu Glu Leu Asp
   185 190 195
   Lys Trp Ala Ser Leu Trp Asn Trp Phe Asn Ile Thr Lys Trp Leu
   200 205 210
   Trp Tyr Ile Lys Ile Phe Ile Met Ile Val Gly Gly Leu Ile Gly
   215 220 225
   Leu Arg Ile Val Phe Ala Val Leu Ser Ile Val Asn Arg Val Arg
   230 235 240
   Gln Gly Tyr Ser Pro Leu Ser Phe Gln Thr Leu Leu Pro Ala Gln
   245 250 255
   Arg Gly Pro Asp Arg Pro Gly Gly Ile Glu Glu Glu Gly Gly
   260 265 269
(2) INFORMATION FOR SEQ ID NO:44:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:44:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
   20 25 30
   Ala Lys Lys Arg Val Val Gln Arg Glu Lys Arg Ala Val Gly Val
   35 40 45
   Leu Gly Ala Met Phe Leu Gly Phe Leu Gly Ala Ala Gly Ser Thr
   50 55 60
   Met Gly Ala Ala Ser Met Thr Leu Thr Val Gln Ala Arg Gln Leu
   65 70 75
   Leu Ser Gly Ile Val Gln Gln Gln Asn Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Ile Leu Ala Met Glu Arg Tyr Leu Lys Asp
   110 115 120
   Gln Gln Leu Leu Gly Ile Trp Gly Cys Ser Gly Lys Leu Ile Cys
   125 130 135
   Thr Thr Ala Val Pro Trp Asn Thr Ser Trp Ser Asn Lys Ser Leu
   140 145 150
   Glu Lys Ile Trp Asn Asn Met Thr Trp Met Glu Trp Glu Arg Glu
   155 160 165
   Ile Asp Asn Tyr Thr Gly Leu Ile Tyr Ser Leu Ile Gly Glu Ser
   170 175 180
   Gln Asn Gln Gln Glu Lys Asn Glu Gln Asp Leu Leu Glu Leu Asp
   185 190 195
   Lys Trp Ala Ser Leu Trp Asn Trp Phe Asn Ile Thr Lys Trp Leu
   200 205 210
   Trp Tyr Ile Lys Ile Phe Ile Met Ile Val Gly Gly Leu Val Gly
   215 220 225
   Leu Arg Ile Val Phe Ala Val Leu Ser Ile Val Asn Arg Val Arg
   230 235 240
   Gln Gly Tyr Ser Pro Leu Ser Phe Gln Thr Leu Leu Pro Ala Gln
   245 250 255
   Arg Gly Pro Asp Arg Pro Gly Gly Ile Glu Glu Glu Gly Gly
   260 265 269
(2) INFORMATION FOR SEQ ID NO:45:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:45:
   Gly Gly Gly Asp Met Lys Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Ser Arg Val Val Gln Arg Glu Lys Arg Ala Val Gly Val
                   35                  40                  45
   Leu Gly Ala Met Phe Leu Gly Leu Leu Gly Ala Ala Gly Ser Thr
                   50                  55                  60
   Met Gly Ala Ala Ser Met Thr Leu Thr Val Gln Ala Arg Gln Leu
                   65                  70                  75
   Leu Ser Gly Ile Val Gln Gln Gln Asn Asn Leu Leu Arg Ala Ile
                   80                  85                  90
   Glu Ala Gln Gln His Leu Ser Gln Leu Thr Val Trp Gly Ile Lys
                   95                 100                 105
   Gln Leu Gln Ala Arg Val Leu Ala Val Glu Arg Tyr Leu Lys Asp
                  110                 115                 120
   Gln Gln Leu Leu Gly Ile Trp Gly Cys Ser Gly Lys Leu Ile Cys
                  125                 130                 135
   Pro Thr Ala Val Pro Trp Asn Ala Ser Trp Ser Asn Arg Ser Leu
                  140                 145                 150
   Gln Tyr Ile Trp Asn Asn Met Thr Trp Ile Glu Trp Glu Arg Glu
                  155                 160                 165
   Ile Asp Asn Tyr Thr Asp Ile Ile Tyr Ser Leu Ile Glu Lys Ser
                  170                 175                 180
   Gln Asn Gln Gln Glu Lys Asn Glu Gln Glu Leu Leu Glu Leu Asp
                  185                 190                 195
   Gln Trp Ala Ser Leu Trp Asn Trp Phe Ser Ile Thr Lys Trp Leu
                       200                 205                 210
   Trp Tyr Ile Lys Leu Phe Ile Met Ile Val Gly Gly Leu Ile Gly
                   215                 220                 225
   Leu Arg Ile Val Phe Ala Ile Leu Ser Ile Val Asn Arg Ala Arg
                   230                 235                 240
   Gln Gly Tyr Ser Pro Leu Ser Phe Gln Thr Leu Leu Pro Ala Pro
                   245                 250                 255
   Arg Gly Leu Asp Arg Pro Glu Gly Ile Gly Glu Glu Gly Gly
                   260                 265             269
(2) INFORMATION FOR SEQ ID NO:46:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:46:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Lys
   20 25 30
   Ala Lys Arg Arg Val Val Gln Arg Glu Lys Arg Ala Val Gly Thr
   35 40 45
   Leu Gly Ala Met Phe Leu Gly Phe Leu Gly Ala Ala Gly Ser Thr
   50 55 60
   Met Gly Ala Ala Ser Met Thr Leu Thr Val Gln Ala Arg Leu Leu
   65 70 75
   Leu Ser Gly Ile Val Gln Gln Gln Asn Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Lys Asp
   110 115 120
   Arg Gln Leu Leu Gly Ile Trp Gly Cys Ser Gly Lys Pro Ile Cys
   125 130 135
   Thr Thr Ser Val Pro Trp Asn Ser Ser Trp Ser Asn Lys Ser Leu
   140 145 150
   Glu Gln Ile Trp Asn Asn Met Thr Trp Leu Glu Trp Glu Arg Glu
   155 160 165
   Ile Asp Asn Tyr Thr Ser Leu Ile Tyr Ser Leu Ile Lys Glu Ser
   170 175 180
   Gln Asn Gln Gln Glu Lys Asn Glu Gln Glu Leu Leu Glu Leu Asp
   185 190 195
   Lys Trp Ala Ser Leu Trp Asn Trp Phe Asn Ile Thr Glu Trp Leu
   200 205 210
   Trp Tyr Ile Lys Ile Phe Ile Met Ile Val Gly Gly Leu Val Gly
   215 220 225
   Leu Arg Ile Val Phe Thr Val Leu Ser Ile Val Asn Arg Val Arg
   230 235 240
   Gln Gly Tyr Ser Pro Leu Ser Phe Gln Thr Arg Leu Pro Ala Pro
   245 250 255
   Arg Gly Pro Asp Arg Pro Glu Gly Ile Glu Glu Glu Gly Gly
   260 265 269
(2) INFORMATION FOR SEQ ID NO:47:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:47:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Thr Lys
                    20                  25                  30
   Ala Lys Arg Arg Val Val Gln Arg Glu Lys Arg Ala Val Gly Val
                    35                  40                  45
   Ile Gly Ala Met Phe Leu Gly Phe Leu Gly Ala Ala Gly Ser Thr
                    50                  55                  60
   Met Gly Ala Ala Ser Met Ala Leu Thr Val Gln Ala Arg Gln Leu
                    65                  70                  75
   Leu Ser Gly Ile Val Gln Gln Gln Asn Asn Leu Leu Arg Ala Ile
                    80                  85                  90
   Glu Ala Gln Gln His Leu Leu Gln Leu Thr Val Trp Gly Ile Lys
                    95                 100                 105
   Gln Leu Gln Ala Arg Val Leu Ala Val Glu Arg Tyr Leu Lys Asp
                   110                 115                 120
   Gln Gln Leu Leu Gly Ile Trp Gly Cys Ser Gly Lys Leu Ile Cys
                   125                 130                 135
   Thr Thr Ala Val Pro Trp Asn Thr Ser Trp Ser Asn Lys Ser Leu
                   140                 145                 150
   Glu Glu Ile Trp Asp Asn Met Thr Trp Met Glu Trp Glu Arg Glu
   155 160 165
   Ile Asn Asn Tyr Thr Gly Leu Ile Tyr Thr Leu Ile Glu Gln Ser
   170 175 180
   Gln Asn Gln Gln Glu Lys Asn Glu Gln Glu Leu Leu Ala Leu Asp
   185 190 195
   Thr Trp Ala Ser Leu Trp Asn Trp Phe Ser Ile Ser Asn Trp Leu
   200 205 210
   Trp Tyr Ile Arg Ile Phe Ile Met Ile Val Gly Gly Leu Val Gly
   215 220 225
   Leu Arg Ile Val Phe Ala Val Leu Ser Ile Val Asn Arg Val Arg
   230 235 240
   Gln Gly Tyr Ser Pro Leu Ser Phe Gln Thr Arg Leu Pro Thr Pro
   245 250 255
   Arg Gly Pro Asp Arg Pro Glu Gly Ile Glu Glu Glu Gly Gly
   260 265 269
(2) INFORMATION FOR SEQ ID NO:48:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:48:
   Gly Gly Gly Asp Met Arg Asp Asn Trp Arg Ser Glu Leu Tyr Lys
    1               5                  10                  15
   Tyr Lys Val Val Lys Ile Glu Pro Ile Gly Val Ala Pro Thr Lys
                   20                  25                  30
   Ala Lys Arg Arg Val Val Gln Arg Glu Lys Arg Ala Val Gly Thr
                   35                  40                  45
   Ile Gly Val Met Phe Leu Gly Phe Leu Gly Ala Ala Gly Ser Thr
                   50                  55                  60
   Met Gly Ala Ala Ser Ile Thr Leu Thr Val Gln Ala Arg Gln Leu
                   65                  70                  75
   Leu Ser Gly Ile Val Gln Gln Gln Asn Asn Leu Leu Arg Ala Ile
                   80                  85                  90
   Lys Ala Gln Gln His Leu Leu Gln Leu Thr Val Trp Gly Ile Lys
                   95                 100                 105
   Gln Leu Gln Ala Arg Val Leu Ala Ile Glu Arg Phe Leu Arg Asp
                  110                 115                 120
   Gln Gln Leu Leu Gly Ile Trp Gly Cys Ser Gly Lys Leu Ile Cys
                  125                 130                 135
   Thr Thr Thr Val Pro Trp Asn Thr Ser Trp Ser Asn Lys Ser Leu
                  140                 145                 150
   Lys Gln Ile Trp Asp Asn Leu Thr Trp Met Glu Trp Glu Arg Glu
                  155                 160                 165
   Ile Asp Asn Tyr Thr Gly Ile Ile Phe Asn Leu Ile Glu Glu Ala
                  170                 175                 180
   Gln Asn Gln Gln Glu Lys Asn Glu Gln Asp Leu Leu Glu Leu Asp
                  185                 190                 195
   Lys Trp Ala Gly Leu Trp Asn Trp Phe Ser Ile Thr Asn Trp Leu
                  200                 205                 210
   Trp Tyr Ile Lys Ile Phe Ile Met Ile Val Gly Gly Leu Ile Gly
                  215                 220                 225
   Leu Arg Ile Val Phe Ala Val Leu Ser Ile Val Asn Arg Val Arg
                  230                 235                 240
   Gln Gly Tyr Ser Pro Leu Ser Phe Gln Thr His Leu Pro Thr Pro
                  245                 250                 255
   Arg Gly Pro Asp Arg Pro Glu Gly Thr Gly Glu Glu Gly Gly
                  260                 265                 269
(2) INFORMATION FOR SEQ ID NO:49:
   (i) SEQUENCE CHARACTERISTICS:
      (A) LENGTH: 233 amino acids
      (B) TYPE: Amino Acid
      (D) TOPOLOGY: Linear
   (xi) SEQUENCE DESCRIPTION: SEQ ID NO:49:
   Gly Gly Gly Asp Met Arg Asp Asn Trp Arg Ser Glu Leu Tyr Lys
    1               5                  10                  15
   Tyr Lys Val Val Arg Ile Glu Pro Leu Gly Ile Ala Pro Thr Lys
                   20                  25                  30
   Ala Lys Arg Arg Val Val Gln Arg Glu Lys Arg Ala Val Gly Ile
                   35                  40                  45
   Gly Ala Met Phe Leu Gly Phe Leu Gly Ala Ala Gly Ser Thr Met
                   50                  55                  60
   Gly Ala Ala Ser Ile Thr Leu Thr Val Gln Ala Arg Leu Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                 100                 105
   Leu Gln Ala Arg Val Leu Ala Val Glu Arg Tyr Leu Arg Asp Gln
                       110                 115                 120
   Gln Leu Leu Gly Ile Trp Gly Cys Ser Gly Lys Leu Ile Cys Thr
                       125                 130                 135
   Thr Ala Val Pro Trp Asn Ala Ser Trp Ser Asn Lys Ser Leu Asp
                       140                 145                 150
   Gln Ile Trp Asp Asn Met Thr Trp Met Gln Trp Glu Arg Glu Ile
                       155                 160                 165
   Glu Asn Tyr Thr Ser Leu Ile Tyr Asn Leu Ile Glu Glu Ser Gln
                       170                 175                 180
   Asn Gln Gln Glu Lys Asn Glu Gln Asp Leu Leu Glu Leu Asp Lys
                       185                 190                 195
   Trp Ala Ser Leu Trp Ser Trp Phe Ser Ile Thr Asn Trp Leu Trp
                       200                 205                 210
   Tyr Ile Arg Ile Phe Ile Met Ile Val Gly Gly Leu Ile Gly Leu
                       215                 220                 225
   Arg Ile Val Phe Ala Val Leu Ser
                       230         233
(2) INFORMATION FOR SEQ ID NO:50:
   (i) SEQUENCE CHARACTERISTICS:
      (A) LENGTH: 233 amino acids
      (B) TYPE: Amino Acid
      (D) TOPOLOGY: Linear
   (xi) SEQUENCE DESCRIPTION: SEQ ID NO:50:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Gln Arg Glu Lys Arg Ala Val Gly Ile
                   35                  40                  45
   Gly Ala Val Phe Leu Gly Phe Leu Gly Ala Ala Gly Ser Thr Met
                   50                  55                  60
   Gly Ala Ala Ser Ile Thr Leu Thr Val Gln Ala Arg Leu Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                  100                 105
   Leu Gln Ala Arg Val Leu Ala Val Glu Arg Tyr Leu Arg Asp Gln
                   110                 115                 120
   Gln Leu Leu Gly Ile Trp Gly Cys Ser Gly Lys Leu Ile Cys Thr
                   125                 130                 135
   Thr Ala Val Pro Trp Asn Ser Ser Trp Ser Asn Lys Ser Leu Asp
                   140                 145                 150
   Gln Ile Trp Asn Asn Met Thr Trp Met Gln Trp Glu Arg Glu Ile
                   155                 160                 165
   Glu Asn Tyr Thr Ser Leu Ile Tyr Asn Leu Ile Glu Glu Ser Gln
                   170                 175                 180
   Asn Gln Gln Glu Lys Asn Glu Gln Glu Leu Leu Glu Leu Asp Lys
                   185                 190                 195
   Trp Ala Ser Leu Trp Asn Trp Phe Ser Ile Thr Asn Trp Leu Trp
                   200                 205                 210
   Tyr Ile Lys Ile Phe Ile Ile Ile Val Gly Gly Leu Ile Gly Leu
                   215                 220                 225
   Arg Ile Val Phe Ala Val Leu Ser
                   230         233
(2) INFORMATION FOR SEQ ID NO:51:
   (i) SEQUENCE CHARACTERISTICS:
      (A) LENGTH: 233 amino acids
      (B) TYPE: Amino Acid
      (D) TOPOLOGY: Linear
   (xi) SEQUENCE DESCRIPTION: SEQ ID NO:51:
   Ala Gly Gly Asp Met Arg Asp Asn Trp Arg Ser Glu Leu Tyr Lys
    1               5                  10                  15
   Tyr Lys Val Ile Gln Ile Glu Pro Leu Gly Val Ala Pro Thr Lys
                   20                  25                  30
   Ala Lys Arg Arg Val Val Gln Arg Glu Lys Arg Ala Val Gly Ile
                   35                  40                  45
   Gly Ala Val Leu Phe Gly Phe Leu Gly Ala Ala Gly Ser Thr Met
                   50                  55                  60
   Gly Ala Ala Ser Leu Thr Leu Thr Val Gln Ala Arg Gln Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                 100                 105
   Leu Gln Ala Arg Val Leu Ala Leu Glu Arg Tyr Leu Lys Asp Gln
                  110                 115                 120
   Gln Leu Leu Gly Ile Trp Gly Cys Ser Gly Lys Leu Ile Cys Thr
                  125                 130                 135
   Thr Ala Val Pro Trp Asn Ala Ser Trp Ser Asn Lys Ser Leu Asn
                  140                 145                 150
   Asp Ile Trp Asp Asn Met Thr Trp Met Gln Trp Asp Arg Glu Ile
                  155                 160                 165
   Asn Lys Tyr Thr Asp Ser Ile Tyr Gln Leu Ile Glu Glu Ser Gln
                  170                 175                 180
   Asn Gln Gln Glu Lys Asn Glu Gln Asp Leu Leu Lys Leu Asp Glu
                  185                 190                 195
   Trp Ala Ser Leu Trp Asn Trp Phe Ser Ile Ser Lys Trp Leu Trp
                  200                 205                 210
   Tyr Ile Lys Ile Phe Ile Met Ile Val Gly Gly Leu Val Gly Leu
                  215                 220                 225
   Arg Ile Val Phe Ala Val Leu Ser
                  230         233
(2) INFORMATION FOR SEQ ID NO:52:
   (i) SEQUENCE CHARACTERISTICS:
      (A) LENGTH: 105 amino acids
      (B) TYPE: Amino Acid
      (D) TOPOLOGY: Linear
   (xi) SEQUENCE DESCRIPTION: SEQ ID NO:52:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Thr Arg
                    20                  25                  30
   Ala Lys Arg Arg Val Val Gln Arg Glu Arg Arg Ala Val Gly Ala
                    35                  40                  45
   Leu Gly Ala Met Phe Leu Gly Phe Leu Gly Ala Ala Gly Ser Thr
                    50                  55                  60
   Met Gly Ala Ala Ser Leu Thr Leu Thr Val Gln Ala Arg Gln Leu
                    65                  70                  75
   Leu Ser Gly Ile Val Gln Gln Gln Asn Asn Leu Leu Lys Ala Ile
                    80                  85                  90
   Glu Ala Gln Gln His Leu Leu Gln Leu Thr Val Trp Gly Ile Lys
                    95                 100                 105
(2) INFORMATION FOR SEQ ID NO:53:
   (i) SEQUENCE CHARACTERISTICS:
      (A) LENGTH: 105 amino acids
      (B) TYPE: Amino Acid
      (D) TOPOLOGY: Linear
   (xi) SEQUENCE DESCRIPTION: SEQ ID NO:53:
   Gly Gly Gly Asp Met Lys Asp Asn Trp Arg Ser Lys Leu Tyr Lys
   1 5 10 15
   Tyr Lys Val Val Lys Ile Glu Pro Leu Gly Val Ala Pro Thr Arg
   20 25 30
   Ala Lys Arg Arg Val Val Gln Arg Glu Lys Arg Ala Val Gly Thr
   35 40 45
   Ile Gly Ala Met Phe Leu Gly Phe Leu Gly Ala Ala Gly Ser Thr
   50 55 60
   Met Gly Ala Ala Ser Ile Thr Leu Met Val Gln Ala Arg Gln Leu
   65 70 75
   Leu Ser Gly Ile Val Gln Gln Gln Arg Asn Leu Leu Arg Ala Ile
   80 85 90
   Glu Ala Gln Gln His Leu Leu Gln Leu Thr Val Trp Gly Ile Lys
   95 100 105
(2) INFORMATION FOR SEQ ID NO:54:
   (i) SEQUENCE CHARACTERISTICS:
      (A) LENGTH: 105 amino acids
      (B) TYPE: Amino Acid
      (D) TOPOLOGY: Linear
   (xi) SEQUENCE DESCRIPTION: SEQ ID NO:54:
   Gly Gly Gly Asp Met Arg Asp Asn Trp Arg Ser Glu Leu Tyr Lys
   1 5 10 15
   Tyr Lys Val Val Lys Ile Glu Pro Leu Gly Val Ala Pro Thr Arg
   20 25 30
   Ala Lys Arg Arg Val Val Gln Arg Glu Arg Arg Ala Val Gly Ala
   35 40 45
   Leu Gly Ala Met Phe Leu Gly Phe Leu Gly Ala Ala Gly Ser Thr
   50 55 60
   Met Gly Ala Ala Ser Leu Thr Leu Thr Val Gln Ala Arg Gln Leu
   65 70 75
   Leu Ser Gly Ile Val Gln Gln Gln Asn Asn Leu Leu Lys Ala Ile
   80 85 90
   Glu Ala Gln Gln His Leu Leu Gln Leu Thr Val Trp Gly Ile Lys
   95 100 105
(2) INFORMATION FOR SEQ ID NO:55:
   (i) SEQUENCE CHARACTERISTICS:
      (A) LENGTH: 105 amino acids
      (B) TYPE: Amino Acid
      (D) TOPOLOGY: Linear
   (xi) SEQUENCE DESCRIPTION: SEQ ID NO:55:
   Gly Gly Gly Asp Met Lys Asp Asn Trp Arg Ser Lys Leu Tyr Lys
    1               5                  10                  15
   Tyr Lys Val Val Lys Ile Glu Pro Leu Gly Val Ala Pro Thr Arg
                   20                  25                  30
   Ala Lys Arg Arg Val Val Gln Arg Glu Lys Arg Ala Val Gly Thr
                   35                  40                  45
   Ile Gly Ala Met Phe Leu Gly Phe Leu Gly Ala Ala Gly Ser Thr
                   50                  55                  60
   Met Gly Ala Ala Ser Ile Thr Leu Met Val Gln Ala Arg Gln Leu
                   65                  70                  75
   Leu Ser Gly Ile Val Gln Gln Gln Arg Asn Leu Leu Arg Ala Ile
                   80                  85                  90
   Glu Ala Gln Gln His Leu Leu Gln Leu Thr Val Trp Gly Ile Lys
                   95                 100                 105
(2) INFORMATION FOR SEQ ID NO:56:
   (i) SEQUENCE CHARACTERISTICS:
      (A) LENGTH: 93 amino acids
      (B) TYPE: Amino Acid
      (D) TOPOLOGY: Linear
   (xi) SEQUENCE DESCRIPTION: SEQ ID NO:56:
   Gly Gly Gly Asn Met Arg Asp Asn Trp Arg Ser Glu Leu Tyr Lys
    1               5                  10                  15
   Tyr Lys Val Val Lys Ile Glu Pro Leu Gly Val Ala Pro Thr Arg
                   20                  25                  30
   Ala Lys Arg Arg Val Val Gln Arg Glu Lys Arg Ala Val Gly Ile
                   35                  40                  45
   Gly Ala Val Phe Leu Gly Phe Leu Gly Ala Ala Gly Ser Thr Met
                   50                  55                  60
   Gly Ala Arg Ser Met Thr Leu Thr Val Gln Ala Arg Leu Leu Leu
                   65                  70                  75
   Ser Gly Ile Val Gln Gln Gln Asn Asn Leu Leu Arg Ala Ile Glu
                   80                  85                  90
   Ala Gln Gln
           93
(2) INFORMATION FOR SEQ ID NO:57:
   (i) SEQUENCE CHARACTERISTICS:
      (A) LENGTH: 94 amino acids
      (B) TYPE: Amino Acid
      (D) TOPOLOGY: Linear
   (xi) SEQUENCE DESCRIPTION: SEQ ID NO:57:
(2) INFORMATION FOR SEQ ID NO:58:
   (i) SEQUENCE CHARACTERISTICS:
      (A) LENGTH: 79 amino acids
      (B) TYPE: Amino Acid
      (D) TOPOLOGY: Linear
   (xi) SEQUENCE DESCRIPTION: SEQ ID NO:58:
   Gly Gly Gly Asp Met Arg Asp Asn Trp Arg Ser Glu Leu Tyr Lys
    1               5                  10                  15
   Tyr Lys Val Val Arg Ile Glu Pro Leu Gly Ile Ala Pro Thr Arg
                   20                  25                  30
   Ala Lys Arg Arg Glu Val Gln Arg Glu Lys Arg Ala Val Gly Thr
                   35                  40                  45
   Leu Gly Ala Met Phe Leu Gly Phe Leu Gly Ala Ala Gly Ser Thr
                   50                  55                  60
   Met Gly Ala Ala Ser Val Ala Leu Thr Val Pro Leu Arg Arg Ile
                   65                  70                  75
   Arg Ser Cys Xaa
               79
(2) INFORMATION FOR SEQ ID NO:59:
   (i) SEQUENCE CHARACTERISTICS:
      (A) LENGTH: 79 amino acids
      (B) TYPE: Amino Acid
      (D) TOPOLOGY: Linear
   (xi) SEQUENCE DESCRIPTION: SEQ ID NO:59:
   Gly Gly Gly Asp Met Arg Asp Asn Trp Arg Ser Glu Leu Tyr Lys
    1               5                  10                  15
   Tyr Lys Val Val Arg Ile Glu Pro Leu Gly Val Ala Pro Thr Lys
                   20                  25                  30
   Ala Lys Arg Arg Val Val Gln Gly Glu Lys Arg Ala Val Gly Thr
                   35                  40                  45
   Ile Gly Ala Met Phe Leu Gly Phe Leu Gly Ala Ala Gly Ser Thr
                   50                  55                  60
   Met Gly Ala Arg Ser Ile Thr Leu Thr Val Pro Leu Arg Arg Ile
                   65                  70                  75
   Arg Ser Cys Xaa
               79
(2) INFORMATION FOR SEQ ID NO:60:
   (i) SEQUENCE CHARACTERISTICS:
      (A) LENGTH: 79 amino acids
      (B) TYPE: Amino Acid
      (D) TOPOLOGY: Linear
   (xi) SEQUENCE DESCRIPTION: SEQ ID NO:60:
   Gly Gly Gly Asp Met Arg Asp Asn Trp Arg Ser Glu Leu Tyr Lys
    1               5                  10                  15
   Tyr Lys Val Val Arg Ile Glu Pro Leu Gly Val Ala Pro Thr Lys
                   20                  25                  30
   Ala Lys Arg Arg Val Val Gln Arg Glu Lys Arg Ala Val Gly Thr
                   35                  40                  45
   Ile Gly Ala Met Phe Leu Gly Phe Leu Gly Ala Ala Gly Ser Thr
                   50                  55                  60
   Met Gly Ala Ala Ser Ile Thr Leu Thr Val Pro Val Arg Arg Ile
                   65                  70                  75
   Arg Ser Cys Xaa
               79
(2) INFORMATION FOR SEQ ID NO:61:
   (i) SEQUENCE CHARACTERISTICS:
      (A) LENGTH: 55 amino acids
      (B) TYPE: Amino Acid
      (D) TOPOLOGY: Linear
   (xi) SEQUENCE DESCRIPTION: SEQ ID NO:61:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Arg
                   20                  25                  30
   Ala Lys Arg Arg Val Val Gln Arg Glu Lys Arg Ala Val Gly Thr
                   35                  40                  45
   Ile Gly Ala Met Phe Leu Gly Phe Leu Gly
                   50                  55
(2) INFORMATION FOR SEQ ID NO:62:
   (i) SEQUENCE CHARACTERISTICS:
      (A) LENGTH: 55 amino acids
      (B) TYPE: Amino Acid
      (D) TOPOLOGY: Linear
   (xi) SEQUENCE DESCRIPTION: SEQ ID NO:62:
   Gly Gly Gly Asn Met Arg Asp Asn Trp Arg Ser Glu Leu Tyr Lys
    1               5                  10                  15
   Tyr Lys Val Val Lys Ile Glu Pro Leu Gly Val Ala Pro Thr Arg
                   20                  25                  30
   Ala Lys Arg Arg Val Val Gln Arg Glu Lys Arg Ala Ile Gly Thr
                   35                  40                  45
   Ile Gly Ala Leu Phe Leu Gly Phe Leu Gly
                   50                  55
(2) INFORMATION FOR SEQ ID NO:63:
   (i) SEQUENCE CHARACTERISTICS:
      (A) LENGTH: 54 amino acids
      (B) TYPE: Amino Acid
      (D) TOPOLOGY: Linear
   (xi) SEQUENCE DESCRIPTION: SEQ ID NO:63:
   Gly Gly Gly Asn Met Lys Asp Asn Trp Arg Ser Glu Leu Tyr Lys
    1               5                  10                  15
   Tyr Lys Val Val Lys Ile Glu Pro Leu Gly Val Ala Pro Thr Arg
                   20                  25                  30
   Ala Lys Arg Arg Val Val Gln Arg Glu Lys Arg Ala Val Thr Met
                   35                  40                  45
   Gly Ala Leu Phe Leu Gly Phe Leu Gly
                   50              54
(2) INFORMATION FOR SEQ ID NO:64:
   (i) SEQUENCE CHARACTERISTICS:
      (A) LENGTH: 54 amino acids
      (B) TYPE: Amino Acid
      (D) TOPOLOGY: Linear
   (xi) SEQUENCE DESCRIPTION: SEQ ID NO:64:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Thr Arg
                    20                  25                  30
   Ala Lys Arg Arg Val Val Gln Arg Glu Lys Arg Ala Val Gly Leu
                    35                  40                  45
   Gly Ala Met Phe Leu Gly Phe Leu Gly
                    50              54
(2) INFORMATION FOR SEQ ID NO:65:
   (i) SEQUENCE CHARACTERISTICS:
      (A) LENGTH: 41 amino acids
      (B) TYPE: Amino Acid
      (D) TOPOLOGY: Linear
   (xi) SEQUENCE DESCRIPTION: SEQ ID NO:65:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Thr Thr
                    20                  25                  30
   Ala Lys Arg Arg Val Met Gln Arg Glu Lys Arg
                    35                  40  41
(2) INFORMATION FOR SEQ ID NO:66:
   (i) SEQUENCE CHARACTERISTICS:
      (A) LENGTH: 34 amino acids
      (B) TYPE: Amino Acid
      (D) TOPOLOGY: Linear
   (xi) SEQUENCE DESCRIPTION: SEQ ID NO:66:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Thr Thr
                    20                  25                  30
   Ala Lys Arg Arg
                34
(2) INFORMATION FOR SEQ ID NO:67:
   (i) SEQUENCE CHARACTERISTICS:
      (A) LENGTH: 270 amino acids
      (B) TYPE: Amino Acid
      (D) TOPOLOGY: Linear
   (xi) SEQUENCE DESCRIPTION: SEQ ID NO:67:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Arg
                   20                  25                  30
   Ala Lys Arg Arg Val Val Glu Arg Glu Lys Arg Ala Val Gly Xaa
                   35                  40                  45
   Leu Gly Ala Val Phe Leu Gly Phe Leu Gly Ala Ala Gly Ser Thr
                   50                  55                  60
   Met Gly Ala Ala Ser Ile Thr Leu Thr Val Gln Ala Arg Gln Leu
                   65                  70                  75
   Leu Ser Gly Ile Val Gln Gln Gln Ser Asn Leu Leu Arg Ala Ile
                   80                  85                  90
   Glu Ala Gln Gln His Leu Leu Lys Leu Thr Val Trp Gly Ile Lys
                   95                  100                 105
   Gln Leu Gln Ala Arg Val Leu Ala Val Glu Arg Tyr Leu Lys Asp
                   110                 115                 120
   Gln Gln Leu Leu Gly Ile Trp Gly Cys Ser Gly Lys Leu Ile Cys
                   125                 130                 135
   Thr Thr Asn Val Pro Trp Asn Ser Ser Trp Ser Asn Lys Ser Xaa
                   140                 145                 150
   Gln Ser Xaa Ile Trp Asp Asn Met Thr Trp Leu Gln Trp Asp Lys
                   155                 160                 165
   Glu Ile Ser Asn Tyr Thr Xaa Ile Ile Tyr Asn Leu Ile Glu Glu
                   170                 175                 180
   Ser Gln Asn Gln Gln Glu Lys Asn Glu Gln Asp Leu Leu Ala Leu
                   185                 190                 195
   Asp Lys Trp Ala Asn Leu Trp Asn Trp Phe Asp Ile Ser Asn Trp
                   200                 205                 210
   Leu Trp Tyr Ile Xaa Ile Phe Ile Met Ile Val Gly Gly Leu Ile
                   215                 220                 225
   Gly Leu Arg Ile Val Phe Ala Val Leu Ser Ile Ile Asn Arg Val
                   230                 235                 240
   Arg Gln Gly Tyr Ser Pro Leu Ser Phe Gln Thr Leu Thr Pro Asn
                   245                 250                 255
   Pro Arg Xaa Pro Asp Arg Pro Gly Arg Ile Glu Glu Glu Gly Gly
   260 265 270
(2) INFORMATION FOR SEQ ID NO:68:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:68:
   Thr Gly Gly Asn Met Arg Asp Asn Trp Arg Ser Glu Leu Tyr Lys
   1 5 10 15
   Tyr Lys Val Val Lys Ile Glu Pro Leu Gly Val Ala Pro Thr Pro
   20 25 30
   Ala Lys Arg Arg Val Val Gln Arg Glu Lys Arg Ala Val Gly Ile
   35 40 45
   Gly Ala Val Phe Ile Gly Phe Leu Gly Ala Ala Gly Ser Thr Met
   50 55 60
   Gly Ala Ala Ser Ile Thr Leu Thr Val Gln Ala Arg Gln Leu Leu
   65 70 75
   Ser Gly Ile Val Gln Gln Gln Ser Asn Leu Leu Arg Ala Ile Glu
   80 85 90
   Ala Gln Gln His Leu Leu Lys Leu Thr Val Trp Gly Ile Lys Gln
   95 100 105
   Leu Gln Ala Arg Val Leu Ala Val Glu Arg Tyr Leu Lys Asp Gln
   110 115 120
   Gln Leu Leu Gly Ile Trp Gly Cys Ser Gly Lys Leu Ile Cys Thr
   125 130 135
   Thr Asn Val Pro Trp Asn Ser Ser Trp Ser Asn Arg Thr Gln Ser
   140 145 150
   Glu Ile Trp Asn Asn Met Thr Trp Leu Gln Trp Asp Lys Glu Ile
   155 160 165
   Ser Asn Tyr Thr Asp Ile Ile Tyr Asn Leu Ile Glu Glu Ser Gln
   170 175 180
   Ile Gln Gln Glu Lys Asn Glu Gln Glu Leu Leu Ala Leu Asp Lys
   185 190 195
   Trp Ala Asn Leu Trp Asn Trp Phe Asp Ile Ser Lys Trp Leu Trp
   200 205 210
   Tyr Ile Arg Ile Phe Ile Met Ile Val Gly Gly Leu Ile Gly Leu
   215 220 225
   Arg Ile Val Phe Ala Val Leu Ser Ile Ile Asn Arg Val Arg Gln
   230 235 240
   Gly Tyr Ser Pro Leu Ser Phe Gln Ile His Thr Pro Asn Pro Arg
   245 250 255
   Gly Pro Asp Arg Pro Glu Arg Ile Glu Glu Glu Gly Gly
   260 265 268
(2) INFORMATION FOR SEQ ID NO:69:
   (i) SEQUENCE CHARACTERISTICS:
      (A) LENGTH: 267 amino acids
      (B) TYPE: Amino Acid
      (D) TOPOLOGY: Linear
   (xi) SEQUENCE DESCRIPTION: SEQ ID NO:69:
   Gly Gly Gly Asp Met Arg Asp Asn Trp Lys Ser Glu Leu Tyr Lys
     1               5                  10                  15
   Tyr Lys Val Val Lys Ile Glu Pro Leu Gly Val Ala Pro Thr Arg
                    20                  25                  30
   Ala Lys Arg Arg Val Val Glu Arg Glu Lys Arg Ala Val Gly Leu
                    35                  40                  45
   Gly Ala Ile Phe Leu Gly Phe Leu Gly Ala Ala Gly Ser Thr Met
                    50                  55                  60
   Gly Ala Ala Ser Ile Thr Leu Thr Val Gln Ala Arg Gln Leu Leu
                    65                  70                  75
   Ser Gly Ile Val Gln Gln Gln Ser Asn Leu Leu Arg Ala Ile Glu
                    80                  85                  90
   Ala Gln Gln His Leu Leu Lys Leu Thr Val Trp Gly Ile Lys Gln
                    95                 100                 105
   Leu Gln Ala Arg Val Leu Ala Val Glu Arg Tyr Leu Gln Asp Gln
                   110                 115                 120
   Gln Leu Leu Gly Ile Trp Gly Cys Ser Gly Lys Leu Ile Cys Thr
                   125                 130                 135
   Thr Thr Val Pro Trp Asn Ser Ser Trp Ser Asn Lys Ser Gln Glu
                   140                 145                 150
   Asp Ile Trp Asn Asn Met Thr Trp Leu Gln Trp Glu Lys Glu Ile
                   155                 160                 165
   Ser Ser Tyr Thr Gly Ile Ile Tyr Gln Leu Ile Glu Glu Ser Gln
                   170                 175                 180
   Asn Gln Gln Glu Lys Asn Glu Leu Asp Leu Leu Ala Leu Asp Lys
                   185                 190                 195
   Trp Ala Asn Leu Asn Trp Phe Asn Ile Ser Asn Trp Leu Trp Tyr
                   200                 205                 210
   Ile Arg Leu Phe Val Ile Ile Val Gly Gly Leu Ile Gly Leu Arg
                   215                 220                 225
   Ile Val Phe Thr Val Leu Ser Ile Ile Asn Arg Val Arg Gln Gly
                   230                 235                 240
   Tyr Ser Pro Leu Ser Phe Gln Thr Leu Ala Pro Ile Pro Glu Gly
                   245                 250                 255
   Leu Gly Arg Pro Gly Arg Ile Glu Glu Glu Gly Gly
                   260                 265     267
(2) INFORMATION FOR SEQ ID NO:70:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:70:
   Gly Gly Gly Asp Met Arg Asp Asn Trp Arg Ser Glu Leu Tyr Lys
    1               5                  10                  15
   Tyr Lys Val Val Lys Ile Glu Pro Leu Gly Val Ala Pro Thr Lys
                   20                  25                  30
   Ala Lys Arg Arg Val Val Ala Arg Glu Lys Arg Ala Ile Gly Met
                   35                  40                  45
   Gly Ala Phe Phe Leu Gly Phe Leu Gly Ala Ala Gly Ser Thr Met
                   50                  55                  60
   Gly Ala Ala Ser Ile Thr Leu Thr Val Gln Ala Arg Arg Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Lys Leu Thr Val Trp Gly Ile Lys Gln
                   95                  100                 105
   Leu Gln Ala Arg Ile Leu Ala Val Glu Arg Tyr Leu Lys Asp Gln
                   110                 115                 120
   Gln Leu Leu Gly Ile Trp Gly Cys Ser Gly Lys Ile Ile Cys Pro
                   125                 130                 135
   Thr Asn Val Pro Trp Asn Ser Ser Trp Ser Asn Lys Ser Gln Ser
                   140                 145                 150
   Asp Ile Trp Asp Lys Met Thr Trp Leu Glu Trp Asp Lys Glu Val
                   155                 160                 165
   Ser Asn Tyr Thr Gln Val Ile Tyr Asn Leu Ile Glu Glu Ser Gln
                   170                 175                 180
   Thr Gln Gln Glu Ile Asn Glu Arg Asp Leu Leu Ala Leu Asp Lys
                   185                 190                 195
   Trp Ala Asn Leu Trp Asn Trp Phe Asp Ile Ser Asn Trp Leu Trp
                   200                 205                 210
   Tyr Ile Lys Ile Phe Ile Met Ile Val Gly Gly Leu Ile Gly Leu
                   215                 220                 225
   Arg Ile Val Phe Ala Val Leu Ser Ile Ile Asn Arg Val Arg Gln
                   230                 235                 240
   Gly Tyr Ser Pro Leu Ser Phe Gln Thr Leu Thr His His Gln Arg
                   245                 250                 255
   Glu Pro Asp Arg Pro Glu Arg Ile Glu Glu Gly Gly Gly
                   260                 265         268
(2) INFORMATION FOR SEQ ID NO:71:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:71:
   Gly Gly Gly Asn Met Arg Asp Asn Trp Arg Ser Glu Leu Tyr Lys
    1               5                  10                  15
   Tyr Lys Val Val Lys Ile Glu Pro Leu Gly Val Ala Pro Ser Arg
                   20                  25                  30
   Ala Lys Arg Arg Val Val Glu Arg Glu Lys Arg Ala Val Gly Ile
                   35                  40                  45
   Gly Ala Val Phe Leu Gly Phe Leu Gly Ala Ala Gly Ser Thr Met
                   50                  55                  60
   Gly Ala Ala Ser Ile Thr Leu Thr Ala Gln Ala Arg Gln Leu Leu
                   65                  70                  75
   Ser Gly Ile Val Gln Gln Gln Ser Asn Leu Leu Arg Ala Ile Glu
                   80                  85                  90
   Ala Gln Gln His Met Leu Lys Leu Thr Val Trp Gly Ile Lys Gln
                   95                 100                 105
   Leu Gln Ala Arg Val Leu Ala Val Glu Arg Tyr Leu Lys Asp Gln
                  110                 115                 120
   Gln Leu Leu Gly Ile Trp Gly Cys Ser Gly Lys Leu Ile Cys Thr
                  125                 130                 135
   Thr Asn Val Pro Trp Asn Ser Ser Trp Ser Asn Lys Ser Met Asn
                  140                 145                 150
   Glu Ile Trp Asp Asn Met Thr Trp Leu Gln Trp Asp Lys Glu Ile
                  155                 160                 165
   Ser Asn Tyr Thr Gln Ile Ile Tyr Asn Leu Ile Glu Glu Ser Gln
                   170                 175                 180
   Asn Gln Gln Glu Lys Asn Glu Gln Asp Leu Leu Ala Leu Asp Lys
                   185                 190                 195
   Trp Ala Ser Leu Trp Asn Trp Phe Asp Ile Ser Arg Trp Leu Trp
                   200                 205                 210
   Tyr Ile Lys Ile Phe Ile Met Ile Val Gly Gly Leu Ile Gly Leu
                   215                 220                 225
   Arg Ile Val Phe Ala Val Leu Ser Val Ile Asn Arg Val Arg Gln
                   230                 235                 240
   Gly Tyr Ser Pro Leu Ser Phe Gln Ile Arg Thr Pro Asn Pro Lys
                   245                 250                 255
   Glu Pro Asp Arg Leu Gly Arg Ile Asp Gly Glu Gly Gly
                   260                 265         268
(2) INFORMATION FOR SEQ ID NO:72:
   (i) SEQUENCE CHARACTERISTICS:
      (A) LENGTH: 268 amino acids
      (B) TYPE: Amino Acid
      (D) TOPOLOGY: Linear
   (xi) SEQUENCE DESCRIPTION: SEQ ID NO:72:
   Gly Gly Gly Asn Met Arg Asp Asn Trp Arg Ser Glu Leu Tyr Lys
   1 5 10 15
   Tyr Lys Val Val Lys Ile Glu Pro Leu Gly Val Ala Pro Thr Arg
   20 25 30
   Ala Lys Arg Arg Val Val Glu Arg Glu Lys Arg Ala Ile Gly Met
   35 40 45
   Gly Ala Val Phe Ile Gly Phe Leu Gly Ala Ala Gly Ser Thr Met
   50 55 60
   Gly Ala Ala Ser Ile Thr Leu Met Val Gln Ala Arg Gln Leu Leu
   65 70 75
   Ser Gly Ile Val Gln Gln Gln Ser Asn Leu Leu Arg Ala Ile Glu
   80 85 90
   Ala Gln Gln His Leu Leu Arg Leu Thr Val Trp Gly Ile Lys Gln
   95 100 105
   Leu Gln Ala Arg Val Leu Ala Leu Glu Arg Tyr Leu Arg Asp Gln
   110 115 120
   Gln Leu Leu Gly Ile Trp Gly Cys Ser Gly Lys Leu Ile Cys Thr
   125 130 135
   Thr Asn Val Pro Trp Asn Ser Ser Trp Ser Asn Lys Ser Tyr Ser
   140 145 150
   Glu Ile Trp Asp Asn Met Thr Trp Leu Gln Trp Asp Lys Glu Ile
   155 160 165
   Asn Asn Tyr Thr Glu Leu Ile Tyr Ser Leu Ile Glu Asp Ser Gln
   170 175 180
   Asn Gln Gln Glu Lys Asn Glu Gln Asp Leu Leu Ala Leu Asp Lys
   185 190 195
   Trp Ala Asn Leu Trp Asn Trp Phe Asp Ile Ser Asn Trp Leu Trp
   200 205 210
   Tyr Ile Lys Ile Phe Ile Met Ile Val Gly Gly Leu Ile Gly Leu
   215 220 225
   Arg Ile Ile Phe Ala Val Leu Ser Ile Ile Asn Arg Val Arg Gln
   230 235 240
   Gly Tyr Ser Pro Leu Ser Phe Gln Thr His Thr Pro Asn Pro Arg
   245 250 255
   Gly Leu Asp Arg Pro Gly Arg Ile Glu Glu Glu Gly Gly
   260 265 268
(2) INFORMATION FOR SEQ ID NO:73:
   (i) SEQUENCE CHARACTERISTICS:
      (A) LENGTH: 243 amino acids
      (B) TYPE: Amino Acid
      (D) TOPOLOGY: Linear
   (xi) SEQUENCE DESCRIPTION: SEQ ID NO:73:
   Gly Gly Gly Asp Met Arg Asp Asn Trp Arg Ser Glu Leu Tyr Lys
    1               5                  10                  15
   Tyr Lys Asp Val Lys Ile Glu Pro Leu Gly Val Ala Pro Thr Arg
                   20                  25                  30
   Ala Lys Arg Arg Val Val Glu Arg Glu Lys Arg Ala Val Gly Leu
                   35                  40                  45
   Gly Ala Val Phe Leu Gly Phe Leu Gly Ala Ala Gly Ser Thr Met
                   50                  55                  60
   Gly Ala Ala Ser Ile Thr Leu Thr Val Gln Ala Arg Gln Leu Leu
                   65                  70                  75
   Ser Gly Ile Val Gln Gln Gln Ser Asn Leu Leu Met Ala Ile Glu
                   80                  85                  90
   Ala Gln Gln His Leu Leu Lys Leu Thr Val Trp Gly Ile Lys Gln
                   95                 100                 105
   Leu Gln Ala Arg Val Leu Ala Leu Glu Arg Tyr Leu Lys Asp Gln
                  110                 115                 120
   Gln Leu Leu Gly Ile Trp Gly Cys Ser Gly Lys Leu Ile Cys Thr
                       125                 130                 135
   Thr Thr Val Pro Trp Asn Ser Ser Trp Ser Asn Lys Thr Tyr Ser
                       140                 145                 150
   Asp Ile Trp Asp Asn Met Thr Trp Leu Gln Trp Asp Lys Glu Ile
                       155                 160                 165
   Ser Asn Tyr Thr Lys Ile Ile Tyr Ala Leu Ile Glu Glu Ser Ala
                       170                 175                 180
   Asn Gln Gln Glu Lys Asn Glu Gln Asp Leu Leu Ala Leu Asp Lys
                       185                 190                 195
   Trp Thr Ser Leu Trp Ser Trp Phe Asp Ile Thr Lys Trp Leu Trp
                       200                 205                 210
   Tyr Ile Arg Ile Phe Ile Met Ile Val Gly Gly Leu Ile Gly Leu
                       215                 220                 225
   Arg Ile Val Phe Ala Val Leu Asn Ile Ile Asn Arg Val Arg Gln
                       230                 235                 240
   Gly Tyr Ser
           243
(2) INFORMATION FOR SEQ ID NO:74:
   (i) SEQUENCE CHARACTERISTICS:
      (A) LENGTH: 104 amino acids
      (B) TYPE: Amino Acid
      (D) TOPOLOGY: Linear
   (xi) SEQUENCE DESCRIPTION: SEQ ID NO:74:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Thr Arg
                    20                  25                  30
   Ala Lys Arg Arg Val Val Glu Arg Glu Lys Arg Ala Val Gly Leu
                    35                  40                  45
   Gly Ala Val Phe Ile Gly Phe Leu Gly Ala Ala Gly Ser Thr Met
                    50                  55                  60
   Gly Ala Ala Ser Ile Thr Leu Thr Val Gln Ala Arg Gln Leu Leu
                    65                  70                  75
   Ser Gly Ile Val Gln Gln Gln Ser Asn Leu Leu Arg Ala Ile Glu
                    80                  85                  90
   Ala Gln Gln His Leu Leu Lys Leu Thr Val Trp Gly Ile Lys
                    95                 100             104
(2) INFORMATION FOR SEQ ID NO:75:
   (i) SEQUENCE CHARACTERISTICS:
      (A) LENGTH: 54 amino acids
      (B) TYPE: Amino Acid
      (D) TOPOLOGY: Linear
   (xi) SEQUENCE DESCRIPTION: SEQ ID NO:75:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Ser Arg
                    20                  25                  30
   Ala Lys Arg Arg Val Val Trp Arg Glu Lys Arg Ala Val Val Glu
                    35                  40                  45
   Ile Gly Ala Val Phe Leu Gly Phe Leu
                    50              54
(2) INFORMATION FOR SEQ ID NO:76:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:76:
   Gly Gly Gly Asp Met Arg Asp Asn Trp Arg Ser Glu Leu Tyr Lys
   1 5 10 15
   Tyr Lys Val Val Glu Ile Lys Pro Leu Gly Val Ala Pro Thr Xaa
   20 25 30
   Ala Lys Arg Arg Val Val Glu Arg Glu Lys Arg Ala Val Gly Xaa
   35 40 45
   Ile Gly Ala Val Phe Leu Gly Phe Leu Gly Ala Ala Gly Ser Thr
   50 55 60
   Met Gly Ala Ala Ser Ile Thr Leu Thr Val Gln Ala Arg Gln Leu
   65 70 75
   Leu Ser Gly Ile Val Gln Gln Gln Ser Asn Leu Leu Arg Ala Ile
   80 85 90
   Glu Ala Gln Gln His Xaa Leu Gln Leu Thr Val Trp Gly Ile Lys
   95 100 105
   Gln Leu Gln Thr Arg Val Leu Ala Ile Glu Arg Tyr Leu Lys Asp
   110 115 120
   Gln Gln Leu Leu Gly Ile Trp Gly Cys Ser Gly Lys Leu Ile Cys
   125 130 135
   Thr Thr Xaa Val Pro Trp Asn Ser Ser Trp Ser Asn Arg Ser Gln
   140 145 150
   Thr Asp Ile Trp Asp Asn Met Thr Trp Met Gln Trp Asp Arg Glu
   155 160 165
   Ile Ser Asn Tyr Thr Asp Thr Ile Tyr Arg Leu Leu Glu Asp Ser
   170 175 180
   Gln Asn Gln Gln Glu Arg Asn Glu Lys Asp Leu Leu Ala Leu Asp
   185 190 195
   Ser Trp Lys Asn Leu Trp Asn Trp Phe Ser Ile Thr Asn Trp Leu
   200 205 210
   Trp Tyr Ile Lys Ile Phe Ile Met Ile Val Gly Gly Leu Ile Gly
   215 220 225
   Leu Arg Ile Ile Phe Ala Val Leu Ser Ile Val Asn Arg Val Arg
   230 235 240
   Gln Gly Tyr Ser Pro Leu Ser Phe Gln Thr Leu Thr Pro Asn Pro
   245 250 255
   Arg Gly Pro Asp Arg Leu Gly Arg Ile Glu Glu Glu Gly Gly
   260 265 269
(2) INFORMATION FOR SEQ ID NO:77:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:77:
   Gly Gly Gly Asp Met Arg Asp Asn Trp Arg Ser Glu Leu Tyr Lys
    1               5                  10                  15
   Tyr Lys Val Val Glu Ile Lys Pro Leu Gly Val Ala Pro Thr Lys
                   20                  25                  30
   Ala Lys Arg Arg Val Val Glu Arg Glu Lys Arg Ala Val Gly Ile
                   35                  40                  45
   Gly Ala Val Phe Leu Gly Phe Leu Gly Ala Ala Gly Ser Thr Met
                   50                  55                  60
   Gly Ala Ala Ser Val Thr Leu Thr Val Gln Ala Arg Gln Leu Leu
                   65                  70                  75
   Phe Gly Ile Val Gln Gln Gln Ser Asn Leu Leu Arg Ala Ile Glu
                   80                  85                  90
   Ala Gln His Gly Leu Leu Gln Leu Thr Val Trp Gly Ile Lys Gln
                   95                  100                 105
   Leu Gln Thr Arg Val Leu Ala Ile Glu Arg Tyr Leu Lys Asp Gln
                   110                 115                 120
   Gln Leu Leu Gly Ile Trp Gly Cys Ser Gly Lys Leu Ile Cys Thr
                   125                 130                 135
   Thr Ala Val Ala Trp Asn Ser Ser Trp Ser Asn Lys Ser Gln Ser
                   140                 145                 150
   Asp Ile Trp Asp Asn Met Thr Trp Met Glu Trp Asp Arg Glu Ile
                   155                 160                 165
   Ser Asn Tyr Thr Asp Ile Ile Tyr Lys Leu Leu Glu Asp Ser Gln
                   170                 175                 180
   Asn Gln Gln Glu Lys Asn Glu Lys Asp Leu Leu Ala Leu Asp Ser
                   185                 190                 195
   Trp Lys Asn
           198
(2) INFORMATION FOR SEQ ID NO:78:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:78:
   Gly Gly Gly Asp Met Arg Asp Asn Trp Arg Ser Glu Leu Tyr Lys
    1               5                  10                  15
   Tyr Lys Val Val Glu Ile Lys Pro Leu Gly Val Ala Pro Thr Glu
                   20                  25                  30
   Ala Lys Arg Arg Val Val Glu Arg Glu Lys Arg Ala Val Gly Ile
                   35                  40                  45
   Gly Ala Val Phe Leu Gly Phe Leu Gly Ala Ala Gly Ser Thr Met
                   50                  55                  60
   Gly Ala Ala Ser Ile Thr Leu Thr Val Gln Ala Arg Gln Leu Leu
                   65                  70                  75
   Ser Gly Ile Val Gln Gln Gln Ser Asn Leu Leu Arg Ala Ile Glu
                   80                  85                  90
   Ala Arg Gln Gly Met Leu Gln Leu Thr Val Trp Gly Ile Lys Gln
                   95                 100                 105
   Leu Gln Ala Arg Val Leu Ala Ile Glu Arg Tyr Leu Gln Asp Gln
                  110                 115                 120
   Gln Leu Leu Gly Leu Trp Gly Cys Ser Gly Lys Leu Ile Cys Thr
                  125                 130                 135
   Thr Thr Val Pro Trp Asn Ser Ser Trp Ser Asn Lys Ser Lys Thr
                  140                 145                 150
   Asp Ile Trp Asp Asn Met Thr Trp Met Gln Trp Asp Arg Glu Ile
                  155                 160                 165
   Ser Asn Tyr Thr Asp Thr Ile Tyr Lys Leu Leu Glu Asp Ser Gln
                  170                 175                 180
   Asn Gln Gln Glu Lys Asn Glu Lys Asp Leu Leu Ala Leu Asp Ser
                  185                 190                 195
   Trp Asn Asn
           198
(2) INFORMATION FOR SEQ ID NO:79:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:79:
   Gly Gly Gly Asp Met Arg Asn Asn Trp Arg Ser Glu Leu Tyr Lys
     1               5                  10                  15
   Tyr Lys Val Val Glu Ile Lys Pro Leu Gly Val Ala Pro Thr Thr
                    20                  25                  30
   Ala Lys Arg Arg Val Val Glu Arg Glu Lys Arg Ala Val Gly Ile
                    35                  40                  45
   Gly Ala Val Phe Leu Gly Phe Leu Gly Ala Ala Gly Ser Thr Met
                    50                  55                  60
   Gly Ala Ala Ser Met Thr Leu Thr Val Gln Ala Arg Gln Leu Leu
                    65                  70                  75
   Ser Gly Ile Val Gln Gln Gln Ser Asn Leu Leu Arg Ala Ile Glu
                    80                  85                  90
   Ala Gln Gln Gly Leu Leu Gln Leu Thr Val Trp Gly Ile Lys Gln
                    95                 100                 105
   Leu Gln Thr Arg Val Leu Ala Ile Glu Arg Tyr Leu Lys Asp Gln
                   110                 115                 120
   Gln Leu Leu Gly Ile Trp Gly Arg Ser Gly Lys Leu Ile Cys Thr
                   125                 130                 135
   Thr Asn Val Pro Trp Asn Ser Ser Trp Ser Asn Arg Ser Gln Thr
                   140                 145                 150
   Asp Ile Trp Asp Asn Met Thr Trp Met Gln Trp Asp Arg Glu Ile
                   155                 160                 165
   Ser Asn Tyr Thr Asp Thr Ile Tyr Arg Leu Leu Glu Asp Ser Gln
                   170                 175                 180
   Asn Gln Gln Glu Arg Asn Glu Lys Asp Leu Leu Ala Leu Asp Ser
                   185                 190                 195
   Trp Lys Asn
           198
(2) INFORMATION FOR SEQ ID NO:80:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:80:
   Gly Gly Gly Glu Met Arg Asp Asn Trp Arg Ser Glu Leu Tyr Lys
    1               5                  10                  15
   Tyr Lys Val Val Glu Ile Lys Pro Leu Gly Val Ala Pro Thr Thr
                   20                  25                  30
   Ala Lys Arg Arg Val Val Glu Arg Glu Lys Arg Ala Val Gly Ile
                   35                  40                  45
   Gly Ala Val Phe Leu Gly Phe Leu Gly Ala Ala Gly Ser Thr Met
                   50                  55                  60
   Gly Ala Ala Ser Met Thr Val Thr Val Gln Ala Arg Gln Leu Leu
                   65                  70                  75
   Ser Gly Ile Val Gln Gln Gln Ser Asn Leu Leu Arg Ala Ile Glu
                   80                  85                  90
   Ala Gln Gln Gly Leu Leu Gln Leu Thr Ile Trp Gly Ile Lys Gln
                   95                  100                 105
   Leu Gln Ala Arg Val Leu Ala Ile Glu Arg Tyr Leu Lys Glu Gln
                   110                 115                 120
   Gln Leu Leu Gly Ile Trp Gly Cys Ser Gly Lys Leu Ile Cys Thr
                   125                 130                 135
   Thr Thr Val Pro Trp Asn Ser Ser Trp Ser Asn Arg Ser Gln Thr
                   140                 145                 150
   Asp Ile Trp Asp Asn Met Thr Trp Met Gln Trp Asp Arg Glu Ile
                   155                 160                 165
   Ser Asn Tyr Thr Glu Thr Ile Tyr Arg Leu Leu Glu Asp Ser Gln
                   170                 175                 180
   Asn Gln Gln Glu Arg Asn Glu Lys Asp Leu Leu Ala Leu Asp Ser
                   185                 190                 195
   Trp Lys Asn
           198
(2) INFORMATION FOR SEQ ID NO:81:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:81:
   Gly Gly Gly Asp Met Arg Asp Asn Trp Arg Ser Glu Leu Tyr Lys
   1               5                   10                  15
   Tyr Lys Val Val Glu Ile Lys Pro Leu Gly Val Ala Pro Thr Thr
                   20                  25                  30
   Ala Lys Arg Arg Val Val Glu Arg Glu Lys Arg Ala Val Gly Leu
                   35                  40                  45
   Gly Ala Val Phe Leu Gly Phe Leu Gly Ala Ala Gly Ser Thr Met
                   50                  55                  60
   Gly Ala Ala Ser Ile Thr Leu Thr Val Gln Ala Arg Gln Leu Leu
                   65                  70                  75
   Ser Gly Ile Val Gln Gln Gln Ser Asn Leu Leu Arg Ala Ile Glu
                   80                  85                  90
   Ala Gln Gln Gly Leu Leu Gln Leu Thr Val Trp Gly Ile Lys Gln
                   95                  100                 105
   Leu Gln Thr Arg Val Leu Ala Ile Glu Arg Tyr Leu Lys Asp Gln
                   110                 115                 120
   Gln Leu Leu Gly Met Trp Gly Cys Ser Gly Lys Leu Ile Cys Thr
                   125                 130                 135
   Thr Ala Val Pro Trp Asn Ser Ser Trp Ser Asn Arg Ser Gln Thr
                   140                 145                 150
   Asp Ile Trp Asp Asn Met Thr Trp Met Gln Trp Asp Arg Glu Ile
                   155                 160                 165
   Ser Asn Tyr Thr Asn Thr Ile Tyr Arg Leu Leu Glu Asp Ser Gln
                   170                 175                 180
   Asn Gln Gln Glu Arg Asn Glu Lys Asp Leu Leu Ala Leu Asp Ser
                   185                 190                 195
   Trp Lys Asn
           198
(2) INFORMATION FOR SEQ ID NO:82:
   (i) SEQUENCE CHARACTERISTICS:
      (A) LENGTH: 121 amino acids
      (B) TYPE: Amino Acid
      (D) TOPOLOGY: Linear
   (xi) SEQUENCE DESCRIPTION: SEQ ID NO:82:
   Gly Gly Gly Asn Met Lys Asp Asn Trp Arg Asn Glu Leu Tyr Lys
   1               5                   10                  15
   Tyr Lys Val Val Glu Ile Lys Pro Leu Gly Ile Ala Pro Thr Gly
                   20                  25                  30
   Ser Lys Arg Arg Val Val Glu Arg Glu Lys Arg Ala Val Gly Ile
                   35                  40                  45
   Gly Ala Val Leu Leu Gly Phe Leu Gly Ala Ala Gly Ser Thr Met
                   50                  55                  60
   Ala Ala Ala Ser Ile Thr Leu Thr Val Gln Ala Arg Gln Leu Leu
                   65                  70                  75
   Ser Gly Ile Val Gln Gln Gln Ser Asn Leu Leu Arg Ala Ile Glu
                   80                  85                  90
   Ala Gln Gln Gly Met Leu Gln Leu Thr Val Trp Gly Ile Lys Gln
                   95                  100                 105
   Leu Gln Thr Arg Val Leu Ala Ile Glu Arg Tyr Leu Lys Asp Gln
                   110                 115                 120
   Gln
   121
(2) INFORMATION FOR SEQ ID NO:83:
   (i) SEQUENCE CHARACTERISTICS:
      (A) LENGTH: 72 amino acids
      (B) TYPE: Amino Acid
      (D) TOPOLOGY: Linear
   (xi) SEQUENCE DESCRIPTION: SEQ ID NO:83:
   Gly Gly Gly Asp Met Arg Asn Asn Trp Arg Ser Glu Leu Tyr Lys
     1             5                   10                  15
   Tyr Lys Val Val Glu Ile Lys Pro Leu Gly Val Ala Pro Thr Thr
                   20                  25                  30
   Pro Lys Arg Arg Val Val Glu Arg Glu Lys Arg Ala Val Gly Ile
                   35                  40                  45
   Gly Ala Val Phe Leu Gly Phe Leu Gly Ala Ala Gly Ser Thr Met
                   50                  55                  60
   Gly Ala Ala Ser Ile Thr Leu Thr Val Pro Leu Arg
                   65                  70      72
(2) INFORMATION FOR SEQ ID NO:84:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:84:
   Gly Gly Gly Asp Met Arg Asp Asn Trp Arg Ser Glu Leu Tyr Lys
   1               5                   10                  15
   Tyr Lys Val Val Arg Ile Glu Pro Leu Gly Xaa Ala Pro Thr Xaa
                   20                  25                  30
   Ala Lys Arg Arg Val Val Glu Arg Glu Lys Arg Ala Ile Gly Leu
                   35                  40                  45
   Gly Ala Xaa Phe Leu Gly Phe Leu Gly Ala Ala Gly Ser Thr Met
                   50                  55                  60
   Gly Ala Ala Ser Leu Thr Leu Thr Val Gln Ala Arg Gln Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                  100                 105
   Leu Gln Ala Arg Val Leu Ala Val Glu Arg Tyr Leu Lys Asp Gln
                   110                 115                 120
   Gln Leu Leu Gly Ile Trp Gly Cys Ser Gly Lys His Ile Cys Thr
                   125                 130                 135
   Thr Xaa Val Pro Trp Asn Ser Ser Trp Ser Asn Arg Ser Leu Asp
                   140                 145                 150
   Glu Ile Trp Gln Asn Met Thr Trp Met Glu Trp Glu Arg Glu Ile
                   155                 160                 165
   Asp Asn Tyr Thr Gly Leu Ile Tyr Ser Leu Ile Glu Glu Ser Gln
                   170                 175                 180
   Ile Gln Gln Glu Lys Asn Glu Lys Glu Leu Leu Glu Leu Asp Lys
                   185                 190                 195
   Trp Ala Ser Leu Trp Asn Trp Phe Ser Ile Thr Lys Trp Leu Trp
                   200                 205                 210
   Tyr Ile Lys Ile Phe Ile Met Ile Val Gly Gly Leu Ile Gly Leu
                   215                 220                 225
   Arg Ile Val Phe Ala Val Leu Ser Ile Val Asn Arg Val Arg Gln
                   230                 235                 240
   Gly Tyr Ser Pro Leu Ser Phe Gln Thr Leu Leu Pro Ala Pro Arg
                   245                 250                 255
   Gly Xaa Pro Asp Arg Pro Glu Gly Ile Glu Glu Glu Gly Gly
                   260                 265                 269
(2) INFORMATION FOR SEQ ID NO:85:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:85:
   Gly Gly Gly Asp Met Lys Asp Asn Trp Arg Asn Glu Leu Tyr Lys
    1               5                  10                  15
   Tyr Lys Val Val Arg Ile Glu Pro Leu Gly Ile Ala Pro Thr Arg
                   20                  25                  30
   Ala Lys Arg Arg Val Val Glu Arg Glu Lys Arg Ala Ile Gly Leu
                   35                  40                  45
   Gly Ala Val Phe Leu Gly Phe Leu Gly Ala Ala Gly Ser Thr Met
                   50                  55                  60
   Gly Ala Val Ser Val Ala Leu Thr Gly Gln Ala Arg Gln Leu Leu
                   65                  70                  75
   Ser Gly Ile Val Gln Gln Gln Asn Asn Leu Leu Arg Ala Ile Glu
                   80                  85                  90
   Ala Gln Gln His Met Leu Gln Leu Thr Val Trp Gly Ile Lys Gln
                   95                 100                 105
   Leu Gln Ala Arg Val Leu Ala Val Glu Ser Tyr Leu Lys Asp Gln
                  110                 115                 120
   Gln Leu Leu Gly Ile Trp Gly Cys Ser Gly Lys His Ile Cys Thr
                  125                 130                 135
   Thr Thr Val Pro Trp Asn Ser Ser Trp Ser Asn Lys Ser Leu Glu
                  140                 145                 150
   Glu Ile Trp Asn Asn Met Thr Trp Ile Glu Trp Glu Arg Glu Ile
                  155                 160                 165
   Asp Asn Tyr Thr Gly Val Ile Tyr Ser Leu Ile Glu Asn Ser Gln
                  170                 175                 180
   Ile Gln Gln Glu Lys Asn Glu Gln Asp Leu Leu Gln Leu Asp Lys
                  185                 190                 195
   Trp Ala Ser
           198
(2) INFORMATION FOR SEQ ID NO:86:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:86:
   Gly Gly Gly Asp Met Arg Asp Asn Trp Arg Ser Glu Leu Tyr Lys
     1               5                  10                  15
   Tyr Lys Val Val Lys Ile Glu Pro Ile Gly Val Ala Pro Thr Lys
                    20                  25                  30
   Ala Arg Arg Arg Val Val Glu Arg Glu Lys Arg Ala Ile Gly Leu
                    35                  40                  45
   Gly Ala Val Phe Leu Gly Phe Leu Gly Ala Ala Gly Ser Thr Met
                    50                  55                  60
   Gly Ala Ala Ser Val Thr Leu Thr Val Gln Ala Arg Gln Leu Met
                    65                  70                  75
   Ser Gly Ile Val His Gln Gln Asn Asn Leu Leu Arg Ala Ile Glu
                    80                  85                  90
   Ala Gln Gln His Leu Leu Gln Leu Thr Val Trp Gly Ile Lys Gln
                    95                 100                 105
   Leu Gln Ala Arg Val Leu Ala Val Glu Arg Tyr Leu Arg Asp Gln
                   110                 115                 120
   Gln Leu Leu Gly Ile Trp Gly Cys Ser Gly Arg His Ile Cys Thr
                   125                 130                 135
   Thr Asn Val Pro Trp Asn Ser Ser Trp Ser Asn Arg Ser Leu Asp
                   140                 145                 150
   Glu Ile Trp Gln Asn Met Thr Trp Met Glu Trp Glu Arg Glu Ile
                   155                 160                 165
   Asp Asn Tyr Thr Gly Leu Ile Tyr Ser Leu Ile Glu Glu Ser Gln
                   170                 175                 180
   Ile Gln Gln Glu Lys Asn Glu Lys Glu Leu Leu Glu Leu Asp Lys
                   185                 190                 195
   Trp Ala Ser
           198
(2) INFORMATION FOR SEQ ID NO:87:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:87:
   Gly Gly Gly Asp Met Arg Asp Asn Trp Ile Ser Glu Leu Tyr Lys
    1               5                  10                  15
   Tyr Lys Val Val Arg Ile Glu Pro Leu Gly Val Ala Pro Thr Lys
                   20                  25                  30
   Ala Lys Arg Arg Val Val Glu Arg Glu Lys Arg Ala Ile Gly Leu
                   35                  40                  45
   Gly Ala Met Phe Leu Gly Phe Leu Gly Ala Ala Gly Ser Thr Met
                   50                  55                  60
   Gly Ala Ala Ser Leu Thr Leu Thr Val Gln Ala Arg Gln Leu Leu
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                 100                 105
   Leu Gln Ala Arg Val Leu Ala Val Glu Arg Tyr Leu Gln Asp Gln
                  110                 115                 120
   Arg Leu Leu Gly Met Trp Gly Cys Ser Gly Lys His Ile Cys Thr
                  125                 130                 135
   Thr Phe Val Pro Trp Asn Ser Ser Trp Ser Asn Arg Ser Leu Asp
                  140                 145                 150
   Asp Ile Trp Asn Asn Met Thr Trp Met Gln Trp Glu Lys Glu Ile
                  155                 160                 165
   Ser Asn Tyr Thr Gly Ile Ile Tyr Asn Leu Ile Glu Glu Ser Gln
                  170                 175                 180
   Ile Gln Gln Glu Lys Asn Glu Lys Glu Leu Leu Glu Leu Asp Lys
                  185                 190                 195
   Trp Ala Ser
           198
(2) INFORMATION FOR SEQ ID NO:88:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:88:
   Gly Gly Gly Asp Met Arg Asp Asn Trp Arg Ser Glu Leu Tyr Lys
   1 5 10 15
   Tyr Lys Val Val Gln Ile Glu Pro Leu Gly Val Ala Pro Thr Arg
   20 25 30
   Ala Lys Arg Arg Val Val Glu Arg Glu Lys Arg Ala Ile Gly Leu
   35 40 45
   Gly Ala Met Phe Leu Gly Phe Leu Gly Ala Ala Gly Ser Thr Met
   50 55 60
   Gly Ala Arg Ser Val Thr Leu Thr Val Gln Ala Arg Gln Leu Met
   65 70 75
   Ser Gly Ile Val Gln Gln Gln Asn Asn Leu Leu Arg Ala Ile Glu
   80 85 90
   Ala Gln Gln His Leu Leu Gln Leu Thr Val Trp Gly Ile Lys Gln
   95 100 105
   Leu Gln Ala Arg Ile Leu Ala Val Glu Arg Tyr Leu Lys Asp Gln
   110 115 120
   Gln Leu Leu Gly Ile Trp Gly Cys Ser Gly Lys His Ile Cys Thr
   125 130 135
   Thr Asn Val Pro Trp Asn Ser Ser Trp Ser Asn Arg Ser Leu Asn
   140 145 150
   Glu Ile Trp Gln Asn Met Thr Trp Met Glu Trp Glu Arg Glu Ile
   155 160 165
   Asp Asn Tyr Thr Gly Leu Ile Tyr Ser Leu Ile Glu Glu Ser Gln
   170 175 180
   Thr Gln Gln Glu Lys Asn Glu Lys Glu Leu Leu Glu Leu Asp Lys
   185 190 195
   Trp Ala Ser
   198
(2) INFORMATION FOR SEQ ID NO:89:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:89:
   Gly Gly Gly Asp Met Arg Asp Asn Trp Arg Ser Glu Leu Tyr Lys
     1               5                  10                  15
   Tyr Lys Val Val Lys Ile Glu Pro Leu Gly Val Ala Pro Thr Arg
                    20                  25                  30
   Ala Lys Arg Arg Val Val Glu Arg Glu Lys Arg Ala Ile Gly Leu
                    35                  40                  45
   Gly Ala Met Phe Leu Gly Phe Leu Gly Ala Ala Gly Ser Thr Met
                    50                  55                  60
   Gly Ala Arg Ser Leu Thr Leu Thr Val Gln Ala Arg Gln Leu Leu
                    65                  70                  75
   Ser Gly Ile Val Gln Gln Gln Asn Asn Leu Leu Arg Ala Ile Glu
                    80                  85                  90
   Ala Gln Gln His Leu Leu Gln Leu Thr Val Trp Gly Ile Lys Gln
                    95                 100                 105
   Leu Gln Ala Arg Ile Leu Ala Val Glu Arg Tyr Leu Lys Asp Gln
                   110                 115                 120
   Gln Leu Leu Gly Ile Trp Gly Cys Ser Gly Lys Leu Ile Cys Thr
                   125                 130                 135
   Thr Thr Val Pro Trp Asn Ser Ser Trp Ser Asn Arg Ser Leu Asn
                   140                 145                 150
   Asp Ile Trp Gln Asn Met Thr Trp Met Glu Trp Glu Arg Glu Ile
                   155                 160                 165
   Asp Asn Tyr Thr Gly Leu Ile Tyr Arg Leu Ile Glu Glu Ser Gln
                   170                 175                 180
   Thr Gln Gln Glu Lys Asn Glu Gln Glu Leu Leu Glu Leu Asp Lys
                   185                 190                 195
   Trp Ala Ser
           198
(2) INFORMATION FOR SEQ ID NO:90:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:90:
   Gly Gly Gly Asp Met Arg Asp Asn Trp Arg Ser Glu Leu Tyr Lys
    1               5                  10                  15
   Tyr Lys Val Ile Lys Ile Glu Pro Leu Gly Leu Ala Pro Thr Arg
                   20                  25                  30
   Ala Lys Arg Arg Val Val Ala Arg Glu Lys Arg Ala Ile Gly Leu
                   35                  40                  45
   Gly Ala Val Phe Leu Gly Phe Leu Gly Ala Ala Gly Ser Thr Met
                   50                  55                  60
   Gly Ala Ala Ser Leu Thr Leu Thr Val Gln Ala Arg Gln Leu Met
                   65                  70                  75
   Ser Gly Ile Val Gln Gln Gln Asn Asn Leu Leu Arg Ala Ile Glu
                   80                  85                  90
   Ala Gln Gln His Leu Leu Gln Leu Thr Val Trp Gly Ile Lys Gln
                   95                  100                 105
   Leu Gln Ala Arg Val Leu Ala Val Glu Ser Tyr Leu Lys Asp Gln
                   110                 115                 120
   Gln Leu Leu Gly Ile Trp Gly Cys Ser Gly Arg His Ile Cys Pro
                   125                 130                 135
   Thr Gln Val Pro Trp Asn Ser Ser Trp Ser Asn Lys Ser Leu Asp
                   140                 145                 150
   Thr Ile Trp Gly Asn Met Thr Trp Met Glu Trp Glu Arg Glu Ile
                   155                 160                 165
   Ser Asn Tyr Thr Gly Leu Ile Tyr Asp Leu Ile Glu Glu Ser Gln
                   170                 175                 180
   Ile Gln Gln Glu Lys Asn Glu Lys Asp Leu Leu Glu Leu Asp Lys
                   185                 190                 195
   Trp Ala Ser
           198
(2) INFORMATION FOR SEQ ID NO:91:
   (i) SEQUENCE CHARACTERISTICS:
      (A) LENGTH: 104 amino acids
      (B) TYPE: Amino Acid
      (D) TOPOLOGY: Linear
   (xi) SEQUENCE DESCRIPTION: SEQ ID NO:91:
   Gly Gly Gly Asp Met Arg Asp Asn Trp Arg Asn Glu Leu Tyr Lys
   1 5 10 15
   Tyr Lys Val Val Arg Ile Glu Pro Leu Gly Leu Ala Pro Thr Lys
   20 25 30
   Ala Arg Arg Arg Val Val Glu Arg Glu Lys Arg Ala Ile Gly Leu
   35 40 45
   Gly Ala Leu Phe Leu Gly Phe Leu Gly Ala Ala Gly Ser Thr Met
   50 55 60
   Gly Ala Ala Ser Leu Thr Leu Thr Val Gln Ala Arg Gln Leu Leu
   65 70 75
   Ser Gly Ile Val Gln Gln Gln Asn Asn Leu Leu Arg Ala Ile Glu
   80 85 90
   Ala Gln Gln His Leu Leu Gln Leu Thr Val Trp Gly Ile Lys
   95 100 104
(2) INFORMATION FOR SEQ ID NO:92:
   (i) SEQUENCE CHARACTERISTICS:
      (A) LENGTH: 70 amino acids
      (B) TYPE: Amino Acid
      (D) TOPOLOGY: Linear
   (xi) SEQUENCE DESCRIPTION: SEQ ID NO:92:
   Gly Gly Gly Asp Met Arg Asp Asn Trp Arg Ser Glu Leu Tyr Lys
   1 5 10 15
   Tyr Lys Val Val Arg Ile Glu Pro Leu Gly Ile Ala Pro Thr Met
   20 25 30
   Ser Lys Arg Arg Val Val Glu Arg Glu Lys Arg Ala Ile Gly Leu
   35 40 45
   Gly Ala Leu Phe Leu Gly Phe Leu Gly Ala Ala Gly Ser Thr Met
   50 55 60
   Gly Ala Ala Thr Leu Thr Leu Thr Val Xaa
   65 70
(2) INFORMATION FOR SEQ ID NO:93:
   (i) SEQUENCE CHARACTERISTICS:
      (A) LENGTH: 70 amino acids
      (B) TYPE: Amino Acid
      (D) TOPOLOGY: Linear
   (xi) SEQUENCE DESCRIPTION: SEQ ID NO:93:
   Gly Gly Gly Asp Met Arg Asp Asn Trp Arg Ser Glu Leu Tyr Lys
    1               5                  10                  15
   Tyr Lys Val Val Arg Ile Glu Pro Leu Gly Leu Ala Pro Thr Glu
                   20                  25                  30
   Ala Lys Arg Arg Val Val Glu Arg Glu Lys Arg Ala Ile Gly Leu
                   35                  40                  45
   Gly Ala Met Phe Leu Gly Phe Leu Gly Ala Ala Gly Ser Thr Met
                   50                  55                  60
   Gly Ala Ala Ser Met Thr Leu Thr Val Xaa
                   65                  70
(2) INFORMATION FOR SEQ ID NO:94:
   (i) SEQUENCE CHARACTERISTICS:
      (A) LENGTH: 53 amino acids
      (B) TYPE: Amino Acid
      (D) TOPOLOGY: Linear
   (xi) SEQUENCE DESCRIPTION: SEQ ID NO:94:
   Gly Gly Gly Asp Met Arg Asp Asn Arg Arg Ser Glu Leu Tyr Lys
    1               5                  10                  15
   Tyr Lys Val Val Lys Ile Glu Pro Leu Gly Val Ala Pro Thr Lys
                   20                  25                  30
   Thr Lys Arg Arg Val Val Glu Arg Glu Glu Arg Ala Ile Gly Leu
                   35                  40                  45
   Gly Ala Met Phe Leu Gly Phe Leu
                   50          53
(2) INFORMATION FOR SEQ ID NO:95:
   (i) SEQUENCE CHARACTERISTICS:
      (A) LENGTH: 41 amino acids
      (B) TYPE: Amino Acid
      (D) TOPOLOGY: Linear
   (xi) SEQUENCE DESCRIPTION: SEQ ID NO:95:
   Gly Gly Gly Asp Met Arg Asp Asn Trp Arg Ser Glu Leu Tyr Lys
    1               5                  10                  15
   Tyr Lys Val Val Arg Ile Glu Pro Leu Gly Ile Ala Pro Thr Met
                   20                  25                  30
   Ala Lys Arg Arg Val Val Glu Arg Glu Lys Arg
                   35                  40  41
(2) INFORMATION FOR SEQ ID NO:96:
   (i) SEQUENCE CHARACTERISTICS:
      (A) LENGTH: 269 amino acids
      (B) TYPE: Amino Acid
      (D) TOPOLOGY: Linear
   (xi) SEQUENCE DESCRIPTION: SEQ ID NO:96:
   Gly Gly Gly Asn Ile Lys Asp Asn Trp Arg Ser Glu Leu Tyr Lys
   1 5 10 15
   Tyr Lys Val Val Gln Ile Glu Pro Leu Gly Ile Ala Pro Thr Arg
   20 25 30
   Ala Lys Arg Arg Val Val Glu Arg Glu Lys Arg Ala Val Gly Xaa
   35 40 45
   Ile Gly Ala Met Ile Phe Gly Phe Leu Gly Ala Ala Gly Ser Thr
   50 55 60
   Met Gly Ala Ala Ser Ile Thr Leu Thr Val Gln Ala Arg Gln Leu
   65 70 75
   Leu Ser Gly Ile Val Gln Gln Gln Ser Asn Leu Leu Arg Ala Ile
   80 85 90
   Glu Ala Gln Gln His Leu Leu Gln Leu Thr Val Trp Gly Ile Lys
   95 100 105
   Gln Leu Gln Ala Arg Val Leu Ala Val Glu Arg Tyr Leu Lys Asp
   110 115 120
   Gln Lys Phe Leu Gly Leu Trp Gly Cys Ser Gly Lys Ile Ile Cys
   125 130 135
   Thr Thr Ala Val Pro Trp Asn Ser Thr Trp Ser Asn Arg Ser Phe
   140 145 150
   Glu Glu Ile Trp Asn Asn Met Thr Trp Ile Glu Trp Glu Arg Glu
   155 160 165
   Ile Ser Asn Tyr Thr Asn Gln Ile Tyr Glu Ile Leu Thr Glu Ser
   170 175 180
   Gln Asn Gln Gln Asp Arg Asn Glu Lys Asp Leu Leu Glu Leu Asp
   185 190 195
   Lys Trp Ala Ser Leu Trp Asn Trp Phe Asp Ile Thr Asn Trp Leu
   200 205 210
   Trp Tyr Ile Lys Ile Phe Ile Met Ile Val Gly Gly Leu Ile Gly
   215 220 225
   Leu Arg Ile Ile Phe Ala Val Leu Ser Ile Val Asn Arg Val Arg
   230 235 240
   Gln Gly Tyr Ser Pro Leu Ser Phe Gln Thr Pro Xaa His His Gln
   245 250 255
   Arg Glu Pro Asp Arg Pro Glu Arg Ile Glu Glu Gly Gly Gly
   260 265 269
(2) INFORMATION FOR SEQ ID NO:97:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:97:
   Gly Gly Gly Asn Ile Lys Asp Asn Trp Arg Ser Glu Leu Tyr Lys
    1               5                  10                  15
   Tyr Lys Val Val Gln Ile Glu Pro Leu Gly Ile Ala Pro Thr Arg
                   20                  25                  30
   Ala Lys Arg Arg Val Val Glu Arg Glu Lys Arg Ala Val Gly Ile
                   35                  40                  45
   Gly Ala Met Ile Phe Gly Phe Leu Gly Ala Ala Gly Ser Thr Met
                   50                  55                  60
   Gly Ala Ala Ser Ile Thr Leu Thr Val Gln Ala Arg Gln Leu Val
                   65                  70                  75
   Ser Gly Ile Val Gln Gln Gln Ser Asn Leu Leu Arg Ala Ile Glu
                   80                  85                  90
   Ala Gln Gln His Leu Leu Gln Leu Thr Val Trp Gly Ile Lys Gln
                   95                 100                 105
   Leu Gln Ala Arg Val Leu Ala Val Glu Arg Tyr Leu Lys Asp Gln
                  110                 115                 120
   Lys Phe Leu Gly Leu Trp Gly Cys Ser Gly Lys Ile Ile Cys Thr
                  125                 130                 135
   Thr Ala Val Pro Trp Asn Ser Thr Trp Ser Asn Arg Ser Phe Glu
                  140                 145                 150
   Glu Ile Trp Ser Asn Met Thr Trp Ile Glu Trp Glu Arg Glu Ile
                  155                 160                 165
   Ser Asn Tyr Thr Asn Gln Ile Tyr Glu Ile Leu Thr Glu Ser Gln
                  170                 175                 180
   Asn Gln Gln Asp Arg Asn Glu Lys Asp Leu Leu Glu Leu Asp Lys
                  185                 190                 195
   Trp Ala Ser
          198
(2) INFORMATION FOR SEQ ID NO:98:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:98:
   Gly Gly Gly Asn Ile Lys Asp Asn Trp Arg Ser Glu Leu Tyr Lys
    1               5                  10                  15
   Tyr Lys Val Val Gln Ile Glu Pro Leu Gly Ile Ala Pro Thr Arg
                   20                  25                  30
   Ala Lys Arg Arg Val Val Glu Arg Glu Lys Arg Ala Val Gly Ile
                   35                  40                  45
   Gly Ala Met Ile Phe Gly Phe Leu Gly Ala Ala Gly Ser Thr Met
                   50                  55                  60
   Gly Ala Ala Ser Ile Thr Leu Thr Val Gln Ala Arg Gln Leu Leu
                   65                  70                  75
   Ser Gly Ile Val Gln Gln Gln Ser Asn Leu Leu Arg Ala Ile Glu
                   80                  85                  90
   Ala Gln Gln His Leu Leu Gln Leu Thr Val Trp Gly Ile Lys Gln
                   95                 100                 105
   Leu Gln Ala Arg Val Leu Ala Val Glu Arg Tyr Leu Lys Asp Gln
                  110                 115                 120
   Lys Phe Leu Gly Leu Trp Gly Cys Ser Gly Lys Ile Ile Cys Thr
                  125                 130                 135
   Thr Ala Val Pro Trp Asn Ser Thr Trp Ser Asn Arg Ser Phe Glu
                  140                 145                 150
   Glu Ile Trp Asn Asn Met Thr Trp Thr Glu Trp Glu Arg Glu Ile
                  155                 160                 165
   Ser Asn Tyr Thr Asn Gln Ile Tyr Asp Ile Leu Thr Glu Ser Gln
                  170                 175                 180
   Asn Gln Gln Asp Arg Asn Glu Lys Asp Leu Leu Gly Leu Asp Lys
                  185                 190                 195
   Trp Ala Ser
           198
(2) INFORMATION FOR SEQ ID NO:99:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:99:
   Gly Gly Gly Asn Ile Lys Asp Asn Trp Arg Ser Glu Leu Tyr Lys
    1               5                  10                  15
   Tyr Lys Val Val Gln Ile Glu Pro Leu Gly Ile Ala Pro Thr Arg
                   20                  25                  30
   Ala Lys Arg Arg Val Val Glu Arg Glu Lys Arg Ala Val Gly Ile
                   35                  40                  45
   Gly Ala Met Ile Phe Gly Phe Leu Gly Ala Ala Gly Ser Thr Met
                   50                  55                  60
   Gly Ala Ala Ser Ile Thr Leu Thr Val Gln Ala Arg Gln Leu Leu
                   65                  70                  75
   Ser Gly Ile Val Gln Gln Gln Ser Asn Leu Leu Arg Ala Ile Glu
                   80                  85                  90
   Ala Gln Gln His Met Leu Gln Leu Thr Val Trp Gly Ile Lys Gln
                   95                 100                 105
   Leu Gln Ala Arg Val Leu Ala Val Glu Arg Tyr Leu Lys Asp Gln
                  110                 115                 120
   Lys Phe Leu Gly Leu Trp Gly Cys Ser Gly Lys Ile Ile Cys Thr
                  125                 130                 135
   Thr Ala Val Pro Trp Asn Ser Thr Trp Ser Asn Lys Ser Phe Glu
                  140                 145                 150
   Glu Ile Trp Asn Asn Met Thr Trp Thr Glu Trp Glu Arg Glu Ile
                  155                 160                 165
   Ser Asn Tyr Thr Asn Gln Ile Tyr Glu Ile Leu Thr Glu Ser Gln
                  170                 175                 180
   Asn Gln Gln Asp Arg Asn Glu Lys Asp Leu Leu Glu Leu Asp Lys
                  185                 190                 195
   Trp Ala Ser
           198
(2) INFORMATION FOR SEQ ID NO:100:
   (i) SEQUENCE CHARACTERISTICS:
      (A) LENGTH: 189 amino acids
      (B) TYPE: Amino Acid
      (D) TOPOLOGY: Linear
   (xi) SEQUENCE DESCRIPTION: SEQ ID NO:100:
   Gly Gly Gly Asn Ile Lys Asp Asn Trp Arg Ser Glu Leu Tyr Lys
     1               5                  10                  15
   Tyr Lys Val Val Gln Ile Glu Pro Leu Gly Ile Ala Pro Thr Arg
                    20                  25                  30
   Ala Lys Arg Arg Val Val Glu Arg Glu Lys Arg Ala Val Gly Ile
                    35                  40                  45
   Gly Ala Met Ile Phe Gly Phe Leu Gly Ala Ala Gly Ser Thr Met
                    50                  55                  60
   Gly Ala Ala Ser Ile Thr Leu Thr Val Gln Ala Arg Gln Leu Leu
                    65                  70                  75
   Ser Gly Ile Val Gln Gln Ser Asn Leu Leu Arg Ala Glu Ala Gln
                    80                  85                  90
   Gln His Leu Leu Gln Leu Thr Val Trp Gly Gln Leu Gln Ala Arg
                    95                 100                 105
   Val Ala Val Glu Arg Tyr Leu Lys Asp Gln Lys Leu Gly Leu Trp
                   110                 115                 120
   Cys Ser Gly Lys Ile Ile Cys Thr Thr Ala Val Pro Trp Asn Ser
                   125                 130                 135
   Thr Trp Ser Asn Arg Ser Phe Glu Glu Ile Trp Asn Asn Met Trp
                   140                 145                 150
   Ile Glu Trp Arg Glu Ile Ser Asn Tyr Thr Asn Gln Ile Tyr Glu
                   155                 160                 165
   Ile Leu Thr Glu Ser Gln Asn Gln Gln Asp Arg Asn Glu Lys Asp
                   170                 175                 180
   Leu Leu Glu Leu Asp Lys Trp Ala Ser
                   185             189
(2) INFORMATION FOR SEQ ID NO:101:
   (i) SEQUENCE CHARACTERISTICS:
      (A) LENGTH: 198 amino acids
      (B) TYPE: Amino Acid
      (D) TOPOLOGY: Linear
   (xi) SEQUENCE DESCRIPTION: SEQ ID NO:101:
   Gly Gly Gly Asn Ile Lys Asp Asn Trp Arg Ser Glu Leu Tyr Lys
   1 5 10 15
   Tyr Lys Val Val Gln Ile Glu Pro Leu Gly Ile Ala Pro Thr Arg
   20 25 30
   Ala Lys Arg Arg Val Val Glu Arg Glu Lys Arg Ala Val Gly Ile
   35 40 45
   Gly Ala Met Ile Phe Gly Phe Leu Gly Ala Ala Gly Ser Thr Met
   50 55 60
   Gly Ala Ala Ser Ile Thr Leu Thr Val Gln Ala Arg Gln Leu Leu
   65 70 75
   Ser Gly Ile Val Gln Gln Gln Ser Asn Leu Leu Arg Ala Ile Glu
   80 85 90
   Ala Gln Gln His Leu Leu Gln Leu Thr Val Trp Gly Ile Lys Gln
   95 100 105
   Leu Gln Ala Arg Val Leu Ala Val Glu Arg Tyr Leu Lys Asp Gln
   110 115 120
   Lys Phe Leu Gly Leu Trp Gly Cys Ser Gly Lys Ile Ile Cys Thr
   125 130 135
   Thr Ala Val Pro Trp Asn Ser Thr Trp Ser Asn Arg Ser Leu Glu
   140 145 150
   Glu Ile Trp Asn Asn Met Thr Trp Ile Glu Trp Glu Arg Glu Ile
   155 160 165
   Ser Asn Tyr Thr Asn Arg Ile Tyr Glu Ile Leu Thr Lys Ser Gln
   170 175 180
   Asp Gln Gln Asp Arg Asn Glu Lys Asp Leu Leu Glu Leu Asp Lys
   185 190 195
   Trp Ala Ser
   198
(2) INFORMATION FOR SEQ ID NO:102:
   (i) SEQUENCE CHARACTERISTICS:
      (A) LENGTH: 70 amino acids
      (B) TYPE: Amino Acid
      (D) TOPOLOGY: Linear
   (xi) SEQUENCE DESCRIPTION: SEQ ID NO:102:
   Gly Gly Gly Asn Ile Lys Asp Asn Trp Arg Ser Glu Leu Tyr Lys
    1               5                  10                  15
   Tyr Lys Val Val Gln Ile Glu Pro Leu Gly Ile Ala Pro Thr Arg
                   20                  25                  30
   Ala Lys Arg Arg Val Val Glu Arg Glu Lys Arg Ala Val Gly Ile
                   35                  40                  45
   Gly Ala Met Ile Phe Gly Phe Leu Gly Ala Ala Gly Ser Thr Met
                   50                  55                  60
   Gly Ala Pro Ser Ile Thr Leu Thr Val Xaa
                   65                  70
(2) INFORMATION FOR SEQ ID NO:103:
   (i) SEQUENCE CHARACTERISTICS:
      (A) LENGTH: 213 amino acids
      (B) TYPE: Amino Acid
      (D) TOPOLOGY: Linear
   (xi) SEQUENCE DESCRIPTION: SEQ ID NO:103:
   Xaa Gly Gly Asp Met Lys Asp Ile Trp Arg Thr Glu Leu Tyr Asn
   1 5 10 15
   Tyr Lys Val Val Arg Ile Lys Pro Xaa Ser Val Ala Pro Thr Lys
   20 25 30
   Xaa Xaa Arg Pro Xaa Ile Xaa Xaa Xaa Xaa Xaa His Arg Xaa Lys
   35 40 45
   Arg Ala Val Gly Xaa Leu Gly Met Leu Phe Leu Gly Val Leu Ser
   50 55 60
   Ala Ala Gly Ser Thr Met Gly Ala Ala Ala Thr Xaa Leu Thr Val
   65 70 75
   Gln Thr Xaa Xaa Leu Leu Lys Gly Ile Val Gln Gln Gln Asp Asn
   80 85 90
   Leu Leu Arg Ala Ile Xaa Ala Gln Gln His Leu Leu Xaa Leu Ser
   95 100 105
   Val Trp Gly Xaa Xaa Gln Leu Xaa Ala Arg Leu Leu Ala Xaa Glu
   110 115 120
   Thr Xaa Leu Gln Xaa Gln Gln Leu Leu Ser Leu Trp Gly Cys Lys
   125 130 135
   Gly Lys Leu Val Cys Tyr Thr Xaa Val Xaa Trp Asn Asn Ser Trp
   140 145 150
   Xaa Xaa Xaa Xaa Xaa Xaa Xaa Xaa Xaa Asn Ser Ser Xaa Xaa Xaa
   155 160 165
   Xaa Ile Trp Asp Asn Leu Thr Trp Gln Xaa Trp Asp Arg Leu Xaa
   170 175 180
   Ser Asn Xaa Xaa Xaa Xaa Ile Tyr Xaa Glu Xaa Gln Xaa Ala Gln
   185 190 195
   Xaa Gln Gln Glu Lys Asn Glu Lys Xaa Leu Leu Glu Leu Asp Glu
   200 205 210
   Trp Ala Ser
   213
(2) INFORMATION FOR SEQ ID NO:104:
   (i) SEQUENCE CHARACTERISTICS:
      (A) LENGTH: 200 amino acids
      (B) TYPE: Amino Acid
      (D) TOPOLOGY: Linear
   (xi) SEQUENCE DESCRIPTION: SEQ ID NO:104:
   Ile Gly Gly Asp Met Lys Asp Ile Trp Arg Thr Glu Leu Phe Asn
    1               5                  10                  15
   Tyr Lys Val Val Arg Val Lys Pro Phe Ser Val Ala Pro Thr Arg
                   20                  25                  30
   Ile Ala Arg Pro Val Ile Ser Thr Arg Thr His Arg Glu Lys Arg
                   35                  40                  45
   Ala Val Gly Leu Gly Met Leu Phe Leu Gly Val Leu Ser Ala Ala
                   50                  55                  60
   Gly Ser Thr Met Gly Ala Ala Ala Thr Thr Leu Ala Val Gln Thr
                   65                  70                  75
   His Thr Leu Leu Lys Gly Ile Val Gln Gln Gln Asp Asn Leu Leu
                   80                  85                  90
   Arg Ala Ile Gln Ala Gln Gln Gln Leu Leu Arg Leu Ser Xaa Trp
                   95                 100                 105
   Gly Ile Arg Gln Leu Arg Ala Arg Leu Leu Ala Leu Glu Thr Leu
                  110                 115                 120
   Leu Gln Asn Gln Gln Leu Leu Ser Leu Trp Gly Cys Lys Gly Lys
                  125                 130                 135
   Leu Val Cys Tyr Thr Ser Val Lys Trp Asn Arg Thr Trp Ile Gly
                  140                 145                 150
   Asn Glu Ser Ile Trp Asp Thr Leu Thr Trp Gln Glu Trp Asp Arg
                  155                 160                 165
   Gln Ile Ser Asn Ile Ser Ser Thr Ile Tyr Glu Glu Ile Gln Lys
                  170                 175                 180
   Ala Gln Val Gln Gln Glu Gln Asn Glu Lys Lys Leu Leu Glu Leu
                  185                 190                 195
   Asp Glu Trp Ala Ser
                  200
(2) INFORMATION FOR SEQ ID NO:105:
   (i) SEQUENCE CHARACTERISTICS:
      (A) LENGTH: 204 amino acids
      (B) TYPE: Amino Acid
      (D) TOPOLOGY: Linear
   (xi) SEQUENCE DESCRIPTION: SEQ ID NO:105:
   Val Gly Gly Asp Met Lys Asp Ile Trp Arg Thr Lys Leu Tyr Asn
    1               5                  10                  15
   Tyr Lys Val Val Gln Ile Lys Pro Phe Ser Val Ala Pro Thr Lys
                   20                  25                  30
   Met Ser Arg Pro Ile Ile Asn Ile His Thr Pro His Arg Glu Lys
                   35                  40                  45
   Arg Ala Val Gly Leu Gly Met Leu Phe Leu Gly Val Leu Ser Ala
                   50                  55                  60
   Ala Gly Ser Thr Met Gly Ala Ala Ala Thr Ala Leu Thr Val Arg
                   65                  70                  75
   Thr His Ser Val Leu Lys Gly Ile Val Gln Gln Gln Asp Asn Leu
                   80                  85                  90
   Leu Arg Ala Ile Gln Ala Gln Gln His Leu Leu Arg Leu Ser Val
                   95                 100                 105
   Trp Gly Ile Arg Gln Leu Arg Ala Arg Leu Gln Ala Leu Glu Thr
                  110                 115                 120
   Leu Ile Gln Asn Gln Gln Arg Leu Asn Leu Trp Gly Cys Lys Gly
                  125                 130                 135
   Lys Leu Ile Cys Tyr Thr Ser Val Lys Trp Asn Thr Ser Trp Ser
                  140                 145                 150
   Gly Arg Tyr Asn Asp Asp Ser Ile Trp Asp Asn Leu Thr Trp Gln
                  155                 160                 165
   Gln Trp Asp Gln His Ile Asn Asn Val Ser Ser Ile Ile Tyr Asp
                  170                 175                 180
   Glu Ile Gln Ala Ala Gln Asp Gln Gln Glu Lys Asn Val Lys Ala
                  185                 190                 195
   Leu Leu Glu Leu Asp Glu Trp Ala Ser
                  200                 204
(2) INFORMATION FOR SEQ ID NO:106:
   (i) SEQUENCE CHARACTERISTICS:
      (A) LENGTH: 204 amino acids
      (B) TYPE: Amino Acid
      (D) TOPOLOGY: Linear
   (xi) SEQUENCE DESCRIPTION: SEQ ID NO:106:
   Thr Gly Gly Asn Met Lys Asp Ile Trp Arg Ser Glu Leu Tyr Lys
    1               5                  10                  15
   Tyr Lys Val Val Arg Ile Glu Pro Leu Ser Val Ala Pro Thr Lys
                   20                  25                  30
   Ala Arg Arg His Thr Val Ala Arg Gln Lys Asp Arg Gln Lys Arg
                   35                  40                  45
   Ala Ala Phe Gly Leu Gly Ala Leu Phe Leu Gly Phe Leu Gly Ala
                   50                  55                  60
   Ala Gly Ser Thr Met Gly Ala Ala Ala Val Thr Leu Thr Val Gln
                   65                  70                  75
   Ala Arg Gln Leu Leu Ser Gly Ile Val Gln Gln Gln Asn Asn Leu
                   80                  85                  90
   Leu Lys Ala Ile Glu Ala Gln Gln His Leu Leu Gln Leu Ser Ile
                   95                  100                 105
   Trp Gly Val Lys Gln Leu Gln Ala Arg Leu Leu Ala Val Glu Arg
                   110                 115                 120
   Tyr Leu Gln Asp Gln Gln Ile Leu Gly Leu Trp Gly Cys Ser Gly
                   125                 130                 135
   Lys Ala Val Cys Tyr Thr Thr Val Pro Trp Asn Asn Ser Trp Pro
                   140                 145                 150
   Gly Ser Asn Ser Thr Asp Asp Ile Trp Gly Asn Leu Thr Trp Gln
                   155                 160                 165
   Gln Trp Asp Lys Leu Val Ser Asn Tyr Thr Gly Lys Ile Phe Gly
                   170                 175                 180
   Leu Leu Glu Glu Ala Gln Ser Gln Gln Glu Lys Asn Glu Arg Asp
                   185                 190                 195
   Leu Leu Glu Leu Asp Gln Trp Ala Ser
                   200             204
(2) INFORMATION FOR SEQ ID NO:107:
   (i) SEQUENCE CHARACTERISTICS:
      (A) LENGTH: 30 amino acids
      (B) TYPE: Amino Acid
      (D) TOPOLOGY: Linear
   (xi) SEQUENCE DESCRIPTION: SEQ ID NO:107:
   Glu Trp Asp Arg Glu Ile Asn Asn Tyr Thr Ser Leu Ile His Ser
   1 5 10 15
   Leu Ile Glu Glu Ser Gln Asn Gln Gln Glu Lys Asn Glu Gln Glu
   20 25 30
(2) INFORMATION FOR SEQ ID NO:108:
   (i) SEQUENCE CHARACTERISTICS:
      (A) LENGTH: 36 amino acids
      (B) TYPE: Amino Acid
      (D) TOPOLOGY: Linear
   (xi) SEQUENCE DESCRIPTION: SEQ ID NO:108:
   Tyr Thr Ser Leu Ile His Ser Leu Ile Glu Glu Ser Gln Asn Gln
   1 5 10 15
   Gln Glu Lys Asn Glu Gln Glu Leu Leu Glu Leu Asp Lys Trp Ala
   20 25 30
   Ser Leu Trp Asn Trp Phe
   35 36
(2) INFORMATION FOR SEQ ID NO:109:
   (i) SEQUENCE CHARACTERISTICS:
      (A) LENGTH: 46 amino acids
      (B) TYPE: Amino Acid
      (D) TOPOLOGY: Linear
   (xi) SEQUENCE DESCRIPTION: SEQ ID NO:109:
   Trp Met Glu Trp Glu Arg Glu Ile Asp Asn Tyr Thr Xaa Leu Ile
   1 5 10 15
   Tyr Thr Leu Ile Glu Glu Ser Gln Asn Gln Gln Glu Lys Asn Glu
   20 25 30
   Gln Glu Leu Leu Glu Leu Asp Lys Trp Ala Ser Leu Trp Asn Trp
   35 40 45
   Phe
   46
(2) INFORMATION FOR SEQ ID NO:110:
   (i) SEQUENCE CHARACTERISTICS:
      (A) LENGTH: 46 amino acids
      (B) TYPE: Amino Acid
      (D) TOPOLOGY: Linear
   (xi) SEQUENCE DESCRIPTION: SEQ ID NO:110:
   Trp Leu Gln Trp Asp Lys Glu Ile Ser Asn Tyr Thr Xaa Ile Ile
    1               5                  10                  15
   Tyr Asn Leu Ile Glu Glu Ser Gln Asn Gln Gln Glu Lys Asn Glu
                   20                  25                  30
   Gln Asp Leu Leu Ala Leu Asp Lys Trp Ala Asn Leu Trp Asn Trp
                   35                  40                  45
   Phe
    46
(2) INFORMATION FOR SEQ ID NO:111:
   (i) SEQUENCE CHARACTERISTICS:
      (A) LENGTH: 46 amino acids
      (B) TYPE: Amino Acid
      (D) TOPOLOGY: Linear
   (xi) SEQUENCE DESCRIPTION: SEQ ID NO:111:
   Trp Met Gln Trp Asp Arg Glu Ile Ser Asn Tyr Thr Asp Thr Ile
    1               5                  10                  15
   Tyr Arg Leu Leu Glu Asp Ser Gln Asn Gln Gln Glu Arg Asn Glu
                   20                  25                  30
   Lys Asp Leu Leu Ala Leu Asp Ser Trp Lys Asn Leu Trp Asn Trp
                   35                  40                  45
   Phe
    46
(2) INFORMATION FOR SEQ ID NO:112:
   (i) SEQUENCE CHARACTERISTICS:
      (A) LENGTH: 46 amino acids
      (B) TYPE: Amino Acid
      (D) TOPOLOGY: Linear
   (xi) SEQUENCE DESCRIPTION: SEQ ID NO:112:
   Trp Met Glu Trp Glu Arg Glu Ile Asp Asn Tyr Thr Gly Leu Ile
    1               5                  10                  15
   Tyr Ser Leu Ile Glu Glu Ser Gln Ile Gln Gln Glu Lys Asn Glu
                   20                  25                  30
   Lys Glu Leu Leu Glu Leu Asp Lys Trp Ala Ser Leu Trp Asn Trp
                   35                  40                  45
   Phe
    46
(2) INFORMATION FOR SEQ ID NO:113:
   (i) SEQUENCE CHARACTERISTICS:
      (A) LENGTH: 40 amino acids
      (B) TYPE: Amino Acid
      (D) TOPOLOGY: Linear
   (xi) SEQUENCE DESCRIPTION: SEQ ID NO:113:
   Trp Ile Glu Trp Glu Arg Glu Ile Ser Asn Tyr Thr Asn Gln Ile
    1               5                  10                  15
   Tyr Glu Ile Leu Thr Glu Ser Gln Asn Gln Gln Asp Arg Asn Glu
                   20                  25                  30
   Lys Asp Leu Leu Glu Leu Asp Lys Trp Ala
                   35                  40